# EUROPEAN PATENT APPLICATION

(11) **EP 4 796 109 A2**
(43) Date of publication of application: **26.08.2026**
(21) Application number: 26177936.7
(22) Date of filing: 29.06.2016
(51) Int. Cl.: A61K 9/16

(54) **BIOMARKERS FOR NANOPARTICLE COMPOSITIONS**

(30) Priority: 29.06.2015 US 201562186309 P
(62) Document number(s) of the earlier application(s) in accordance with Art. 76 EPC: 16818726.8 / 3 313 381
(71) Applicant: Abraxis BioScience, LLC, Summit, New Jersey 07901 (US)
(72) Inventor: DESAI, Neil P., Pacific Palisades, CA 90272 (US)
(74) Representative: Jones Day

(57) **Abstract**

The present invention provides methods and compositions for treating a hyperplasia (such as cancer, restenosis, or pulmonary hypertension) by administering a composition comprising nanoparticles that comprise an mTOR inhibitor (such as a limus drug) and an albumin based upon the status of an mTOR-activating aberration.

## Description

### CROSS REFERENCE TO RELATED APPLICATIONS

This application claims priority benefit of U.S. Provisional Application No. 62/186,309, filed June 29, 2015, the contents of which are incorporated herein by reference in their entirety.

### TECHNICAL FIELD

The present invention relates to methods and compositions for treating hyperplasia such as cancer. In particular, the present invention relates to methods and compositions for determining responsiveness and/or likelihood of successful treatment comprising administering compositions comprising nanoparticles that comprise an mTOR inhibitor (*e.g*. a limus drug) and an albumin. The present invention also relates to methods and compositions for treating pediatric solid tumors.

### BACKGROUND

The mammalian target of rapamycin (mTOR) is a conserved serine/threonine kinase that serves as a central hub of signaling in the cell to integrate intracellular and extracellular signals and to regulate cellular growth and homeostasis. Activation of the mTOR pathway is associated with cell proliferation and survival, while inhibition of mTOR signaling leads to inflammation and cell death. Dysregulation of the mTOR signaling pathway has been implicated in an increasing number of human diseases, including cancer and autoimmune disorders. Consequently, mTOR inhibitors have found wide applications in treating diverse pathological conditions such as solid tumors, organ transplantation, restenosis, and rheumatoid arthritis. However, a pressing issue in the application of mTOR inhibitors is the variability of treatment response among different individuals having the same disease or condition. Given the large number of genes involved in the extended signaling network of mTOR, a reliable set of predictive biomarkers is much needed to guide selection of an effective treatment plan for individual patients.

Sirolimus (INN/USAN), also known as rapamycin, is an immunosuppressant drug used to prevent rejection in organ transplantation; it is especially useful in kidney transplants. Sirolimus-eluting stents were approved in the United States to treat coronary restenosis. Additionally, sirolimus has been demonstrated as an effective inhibitor of tumor growth in various cell lines and animal models. Other limus drugs, such as analogs of rapamycin, have been designed to improve the pharmacokinetic and pharmacodynamic properties of sirolimus. For example, Temsirolimus was approved in the United States and Europe for the treatment of renal cell carcinoma. Everolimus was approved in the U.S. for treatment of advanced breast cancer, pancreatic neuroendocrine tumors, advanced renal cell carcinoma, and subependymal giant cell astrocytoma (SEGA) associated with Tuberous Sclerosis. The mode of action of rapamycin is to bind the cytosolic protein FK-binding protein 12 (FKBP12), and the sirolimus-FKBP12 complex in turn inhibits the mTOR pathway by directly binding to the mTOR Complex 1 (mTORC1).

The disclosures of all publications, patents, patent applications and published patent applications referred to herein are hereby incorporated herein by reference in their entirety.

### BRIEF SUMMARY OF THE INVENTION

The present invention provides methods of treating a hyperplasia (such as cancer, restenosis and pulmonary hypertension) in an individual, comprising administering to the individual an effective amount of a composition comprising nanoparticles comprising an mTOR inhibitor (such as a limus drug) and an albumin, wherein the status of an mTOR-activating aberration is used as a basis for selecting the individual for treatment.

In one aspect of the present application, there is provided a method of treating a hyperplasia in an individual comprising administering to the individual an effective amount of a composition comprising nanoparticles comprising an mTOR inhibitor and an albumin, wherein the individual is selected for treatment on the basis of having an mTOR-activating aberration. In some embodiments, the method further comprises assessing the mTOR-activating aberration in the individual.

In another aspect of the present application, there is provided a method of selecting an individual having a hyperplasia for treatment with a composition comprising nanoparticles comprising an mTOR inhibitor and an albumin, wherein the method comprises: assessing an mTOR-activating aberration in the individual; and selecting or recommending the individual for treatment based on the individual having the mTOR-activating aberration. In some embodiments, the method further comprises administering the composition comprising nanoparticles comprising an mTOR inhibitor and an albumin to the selected individual.

In some embodiments according to any one of the methods described above, the hyperplasia is selected from the group consisting of cancer, restenosis, and pulmonary hypertension. In some embodiments, the cancer is selected from the group consisting of pancreatic neuroendocrine cancer, endometrial cancer, breast cancer, renal cell carcinoma, lymphangioleiomyomatosis (LAM), prostate cancer, lymphoma, bladder cancer, endometrial cancer, and ovary cancer.

In some embodiments according to any one of the methods described above, the mTOR-activating aberration comprises a mutation in an mTOR-associated gene. In some embodiments, the mTOR-activating aberration comprises a copy number variation of an mTOR-associated gene. In some embodiments, the mTOR-activating aberration is assessed by gene sequencing. In some embodiments, the gene sequencing is based on sequencing of DNA in a tumor sample. In some embodiments, the gene sequencing is based on sequencing of circulating DNA or cell-free DNA isolated from a blood sample.

In some embodiments according to any one of the methods described above, the mTOR-activating aberration comprises an aberrant expression level of an mTOR-associated gene.

In some embodiments according to any one of the methods described above, the mTOR-activating aberration comprises an aberrant phosphorylation level of the protein encoded by the mTOR-associated gene. In some embodiments, the mTOR-activating aberration comprises an aberrant phosphorylation level of a protein encoded by an mTOR-associated gene selected from the group consisting of AKT, S6K, S6, 4EBP1, and SPARC. In some embodiments, the aberrant phosphorylation level is determined by immunohistochemistry.

In some embodiments according to any one of the methods described above, the mTOR-activating aberration comprises an aberrant activity level of an mTOR-associated gene.

In some embodiments according to any one of the methods described above, the mTOR-activating aberration leads to activation of mTORC1 (including for example activation of mTORC1 but not mTORC2).

In some embodiments according to any one of the methods described above, the mTOR-activating aberration leads to activation of mTORC2 (including for example activation of mTORC2 but not mTORC1).

In some embodiments according to any one of the methods described above, the mTOR-activating aberration leads to activation of both mTORC1 and mTORC2.

In some embodiments according to any one of the methods described above, the mTOR-activating aberration is an aberration in at least one mTOR-associated gene selected from the group consisting of AKT1, FLT3, MTOR, PIK3CA, PIK3CG, TSC1, TSC2, RHEB, STK11, NF1, NF2, PTEN, TP53, FGFR4, KRAS, NRAS, and BAP1. In some embodiments, the at least one mTOR-associated gene comprises MTOR. In some embodiments, the mTOR-activating aberration comprises an activating mutation of MTOR. In some embodiments, the at least one mTOR-associated gene comprises TSC1 or TSC2. In some embodiments, the mTOR-activating aberration comprises a loss of heterozygosity of TSC1 or TSC2. In some embodiments, the mTOR-activating aberration comprises a loss of function mutation in TSC1 or TSC2. In some embodiments, the at least one mTOR-associated gene comprises RHEB. In some embodiments, the mTOR-activating aberration comprises a loss of function mutation in RHEB. In some embodiments, the at least one mTOR-associated gene comprises NF1. In some embodiments, the mTOR-activating aberration comprises a loss of function mutation of NF1. In some embodiments, the at least one mTOR-associated gene comprises NF2. In some embodiments, the mTOR-activating aberration comprises a loss of function mutation of NF2. In some embodiments, the mTOR-associated gene comprises PTEN. In some embodiments, the mTOR-activating aberration comprises a deletion of PTEN. In some embodiments, the mTOR-associated gene comprises PIK3CA. In some embodiments, the mTOR-activating aberration comprises a loss of function mutation in PIK3CA. In some embodiments, the mTOR-associated gene comprises PIK3CG. In some embodiments, the mTOR-activating aberration comprises a loss of function mutation in PIK3CG. In some embodiments, the mTOR-associated gene comprises AKT1. In some embodiments, the mTOR-activating aberration comprises an activating mutation in AKT1. In some embodiments, the mTOR-associated gene comprises TP53. In some embodiments, the mTOR-activating aberration comprises a loss of function mutation in TP53.

In some embodiments according to any one of the methods described above, the mutational status of TFE3 is further used as a basis for selecting the individual. In some embodiments, the mutational status of TFE3 comprises translocation of TFE3.

In some embodiments according to any one of the methods described above, the method further comprises administering to the individual an effective amount of a second therapeutic agent.

In some embodiments according to any one of the methods described above, the individual is human.

In some embodiments according to any one of the methods described above, the composition comprises nanoparticles comprising the mTOR inhibitor and the albumin is administered intravenously. In some embodiments, the composition comprises nanoparticles comprising the mTOR inhibitor and the albumin is administered subcutaneously.

In some embodiments according to any one of the methods described above, the nanoparticles in the composition comprise the mTOR inhibitor associated (i.e., coated) with the albumin.

In some embodiments according to any one of the methods described above, the nanoparticles in the composition have an average diameter of no greater than about 150 nm (including for example no more than about any of 120 nm or 100 nm).

In some embodiments according to any one of the methods described above, the ratio of the mTOR inhibitor to the albumin in the nanoparticles is about 1:1 to about 9:1.

In some embodiments according to any one of the methods described above, the albumin is human serum albumin.

In some embodiments according to any one of the methods described above, the mTOR inhibitor is a limus drug. In some embodiments, the limus drug is sirolimus.

In some embodiments according to any one of the methods described above, the dose of the mTOR inhibitor in the composition is about 10 mg/m² to about 150 mg/m² (including for example any of about 20 mg/m² to about 45 mg/m², about 45 mg/m² to about 100 mg/m², about 75 mg/m2 to about 100 mg/m², about 20 mg/m², about 45 mg/m², about 65 mg/m², about 75 mg/m², or about 100 mg/m²).

In one aspect of the present application there is provided a kit comprising a composition comprising nanoparticles comprising an mTOR inhibitor and an albumin; and an agent for assessing an mTOR-activating aberration.

Also provided are compositions (such as pharmaceutical compositions), medicine, kits, and unit dosages useful for methods described herein.

These and other aspects and advantages of the present invention will become apparent from the subsequent detailed description and the appended claims. It is to be understood that one, some, or all of the properties of the various embodiments described herein may be combined to form other embodiments of the present invention.

### BRIEF DESCRIPTION OF THE FIGURES

FIG. 1 shows antitumor activity of single agents in UMUC3 bladder cancer mouse xenograft model during part A of the nonclinical study of Example 2.
FIG. 2A shows tumor volume changes following single agent treatments, including rapamycin, everolimus, and ABI-009 at three different doses, in UMUC3 bladder cancer mouse xenograft model during part A of the nonclinical study of Example 2.
FIG. 2B shows tumor volume changes following single agent treatments, including ABI-009, mitomycin C, cisplatin, gemcitabine, valrubicin, and docetaxel, in UMUC3 bladder cancer mouse xenograft model during part A of the nonclinical study of Example 2.
FIG. 2C shows body weight changes following single agent treatments, including rapamycin, everolimus, and ABI-009 at three different doses, in UMUC3 bladder cancer mouse xenograft model during part A of the nonclinical study of Example 2.
FIG. 2D shows body weight changes following single agent treatments, including ABI-009, mitomycin C, cisplatin, gemcitabine, valrubicin, and docetaxel, in UMUC3 bladder cancer mouse xenograft model during part A of the nonclinical study of Example 2.
FIG. 3A shows survival curves of mice with UMUC3 bladder cancer xenograft following single agent treatments, including rapamycin, everolimus, and ABI-009 at three different doses during part A of the nonclinical study of Example 2.
FIG. 3B shows survival curves of mice with UMUC3 bladder cancer xenograft following single agent treatments, including ABI-009, mitomycin C, cisplatin, gemcitabine, valrubicin, and docetaxel during part A of the nonclinical study of Example 2.
FIG. 4 shows antitumor activity of combination treatments in UMUC3 bladder cancer mouse xenograft model during part B of the nonclinical study of Example 2.
FIG. 5A shows tumor volume changes following combination treatments, including ABI-009, mitomycin C, cisplatin, gemcitabine, valrubicin, and docetaxel, in UMUC3 bladder cancer mouse xenograft model during part B of the nonclinical study of Example 2.
FIG. 5B shows tumor volume changes following combination treatments, i combination of ABI-009 with mitomycin C (MMC), combination of ABI-009 with cisplatin (Cis), combination of ABI-009 with gemcitabine (Gem), combination of ABI-009 with valrubicin (Val), and combination of ABI-009 with docetaxel (Doc), in UMUC3 bladder cancer mouse xenograft model during part B of the nonclinical study of Example 2.
FIG. 5C shows body weight changes following combination treatments, including ABI-009, mitomycin C, cisplatin, gemcitabine, valrubicin, and docetaxel, in UMUC3 bladder cancer mouse xenograft model during part B of the nonclinical study of Example 2.
FIG. 5D shows body weight changes following combination treatments, including combination of ABI-009 with mitomycin C (MMC), combination of ABI-009 with cisplatin (Cis), combination of ABI-009 with gemcitabine (Gem), combination of ABI-009 with valrubicin (Val), and combination of ABI-009 with docetaxel (Doc), in UMUC3 bladder cancer mouse xenograft model during part B of the nonclinical study of Example 2.
FIG. 6A shows survival curves of mice with UMUC3 bladder cancer xenograft following single agent treatments in part B of the nonclinical study of Example 2, including ABI-009, mitomycin C, cisplatin, gemcitabine, valrubicin, or docetaxel.
FIG. 6B shows survival curves of mice with UMUC3 bladder cancer xenograft following ABI-009 single agent or combination treatments in part B of the nonclinical study of Example 2, including combination of ABI-009 with mitomycin C (MMC), combination of ABI-009 with cisplatin (Cis), combination of ABI-009 with gemcitabine (Gem), combination of ABI-009 with valrubicin (Val), and combination of ABI-009 with docetaxel (Doc).
FIG. 7A shows comparison of tumor volume changes following single agent treatments (ABI-009, or mitomycin C) versus combination treatment (ABI-009 and mitomycin C) in UMUC3 bladder cancer mouse xenograft model.
FIG. 7B shows comparison of percent survival following single agent treatments (ABI-009, or mitomycin C) versus combination treatment (ABI-009 and mitomycin C) in UMUC3 bladder cancer mouse xenograft model.
FIG. 7C shows comparison of tumor volume changes following single agent treatments (ABI-009, or cisplatin) versus combination treatment (ABI-009 and cisplatin) in UMUC3 bladder cancer mouse xenograft model.
FIG. 7D shows comparison of percent survival following single agent treatments (ABI-009, or cisplatin) versus combination treatment (ABI-009 and cisplatin) in UMUC3 bladder cancer mouse xenograft model.
FIG. 7E shows comparison of tumor volume changes following single agent treatments (ABI-009, or gemcitabine) versus combination treatment (ABT-009 and gemcitabine) in UMUC3 bladder cancer mouse xenograft model.
FIG. 7F shows comparison of percent survival following single agent treatments (ABI-009, or gemcitabine) versus combination treatment (ABI-009 and gemcitabine) in UMUC3 bladder cancer mouse xenograft model.
FIG. 7G shows comparison of tumor volume changes following single agent treatments (ABI-009, or valrubicin) versus combination treatment (ABI-009 and valrubicin) in UMUC3 bladder cancer mouse xenograft model.
FIG. 7H shows comparison of percent survival following single agent treatments (ABI-009, or valrubicin) versus combination treatment (ABI-009 and valrubicin) in UMUC3 bladder cancer mouse xenograft model.
FIG. 7I shows comparison of tumor volume changes following single agent treatments (ABI-009, or docetaxel) versus combination treatment (ABI-009 and docetaxel) in UMUC3 bladder cancer mouse xenograft model.
FIG. 7J shows comparison of percent survival following single agent treatments (ABI-009, or docetaxel) versus combination treatment (ABI-009 and docetaxel) in UMUC3 bladder cancer mouse xenograft model.
FIG. 8 shows experimental design schema for the Phase I clinical study described in Example 6.

### DETAILED DESCRIPTION OF THE INVENTION

The present invention provides methods of treatment of an individual having a hyperplasia (such as cancer, restenosis, or pulmonary hypertension) with a nanoparticle composition comprising an mTOR inhibitor (such as a limus drug) and an albumin, wherein the level and/or mutational status of one or more biomarkers associated with the mTOR pathway is used as a basis of selecting the individual for the treatment. Aberrations in the sequence, expression level, phosphorylation, and/or activity level of any one or combinations of the biomarkers described herein are associated with hyperactivation of the mTOR pathway (hereinafter referred to as "mTOR-activating aberrations"), which in turn correlate with responses of the individual to treatment involving the nanoparticle composition.

In one aspect, there is provided a method of treating a hyperplasia (such as cancer, restenosis, or pulmonary hypertension) in an individual having an mTOR-activating aberration, comprising administering to the individual an effective amount of a composition comprising nanoparticles comprising an mTOR inhibitor (such as a limus drug) and an albumin.

In another aspect, there is provided a method of treating a hyperplasia (such as cancer, restenosis, or pulmonary hypertension) in an individual comprising administering to the individual an effective amount of a composition comprising nanoparticles comprising an mTOR inhibitor (such as a limus drug) and an albumin, wherein the individual is selected for treatment based on the individual having an mTOR-activating aberration.

In another aspect, there is provided a method of selecting (including identifying) an individual for treatment with a composition comprising nanoparticles comprising an mTOR inhibitor (such as a limus drug) and an albumin, wherein the method comprises assessing the mTOR-activating aberration.

Also provided are compositions (such as pharmaceutical compositions), medicine, kits, and unit dosages useful for the methods described herein.

### Definitions

As used herein, "treatment" or "treating" is an approach for obtaining beneficial or desired results including clinical results. For purposes of this invention, beneficial or desired clinical results include, but are not limited to, one or more of the following: alleviating one or more symptoms resulting from the disease, diminishing the extent of the disease, stabilizing the disease (*e.g*., preventing or delaying the worsening of the disease), preventing or delaying the spread *(e.g.,* metastasis) of the disease, preventing or delaying the recurrence of the disease, delay or slowing the progression of the disease, ameliorating the disease state, providing a remission (partial or total) of the disease, decreasing the dose of one or more other medications required to treat the disease, delaying the progression of the disease, increasing the quality of life, and/or prolonging survival. Also encompassed by "treatment" is a reduction of a pathological consequence of a hyperplasia, such as cancer, restenosis, or pulmonary hypertension. The methods of the invention contemplate any one or more of these aspects of treatment.

The term "individual" refers to a mammal and includes, but is not limited to, human, bovine, horse, feline, canine, rodent, or primate. In some embodiments, the individual is a human.

As used herein, an "at risk" individual is an individual who is at risk of developing a hyperplasia (*e.g*. cancer, restenosis, or pulmonary hypertension). An individual "at risk" may or may not have detectable disease, and may or may not have displayed detectable disease prior to the treatment methods described herein. "At risk" denotes that an individual has one or more socalled risk factors, which are measurable parameters that correlate with development of a hyperplasia (*e.g*. cancer, restenosis, or pulmonary hypertension), which are described herein. An individual having one or more of these risk factors has a higher probability of developing hyperplasia (*e.g*. cancer, restenosis, or pulmonary hypertension) than an individual without these risk factor(s).

"Adjuvant setting" refers to a clinical setting in which an individual has had a history of a hyperplasia (*e.g*. cancer, restenosis, or pulmonary hypertension), and generally (but not necessarily) been responsive to therapy, which includes, but is not limited to, surgery (*e.g*., surgery resection), radiotherapy, and chemotherapy. However, because of their history of a hyperplasia (*e.g*. cancer, restenosis, or pulmonary hypertension), these individuals are considered at risk of development of the disease. Treatment or administration in the "adjuvant setting" refers to a subsequent mode of treatment. The degree of risk *(e.g.,* when an individual in the adjuvant setting is considered as "high risk" or "low risk") depends upon several factors, most usually the extent of disease when first treated.

"Neoadjuvant setting" refers to a clinical setting in which the method is carried out before the primary/definitive therapy.

As used herein, "delaying" the development of a hyperplasia (*e.g*. cancer, restenosis, or pulmonary hypertension) means to defer, hinder, slow, retard, stabilize, and/or postpone development of the disease. This delay can be of varying lengths of time, depending on the history of the disease and/or individual being treated. As is evident to one skilled in the art, a sufficient or significant delay can, in effect, encompass prevention, in that the individual does not develop the disease. A method that "delays" development of a hyperplasia (*e.g*. cancer, restenosis, or pulmonary hypertension) is a method that reduces probability of disease development in a given time frame and/or reduces the extent of the disease in a given time frame, when compared to not using the method. Such comparisons are typically based on clinical studies, using a statistically significant number of subjects. Hyperplasia (*e.g*. cancer, restenosis, or pulmonary hypertension) development can be detectable using standard methods, including, but not limited to, computerized axial tomography (CAT Scan), Magnetic Resonance Imaging (MRI), abdominal ultrasound, clotting tests, arteriography, or biopsy. Development may also refer to hyperplasia (*e.g*. cancer, restenosis, or pulmonary hypertension) progression that may be initially undetectable and includes occurrence, recurrence, and onset.

The term "effective amount" used herein refers to an amount of a compound or composition sufficient to treat a specified disorder, condition or disease such as ameliorate, palliate, lessen, and/or delay one or more of its symptoms. For therapeutic use, beneficial or desired results include, *e.g*., decreasing one or more symptoms resulting from the disease (biochemical, histologic and/or behavioral), including its complications and intermediate pathological phenotypes presenting during development of the disease, increasing the quality of life of those suffering from the disease, decreasing the dose of other medications required to treat the disease, enhancing effect of another medication, delaying the progression of the disease, and/or prolonging survival of patients. In reference to a hyperplasia (*e.g.* cancer, restenosis, or pulmonary hypertension), an effective amount comprises an amount sufficient to cause a hyperplastic tissue (such as a tumor) to shrink and/or to decrease the growth rate of the hyperplastic tissue (such as to suppress hyperplastic or tumor growth) or to prevent or delay other unwanted cell proliferation in the hyperplasia. In some embodiments, an effective amount is an amount sufficient to delay development of a hyperplasia (*e.g*. cancer, restenosis, or pulmonary hypertension). In some embodiments, an effective amount is an amount sufficient to prevent or delay recurrence. An effective amount can be administered in one or more administrations. In the case of cancer, the effective amount of the drug or composition may: (i) reduce the number of tumor cells; (ii) reduce the tumor size; (iii) inhibit, retard, slow to some extent and preferably stop a tumor cell infiltration into peripheral organs; (iv) inhibit (i.e., slow to some extent and preferably stop) tumor metastasis; (v) inhibit tumor growth; (vi) prevent or delay occurrence and/or recurrence of tumor; and/or (vii) relieve to some extent one or more of the symptoms associated with the cancer.

The term "simultaneous administration," as used herein, means that a first therapy and second therapy in a combination therapy are administered with a time separation of no more than about 15 minutes, such as no more than about any of 10, 5, or 1 minutes. When the first and second therapies are administered simultaneously, the first and second therapies may be contained in the same composition *(e.g.,* a composition comprising both a first and second therapy) or in separate compositions *(e.g.,* a first therapy in one composition and a second therapy is contained in another composition).

As used herein, the term "sequential administration" means that the first therapy and second therapy in a combination therapy are administered with a time separation of more than about 15 minutes, such as more than about any of 20, 30, 40, 50, 60, or more minutes. Either the first therapy or the second therapy may be administered first. The first and second therapies are contained in separate compositions, which may be contained in the same or different packages or kits.

As used herein, the term "concurrent administration" means that the administration of the first therapy and that of a second therapy in a combination therapy overlap with each other.

As used herein, by "pharmaceutically acceptable" or "pharmacologically compatible" is meant a material that is not biologically or otherwise undesirable, *e.g*., the material may be incorporated into a pharmaceutical composition administered to a patient without causing any significant undesirable biological effects or interacting in a deleterious manner with any of the other components of the composition in which it is contained. Pharmaceutically acceptable carriers or excipients have preferably met the required standards of toxicological and manufacturing testing and/or are included on the Inactive Ingredient Guide prepared by the U.S. Food and Drug administration.

An "adverse event" or "AE" as used herein refers to any untoward medical occurrence in an individual receiving a marketed pharmaceutical product or in an individual who is participating on a clinical trial who is receiving an investigational or non-investigational pharmaceutical agent. The AE does not necessarily have a causal relationship with the individual's treatment. Therefore, an AE can be any unfavorable and unintended sign, symptom, or disease temporally associated with the use of a medicinal product, whether or not considered to be related to the medicinal product. An AE includes, but is not limited to: an exacerbation of a pre-existing illness; an increase in frequency or intensity of a pre-existing episodic event or condition; a condition detected or diagnosed after study drug administration even though it may have been present prior to the start of the study; and continuously persistent disease or symptoms that were present at baseline and worsen following the start of the study. An AE generally does not include: medical or surgical procedures (*e.g*., surgery, endoscopy, tooth extraction, or transfusion); however, the condition that leads to the procedure is an adverse event; pre-existing diseases, conditions, or laboratory abnormalities present or detected at the start of the study that do not worsen; hospitalizations or procedures that are done for elective purposes not related to an untoward medical occurrence (*e.g*., hospitalizations for cosmetic or elective surgery or social/convenience admissions); the disease being studied or signs/symptoms associated with the disease unless more severe than expected for the individual's condition; and overdose of study drug without any clinical signs or symptoms.

A "serious adverse event" or (SAE) as used herein refers to any untoward medical occurrence at any dose including, but not limited to, that: a) is fatal; b) is life-threatening (defined as an immediate risk of death from the event as it occurred); c) results in persistent or significant disability or incapacity; d) requires in-patient hospitalization or prolongs an existing hospitalization (exception: Hospitalization for elective treatment of a pre-existing condition that did not worsen during the study is not considered an adverse event. Complications that occur during hospitalization are AEs and if a complication prolongs hospitalization, then the event is serious); e) is a congenital anomaly/birth defect in the offspring of an individual who received medication; or f) conditions not included in the above definitions that may jeopardize the individual or may require intervention to prevent one of the outcomes listed above unless clearly related to the individual's underlying disease. "Lack of efficacy" (progressive disease) is not considered an AE or SAE. The signs and symptoms or clinical sequelae resulting from lack of efficacy should be reported if they fulfill the AE or SAE definitions.

The following definitions may be used to evaluate response based on target lesions: "complete response" or "CR" refers to disappearance of all target lesions; "partial response" or "PR" refers to at least a 30% decrease in the sum of the longest diameters (SLD) of target lesions, taking as reference the baseline SLD; "stable disease" or "SD" refers to neither sufficient shrinkage of target lesions to qualify for PR, nor sufficient increase to qualify for PD, taking as reference the nadir SLD since the treatment started; and "progressive disease" or "PD" refers to at least a 20% increase in the SLD of target lesions, taking as reference the nadir SLD recorded since the treatment started, or, the presence of one or more new lesions.

The following definitions of response assessments may be used to evaluate a non-target lesion: "complete response" or "CR" refers to disappearance of all non-target lesions; "stable disease" or "SD" refers to the persistence of one or more non-target lesions not qualifying for CR or PD; and "progressive disease" or "PD" refers to the "unequivocal progression" of existing non-target lesion(s) or appearance of one or more new lesion(s) is considered progressive disease (if PD for the subject is to be assessed for a time point based solely on the progression of non-target lesion(s), then additional criteria are required to be fulfilled.

"Progression free survival" (PFS) indicates the length of time during and after treatment that the cancer does not grow. Progression-free survival includes the amount of time individuals have experienced a complete response or a partial response, as well as the amount of time individuals have experienced stable disease.

"Correlate" or "correlating" is meant comparing, in any way, the performance and/or results of a first analysis or protocol with the performance and/or results of a second analysis or protocol. For example one may use the results of a first analysis or protocol to determine whether a second analysis or protocol should be performed. With respect to the embodiment of gene expression analysis or protocol, one may use the results of the gene expression analysis or protocol to determine whether a specific therapeutic regimen should be performed.

"Predicting" or "prediction" is used herein to refer to the likelihood that an individual is likely to respond either favorably or unfavorably to a treatment regimen.

As used herein, "at the time of starting treatment" or "baseline" refers to the time period at or prior to the first exposure to the treatment.

A method of "aiding assessment" as used herein refers to methods that assist in making a clinical determination and may or may not be conclusive with respect to the assessment.

"Likely to respond" or "responsiveness" as used herein refers to any kind of improvement or positive response either clinical or non-clinical selected from, but not limited to, measurable reduction in tumor size or evidence of disease or disease progression, complete response, partial response, stable disease, increase or elongation of progression free survival, or increase or elongation of overall survival.

As used herein, "sample" refers to a composition which contains a molecule which is to be characterized and/or identified, for example, based on physical, biochemical, chemical, physiological, and/or genetic characteristics.

"Cells," as used herein, is understood to refer not only to the particular subject cell, but to the progeny or potential progeny of such a cell. Because certain modifications may occur in succeeding generations due to either mutation or environmental influences, such progeny may not, in fact, be identical to the parent cell, but are still included within the scope of the term as used herein.

The mTOR-activing aberration determined "before or upon initiation of treatment" is the mTOR-activing aberration determined in an individual before or upon the individual receives the first administration of a treatment modality described herein.

An individual who "may be suitable", which includes an individual who is "suitable" for treatment(s) described herein, is an individual who is more likely than not to benefit from administration of said treatments. Conversely, an individual who "may not be suitable" or "may be unsuitable", which includes an individual who is "unsuitable" for treatment(s) described herein, is an individual who is more likely than not to fail to benefit from administration of said treatments.

As used herein, "mTOR inhibitor nanoparticle composition" refers to a composition comprising nanoparticles comprising an mTOR inhibitor (such as a limus drug) and an albumin. "Limus nanoparticle composition" refers to a composition comprising nanoparticles comprising a limus drug (such as Sirolimus) and an albumin.

It is understood that aspect and embodiments of the invention described herein include "consisting" and/or "consisting essentially of" aspects and embodiments.

Reference to "about" a value or parameter herein includes (and describes) variations that are directed to that value or parameter per se. For example, description referring to "about X" includes description of "X".

The term "about X-Y" used herein has the same meaning as "about X to about Y."

As used herein and in the appended claims, the singular forms "a," "or," and "the" include plural referents unless the context clearly dictates otherwise.

As is apparent to one skilled in the art, an individual assessed, selected for, and/or receiving treatment is an individual in need of such activities.

### Methods of Treatment Based on Status of an mTOR-activating Aberration

The present invention in one aspect provides methods of treating hyperplasia (such as cancer, restenosis or pulmonary hypertension) based on the status of one or more mTOR-activating aberrations in one or more mTOR-associated genes.

In some embodiments, there is provided a method of treating a hyperplasia (such as cancer, restenosis, or pulmonary hypertension) in an individual comprising administering to the individual an effective amount of a composition comprising nanoparticles comprising an mTOR inhibitor (such as a limus drug) and an albumin, wherein the individual is selected for treatment based on the individual having an mTOR-activating aberration. In some embodiments, there is provided a method of treating a hyperplasia (such as cancer, restenosis, or pulmonary hypertension) in an individual comprising administering to the individual an effective amount of a composition comprising nanoparticles comprising a limus drug (such as sirolimus) and an albumin (including nanoparticles having an average diameter of no greater than about 150 nm), wherein the individual is selected for treatment based on the individual having an mTOR-activating aberration. In some embodiments, there is provided a method of treating a hyperplasia (such as cancer, restenosis, or pulmonary hypertension) in an individual comprising administering to the individual an effective amount of a composition comprising nanoparticles comprising sirolimus associated (*e.g*., coated) with albumin (including nanoparticles having an average diameter of no greater than about 150 nm and a weight ratio of albumin to sirolimus in the composition is no more than about 9:1), wherein the individual is selected for treatment based on the individual having an mTOR-activating aberration. In some embodiments, there is provided a method of treating a hyperplasia (such as cancer, restenosis, or pulmonary hypertension) in an individual comprising administering to the individual an effective amount of *Nab*-sirolimus, wherein the individual is selected for treatment based on the individual having an mTOR-activating aberration. In some embodiments, the mTOR-activating aberration comprises a mutation of an mTOR-associated gene. In some embodiments, the mTOR-activating aberration comprises a copy number variation of an mTOR-associated gene. In some embodiments, the mTOR-activating aberration comprises an aberrant expression level of an mTOR-associated gene. In some embodiments, the mTOR-activating aberration comprises an aberrant activity level of an mTOR-associated gene. In some embodiments, the mTOR-activating aberration leads to activation of mTORC1 (including for example activation of mTORC1 but not mTORC2). In some embodiments, the mTOR-activating aberration leads to activation of mTORC2 (including for example activation of mTORC2 but not mTORC1). In some embodiments, the mTOR-activating aberration leads to activation of both mTORC1 and mTORC2. In some embodiments, the mTOR-activating aberration is an aberration in at least one mTOR-associated gene selected from the group consisting of AKT1, FLT3, MTOR, PIK3CA, PIK3CG, TSC1, TSC2, RHEB, STK11, NF1, NF2, PTEN, TP53, FGFR4, KRAS, NRAS, and BAP1. In some embodiments, the mTOR-activating aberration is assessed by gene sequencing. In some embodiments, the gene sequencing is based on sequencing of DNA in a tumor sample. In some embodiments, the gene sequencing is based on sequencing of circulating DNA or cell-free DNA isolated from a blood sample. In some embodiments, the mutational status of TFE3 is further used as a basis for selecting the individual. In some embodiments, the mutational status of TFE3 comprises translocation of TFE3. In some embodiments, the mTOR-activating aberration comprises an aberrant phosphorylation level of the protein encoded by the mTOR-associated gene. In some embodiments, the mTOR-activating aberration comprises an aberrant phosphorylation level of a protein encoded by an mTOR-associated gene selected from the group consisting of AKT, S6K, S6, 4EBP1, and SPARC. In some embodiments, the aberrant phosphorylation level is determined by immunohistochemistry.

In some embodiments, there is provided a method of treating a hyperplasia (such as cancer, restenosis, or pulmonary hypertension) in an individual comprising: (a) assessing an mTOR-activating aberration in the individual; and (b) administering to the individual an effective amount of a composition comprising nanoparticles comprising an mTOR inhibitor (such as a limus drug) and an albumin, wherein the individual is selected for treatment based on having the mTOR-activating aberration. In some embodiments, there is provided a method of treating a hyperplasia (such as cancer, restenosis, or pulmonary hypertension) in an individual comprising: (a) assessing an mTOR-activating aberration in the individual; and (b) administering to the individual an effective amount of a composition comprising nanoparticles comprising a limus drug (such as sirolimus) and an albumin (including nanoparticles having an average diameter of no greater than about 150 nm), wherein the individual is selected for treatment based on having the mTOR-activating aberration. In some embodiments, there is provided a method of treating a hyperplasia (such as cancer, restenosis, or pulmonary hypertension) in an individual comprising: (a) assessing an mTOR-activating aberration in the individual; and (b) administering to the individual an effective amount of a composition comprising nanoparticles comprising sirolimus associated (*e.g*., coated) with albumin (including nanoparticles having an average diameter of no greater than about 150 nm and a weight ratio of albumin to sirolimus in the composition is no more than about 9:1), wherein the individual is selected for treatment based on having the mTOR-activating aberration. In some embodiments, there is provided a method of treating a hyperplasia (such as cancer, restenosis, or pulmonary hypertension) in an individual comprising: (a) assessing an mTOR-activating aberration in the individual; and (b) administering to the individual an effective amount of*Nab*-sirolimus, wherein the individual is selected for treatment based on having the mTOR-activating aberration. In some embodiments, the mTOR-activating aberration comprises a mutation of an mTOR-associated gene. In some embodiments, the mTOR-activating aberration comprises a copy number variation of an mTOR-associated gene. In some embodiments, the mTOR-activating aberration comprises an aberrant expression level of an mTOR-associated gene. In some embodiments, the mTOR-activating aberration comprises an aberrant activity level of an mTOR-associated gene. In some embodiments, the mTOR-activating aberration leads to activation of mTORC1 (including for example activation of mTORC1 but not mTORC2). In some embodiments, the mTOR-activating aberration leads to activation of mTORC2 (including for example activation of mTORC2 but not mTORC1). In some embodiments, the mTOR-activating aberration leads to activation of both mTORC1 and mTORC2. In some embodiments, the mTOR-activating aberration is an aberration in at least one mTOR-associated gene selected from the group consisting of AKT1, FLT3, MTOR, PIK3CA, PIK3CG, TSC1, TSC2, RHEB, STK11, NF1, NF2, PTEN, TP53, FGFR4, KRAS, NRAS, and BAPl. In some embodiments, the mTOR-activating aberration is assessed by gene sequencing. In some embodiments, the gene sequencing is based on sequencing of DNA in a tumor sample. In some embodiments, the gene sequencing is based on sequencing of circulating DNA or cell-free DNA isolated from a blood sample. In some embodiments, the mutational status of TFE3 is further used as a basis for selecting the individual. In some embodiments, the mutational status of TFE3 comprises translocation of TFE3. In some embodiments, the mTOR-activating aberration comprises an aberrant phosphorylation level of the protein encoded by the mTOR-associated gene. In some embodiments, the mTOR-activating aberration comprises an aberrant phosphorylation level of a protein encoded by an mTOR-associated gene selected from the group consisting of AKT, S6K, S6, 4EBP1, and SPARC. In some embodiments, the aberrant phosphorylation level is determined by immunohistochemistry.

In some embodiments, there is provided a method of treating a hyperplasia (such as cancer, restenosis, or pulmonary hypertension) in an individual comprising: (a) assessing an mTOR-activating aberration in the individual; (b) selecting (*e.g*., identifying or recommending) the individual for treatment based on the individual having the mTOR-activating aberration; and (c) administering to the individual an effective amount of a composition comprising nanoparticles comprising an mTOR inhibitor (such as a limus drug) and an albumin. In some embodiments, there is provided a method of treating a hyperplasia (such as cancer, restenosis, or pulmonary hypertension) in an individual comprising: (a) assessing an mTOR-activating aberration in the individual; (b) selecting *(e.g.,* identifying or recommending) the individual for treatment based on the individual having the mTOR-activating aberration; and (c) administering to the individual an effective amount of a composition comprising nanoparticles comprising a limus drug (such as sirolimus) and an albumin (including nanoparticles having an average diameter of no greater than about 150 nm). In some embodiments, there is provided a method of treating a hyperplasia (such as cancer, restenosis, or pulmonary hypertension) in an individual comprising: (a) assessing an mTOR-activating aberration in the individual; (b) selecting (*e.g.,* identifying or recommending) the individual for treatment based on the individual having the mTOR-activating aberration; and (c) administering to the individual an effective amount of a composition comprising nanoparticles comprising sirolimus associated (*e.g*., coated) with albumin (including nanoparticles having an average diameter of no greater than about 150 nm and a weight ratio of albumin to sirolimus in the composition is no more than about 9:1). In some embodiments, there is provided a method of treating a hyperplasia (such as cancer, restenosis, or pulmonary hypertension) in an individual comprising: (a) assessing an mTOR-activating aberration in the individual; (b) selecting (*e.g.*, identifying or recommending) the individual for treatment based on the individual having the mTOR-activating aberration; and (c) administering to the individual an effective amount of *Nab*-sirolimus. In some embodiments, the mTOR-activating aberration comprises a mutation of an mTOR-associated gene. In some embodiments, the mTOR-activating aberration comprises a copy number variation of an mTOR-associated gene. In some embodiments, the mTOR-activating aberration comprises an aberrant expression level of an mTOR-associated gene. In some embodiments, the mTOR-activating aberration comprises an aberrant activity level of an mTOR-associated gene. In some embodiments, the mTOR-activating aberration leads to activation of mTORC1 (including for example activation of mTORC1 but not mTORC2). In some embodiments, the mTOR-activating aberration leads to activation of mTORC2 (including for example activation of mTORC2 but not mTORC1). In some embodiments, the mTOR-activating aberration leads to activation of both mTORC1 and mTORC2. In some embodiments, the mTOR-activating aberration is an aberration in at least one mTOR-associated gene selected from the group consisting of AKT1, FLT3, MTOR, PIK3CA, PIK3CG, TSC1, TSC2, RHEB, STKl 1, NF1, NF2, PTEN, TP53, FGFR4, KRAS, NRAS, and BAP1. In some embodiments, the mTOR-activating aberration is assessed by gene sequencing. In some embodiments, the gene sequencing is based on sequencing of DNA in a tumor sample. In some embodiments, the gene sequencing is based on sequencing of circulating DNA or cell-free DNA isolated from a blood sample. In some embodiments, the mutational status of TFE3 is further used as a basis for selecting the individual. In some embodiments, the mutational status of TFE3 comprises translocation of TFE3. In some embodiments, the mTOR-activating aberration comprises an aberrant phosphorylation level of the protein encoded by the mTOR-associated gene. In some embodiments, the mTOR-activating aberration comprises an aberrant phosphorylation level of a protein encoded by an mTOR-associated gene selected from the group consisting of AKT, S6K, S6, 4EBP1, and SPARC. In some embodiments, the aberrant phosphorylation level is determined by immunohistochemistry.

The present invention in one aspect provides a method of treating a hyperplasia (such as cancer, restenosis, or pulmonary hypertension) in an individual comprising administering to the individual an effective amount of a composition comprising nanoparticles comprising an mTOR inhibitor (such as a limus drug) and an albumin, wherein the individual is selected for treatment on the basis of having an mTOR-activating aberration. In some embodiments, there is provided a method of treating a hyperplasia (such as cancer, restenosis, or pulmonary hypertension) in an individual comprising administering to the individual an effective amount of a composition comprising nanoparticles comprising a limus drug (such as sirolimus) and an albumin (including nanoparticles having an average diameter of no greater than about 150 nm), wherein the individual is selected for treatment on the basis of having an mTOR-activating aberration. In some embodiments, there is provided a method of treating a hyperplasia (such as cancer, restenosis, or pulmonary hypertension) in an individual comprising administering to the individual an effective amount of a composition comprising nanoparticles comprising sirolimus associated (*e.g*., coated) with albumin (including nanoparticles having an average diameter of no greater than about 150 nm and a weight ratio of albumin to sirolimus in the composition is no more than about 9:1), wherein the individual is selected for treatment on the basis of having an mTOR-activating aberration. In some embodiments, there is provided a method of treating a hyperplasia (such as cancer, restenosis, or pulmonary hypertension) in an individual comprising administering to the individual an effective amount of *Nab*-sirolimus, wherein the individual is selected for treatment on the basis of having an mTOR-activating aberration. In some embodiments, the mTOR-activating aberration comprises a mutation of an mTOR-associated gene. In some embodiments, the mTOR-activating aberration comprises a copy number variation of an mTOR-associated gene. In some embodiments, the mTOR-activating aberration comprises an aberrant expression level of an mTOR-associated gene. In some embodiments, the mTOR-activating aberration comprises an aberrant activity level of an mTOR-associated gene. In some embodiments, the mTOR-activating aberration leads to activation of mTORC1 (including for example activation of mTORC1 but not mTORC2). In some embodiments, the mTOR-activating aberration leads to activation of mTORC2 (including for example activation of mTORC2 but not mTORC1). In some embodiments, the mTOR-activating aberration leads to activation of both mTORC1 and mTORC2. In some embodiments, the mTOR-activating aberration is an aberration in at least one mTOR-associated gene selected from the group consisting of AKT1, FLT3, MTOR, PIK3CA, PIK3CG, TSC1, TSC2, RHEB, STK11, NF1, NF2, PTEN, TP53, FGFR4, KRAS, NRAS, and BAP1. In some embodiments, the mTOR-activating aberration is assessed by gene sequencing. In some embodiments, the gene sequencing is based on sequencing of DNA in a tumor sample. In some embodiments, the gene sequencing is based on sequencing of circulating DNA or cell-free DNA isolated from a blood sample. In some embodiments, the mutational status of TFE3 is further used as a basis for selecting the individual. In some embodiments, the mutational status of TFE3 comprises translocation of TFE3. In some embodiments, the mTOR-activating aberration comprises an aberrant phosphorylation level of the protein encoded by the mTOR-associated gene. In some embodiments, the mTOR-activating aberration comprises an aberrant phosphorylation level of a protein encoded by an mTOR-associated gene selected from the group consisting of AKT, S6K, S6, 4EBP1, and SPARC. In some embodiments, the aberrant phosphorylation level is determined by immunohistochemistry.

In some embodiments, there is provided a method of selecting (including identifying or recommending) an individual having a hyperplasia (such as cancer, restenosis, or pulmonary hypertension) for treatment with a composition comprising nanoparticles comprising an mTOR inhibitor (such as a limus drug) and an albumin, wherein the method comprises (a) assessing an mTOR-activating aberration in the individual; and (b) selecting or recommending the individual for treatment based on the individual having the mTOR-activating aberration. In some embodiments, there is provided a method of selecting (including identifying or recommending) an individual having a hyperplasia (such as cancer, restenosis, or pulmonary hypertension) for treatment with a composition comprising a limus drug (such as sirolimus) and an albumin (including nanoparticles having an average diameter of no greater than about 150 nm), wherein the method comprises (a) assessing an mTOR-activating aberration in the individual; and (b) selecting or recommending the individual for treatment based on the individual having the mTOR-activating aberration. In some embodiments, there is provided a method of selecting (including identifying or recommending) an individual having a hyperplasia (such as cancer, restenosis, or pulmonary hypertension) for treatment with a composition comprising nanoparticles comprising sirolimus associated (*e.g*., coated) with albumin (including nanoparticles having an average diameter of no greater than about 150 nm and a weight ratio of albumin to sirolimus in the composition is no more than about 9:1), wherein the method comprises (a) assessing an mTOR-activating aberration in the individual; and (b) selecting or recommending the individual for treatment based on the individual having the mTOR-activating aberration. In some embodiments, there is provided a method of selecting (including identifying or recommending) an individual having a hyperplasia (such as cancer, restenosis, or pulmonary hypertension) for treating with *Nab*-sirolimus, wherein the method comprises (a) assessing an mTOR-activating aberration in the individual; and (b) selecting or recommending the individual for treatment based on the individual having the mTOR-activating aberration. In some embodiments, the mTOR-activating aberration comprises a mutation of an mTOR-associated gene. In some embodiments, the mTOR-activating aberration comprises a copy number variation of an mTOR-associated gene. In some embodiments, the mTOR-activating aberration comprises an aberrant expression level of an mTOR-associated gene. In some embodiments, the mTOR-activating aberration comprises an aberrant activity level of an mTOR-associated gene. In some embodiments, the mTOR-activating aberration leads to activation of mTORC1 (including for example activation of mTORC1 but not mTORC2). In some embodiments, the mTOR-activating aberration leads to activation of mMTORC2 (including for example activation of mTORC2 but not mTORC1). In some embodiments, the mTOR-activating aberration leads to activation of both mTORC1 and mTORC2. In some embodiments, the mTOR-activating aberration is an aberration in at least one mTOR-associated gene selected from the group consisting of AKT1, FLT3, MTOR, PIK3CA, PIK3CG, TSC1, TSC2, RHEB, STKl 1, NF1, NF2, PTEN, TP53, FGFR4, KRAS, NRAS, and BAP1. In some embodiments, the mTOR-activating aberration is assessed by gene sequencing. In some embodiments, the gene sequencing is based on sequencing of DNA in a tumor sample. In some embodiments, the gene sequencing is based on sequencing of circulating DNA or cell-free DNA isolated from a blood sample. In some embodiments, the mutational status of TFE3 is further used as a basis for selecting the individual. In some embodiments, the mutational status of TFE3 comprises translocation of TFE3. In some embodiments, the mTOR-activating aberration comprises an aberrant phosphorylation level of the protein encoded by the mTOR-associated gene. In some embodiments, the mTOR-activating aberration comprises an aberrant phosphorylation level of a protein encoded by an mTOR-associated gene selected from the group consisting of AKT, S6K, S6, 4EBP1, and SPARC. In some embodiments, the aberrant phosphorylation level is determined by immunohistochemistry.

In some embodiments, there is provided a method of selecting (including identifying or recommending) an individual having a hyperplasia (such as cancer, restenosis, or pulmonary hypertension) for treatment with a composition comprising nanoparticles comprising an mTOR inhibitor (such as a limus drug) and an albumin, wherein the method comprises (a) assessing an mTOR-activating aberration in the individual; (b) selecting or recommending the individual for treatment based on the individual having the mTOR-activating aberration; and (c) administering an effective amount of the composition comprising the mTOR inhibitor (such as a limus drug) and the albumin to the selected individual. In some embodiments, there is provided a method of selecting (including identifying or recommending) an individual having a hyperplasia (such as cancer, restenosis, or pulmonary hypertension) for treatment with a composition comprising a limus drug (such as sirolimus) and an albumin (including nanoparticles having an average diameter of no greater than about 150 nm), wherein the method comprises (a) assessing an mTOR-activating aberration in the individual; (b) selecting or recommending the individual for treatment based on the individual having the mTOR-activating aberration; and (c) administering an effective amount of the composition comprising the limus drug (such as sirolimus) and the albumin to the selected individual. In some embodiments, there is provided a method of selecting (including identifying or recommending) an individual having a hyperplasia (such as cancer, restenosis, or pulmonary hypertension) for treatment with a composition comprising nanoparticles comprising sirolimus associated (*e.g*., coated) with albumin (including nanoparticles having an average diameter of no greater than about 150 nm and a weight ratio of albumin to sirolimus in the composition is no more than about 9:1), wherein the method comprises (a) assessing an mTOR-activating aberration in the individual; (b) selecting or recommending the individual for treatment based on the individual having the mTOR-activating aberration; and (c) administering an effective amount of the composition comprising nanoparticles comprising sirolimus associated (*e.g*., coated) with albumin to the selected individual. In some embodiments, there is provided a method of selecting (including identifying or recommending) an individual having a hyperplasia (such as cancer, restenosis, or pulmonary hypertension) for treating with *Nab*-sirolimus, wherein the method comprises (a) assessing an mTOR-activating aberration in the individual; (b) selecting or recommending the individual for treatment based on the individual having the mTOR-activating aberration; and (c) administering an effective amount of *Nab*-sirolimus to the selected individual. In some embodiments, the mTOR-activating aberration comprises a mutation of an mTOR-associated gene. In some embodiments, the mTOR-activating aberration comprises a copy number variation of an mTOR-associated gene. In some embodiments, the mTOR-activating aberration comprises an aberrant expression level of an mTOR-associated gene. In some embodiments, the mTOR-activating aberration comprises an aberrant activity level of an mTOR-associated gene. In some embodiments, the mTOR-activating aberration leads to activation of mTORC1 (including for example activation of mTORC1 but not mTORC2). In some embodiments, the mTOR-activating aberration leads to activation of mTORC2 (including for example activation of mTORC2 but not mTORC1). In some embodiments, the mTOR-activating aberration leads to activation of both mTORC1 and mTORC2. In some embodiments, the mTOR-activating aberration is an aberration in at least one mTOR-associated gene selected from the group consisting of AKT1, FLT3, MTOR, PIK3CA, PIK3CG, TSC1, TSC2, RHEB, STK11, NF1, NF2, PTEN, TP53, FGFR4, KRAS, NRAS, and BAP1. In some embodiments, the mTOR-activating aberration is assessed by gene sequencing. In some embodiments, the gene sequencing is based on sequencing of DNA in a tumor sample. In some embodiments, the gene sequencing is based on sequencing of circulating DNA or cell-free DNA isolated from a blood sample. In some embodiments, the mutational status of TFE3 is further used as a basis for selecting the individual. In some embodiments, the mutational status of TFE3 comprises translocation of TFE3. In some embodiments, the mTOR-activating aberration comprises an aberrant phosphorylation level of the protein encoded by the mTOR-associated gene. In some embodiments, the mTOR-activating aberration comprises an aberrant phosphorylation level of a protein encoded by an mTOR-associated gene selected from the group consisting of AKT, S6K, S6, 4EBP1, and SPARC. In some embodiments, the aberrant phosphorylation level is determined by immunohistochemistry.

Further provided are methods of treating a hyperplasia (such as cancer, restenosis, or pulmonary hypertension) in an individual comprising administering to the individual an effective amount of a composition comprising nanoparticles comprising an mTOR inhibitor (such as a limus drug) and an albumin, wherein the individual has an mTOR-activating aberration. In some embodiments, there is provided a method of treating a hyperplasia (such as cancer, restenosis, or pulmonary hypertension) in an individual comprising administering to the individual an effective amount of a composition comprising nanoparticles comprising a limus drug (such as sirolimus) and an albumin (including nanoparticles having an average diameter of no greater than about 150 nm), wherein the individual has an mTOR-activating aberration. In some embodiments, there is provided a method of treating a hyperplasia (such as cancer, restenosis, or pulmonary hypertension) in an individual comprising administering to the individual an effective amount of a composition comprising nanoparticles comprising sirolimus associated (*e.g*., coated) with albumin (including nanoparticles having an average diameter of no greater than about 150 nm and a weight ratio of albumin to sirolimus in the composition is no more than about 9:1), wherein the individual has an mTOR-activating aberration. In some embodiments, there is provided a method of treating a hyperplasia (such as cancer, restenosis, or pulmonary hypertension) in an individual comprising administering to the individual an effective amount of *Nab*-sirolimus, wherein the individual has an mTOR-activating aberration. In some embodiments, the mTOR-activating aberration comprises a mutation of an mTOR-associated gene. In some embodiments, the mTOR-activating aberration comprises a copy number variation of an mTOR-associated gene. In some embodiments, the mTOR-activating aberration comprises an aberrant expression level of an mTOR-associated gene. In some embodiments, the mTOR-activating aberration comprises an aberrant activity level of an mTOR-associated gene. In some embodiments, the mTOR-activating aberration leads to activation of mTORC1 (including for example activation of mTORC1 but not mTORC2). In some embodiments, the mTOR-activating aberration leads to activation of mTORC2 (including for example activation of mTORC2 but not mTORC1). In some embodiments, the mTOR-activating aberration leads to activation of both mTORC1 and mTORC2. In some embodiments, the mTOR-activating aberration is an aberration in at least one mTOR-associated gene selected from the group consisting of AKT1, FLT3, MTOR, PIK3CA, PIK3CG, TSC1, TSC2, RHEB, STK11, NF1, NF2, PTEN, TP53, FGFR4, KRAS, NRAS, and BAP1. In some embodiments, the mTOR-activating aberration is assessed by gene sequencing. In some embodiments, the gene sequencing is based on sequencing of DNA in a tumor sample. In some embodiments, the gene sequencing is based on sequencing of circulating DNA or cell-free DNA isolated from a blood sample. In some embodiments, the mutational status of TFE3 is further used as a basis for selecting the individual. In some embodiments, the mutational status of TFE3 comprises translocation of TFE3. In some embodiments, the mTOR-activating aberration comprises an aberrant phosphorylation level of the protein encoded by the mTOR-associated gene. In some embodiments, the mTOR-activating aberration comprises an aberrant phosphorylation level of a protein encoded by an mTOR-associated gene selected from the group consisting of AKT, S6K, S6, 4EBP1, and SPARC. In some embodiments, the aberrant phosphorylation level is determined by immunohistochemistry.

Also provided herein are methods of assessing whether an individual with a hyperplasia (such as cancer, restenosis, or pulmonary hypertension) is more likely to respond or less likely to respond to treatment based on the individual having an mTOR-activating aberration, wherein the treatment comprises a composition comprising nanoparticles comprising an mTOR inhibitor (such as a limus drug) and an albumin, the method comprising assessing the mTOR-activating aberration in the individual. In some embodiments, the method further comprises administering to the individual an effective amount of the composition comprising nanoparticles comprising an mTOR inhibitor (such as a limus drug) and an albumin to the individual who is determined to be likely to respond to the treatment. In some embodiments, the presence of the mTOR-activating aberration indicates that the individual is more likely to respond to the treatment, and the absence of the mTOR-activating aberration indicates that the individual is less likely to respond to the treatment. In some embodiments, the amount of the mTOR inhibitor (such as a limus drug) is determined based on the status of the mTOR-activating aberration.

Methods are also provided herein of aiding assessment of whether an individual with hyperplasia (such as cancer, restenosis or pulmonary hypertension) will likely respond to or is suitable for treatment based on the individual having an mTOR-activating aberration, wherein the treatment comprises an effective amount of a composition comprising an mTOR inhibitor (such as a limus drug) and an albumin, the method comprising assessing the mTOR-activating aberration in the individual. In some embodiments, the presence of the mTOR-activating aberration indicates that the individual will likely be responsive to the treatment, and the absence of the mTOR-activating aberration indicates that the individual is less likely to respond to the treatment. In some embodiments, the method further comprises administering an effective amount of a composition comprising nanoparticles comprising an mTOR inhibitor (such as a limus drug) and an albumin.

In addition, methods are provided herein of identifying an individual with hyperplasia (such as cancer, restenosis, or pulmonary hypertension) likely to respond to treatment comprising an effective amount of a composition comprising nanoparticles comprising an mTOR inhibitor (such as a limus drug) and an albumin, the method comprising: (a) assessing an mTOR-activating aberration in the individual; and (b) identifying the individual based on the individual having the mTOR-activating aberration. In some embodiments, the method further comprises administering i) an effective amount of a composition comprising nanoparticles comprising an mTOR inhibitor (such as a limus drug) and an albumin. In some embodiments, the amount of the mTOR inhibitor (such as a limus drug) is determined based on the status of the mTOR-activating aberration.

Also provided herein are methods of adjusting therapy treatment of an individual with hyperplasia (such as cancer, restenosis, or pulmonary hypertension) receiving an effective amount of a composition comprising nanoparticles comprising an mTOR inhibitor (such as a limus drug) and an albumin, the method comprising assessing an mTOR-activating aberration in a sample isolated from the individual, and adjusting the therapy treatment based on the status of the mTOR-activating aberration. In some embodiments, the amount of the mTOR inhibitor (such as a limus drug) is adjusted.

Provided herein are also methods for marketing a therapy comprising an effective amount of a composition comprising nanoparticles comprising an mTOR inhibitor (such as a limus drug) and an albumin for use in a hyperplasia (such as cancer, restenosis, or pulmonary hypertension) in an individual subpopulation, the methods comprising informing a target audience about the use of the therapy for treating the individual subpopulation characterized by the individuals of such subpopulation having a sample which has an mTOR-activating aberration.

In some embodiments of any of the methods described herein, the methods are predictive of and/or result in a measurable reduction in abnormal cell proliferation (including tumor size, degree of stenosis, and pulmonary pressure), evidence of disease or disease progression, objective response (including for example, in the case of cancer, complete response, partial response, and stable disease), increase or elongation of progression free survival, and/or increase or elongation of overall survival. In some embodiments of any of the methods above, an individual is likely to respond to an mTOR inhibitor nanoparticle composition (such as a limus nanoparticle composition, including *Nab*-sirolimus), alone or in combination with another agent, if the individual has an mTOR-activating aberration, wherein the individual's response to the treatment is evident by a measurable reduction in abnormal cell proliferation (including tumor size, degree of stenosis and pulmonary pressure), evidence of disease or disease progression, objective response (including for example, in the case of cancer, complete response, partial response, and stable disease), increase or elongation of progression free survival, and/or increase or elongation of overall survival.

In some embodiments of any of the methods described herein, there is provided a method of inhibiting abnormal cell proliferation (such as tumor growth, abnormal cell growth in a blood vessel or lung) in an individual, comprising administering to the individual an effective amount of a composition comprising nanoparticles comprising an mTOR inhibitor (such as a limus drug) and an albumin, wherein the individual is selected based on the individual having an mTOR-activating aberration. In some embodiments, at least about 10% (including for example at least about any of 20%, 30%, 40%, 60%, 70%, 80%, 90%, or 100%) of the abnormal cell proliferation is inhibited.

In some embodiments of any of the methods described herein, there is provided a method of reducing tumor size in an individual, comprising administering to the individual an effective amount of a composition comprising nanoparticles comprising an mTOR inhibitor (such as a limus drug) and an albumin, wherein the individual is selected based on the individual having an mTOR-activating aberration. In some embodiments, the tumor size is reduced at least about 10% (including for example at least about any of 20%, 30%, 40%, 60%, 70%, 80%, 90%, or 100%).

In some embodiments of any of the methods described herein, there is provided a method of retaining the luminal diameter or cross-section area of a blood vessel in an individual following an endovascular procedure, comprising administering to the individual an effective amount of a composition comprising nanoparticles comprising an mTOR inhibitor (such as a limus drug) and an albumin, wherein the individual is selected based on the individual having an mTOR-activating aberration. In some embodiments, the luminal diameter or cross-section area of the blood vessel is retained at least about 50% (including for example at least about any of 60%, 70%, 80%, 90% or 100%) of the luminal diameter or cross-section area of the blood vessel after the endovascular procedure. In some embodiments, the luminal diameter or cross-section area of the blood vessel is retained for at least about any one of 1, 2, 3, 4, 5, 6, 7, 8, 9, 10, or more years after the endovascular procedure.

In some embodiments of any of the methods described herein, there is provided a method of reducing pulmonary pressure of an individual, comprising administering to the individual an effective amount of a composition comprising nanoparticles comprising an mTOR inhibitor (such as a limus drug) and an albumin, wherein the individual is selected based on the individual having an mTOR-activating aberration. In some embodiments, the pulmonary pressure is reduced by at least about 10% (including for example at least about any of 20%, 30%, 40%, 60%, 70%, 80%, or 90%).

In some embodiments of any of the methods described herein, there is provided a method of inhibiting tumor metastasis in an individual, comprising administering to the individual an effective amount of a composition comprising nanoparticles comprising an mTOR inhibitor (such as a limus drug) and an albumin, wherein the individual is selected based on the individual having an mTOR-activating aberration. In some embodiments, at least about 10% (including for example at least about any of 20%, 30%, 40%, 60%, 70%, 80%, 90%, or 100%) metastasis is inhibited. In some embodiments, the method inhibits metastasis to lymph nodes.

In some embodiments of any of the methods described herein, there is provided a method of prolonging progression-free survival of hyperplasia (such as cancer, restenosis or pulmonary hypertension) in an individual, comprising administering to the individual an effective amount of a composition comprising nanoparticles comprising an mTOR inhibitor (such as a limus drug) and an albumin, wherein the individual is selected based on the individual having an mTOR-activating aberration. In some embodiments, the method prolongs the time to disease progression by at least about any of 1, 2, 3, 4, 5, 6, 7, 8, 9, 10, 11, 12 months, wherein the hyperplasia is cancer. In some embodiments, the method prolongs the time to disease progression by at least about any of 3 months, 6 months, 1 year, 2 years, 3 years, 4 years, 5 years, 6 years, or more, wherein the hyperplasia is restenosis or pulmonary hypertension.

In some embodiments of any of the methods described herein, there is provided a method of prolonging survival of an individual having hyperplasia (such as cancer, restenosis, or pulmonary hypertension), comprising administering to the individual an effective amount of a composition comprising nanoparticles comprising an mTOR inhibitor (such as a limus drug) and an albumin, wherein the individual is selected based on the individual having an mTOR-activating aberration. In some embodiments, the method prolongs the survival of the individual by at least about any of 1, 2, 3, 4, 5, 6, 7, 8, 9, 10, 11, 12, 18, or 24 months, wherein the hyperplasia is cancer. In some embodiments, the method prolongs the survival of the individual by at least about any of 3 months, 6 months, 1 year, 2 years, 3 years, 4 years, 5 years, 6 years, or more, wherein the hyperplasia is restenosis or pulmonary hypertension.

In some embodiments of any of the methods described herein, there is provided a method of relieving one or more of the symptoms (including about any of 1, 2, 3, 4, 5, 6 or more) associated with hyperplasia (such as cancer, restenosis, or pulmonary hypertension), comprising administering to the individual an effective amount of a composition comprising nanoparticles comprising an mTOR inhibitor (such as a limus drug) and an albumin, wherein the individual is selected based on the individual having an mTOR-activating aberration. In some embodiments, the one or more of the symptoms associated with hyperplasia are relieved by at least about 10% (including for example at least about any of 20%, 30%, 40%, 60%, 70%, 80%, 90%, or 100%).

In some embodiments of any of the methods described herein, there is provided a method of improving the quality of life in an individual having hyperplasia (such as cancer, restenosis, or pulmonary hypertension), comprising administering to the individual an effective amount of a composition comprising nanoparticles comprising an mTOR inhibitor (such as a limus drug) and an albumin, wherein the individual is selected based on the individual having an mTOR-activating aberration.

In some embodiments of any of the methods described herein, there is provided a method of reducing AEs and SAEs in an individual having hyperplasia (such as cancer, restenosis, or pulmonary hypertension), comprising administering to the individual an effective amount of a composition comprising nanoparticles comprising an mTOR inhibitor (such as a limus drug) and an albumin, wherein the individual is selected based on the individual having an mTOR-activating aberration.

In some embodiments of any of the methods described herein, the method is predictive of and/or results in an objective response (such as a partial response or complete response).

In some embodiments of any of the methods described herein, the method is predictive of and/or results in improved quality of life.

"MTOR-activating aberration" refers to a genetic aberration, an aberrant expression level and/or an aberrant activity level of one or more mTOR-associated gene that may lead to hyperactivation of the mTOR signaling pathway. "Hyperactivate" refers to increase of an activity level of a molecule (such as a protein or protein complex) or a signaling pathway (such as the mTOR a signaling pathway) to a level that is above a reference activity level or range, such as at least about any of 10%, 20%, 30%, 40%, 60%, 70%, 80%, 90%, 100%, 200%, 500% or more above the reference activity level or the median of the reference activity range. In some embodiments, the reference activity level is a clinically accepted normal activity level in a standardized test, or an activity level in a healthy individual (or tissue or cell isolated from the individual) free of the mTOR-activating aberration.

The mTOR-activating aberration contemplated herein may include one type of aberration in one mTOR-associated gene, more than one type (such as at least about any of 2, 3, 4, 5, 6, or more) of aberrations in one mTOR-associated gene, one type of aberration in more than one (such as at least about any of 2, 3, 4, 5, 6, or more) mTOR-associated genes, or more than one type (such as at least about any of 2, 3, 4, 5, 6, or more) of aberration in more than one (such as at least about any of 2, 3, 4, 5, 6, or more) mTOR-associated genes. Different types of mTOR-activating aberration may include, but are not limited to, genetic aberrations, aberrant expression levels (*e.g*. overexpression or under-expression), aberrant activity levels (*e.g.* high or low activity levels), and aberrant protein phosphorylation levels. In some embodiments, a genetic aberration comprises a change to the nucleic acid (such as DNA or RNA) or protein sequence (i.e. mutation) or an aberrant epigenetic feature associated with an mTOR-associated gene, including, but not limited to, coding, non-coding, regulatory, enhancer, silencer, promoter, intron, exon, and untranslated regions of the mTOR-associated gene. In some embodiments, the mTOR-activating aberration comprises a mutation of an mTOR-associated gene, including, but not limited to, deletion, frameshift, insertion, indel, missense mutation, nonsense mutation, point mutation, silent mutation, splice site mutation, splice variant, and translocation. In some embodiments, the mutation may be a loss of function mutation for a negative regulator of the mTOR signaling pathway or a gain of function mutation of a positive regulator of the mTOR signaling pathway. In some embodiments, the genetic aberration comprises a copy number variation of an mTOR-associated gene. In some embodiments, the copy number variation of the mTOR-associated gene is caused by structural rearrangement of the genome, including deletions, duplications, inversion, and translocations. In some embodiments, the genetic aberration comprises an aberrant epigenetic feature of an mTOR-associated gene, including, but not limited to, DNA methylation, hydroxymethylation, increased or decreased histone binding, chromatin remodeling, and the like.

The mTOR-activating aberration is determined in comparison to a control or reference, such as a reference sequence (such as a nucleic acid sequence or a protein sequence), a control expression (such as RNA or protein expression) level, a control activity (such as activation or inhibition of downstream targets) level, or a control protein phosphorylation level. The aberrant expression level or the aberrant activity level in an mTOR-associated gene may be above the control level (such as about any of 10%, 20%, 30%, 40%, 60%, 70%, 80%, 90%, 100%, 200%, 500% or more above the control level) if the mTOR-associated gene is a positive regulator (i.e. activator) of the mTOR signaling pathway, or below the control level (such as about any of 10%, 20%, 30%, 40%, 60%, 70%, 80%, 90% or more below the control level) if the mTOR-associated gene is a negative regulator (i.e. inhibitor) of the mTOR signaling pathway. In some embodiments, the control level (*e.g*. expression level or activity level) is the median level (*e.g.* expression level or activity level) of a control population. In some embodiments, the control population is a population having the same hyperplasia (such as cancer, restenosis, or pulmonary hypertension) as the individual being treated. In some embodiments, the control population is a healthy population that does not have the hyperplasia (such as cancer, restenosis, or pulmonary hypertension), and optionally with comparable demographic characteristics (*e.g.* gender, age, ethnicity, etc.) as the individual being treated. In some embodiments, the control level (*e.g.* expression level or activity level) is a level (*e.g.* expression level or activity level) of a healthy tissue from the same individual. A genetic aberration may be determined by comparing to a reference sequence, including epigenetic patterns of the reference sequence in a control sample. In some embodiments, the reference sequence is the sequence (DNA, RNA or protein sequence) corresponding to a fully functional allele of an mTOR-associated gene, such as an allele (*e.g.* the prevalent allele) of the mTOR-associated gene present in a healthy population of individuals that do not have the hyperplasia (such as cancer, restenosis, or pulmonary hypertension), but may optionally have similar demographic characteristics (such as gender, age, ethnicity etc.) as the individual being treated. Exemplary mTOR-associated genes and their reference sequences (i.e. wildtype sequences) are described in the section "Biomarkers" below.

The "status" of an mTOR-activating aberration may refer to the presence or absence of the mTOR-activating aberration in one or more mTOR-associated genes, or the aberrant level (expression or activity level, including phosphorylation level of a protein) of one or more mTOR-associated genes. In some embodiments, the presence of a genetic aberration (such as a mutation or a copy number variation) in one or more mTOR-associated genes as compared to a control indicates that (a) the individual is more likely to respond to treatment or (b) the individual is selected for treatment. In some embodiments, the absence of a genetic aberration in an mTOR-associated gene, or a wild-type mTOR-associated gene compared to a control, indicates that (a) the individual is less likely to respond to treatment or (b) the individual is not selected for treatment. In some embodiments, an aberrant level (such as expression level or activity level, including phosphorylation level of a protein) of one or more mTOR-associated genes is correlated with the likelihood of the individual to respond to treatment. For example, a larger deviation of the level (*e.g.* expression or activity level, including phosphorylation level of a protein) of one or more mTOR-associated genes in the direction of hyper activating the mTOR signaling pathway indicates that the individual is more likely to respond to treatment. In some embodiments, a prediction model based on the level(s) (*e.g.* expression level or activity level, including phosphorylation level of a protein) of one or more mTOR-associated genes is used to predict (a) the likelihood of the individual to respond to treatment and (b) whether to select the individual for treatment. The prediction model, including, for example, coefficient for each level, may be obtained by statistical analysis, such as regression analysis, using clinical trial data.

The expression level, and/or activity level of the one or more mTOR-associated genes, and/or phosphorylation level of one or more proteins encoded by the one or more mTOR-associated genes, and/or the presence or absence of one or more genetic aberrations of the one or more mTOR-associated genes can be useful for determining any of the following: (a) probable or likely suitability of an individual to initially receive treatment(s); (b) probable or likely unsuitability of an individual to initially receive treatment(s); (c) responsiveness to treatment; (d) probable or likely suitability of an individual to continue to receive treatment(s); (e) probable or likely unsuitability of an individual to continue to receive treatment(s); (f) adjusting dosage; (g) predicting likelihood of clinical benefits.

In some embodiments, the mutational status, expression level, or activity level of one or more resistance biomarker (such as TFE3) is further used for selecting an individual for any of the methods of treatment described herein, and/or for determining any of the following: (a) probable or likely suitability of an individual to initially receive treatment(s); (b) probable or likely unsuitability of an individual to initially receive treatment(s); (c) responsiveness to treatment; (d) probable or likely suitability of an individual to continue to receive treatment(s); (e) probable or likely unsuitability of an individual to continue to receive treatment(s); (f) adjusting dosage; (g) predicting likelihood of clinical benefits. In some embodiments, the resistance biomarker is a gene selected from the ONCOPANEL^{™} test. See, for example, Wagle N. et al. Cancer discovery 2.1 (2012): 32-93.

In some embodiments according to any one of the methods of treatment described herein, the mutational status of TFE3 in an individual is used as a basis for selecting the individual. In some embodiments, the mutational status of TFE3 is used in combination with one or more mTOR activating aberration in an individual as a basis for selecting the individual for the treatment. In some embodiments, the mutational status of TFE3 comprises translocation of TFE3. In some embodiments, translocation of TFE3 is used to exclude an individual from the treatment. In some embodiments, translocation of TFE3 in a sample of the individual is assessed by fluorescence in situ hybridization (FISH). In some embodiments, the sample is a blood sample. In some embodiments, the sample is a tumor biopsy. In some embodiments, the sample is obtained prior to initiation of the treatment methods described herein. In some embodiments, the sample is obtained after initiation of the treatment methods described herein.

As used herein, "based upon" includes assessing, determining, or measuring the individual's characteristics as described herein (and preferably selecting an individual suitable for receiving treatment). When the status of an mTOR-activating aberration is "used as a basis" for selection, assessing, measuring, or determining method of treatment as described herein, the mTOR-activating aberration in one or more mTOR-associated genes is determined before and/or during treatment, and the status (including presence, absence, expression level, and/or activity level of the mTOR-activating aberration) obtained is used by a clinician in assessing any of the following: (a) probable or likely suitability of an individual to initially receive treatment(s); (b) probable or likely unsuitability of an individual to initially receive treatment(s); (c) responsiveness to treatment; (d) probable or likely suitability of an individual to continue to receive treatment(s); (e) probable or likely unsuitability of an individual to continue to receive treatment(s); (f) adjusting dosage; or (g) predicting likelihood of clinical benefits.

The methods described herein relate to administration of a composition comprising nanoparticles comprising an mTOR inhibitor (such as a limus drug) and an albumin (hereinafter also referred to as "mTOR inhibitor nanoparticle composition"). "mTOR inhibitor" used herein refers to an inhibitor of mTOR. mTOR is a serine/threonine-specific protein kinase downstream of the phosphatidylinositol 3-kinase (PI3K)/Akt (protein kinase B) pathway, and a key regulator of cell survival, proliferation, stress, and metabolism. mTOR pathway dysregulation has been found in many human carcinomas, and mTOR inhibition produced substantial inhibitory effects on tumor progression. In some embodiments, an mTOR inhibitor is an mTOR kinase inhibitor. mTOR inhibitors described herein include, but are not limited to, BEZ235 (NVP-BEZ235), everolimus (also known as RAD001, Zortress, Certican, and Afinitor), rapamycin (also known as sirolimus or Rapamune), AZD8055, temsirolimus (also known as CCI-779 and Torisel), PI-103, Ku-0063794, INK 128, AZD2014, NVP-BGT226, PF-04691502, CH5132799, GDC-0980 (RG7422), Torin 1, WAY-600, WYE-125132, WYE-687, GSK2126458, PF-05212384 (PKI-587), PP-121, OSI-027, Palomid 529, PP242, XL765, GSK1059615, WYE-354, eforolimus (also known as ridaforolimus or deforolimus), CC115, and CC-223.

In some embodiments, the mTOR inhibitor is a limus drug, which includes sirolimus and its analogues. Examples of limus drugs include, but are not limited to, temsirolimus (CCI-779), everolimus (RAD001), ridaforolimus (AP-23573), deforolimus (MK-8669), zotarolimus (ABT-578), pimecrolimus, and tacrolimus (FK-506). In some embodiments, the limus drug is selected from the group consisting of temsirolimus (CCI-779), everolimus (RAD001), ridaforolimus (AP-23573), deforolimus (MK-8669), zotarolimus (ABT-578), pimecrolimus, and tacrolimus (FK-506).

In some embodiments, the albumin is human serum albumin.

In some embodiments, the mTOR inhibitor (such as a limus drug) is associated (*e.g.,* coated) with the albumin.

In some embodiments, the composition comprising nanoparticles comprising the mTOR inhibitor (such as a limus drug) and the albumin is substantially free of surfactant.

In some embodiments, the composition comprising nanoparticles comprising an mTOR inhibitor and an albumin is *Nab*-sirolimus. "Nab" stands for nanoparticle albumin-bound, and "*Nab*-sirolimus" is an albumin stabilized nanoparticle formulation of sirolimus *Nab*-sirolimus is also known as *Nab*-rapamycin, which has been previously described, for example, see, WO2008109163A1, WO2014151853, WO2008137148A2, and WO2012149451A1.

In some embodiments, the treatment comprises administration of the composition comprising nanoparticles comprising the mTOR inhibitor (such as a limus drug) and the albumin over less than about 50 minutes, such as less than about 40 minutes, less than about 30 minutes, about 30 to about 40 minutes, or about 30 minutes. In some embodiments, the dose of the mTOR inhibitor (such as a limus drug, including sirolimus) in the mTOR inhibitor nanoparticle composition is about 10 mg/m² to about 150 mg/m² (including, for example, about 10 mg/m² to about 50 mg/m², about 50 mg/m² to about 75 mg/m², or about 75 mg/m² to about 150 mg/m²). In some embodiments, the dose of the mTOR inhibitor (such as a limus drug, including sirolimus) in the mTOR inhibitor nanoparticle composition is about 45 mg/m², about 56 mg/m², about 75 mg/m², or about 100 mg/m². In some embodiments, the treatment comprises administration of the composition comprising nanoparticles comprising the mTOR inhibitor (such as a limus drug) and the albumin parenterally. In some embodiments, the treatment comprises administration of the composition comprising nanoparticles comprising the mTOR inhibitor (such as a limus drug) and the albumin intravenously. In some embodiments, the treatment comprises administration of the composition comprising nanoparticles comprising the mTOR inhibitor (such as a limus drug) and the albumin weekly. In some embodiments, the treatment comprises administration of the composition comprising nanoparticles comprising the mTOR inhibitor (such as a limus drug) and the albumin weekly, three out of four weeks, or weekly, two out of three weeks. In some embodiments, the treatment comprises administration of the composition comprising nanoparticles comprising the mTOR inhibitor (such as a limus drug) and the albumin on days 1, 8, 15 of a 28 day cycle. In some embodiments, the treatment comprises administration of the composition comprising nanoparticles comprising the mTOR inhibitor (such as a limus drug) and the albumin on days 1 and 8 of a 21 day cycle. In some embodiments, the treatment comprises at least about 2 cycles (including at least about any of 3, 4, 5, 6, 7, 8, 9, 10 or more) of administration of the composition comprising nanoparticles comprising the mTOR inhibitor (such as a limus drug) and the albumin. In some embodiments of any of the methods, the treatment comprises administration of the composition comprising the mTOR inhibitor (such as a limus drug) and the albumin without any premedication (for example steroid premedication) and/or without G-CSF prophylaxis.

The mTOR-activating aberration in an individual can be assessed or determined by analyzing a sample from the individual. The assessment may be based on fresh tissue samples or archived tissue samples. Suitable samples include, but are not limited to, hyperplasia (such as cancer, including tumor stroma) tissue, normal tissue adjacent to the hyperplasia (such as cancer) tissue, normal tissue distal to the hyperplasia (such as cancer) tissue, or peripheral blood lymphocytes. In some embodiments, the sample is a hyperplasia (such as cancer) tissue. In some embodiments, the sample is a biopsy containing hyperplasia (such as cancer) cells, such as fine needle aspiration of hyperplasia (such as cancer) cells or laparoscopy obtained hyperplasia cells (such as cancer cells, including tumor stroma). In some embodiments, the biopsied cells are centrifuged into a pellet, fixed, and embedded in paraffin prior to the analysis. In some embodiments, the biopsied cells are flash frozen prior to the analysis. In some embodiments, the sample is a plasma sample. In some embodiments, the sample is a blood sample. In some embodiments, the sample is a tumor biopsy.

In some embodiments, the sample comprises a circulating metastatic cancer cell. In some embodiments, the sample is obtained by sorting circulating tumor cells (CTCs) from blood. In some further embodiments, the CTCs have detached from a primary tumor and circulate in a bodily fluid. In some further embodiments, the CTCs have detached from a primary tumor and circulate in the bloodstream. In some embodiments, the CTCs are an indication of metastasis.

In some embodiments, the sample is mixed with an antibody that recognizes a molecule encoded by an mTOR-associated gene (such as a protein) or fragment thereof. In some embodiments, the sample is mixed with a nucleic acid that recognizes nucleic acids associated with the mTOR-associated gene (such as DNA or RNA) or fragment thereof. In some embodiments, the sample is used for sequencing analysis, such as next-generation DNA, RNA and/or exome sequencing analysis.

The mTOR-activating aberration may be assessed before the start of the treatment, at any time during the treatment, and/or at the end of the treatment. In some embodiments, the mTOR-activating aberration is assessed from about 3 days prior to the administration of the mTOR inhibitor nanoparticle composition to about 3 days after the administration of the mTOR inhibitor nanoparticle composition in each cycle of the administration. In some embodiments, the mTOR-activating aberration is assessed on day 1 of each cycle of administration. In some embodiments, the mTOR-activating aberration is assessed in each cycle of administration. In some embodiments, the mTOR-activating aberration is further assessed each 2 cycles after the first 3 cycles of administration.

In some embodiments, the hyperplasia is a cancer. Examples of cancers that may be treated by the methods described herein include, but are not limited to, adenocortical carcinoma, agnogenic myeloid metaplasia, anal cancer, appendix cancer, astrocytoma (*e.g.,* cerebellar and cerebral), basal cell carcinoma, bile duct cancer (*e.g.,* extrahepatic), bladder cancer, bone cancer, (osteosarcoma and malignant fibrous histiocytoma), brain tumor (*e.g.,* glioma, brain stem glioma, cerebellar or cerebral astrocytoma (*e.g.,* pilocytic astrocytoma, diffuse astrocytoma, anaplastic (malignant) astrocytoma), malignant glioma, ependymoma, oligodenglioma, meningioma, craniopharyngioma, haemangioblastonizis, medulloblastoma, supratentorial primitive neuroectodermal tumors, visual pathway and hypothalamic glioma, and glioblastoma), breast cancer, bronchial adenomas/carcinoids, carcinoid tumor (*e.g.,* gastrointestinal carcinoid tumor), carcinoma of unknown primary, central nervous system lymphoma, cervical cancer, colon cancer, colorectal cancer, chronic myeloproliferative disorders, endometrial cancer (*e.g.,* uterine cancer), ependymoma, esophageal cancer, Ewing's family of tumors, eye cancer (*e.g.,* intraocular melanoma and retinoblastoma), gallbladder cancer, gastric (stomach) cancer, gastrointestinal carcinoid tumor, gastrointestinal stromal tumor (GIST), germ cell tumor, (*e.g.,* extracranial, extragonadal, ovarian), gestational trophoblastic tumor, head and neck cancer, hepatocellular (liver) cancer (*e.g.,* hepatic carcinoma and heptoma), hypopharyngeal cancer, islet cell carcinoma (endocrine pancreas), laryngeal cancer, laryngeal cancer, leukemia (except for T-cell leukemia), lip and oral cavity cancer, oral cancer, liver cancer, lung cancer (*e.g*., small cell lung cancer, non-small cell lung cancer, adenocarcinoma of the lung, and squamous carcinoma of the lung), lymphoma (except for T-cell lymphoma), medulloblastoma, melanoma, mesothelioma, metastatic squamous neck cancer, mouth cancer, multiple endocrine neoplasia syndrome, myelodysplastic syndromes, myelodysplastic/myeloproliferative diseases, nasal cavity and paranasal sinus cancer, nasopharyngeal carcinoma, neuroblastoma, neuroendocrine cancer, oropharyngeal cancer, ovarian cancer (*e.g.,* ovarian epithelial cancer, ovarian germ cell tumor, ovarian low malignant potential tumor), pancreatic cancer, parathyroid cancer, penile cancer, cancer of the peritoneal, pharyngeal cancer, pheochromocytoma, pineoblastoma and supratentorial primitive neuroectodermal tumors, pituitary tumor, pleuropulmonary blastoma, primary central nervous system lymphoma (microglioma), pulmonary lymphangiomyomatosis, rectal cancer, renal carcinoma, renal pelvis and ureter cancer (transitional cell cancer), rhabdomyosarcoma, salivary gland cancer, skin cancer (*e.g.,* non-melanoma (*e.g.,* squamous cell carcinoma), melanoma, and Merkel cell carcinoma), small intestine cancer, squamous cell cancer, testicular cancer, throat cancer, thyroid cancer, tuberous sclerosis, urethral cancer, vaginal cancer, vulvar cancer, Wilms' tumor, abnormal vascular proliferation associated with phakomatoses, edema (such as that associated with brain tumors), and Meigs' syndrome.

Thus, in some embodiments, there is provided a method of treating cancer in an individual comprising administering to the individual an effective amount of a composition comprising nanoparticles comprising an mTOR inhibitor (such as a limus drug) and an albumin, wherein the individual is selected for treatment on the basis of having an mTOR-activating aberration. In some embodiments, there is provided a method of treating cancer in an individual comprising: (a) assessing an mTOR-activating aberration in the individual; and (b) administering (for example intravenously) to the individual an effective amount of a composition comprising nanoparticles comprising an mTOR inhibitor (such as a limus drug) and an albumin, wherein the individual is selected for treatment based on having the mTOR-activating aberration. In some embodiments, there is provided a method of selecting an individual having a cancer for treatment with a composition comprising nanoparticles comprising an mTOR inhibitor (such as a limus drug) and an albumin, wherein the method comprises (a) assessing an mTOR-activating aberration in the individual; and (b) selecting or recommending the individual for treatment based on the individual having the mTOR-activating aberration. In some embodiments, there is provided a method of selecting an individual having a cancer for treatment with a composition comprising nanoparticles comprising an mTOR inhibitor (such as a limus drug) and an albumin, wherein the method comprises (a) assessing an mTOR-activating aberration in the individual; (b) selecting or recommending the individual for treatment based on the individual having the mTOR-activating aberration; and (c) administering an effective amount of the composition comprising the mTOR inhibitor (such as a limus drug) and the albumin to the selected individual. In some embodiments, there is provided a method of treating a cancer (such as an mTOR-inhibitor-sensitive cancer) in an individual comprising administering to the individual an effective amount of a composition comprising nanoparticles comprising an mTOR inhibitor (such as a limus drug) and an albumin, wherein the individual has an mTOR-activating aberration. In some embodiments, the composition comprising nanoparticles comprises a limus drug and an albumin, wherein the limus drug in the nanoparticles is associated (*e.g.,* coated) with the albumin. In some embodiments, the composition comprising nanoparticles comprises a limus drug and an albumin, wherein the nanoparticles have an average particle size of no greater than about 150 nm (such as no greater than about 120 nm). In some embodiments, the composition comprising nanoparticles comprises sirolimus and human serum albumin, wherein the nanoparticles comprise sirolimus associated (*e.g.,* coated) with human serum albumin, wherein the nanoparticles have an average particle size of no greater than about 150 nm (such as no greater than about 120 nm, for example about 100 nm), and wherein the weight ratio of human albumin and sirolimus in the composition is about 9:1 or less (such as about 9:1 or about 8:1). In some embodiments, the composition comprising nanoparticles comprises *Nab*-sirolimus. In some embodiments, the mTOR-activating aberration comprises a mutation of an mTOR-associated gene. In some embodiments, the mTOR-activating aberration comprises a copy number variation of an mTOR-associated gene. In some embodiments, the mTOR-activating aberration comprises an aberrant expression level of an mTOR-associated gene. In some embodiments, the mTOR-activating aberration comprises an aberrant activity level of an mTOR-associated gene. In some embodiments, the mTOR-activating aberration leads to activation of mTORCI1 (including for example activation of mTORC1 but not mTORC2). In some embodiments, the mTOR-activating aberration leads to activation of mMTORC2 (including for example activation of mMTORC2 but not mTORC1). In some embodiments, the mTOR-activating aberration leads to activation of both mTORC1 and mTORC2. In some embodiments, the mTOR-activating aberration is an aberration in at least one mTOR-associated gene selected from the group consisting of AKT1, FLT3, MTOR, PIK3CA, PIK3CG, TSC1, TSC2, RHEB, STK11, NF1, NF2, PTEN, TP53, FGFR4, KRAS, NRAS, and BAP1. In some embodiments, the mTOR-activating aberration is assessed by gene sequencing. In some embodiments, the gene sequencing is based on sequencing of DNA in a tumor sample. In some embodiments, the gene sequencing is based on sequencing of circulating DNA or cell-free DNA isolated from a blood sample. In some embodiments, the mutational status of TFE3 is further used as a basis for selecting the individual. In some embodiments, the mutational status of TFE3 comprises translocation of TFE3. In some embodiments, the mTOR-activating aberration comprises an aberrant phosphorylation level of the protein encoded by the mTOR-associated gene. In some embodiments, the mTOR-activating aberration comprises an aberrant phosphorylation level of a protein encoded by an mTOR-associated gene selected from the group consisting of AKT, S6K, S6, 4EBP1, and SPARC. In some embodiments, the aberrant phosphorylation level is determined by immunohistochemistry.

In some embodiments, the cancer is selected from the group consisting of pancreatic neuroendocrine cancer, endometrial cancer, ovarian cancer, breast cancer, renal cell carcinoma, lymphangioleiomyomatosis (LAM), prostate cancer, lymphoma, and bladder cancer. The methods are applicable to cancers of all stages, including stages, I, II, III, and IV, according to the American Joint Committee on Cancer (AJCC) staging groups. In some embodiments, the cancer is an/a: early stage cancer, non-metastatic cancer, primary cancer, advanced cancer, locally advanced cancer, metastatic cancer, cancer in remission, cancer in an adjuvant setting, or cancer in a neoadjuvant setting. In some embodiments, the cancer is solid tumor. In some embodiments, the solid tumor is localized resectable, localized unresectable, or unresectable. In some embodiments, the solid tumor is localized resectable or borderline resectable. In some embodiments, the cancer has been refractory to prior therapy. In some embodiments, the cancer is resistant to the treatment with a non-nanoparticle formulation of a chemotherapeutic agent (such as non-nanoparticle formulation of a limus drug). In some embodiments, the cancer is liquid cancer.

In some embodiments, there is provided a method of treating pancreatic neuroendocrine cancer in an individual comprising administering to the individual an effective amount of a composition comprising nanoparticles comprising an mTOR inhibitor (such as a limus drug) and an albumin, wherein the individual is selected for treatment on the basis of having an mTOR-activating aberration. In some embodiments, there is provided a method of treating pancreatic neuroendocrine cancer in an individual comprising: (a) assessing an mTOR-activating aberration in the individual; and (b) administering (for example intravenously) to the individual an effective amount of a composition comprising nanoparticles comprising an mTOR inhibitor (such as a limus drug) and an albumin, wherein the individual is selected for treatment based on having the mTOR-activating aberration. In some embodiments, there is provided a method of selecting an individual having a pancreatic neuroendocrine cancer for treatment with a composition comprising nanoparticles comprising an mTOR inhibitor (such as a limus drug) and an albumin, wherein the method comprises (a) assessing an mTOR-activating aberration in the individual; and (b) selecting or recommending the individual for treatment based on the individual having the mTOR-activating aberration. In some embodiments, there is provided a method of selecting an individual having a pancreatic neuroendocrine cancer for treatment with a composition comprising nanoparticles comprising an mTOR inhibitor (such as a limus drug) and an albumin, wherein the method comprises (a) assessing an mTOR-activating aberration in the individual; (b) selecting or recommending the individual for treatment based on the individual having the mTOR-activating aberration; and (c) administering an effective amount of the composition comprising the mTOR inhibitor (such as a limus drug) and the albumin to the selected individual. In some embodiments, there is provided a method of treating a pancreatic neuroendocrine cancer (such as an mTOR-inhibitor-sensitive pancreatic neuroendocrine cancer) in an individual comprising administering to the individual an effective amount of a composition comprising nanoparticles comprising an mTOR inhibitor (such as a limus drug) and an albumin, wherein the individual has an mTOR-activating aberration. In some embodiments, the composition comprising nanoparticles comprises a limus drug and an albumin, wherein the limus drug in the nanoparticles is associated (*e.g.,* coated) with the albumin. In some embodiments, the composition comprising nanoparticles comprises a limus drug and an albumin, wherein the nanoparticles have an average particle size of no greater than about 150 nm (such as no greater than about 120 nm). In some embodiments, the composition comprising nanoparticles comprises sirolimus and human serum albumin, wherein the nanoparticles comprise sirolimus associated (*e.g.,* coated) with human serum albumin, wherein the nanoparticles have an average particle size of no greater than about 150 nm (such as no greater than about 120 nm, for example about 100 nm), and wherein the weight ratio of human albumin and sirolimus in the composition is about 9: 1 or less (such as about 9:1 or about 8:1). In some embodiments, the composition comprising nanoparticles comprises *Nab*-sirolimus. In some embodiments, the mTOR-activating aberration comprises a mutation of an mTOR-associated gene. In some embodiments, the mTOR-activating aberration comprises a copy number variation of an mTOR-associated gene. In some embodiments, the mTOR-activating aberration comprises an aberrant expression level of an mTOR-associated gene. In some embodiments, the mTOR-activating aberration comprises an aberrant activity level of an mTOR-associated gene. In some embodiments, the mTOR-activating aberration leads to activation of mTORC1 (including for example activation of mTORC1 but not mTORC2). In some embodiments, the mTOR-activating aberration leads to activation of mTORC2 (including for example activation of mTORC2 but not mTORC1). In some embodiments, the mTOR-activating aberration leads to activation of both mTORC1 and mTORC2. In some embodiments, the mTOR-activating aberration is an aberration in at least one mTOR-associated gene selected from the group consisting of AKT1, FLT3, MTOR, PIK3CA, PIK3CG, TSC1, TSC2, RHEB, STK11, NF1, NF2, PTEN, TP53, FGFR4, KRAS, NRAS, and BAP1. In some embodiments, the mTOR-activating aberration is assessed by gene sequencing. In some embodiments, the gene sequencing is based on sequencing of DNA in a tumor sample. In some embodiments, the gene sequencing is based on sequencing of circulating DNA or cell-free DNA isolated from a blood sample. In some embodiments, the mutational status of TFE3 is further used as a basis for selecting the individual. In some embodiments, the mutational status of TFE3 comprises translocation of TFE3. In some embodiments, the mTOR-activating aberration comprises an aberrant phosphorylation level of the protein encoded by the mTOR-associated gene. In some embodiments, the mTOR-activating aberration comprises an aberrant phosphorylation level of a protein encoded by an mTOR-associated gene selected from the group consisting of AKT, S6K, S6, 4EBP1, and SPARC. In some embodiments, the aberrant phosphorylation level is determined by immunohistochemistry. In some embodiments, the pancreatic neuroendocrine cancer is a functional or a nonfunctional pancreatic neuroendocrine tumor. In some embodiments, the pancreatic neuroendocrine cancer is insulinoma, glucagonoma, somatostatinoma, gastrinoma, VIPoma, GRFoma, or ACTHoma.

In some embodiments, there is provided a method of treating an endometrial cancer in an individual comprising administering to the individual an effective amount of a composition comprising nanoparticles comprising an mTOR inhibitor (such as a limus drug) and an albumin, wherein the individual is selected for treatment on the basis of having an mTOR-activating aberration. In some embodiments, there is provided a method of treating an endometrial cancer in an individual comprising: (a) assessing an mTOR-activating aberration in the individual; and (b) administering (for example intravenously) to the individual an effective amount of a composition comprising nanoparticles comprising an mTOR inhibitor (such as a limus drug) and an albumin, wherein the individual is selected for treatment based on having the mTOR-activating aberration. In some embodiments, there is provided a method of selecting an individual having an endometrial cancer for treatment with a composition comprising nanoparticles comprising an mTOR inhibitor (such as a limus drug) and an albumin, wherein the method comprises (a) assessing an mTOR-activating aberration in the individual; and (b) selecting or recommending the individual for treatment based on the individual having the mTOR-activating aberration. In some embodiments, there is provided a method of selecting an individual having an endometrial cancer for treatment with a composition comprising nanoparticles comprising an mTOR inhibitor (such as a limus drug) and an albumin, wherein the method comprises (a) assessing an mTOR-activating aberration in the individual; (b) selecting or recommending the individual for treatment based on the individual having the mTOR-activating aberration; and (c) administering an effective amount of the composition comprising the mTOR inhibitor (such as a limus drug) and the albumin to the selected individual. In some embodiments, there is provided a method of treating an endometiral cancer (such as an mTOR-inhibitor-sensitive endometrial cancer) in an individual comprising administering to the individual an effective amount of a composition comprising nanoparticles comprising an mTOR inhibitor (such as a limus drug) and an albumin, wherein the individual has an mTOR-activating aberration. In some embodiments, the composition comprising nanoparticles comprises a limus drug and an albumin, wherein the limus drug in the nanoparticles is associated (*e.g.,* coated) with the albumin. In some embodiments, the composition comprising nanoparticles comprises a limus drug and an albumin, wherein the nanoparticles have an average particle size of no greater than about 150 nm (such as no greater than about 120 nm). In some embodiments, the composition comprising nanoparticles comprises sirolimus and human serum albumin, wherein the nanoparticles comprise sirolimus associated (*e.g.,* coated) with human serum albumin, wherein the nanoparticles have an average particle size of no greater than about 150 nm (such as no greater than about 120 nm, for example about 100 nm), and wherein the weight ratio of human albumin and sirolimus in the composition is about 9:1 or less (such as about 9:1 or about 8:1). In some embodiments, the composition comprising nanoparticles comprises *Nab*-sirolimus. In some embodiments, the mTOR-activating aberration comprises a mutation of an mTOR-associated gene. In some embodiments, the mTOR-activating aberration comprises a copy number variation of an mTOR-associated gene. In some embodiments, the mTOR-activating aberration comprises an aberrant expression level of an mTOR-associated gene. In some embodiments, the mTOR-activating aberration comprises an aberrant activity level of an mTOR-associated gene. In some embodiments, the mTOR-activating aberration leads to activation of mTORC1 (including for example activation of mTORC1 but not mTORC2). In some embodiments, the mTOR-activating aberration leads to activation of mTORC2 (including for example activation of mTORC2 but not mTORC1). In some embodiments, the mTOR-activating aberration leads to activation of both mTORC1 and mTORC2. In some embodiments, the mTOR-activating aberration is an aberration in at least one mTOR-associated gene selected from the group consisting of AKT1, FLT3, MTOR, PIK3CA, PIK3CG, TSC1, TSC2, RHEB, STK11, NF1, NF2, PTEN, TP53, FGFR4, KRAS, NRAS, and BAP1. In some embodiments, the mTOR-activating aberration is assessed by gene sequencing. In some embodiments, the gene sequencing is based on sequencing of DNA in a tumor sample. In some embodiments, the gene sequencing is based on sequencing of circulating DNA or cell-free DNA isolated from a blood sample. In some embodiments, the mutational status of TFE3 is further used as a basis for selecting the individual. In some embodiments, the mutational status of TFE3 comprises translocation of TFE3. In some embodiments, the mTOR-activating aberration comprises an aberrant phosphorylation level of the protein encoded by the mTOR-associated gene. In some embodiments, the mTOR-activating aberration comprises an aberrant phosphorylation level of a protein encoded by an mTOR-associated gene selected from the group consisting of AKT, S6K, S6, 4EBP1, and SPARC. In some embodiments, the aberrant phosphorylation level is determined by immunohistochemistry.

In some embodiments, there is provided a method of treating a breast cancer in an individual comprising administering to the individual an effective amount of a composition comprising nanoparticles comprising an mTOR inhibitor (such as a limus drug) and an albumin, wherein the individual is selected for treatment on the basis of having an mTOR-activating aberration. In some embodiments, there is provided a method of treating a breast cancer in an individual comprising: (a) assessing an mTOR-activating aberration in the individual; and (b) administering (for example intravenously) to the individual an effective amount of a composition comprising nanoparticles comprising an mTOR inhibitor (such as a limus drug) and an albumin, wherein the individual is selected for treatment based on having the mTOR-activating aberration. In some embodiments, there is provided a method of selecting an individual having a breast cancer for treatment with a composition comprising nanoparticles comprising an mTOR inhibitor (such as a limus drug) and an albumin, wherein the method comprises (a) assessing an mTOR-activating aberration in the individual; and (b) selecting or recommending the individual for treatment based on the individual having the mTOR-activating aberration. In some embodiments, there is provided a method of selecting an individual having a breast cancer for treatment with a composition comprising nanoparticles comprising an mTOR inhibitor (such as a limus drug) and an albumin, wherein the method comprises (a) assessing an mTOR-activating aberration in the individual; (b) selecting or recommending the individual for treatment based on the individual having the mTOR-activating aberration; and (c) administering an effective amount of the composition comprising the mTOR inhibitor (such as a limus drug) and the albumin to the selected individual. In some embodiments, there is provided a method of treating a breast cancer (such as an mTOR-inhibitor-sensitive breast cancer) in an individual comprising administering to the individual an effective amount of a composition comprising nanoparticles comprising an mTOR inhibitor (such as a limus drug) and an albumin, wherein the individual has an mTOR-activating aberration. In some embodiments, the composition comprising nanoparticles comprises a limus drug and an albumin, wherein the limus drug in the nanoparticles is associated (*e.g.,* coated) with the albumin. In some embodiments, the composition comprising nanoparticles comprises a limus drug and an albumin, wherein the nanoparticles have an average particle size of no greater than about 150 nm (such as no greater than about 120 nm). In some embodiments, the composition comprising nanoparticles comprises sirolimus and human serum albumin, wherein the nanoparticles comprise sirolimus associated (*e.g.,* coated) with human serum albumin, wherein the nanoparticles have an average particle size of no greater than about 150 nm (such as no greater than about 120 nm, for example about 100 nm), and wherein the weight ratio of human albumin and sirolimus in the composition is about 9:1 or less (such as about 9: 1 or about 8:1). In some embodiments, the composition comprising nanoparticles comprises *Nab-*sirolimus. In some embodiments, the mTOR-activating aberration comprises a mutation of an mTOR-associated gene. In some embodiments, the mTOR-activating aberration comprises a copy number variation of an mTOR-associated gene. In some embodiments, the mTOR-activating aberration comprises an aberrant expression level of an mTOR-associated gene. In some embodiments, the mTOR-activating aberration comprises an aberrant activity level of an mTOR-associated gene. In some embodiments, the mTOR-activating aberration leads to activation of mTORC1 (including for example activation of mTORC1 but not mTORC2). In some embodiments, the mTOR-activating aberration leads to activation of mTORC2 (including for example activation of mTORC2 but not mTORC1). In some embodiments, the mTOR-activating aberration leads to activation of both mTORC1 and mTORC2, In some embodiments, the mTOR-activating aberration is an aberration in at least one mTOR-associated gene selected from the group consisting of AKT1, FLT3, MTOR, PIK3CA, PIK3CG, TSC1, TSC2, RHEB, STKl 1, NF1, NF2, PTEN, TP53, FGFR4, KRAS, NRAS, and BAP1. In some embodiments, the mTOR-activating aberration is assessed by gene sequencing. In some embodiments, the gene sequencing is based on sequencing of DNA in a tumor sample. In some embodiments, the gene sequencing is based on sequencing of circulating DNA or cell-free DNA isolated from a blood sample. In some embodiments, the mutational status of TFE3 is further used as a basis for selecting the individual. In some embodiments, the mutational status of TFE3 comprises translocation of TFE3. In some embodiments, the mTOR-activating aberration comprises an aberrant phosphorylation level of the protein encoded by the mTOR-associated gene. In some embodiments, the mTOR-activating aberration comprises an aberrant phosphorylation level of a protein encoded by an mTOR-associated gene selected from the group consisting of AKT, S6K, S6, 4EBP1, and SPARC. In some embodiments, the aberrant phosphorylation level is determined by immunohistochemistry.

In some embodiments, the breast cancer is early stage breast cancer, non-metastatic breast cancer, locally advanced breast cancer, metastatic breast cancer, hormone receptor positive metastatic breast cancer, breast cancer in remission, breast cancer in an adjuvant setting, ductal carcinoma in situ (DCIS), invasive ductal carcinoma (IDC), or breast cancer in a neoadjuvant setting. In some embodiments, the breast cancer is hormone receptor positive metastatic breast cancer. In some embodiments, the breast cancer is ductal carcinoma in situ. In some embodiments, the individual may be a human who has a gene, genetic mutation, or polymorphism associated with breast cancer (*e.g.,* BRCA1, BRCA2, ATM, CHEK2, RAD51, AR, DIRAS3, ERBB2, TP53, AKT, PTEN, and/or PI3K) or has one or more extra copies of a gene (*e.g.,* one or more extra copies of the HER2 gene) associated with breast cancer. In some embodiments, the breast cancer is negative for at least one of estrogen receptor ("ER"), progesterone receptor ("PR") or human epidermal growth factor receptor 2 ("HER2"). In some embodiments, the breast cancer is ER-negative, PR-negative and HER2-negative. In some embodiments, the breast cancer is positive for ER, PR and/or HER2. In some embodiments, the breast cancer is ER-positive.

In some embodiments, there is provided a method of treating a renal cell carcinoma in an individual comprising administering to the individual an effective amount of a composition comprising nanoparticles comprising an mTOR inhibitor (such as a limus drug) and an albumin, wherein the individual is selected for treatment on the basis of having an mTOR-activating aberration. In some embodiments, there is provided a method of treating a renal cell carcinoma in an individual comprising: (a) assessing an mTOR-activating aberration in the individual; and (b) administering (for example intravenously) to the individual an effective amount of a composition comprising nanoparticles comprising an mTOR inhibitor (such as a limus drug) and an albumin, wherein the individual is selected for treatment based on having the mTOR-activating aberration. In some embodiments, there is provided a method of selecting an individual having a renal cell carcinoma for treatment with a composition comprising nanoparticles comprising an mTOR inhibitor (such as a limus drug) and an albumin, wherein the method comprises (a) assessing an mTOR-activating aberration in the individual; and (b) selecting or recommending the individual for treatment based on the individual having the mTOR-activating aberration. In some embodiments, there is provided a method of selecting an individual having a renal cell carcinoma for treatment with a composition comprising nanoparticles comprising an mTOR inhibitor (such as a limus drug) and an albumin, wherein the method comprises (a) assessing an mTOR-activating aberration in the individual; (b) selecting or recommending the individual for treatment based on the individual having the mTOR-activating aberration; and (c) administering an effective amount of the composition comprising the mTOR inhibitor (such as a limus drug) and the albumin to the selected individual. In some embodiments, there is provided a method of treating a renal cell carcinoma (such as an mTOR-inhibitor-sensitive renal cell carcinoma) in an individual comprising administering to the individual an effective amount of a composition comprising nanoparticles comprising an mTOR inhibitor (such as a limus drug) and an albumin, wherein the individual has an mTOR-activating aberration. In some embodiments, the composition comprising nanoparticles comprises a limus drug and an albumin, wherein the limus drug in the nanoparticles is associated (*e.g.,* coated) with the albumin. In some embodiments, the composition comprising nanoparticles comprises a limus drug and an albumin, wherein the nanoparticles have an average particle size of no greater than about 150 nm (such as no greater than about 120 nm). In some embodiments, the composition comprising nanoparticles comprises sirolimus and human serum albumin, wherein the nanoparticles comprise sirolimus associated (*e.g.,* coated) with human serum albumin, wherein the nanoparticles have an average particle size of no greater than about 150 nm (such as no greater than about 120 nm, for example about 100 nm), and wherein the weight ratio of human albumin and sirolimus in the composition is about 9:1 or less (such as about 9:1 or about 8:1). In some embodiments, the composition comprising nanoparticles comprises *Nab*-sirolimus. In some embodiments, the mTOR-activating aberration comprises a mutation of an mTOR-associated gene. In some embodiments, the mTOR-activating aberration comprises a copy number variation of an mTOR-associated gene. In some embodiments, the mTOR-activating aberration comprises an aberrant expression level of an mTOR-associated gene. In some embodiments, the mTOR-activating aberration comprises an aberrant activity level of an mTOR-associated gene. In some embodiments, the mTOR-activating aberration leads to activation of mTORC1 (including for example activation of mTORC1 but not mTORC2). In some embodiments, the mTOR-activating aberration leads to activation of mTORC2 (including for example activation of mTORC2 but not mTORC1). In some embodiments, the mTOR-activating aberration leads to activation of both mTORC1 and mTORC2. In some embodiments, the mTOR-activating aberration is an aberration in at least one mTOR-associated gene selected from the group consisting of AKT1, FLT3, MTOR, PIK3CA, PIK3CG, TSC1, TSC2, RHEB, STK11, NF1, NF2, PTEN, TP53, FGFR4, KRAS, NRAS, and BAP1. In some embodiments, the mTOR-activating aberration is assessed by gene sequencing. In some embodiments, the gene sequencing is based on sequencing of DNA in a tumor sample. In some embodiments, the gene sequencing is based on sequencing of circulating DNA or cell-free DNA isolated from a blood sample. In some embodiments, the mutational status of TFE3 is further used as a basis for selecting the individual. In some embodiments, the mutational status of TFE3 comprises translocation of TFE3. In some embodiments, the mTOR-activating aberration comprises an aberrant phosphorylation level of the protein encoded by the mTOR-associated gene. In some embodiments, the mTOR-activating aberration comprises an aberrant phosphorylation level of a protein encoded by an mTOR-associated gene selected from the group consisting of AKT, S6K, S6, 4EBP1, and SPARC. In some embodiments, the aberrant phosphorylation level is determined by immunohistochemistry.

In some embodiments, the renal cell carcinoma is an adenocarcinoma. In some embodiments, the renal cell carcinoma is a clear cell renal cell carcinoma, papillary renal cell carcinoma (also called chromophilic renal cell carcinoma), chromophobe renal cell carcinoma, collecting duct renal cell carcinoma, granular renal cell carcinoma, mixed granular renal cell carcinoma, and spindle renal cell carcinoma. In some embodiments, the renal cell carcinoma is associated with (1) von Hippel-Lindau (VHL) syndrome, (2) hereditary papillary renal carcinoma (HPRC), (3) familial renal oncocytoma (FRO) associated with Birt-Hogg-Dube syndrome (BHDS), or (4) hereditary renal carcinoma (HRC).

In some embodiments, there is provided a method of treating a lymphangioleiomyomatosis (LAM) in an individual comprising administering to the individual an effective amount of a composition comprising nanoparticles comprising an mTOR inhibitor (such as a limus drug) and an albumin, wherein the individual is selected for treatment on the basis of having an mTOR-activating aberration. In some embodiments, there is provided a method of treating a lymphangioleiomyomatosis in an individual comprising: (a) assessing an mTOR-activating aberration in the individual; and (b) administering (for example intravenously) to the individual an effective amount of a composition comprising nanoparticles comprising an mTOR inhibitor (such as a limus drug) and an albumin, wherein the individual is selected for treatment based on having the mTOR-activating aberration. In some embodiments, there is provided a method of selecting an individual having a lymphangioleiomyomatosis for treatment with a composition comprising nanoparticles comprising an mTOR inhibitor (such as a limus drug) and an albumin, wherein the method comprises (a) assessing an mTOR-activating aberration in the individual; and (b) selecting or recommending the individual for treatment based on the individual having the mTOR-activating aberration. In some embodiments, there is provided a method of selecting an individual having a lymphangioleiomyomatosis for treatment with a composition comprising nanoparticles comprising an mTOR inhibitor (such as a limus drug) and an albumin, wherein the method comprises (a) assessing an mTOR-activating aberration in the individual; (b) selecting or recommending the individual for treatment based on the individual having the mTOR-activating aberration; and (c) administering an effective amount of the composition comprising the mTOR inhibitor (such as a limus drug) and the albumin to the selected individual. In some embodiments, there is provided a method of treating a LAM (such as an mTOR-inhibitor-sensitive LAM) in an individual comprising administering to the individual an effective amount of a composition comprising nanoparticles comprising an mTOR inhibitor (such as a limus drug) and an albumin, wherein the individual has an mTOR-activating aberration. In some embodiments, the composition comprising nanoparticles comprises a limus drug and an albumin, wherein the limus drug in the nanoparticles is associated (e.g., coated) with the albumin. In some embodiments, the composition comprising nanoparticles comprises a limus drug and an albumin, wherein the nanoparticles have an average particle size of no greater than about 150 nm (such as no greater than about 120 nm). In some embodiments, the composition comprising nanoparticles comprises sirolimus and human serum albumin, wherein the nanoparticles comprise sirolimus associated (*e.g.,* coated) with human serum albumin, wherein the nanoparticles have an average particle size of no greater than about 150 nm (such as no greater than about 120 nm, for example about 100 nm), and wherein the weight ratio of human albumin and sirolimus in the composition is about 9: 1 or less (such as about 9: 1 or about 8:1). In some embodiments, the composition comprising nanoparticles comprises *Nab*-sirolimus. In some embodiments, the mTOR-activating aberration comprises a mutation of an mTOR-associated gene. In some embodiments, the mTOR-activating aberration comprises a copy number variation of an mTOR-associated gene. In some embodiments, the mTOR-activating aberration comprises an aberrant expression level of an mTOR-associated gene. In some embodiments, the mTOR-activating aberration comprises an aberrant activity level of an mTOR-associated gene. In some embodiments, the mTOR-activating aberration leads to activation of mTORC1 (including for example activation of mTORC1 but not mTORC2). In some embodiments, the mTOR-activating aberration leads to activation of mTORC2 (including for example activation of mTORC2 but not mTORC1). In some embodiments, the mTOR-activating aberration leads to activation of both mTORC1 and mTORC2. In some embodiments, the mTOR-activating aberration is an aberration in at least one mTOR-associated gene selected from the group consisting of AKT1, FLT3, MTOR, PIK3CA, PIK3CG, TSC1, TSC2, RHEB, STK11, NF1, NF2, PTEN, TP53, FGFR4, KRAS, NRAS, and BAP1. In some embodiments, the mTOR-activating aberration is assessed by gene sequencing. In some embodiments, the gene sequencing is based on sequencing of DNA in a tumor sample. In some embodiments, the gene sequencing is based on sequencing of circulating DNA or cell-free DNA isolated from a blood sample. In some embodiments, the mutational status of TFE3 is further used as a basis for selecting the individual. In some embodiments, the mutational status of TFE3 comprises translocation of TFE3. In some embodiments, the mTOR-activating aberration comprises an aberrant phosphorylation level of the protein encoded by the mTOR-associated gene. In some embodiments, the mTOR-activating aberration comprises an aberrant phosphorylation level of a protein encoded by an mTOR-associated gene selected from the group consisting of AKT, S6K, S6, 4EBP1, and SPARC. In some embodiments, the aberrant phosphorylation level is determined by immunohistochemistry.

In some embodiments, the lymphangioleiomyomatosis is inherited. In some embodiments, the lymphangioleiomyomatosis is a feature of tuberous sclerosis complex. In some embodiments, the lymphangioleiomyomatosis is isolated or sporadic. In some embodiments, the lymphangioleiomyomatosis develops cysts in the lung, lymphatic vessels, and/or kidneys.

In some embodiments, there is provided a method of treating a prostate cancer in an individual comprising administering to the individual an effective amount of a composition comprising nanoparticles comprising an mTOR inhibitor (such as a limus drug) and an albumin, wherein the individual is selected for treatment on the basis of having an mTOR-activating aberration. In some embodiments, there is provided a method of treating a prostate cancer in an individual comprising: (a) assessing an mTOR-activating aberration in the individual; and (b) administering (for example intravenously) to the individual an effective amount of a composition comprising nanoparticles comprising an mTOR inhibitor (such as a limus drug) and an albumin, wherein the individual is selected for treatment based on having the mTOR-activating aberration. In some embodiments, there is provided a method of selecting an individual having a prostate cancer for treatment with a composition comprising nanoparticles comprising an mTOR inhibitor (such as a limus drug) and an albumin, wherein the method comprises (a) assessing an mTOR-activating aberration in the individual; and (b) selecting or recommending the individual for treatment based on the individual having the mTOR-activating aberration. In some embodiments, there is provided a method of selecting an individual having a prostate cancer for treatment with a composition comprising nanoparticles comprising an mTOR inhibitor (such as a limus drug) and an albumin, wherein the method comprises (a) assessing an mTOR-activating aberration in the individual; (b) selecting or recommending the individual for treatment based on the individual having the mTOR-activating aberration; and (c) administering an effective amount of the composition comprising the mTOR inhibitor (such as a limus drug) and the albumin to the selected individual. In some embodiments, there is provided a method of treating a prostate cancer (such as an mTOR-inhibitor-sensitive prostate cancer) in an individual comprising administering to the individual an effective amount of a composition comprising nanoparticles comprising an mTOR inhibitor (such as a limus drug) and an albumin, wherein the individual has an mTOR-activating aberration. In some embodiments, the composition comprising nanoparticles comprises a limus drug and an albumin, wherein the limus drug in the nanoparticles is associated (*e.g.,* coated) with the albumin. In some embodiments, the composition comprising nanoparticles comprises a limus drug and an albumin, wherein the nanoparticles have an average particle size of no greater than about 150 nm (such as no greater than about 120 nm). In some embodiments, the composition comprising nanoparticles comprises sirolimus and human serum albumin, wherein the nanoparticles comprise sirolimus associated (*e.g*., coated) with human serum albumin, wherein the nanoparticles have an average particle size of no greater than about 150 nm (such as no greater than about 120 nm, for example about 100 nm), and wherein the weight ratio of human albumin and sirolimus in the composition is about 9: 1 or less (such as about 9:1 or about 8:1). In some embodiments, the composition comprising nanoparticles comprises *Nab*-sirolimus. In some embodiments, the mTOR-activating aberration comprises a mutation of an mTOR-associated gene. In some embodiments, the mTOR-activating aberration comprises a copy number variation of an mTOR-associated gene. In some embodiments, the mTOR-activating aberration comprises an aberrant expression level of an mTOR-associated gene. In some embodiments, the mTOR-activating aberration comprises an aberrant activity level of an mTOR-associated gene. In some embodiments, the mTOR-activating aberration leads to activation of mTORC1 (including for example activation of mTORC1 but not mTORC2). In some embodiments, the mTOR-activating aberration leads to activation of mTORC2 (including for example activation of mTORC2 but not mTORC1). In some embodiments, the mTOR-activating aberration leads to activation of both mTORC1 and mTORC2. In some embodiments, the mTOR-activating aberration is an aberration in at least one mTOR-associated gene selected from the group consisting of AKT1, FLT3, MTOR, PIK3CA, PIK3CG, TSC1, TSC2, RHEB, STK11, NF1, NF2, PTEN, TP53, FGFR4, KRAS, NRAS, and BAP1. In some embodiments, the mTOR-activating aberration is assessed by gene sequencing. In some embodiments, the gene sequencing is based on sequencing of DNA in a tumor sample. In some embodiments, the gene sequencing is based on sequencing of circulating DNA or cell-free DNA isolated from a blood sample. In some embodiments, the mutational status of TFE3 is further used as a basis for selecting the individual. In some embodiments, the mutational status of TFE3 comprises translocation of TFE3. In some embodiments, the mTOR-activating aberration comprises an aberrant phosphorylation level of the protein encoded by the mTOR-associated gene. In some embodiments, the mTOR-activating aberration comprises an aberrant phosphorylation level of a protein encoded by an mTOR-associated gene selected from the group consisting of AKT, S6K, S6, 4EBP1, and SPARC. In some embodiments, the aberrant phosphorylation level is determined by immunohistochemistry.

In some embodiments, the prostate cancer is an adenocarcinoma. In some embodiments, the prostate cancer is a sarcoma, neuroendocrine tumor, small cell cancer, ductal cancer, or a lymphoma. In some embodiments of any of the methods, the prostate cancer may be androgen independent prostate cancer (AIPC). In some embodiments, the prostate cancer may be androgen dependent prostate cancer. In some embodiments, the prostate cancer may be refractory to hormone therapy. In some embodiments, the prostate cancer may be substantially refractory to hormone therapy. In some embodiments, the individual may be a human who has a gene, genetic mutation, or polymorphism associated with prostate cancer (e.g., RNASEL/HPC1, ELAC2/HPC2, SR-A/MSR1, CHEK2, BRCA2, PON1, OGG1, MIC-1, TLR4, and/or PTEN) or has one or more extra copies of a gene associated with prostate cancer.

In some embodiments, there is provided a method of treating a lymphoma in an individual comprising administering to the individual an effective amount of a composition comprising nanoparticles comprising an mTOR inhibitor (such as a limus drug) and an albumin, wherein the individual is selected for treatment on the basis of having an mTOR-activating aberration. In some embodiments, there is provided a method of treating a lymphoma in an individual comprising: (a) assessing an mTOR-activating aberration in the individual; and (b) administering (for example intravenously) to the individual an effective amount of a composition comprising nanoparticles comprising an mTOR inhibitor (such as a limus drug) and an albumin, wherein the individual is selected for treatment based on having the mTOR-activating aberration. In some embodiments, there is provided a method of selecting an individual having a lymphoma for treatment with a composition comprising nanoparticles comprising an mTOR inhibitor (such as a limus drug) and an albumin, wherein the method comprises (a) assessing an mTOR-activating aberration in the individual; and (b) selecting or recommending the individual for treatment based on the individual having the mTOR-activating aberration. In some embodiments, there is provided a method of selecting an individual having a lymphoma for treatment with a composition comprising nanoparticles comprising an mTOR inhibitor (such as a limus drug) and an albumin, wherein the method comprises (a) assessing an mTOR-activating aberration in the individual; (b) selecting or recommending the individual for treatment based on the individual having the mTOR-activating aberration; and (c) administering an effective amount of the composition comprising the mTOR inhibitor (such as a limus drug) and the albumin to the selected individual. In some embodiments, there is provided a method of treating a lymphoma (such as an mTOR-inhibitor-sensitive lymphoma) in an individual comprising administering to the individual an effective amount of a composition comprising nanoparticles comprising an mTOR inhibitor (such as a limus drug) and an albumin, wherein the individual has an mTOR-activating aberration. In some embodiments, the composition comprising nanoparticles comprises a limus drug and an albumin, wherein the limus drug in the nanoparticles is associated (*e.g.*, coated) with the albumin. In some embodiments, the composition comprising nanoparticles comprises a limus drug and an albumin, wherein the nanoparticles have an average particle size of no greater than about 150 nm (such as no greater than about 120 nm). In some embodiments, the composition comprising nanoparticles comprises sirolimus and human serum albumin, wherein the nanoparticles comprise sirolimus associated (*e.g*., coated) with human serum albumin, wherein the nanoparticles have an average particle size of no greater than about 150 nm (such as no greater than about 120 nm, for example about 100 nm), and wherein the weight ratio of human albumin and sirolimus in the composition is about 9:1 or less (such as about 9:1 or about 8:1). In some embodiments, the composition comprising nanoparticles comprises *Nab-*sirolimus. In some embodiments, the mTOR-activating aberration comprises a mutation of an mTOR-associated gene. In some embodiments, the mTOR-activating aberration comprises a copy number variation of an mTOR-associated gene. In some embodiments, the mTOR-activating aberration comprises an aberrant expression level of an mTOR-associated gene. In some embodiments, the mTOR-activating aberration comprises an aberrant activity level of an mTOR-associated gene. In some embodiments, the mTOR-activating aberration leads to activation of mTORC1 (including for example activation of mTORC1 but not mTORC2). In some embodiments, the mTOR-activating aberration leads to activation of mTORC2 (including for example activation of mTORC2 but not mTORC1). In some embodiments, the mTOR-activating aberration leads to activation of both mTORC1 and mTORC2. In some embodiments, the mTOR-activating aberration is an aberration in at least one mTOR-associated gene selected from the group consisting of AKT1, FLT3, MTOR, PIK3CA, PIK3CG, TSC1, TSC2, RHEB, STK11, NF1, NF2, PTEN, TP53, FGFR4, KRAS, NRAS, and BAP1. In some embodiments, the mTOR-activating aberration is assessed by gene sequencing. In some embodiments, the gene sequencing is based on sequencing of DNA in a tumor sample. In some embodiments, the gene sequencing is based on sequencing of circulating DNA or cell-free DNA isolated from a blood sample. In some embodiments, the mutational status of TFE3 is further used as a basis for selecting the individual. In some embodiments, the mutational status of TFE3 comprises translocation of TFE3. In some embodiments, the mTOR-activating aberration comprises an aberrant phosphorylation level of the protein encoded by the mTOR-associated gene. In some embodiments, the mTOR-activating aberration comprises an aberrant phosphorylation level of a protein encoded by an mTOR-associated gene selected from the group consisting of AKT, S6K, S6, 4EBP1, and SPARC. In some embodiments, the aberrant phosphorylation level is determined by immunohistochemistry.

In some embodiments, the lymphoma is a B-cell lymphoma. Examples of B-cell lymphomas include, but are not limited to, precursor B-cell neoplasms (*e.g*., precursor B-lymphoblastic leukemia/lymphoma) and peripheral B-cell neoplasms (*e.g*., B-cell chronic lymphocytic leukemia/prolymphocytic leukemia/small lymphocytic lymphoma (small lymphocytic (SL) NHL), lymphoplasmacytoid lymphoma/immunocytoma, mantel cell lymphoma, follicle center lymphoma, follicular lymphoma (*e.g*., cytologic grades: I (small cell), II (mixed small and large cell), III (large cell) and/or subtype: diffuse and predominantly small cell type), low grade/follicular non-Hodgkin's lymphoma (NHL), intermediate grade/follicular NHL, marginal zone B-cell lymphoma (*e.g*., extranodal (*e.g*., MALT-type +/- monocytoid B cells) and/or Nodal (*e.g*., +/- monocytoid B cells)), splenic marginal zone lymphoma (*e.g*., +/villous lymphocytes), Hairy cell leukemia, plasmacytoma/plasma cell myeloma (*e.g*., myeloma and multiple myeloma), diffuse large B-cell lymphoma (*e.g*., primary mediastinal (thymic) B-cell lymphoma), intermediate grade diffuse NHL, Burkitt's lymphoma, High-grade B-cell lymphoma, Burkitt-like, high grade immunoblastic NHL, high grade lymphoblastic NHL, high grade small non-cleaved cell NHL, bulky disease NHL, AIDS-related lymphoma, and Waldenstrom's macroglobulinemia). In some embodiments, the lymphoma is Mantle Cell lymphoma. In some embodiments, the lymphoma is a T-cell and/or putative NK-cell lymphoma. Examples of T-cell and/or putative NK-cell lymphomas include, but are not limited to, precursor T-cell neoplasm (precursor T-lymphoblastic lymphoma/leukemia) and peripheral T-cell and NK-cell neoplasms (*e.g.*, T-cell chronic lymphocytic leukemia/prolymphocytic leukemia, and large granular lymphocyte leukemia (LGL) (*e.g*., T-cell type and/or NK-cell type), cutaneous T-cell lymphoma (*e.g*., mycosis fungoides/Sezary syndrome), primary T-cell lymphomas unspecified (*e.g*., cytological categories (*e.g*., medium-sized cell, mixed medium and large cell), large cell, lymphoepitheloid cell, subtype hepatosplenic γδ T-cell lymphoma, and subcutaneous panniculitic T-cell lymphoma), angioimmunoblastic T-cell lymphoma (AILD), angiocentric lymphoma, intestinal T-cell lymphoma (*e.g*., +/- enteropathy associated), adult T-cell lymphoma/leukemia (ATL), anaplastic large cell lymphoma (ALCL) (*e.g.*, CD30+, T- and null-cell types), anaplastic large-cell lymphoma, and Hodgkin's like). In some embodiments, the lymphoma is Hodgkin's disease. For example, the Hodgkin's disease may be lymphocyte predominance, nodular sclerosis, mixed cellularity, lymphocyte depletion, and/or lymphocyte-rich. In some embodiments, the lymphoma is non-Hodgkin's disease.

In some embodiments, there is provided a method of treating a bladder cancer in an individual comprising administering to the individual an effective amount of a composition comprising nanoparticles comprising an mTOR inhibitor (such as a limus drug) and an albumin, wherein the individual is selected for treatment on the basis of having an mTOR-activating aberration. In some embodiments, there is provided a method of treating a bladder cancer in an individual comprising: (a) assessing an mTOR-activating aberration in the individual; and (b) administering (for example intravenously) to the individual an effective amount of a composition comprising nanoparticles comprising an mTOR inhibitor (such as a limus drug) and an albumin, wherein the individual is selected for treatment based on having the mTOR-activating aberration. In some embodiments, there is provided a method of selecting an individual having a bladder cancer for treatment with a composition comprising nanoparticles comprising an mTOR inhibitor (such as a limus drug) and an albumin, wherein the method comprises (a) assessing an mTOR-activating aberration in the individual; and (b) selecting or recommending the individual for treatment based on the individual having the mTOR-activating aberration. In some embodiments, there is provided a method of selecting an individual having a bladder cancer for treatment with a composition comprising nanoparticles comprising an mTOR inhibitor (such as a limus drug) and an albumin, wherein the method comprises (a) assessing an mTOR-activating aberration in the individual; (b) selecting or recommending the individual for treatment based on the individual having the mTOR-activating aberration; and (c) administering an effective amount of the composition comprising the mTOR inhibitor (such as a limus drug) and the albumin to the selected individual. In some embodiments, there is provided a method of treating a bladder cancer (such as an mTOR-inhibitor-sensitive bladder cancer) in an individual comprising administering to the individual an effective amount of a composition comprising nanoparticles comprising an mTOR inhibitor (such as a limus drug) and an albumin, wherein the individual has an mTOR-activating aberration. In some embodiments, the composition comprising nanoparticles comprises a limus drug and an albumin, wherein the limus drug in the nanoparticles is associated (*e.g*., coated) with the albumin. In some embodiments, the composition comprising nanoparticles comprises a limus drug and an albumin, wherein the nanoparticles have an average particle size of no greater than about 150 nm (such as no greater than about 120 nm). In some embodiments, the composition comprising nanoparticles comprises sirolimus and human serum albumin, wherein the nanoparticles comprise sirolimus associated (*e.g.*, coated) with human serum albumin, wherein the nanoparticles have an average particle size of no greater than about 150 nm (such as no greater than about 120 nm, for example about 100 nm), and wherein the weight ratio of human albumin and sirolimus in the composition is about 9:1 or less (such as about 9:1 or about 8:1). In some embodiments, the composition comprising nanoparticles comprises *Nab*-sirolimus. In some embodiments, the mTOR-activating aberration comprises a mutation of an mTOR-associated gene. In some embodiments, the mTOR-activating aberration comprises a copy number variation of an mTOR-associated gene. In some embodiments, the mTOR-activating aberration comprises an aberrant expression level of an mTOR-associated gene. In some embodiments, the mTOR-activating aberration comprises an aberrant activity level of an mTOR-associated gene. In some embodiments, the mTOR-activating aberration leads to activation of mTORC1 (including for example activation of mTORC1 but not mTORC2). In some embodiments, the mTOR-activating aberration leads to activation of mTORC2 (including for example activation of mTORC2 but not mTORC1). In some embodiments, the mTOR-activating aberration leads to activation of both mTORC1 and mTORC2. In some embodiments, the mTOR-activating aberration is an aberration in at least one mTOR-associated gene selected from the group consisting of AKT1, FLT3, MTOR, PIK3CA, PIK3CG, TSC1, TSC2, RHEB, STK11, NF1, NF2, PTEN, TP53, FGFR4, KRAS, NRAS, and BAP1. In some embodiments, the mTOR-activating aberration is assessed by gene sequencing. In some embodiments, the gene sequencing is based on sequencing of DNA in a tumor sample. In some embodiments, the gene sequencing is based on sequencing of circulating DNA or cell-free DNA isolated from a blood sample. In some embodiments, the mutational status of TFE3 is further used as a basis for selecting the individual. In some embodiments, the mutational status of TFE3 comprises translocation of TFE3. In some embodiments, the mTOR-activating aberration comprises an aberrant phosphorylation level of the protein encoded by the mTOR-associated gene. In some embodiments, the mTOR-activating aberration comprises an aberrant phosphorylation level of a protein encoded by an mTOR-associated gene selected from the group consisting of AKT, S6K, S6, 4EBP1, and SPARC. In some embodiments, the aberrant phosphorylation level is determined by immunohistochemistry.

In some embodiments, the bladder cancer is a low grade bladder cancer. In some embodiments, the bladder cancer is a high grade bladder cancer. In some embodiments, the bladder cancer is invasive. In some embodiments, the bladder cancer is non-invasive. In some embodiments, the bladder cancer is non-muscle invasive bladder cancer (NMIBC). In some embodiments, the bladder cancer is BCG refractory or recurrent non-muscle invasive bladder cancer. In some embodiments, the bladder cancer is transitional cell carcinoma or urothelial carcinoma (such as metastatic urothelial carcinoma), including, but not limited to, papillary tumors and flat carcinomas. In some embodiments, the bladder cancer is metastatic urothelial carcinoma. In some embodiments, the bladder cancer is urothelial carcinoma of the bladder. In some embodiments, the bladder cancer is urothelial carcinoma of the ureter. In some embodiments, the bladder cancer is urothelial carcinoma of the urethra. In some embodiments, the bladder cancer is urothelial carcinoma of the renal pelvis. In some embodiments, the bladder cancer is squamous cell carcinoma. In some embodiments, the bladder cancer is non-squamous cell carcinoma. In some embodiments, the bladder cancer is adenocarcinoma. In some embodiments, the bladder cancer is small cell carcinoma.

In some embodiments, there is provided a method of treating an ovarian cancer in an individual comprising administering to the individual an effective amount of a composition comprising nanoparticles comprising an mTOR inhibitor (such as a limus drug) and an albumin, wherein the individual is selected for treatment on the basis of having an mTOR-activating aberration. In some embodiments, there is provided a method of treating an ovarian cancer in an individual comprising: (a) assessing an mTOR-activating aberration in the individual; and (b) administering (for example intravenously) to the individual an effective amount of a composition comprising nanoparticles comprising an mTOR inhibitor (such as a limus drug) and an albumin, wherein the individual is selected for treatment based on having the mTOR-activating aberration. In some embodiments, there is provided a method of selecting an individual having an ovarian cancer for treatment with a composition comprising nanoparticles comprising an mTOR inhibitor (such as a limus drug) and an albumin, wherein the method comprises (a) assessing an mTOR-activating aberration in the individual; and (b) selecting or recommending the individual for treatment based on the individual having the mTOR-activating aberration. In some embodiments, there is provided a method of selecting an individual having an ovarian cancer for treatment with a composition comprising nanoparticles comprising an mTOR inhibitor (such as a limus drug) and an albumin, wherein the method comprises (a) assessing an mTOR-activating aberration in the individual; (b) selecting or recommending the individual for treatment based on the individual having the mTOR-activating aberration; and (c) administering an effective amount of the composition comprising the mTOR inhibitor (such as a limus drug) and the albumin to the selected individual. In some embodiments, there is provided a method of treating an ovarian cancer (such as an mTOR-inhibitor-sensitive ovarian cancer) in an individual comprising administering to the individual an effective amount of a composition comprising nanoparticles comprising an mTOR inhibitor (such as a limus drug) and an albumin, wherein the individual has an mTOR-activating aberration. In some embodiments, the composition comprising nanoparticles comprises a limus drug and an albumin, wherein the limus drug in the nanoparticles is associated (*e.g*., coated) with the albumin. In some embodiments, the composition comprising nanoparticles comprises a limus drug and an albumin, wherein the nanoparticles have an average particle size of no greater than about 150 nm (such as no greater than about 120 nm). In some embodiments, the composition comprising nanoparticles comprises sirolimus and human serum albumin, wherein the nanoparticles comprise sirolimus associated (*e.g.*, coated) with human serum albumin, wherein the nanoparticles have an average particle size of no greater than about 150 nm (such as no greater than about 120 nm, for example about 100 nm), and wherein the weight ratio of human albumin and sirolimus in the composition is about 9:1 or less (such as about 9:1 or about 8:1). In some embodiments, the composition comprising nanoparticles comprises *Nab*-sirolimus. In some embodiments, the mTOR-activating aberration comprises a mutation of an mTOR-associated gene. In some embodiments, the mTOR-activating aberration comprises a copy number variation of an mTOR-associated gene. In some embodiments, the mTOR-activating aberration comprises an aberrant expression level of an mTOR-associated gene. In some embodiments, the mTOR-activating aberration comprises an aberrant activity level of an mTOR-associated gene. In some embodiments, the mTOR-activating aberration leads to activation of mTORC1 (including for example activation of mTORC1 but not mTORC2). In some embodiments, the mTOR-activating aberration leads to activation of mTORC2 (including for example activation of mTORC2 but not mTORC1). In some embodiments, the mTOR-activating aberration leads to activation of both mTORC1 and mTORC2. In some embodiments, the mTOR-activating aberration is an aberration in at least one mTOR-associated gene selected from the group consisting of AKT1, FLT3, MTOR, PIK3CA, PIK3CG, TSC1, TSC2, RHEB, STK11, NF1, NF2, PTEN, TP53, FGFR4, KRAS, NRAS, and BAP1. In some embodiments, the mTOR-activating aberration is assessed by gene sequencing. In some embodiments, the gene sequencing is based on sequencing of DNA in a tumor sample. In some embodiments, the gene sequencing is based on sequencing of circulating DNA or cell-free DNA isolated from a blood sample. In some embodiments, the mutational status of TFE3 is further used as a basis for selecting the individual. In some embodiments, the mutational status of TFE3 comprises translocation of TFE3. In some embodiments, the mTOR-activating aberration comprises an aberrant phosphorylation level of the protein encoded by the mTOR-associated gene. In some embodiments, the mTOR-activating aberration comprises an aberrant phosphorylation level of a protein encoded by an mTOR-associated gene selected from the group consisting of AKT, S6K, S6, 4EBP1, and SPARC. In some embodiments, the aberrant phosphorylation level is determined by immunohistochemistry.

In some embodiments, the ovarian cancer is ovarian epithelial cancer. Exemplary ovarian epithelial cancer histological classifications include: serous cystomas (*e.g.*, serous benign cystadenomas, serous cystadenomas with proliferating activity of the epithelial cells and nuclear abnormalities but with no infiltrative destructive growth, or serous cystadenocarcinomas), mucinous cystomas (*e.g*., mucinous benign cystadenomas, mucinous cystadenomas with proliferating activity of the epithelial cells and nuclear abnormalities but with no infiltrative destructive growth, or mucinous cystadenocarcinomas), endometrioid tumors (*e.g.*, endometrioid benign cysts, endometrioid tumors with proliferating activity of the epithelial cells and nuclear abnormalities but with no infiltrative destructive growth, or endometrioid adenocarcinomas), clear cell (mesonephroid) tumors (*e.g*., benign clear cell tumors, clear cell tumors with proliferating activity of the epithelial cells and nuclear abnormalities but with no infiltrative destructive growth, or clear cell cystadenocarcinomas), unclassified tumors that cannot be allotted to one of the above groups, or other malignant tumors. In some embodiments, the individual may be a human who has a gene, genetic mutation, or polymorphism associated with ovarian cancer (*e.g*., BRCA1 or BRCA2) or has one or more extra copies of a gene associated with ovarian cancer (*e.g.*, one or more extra copies of the HER2 gene). In some embodiments, the ovarian cancer is an ovarian germ cell tumor. Exemplary histologic subtypes include dysgerminomas or other germ cell tumors (*e.g*., endodermal sinus tumors such as hepatoid or intestinal tumors, embryonal carcinomas, olyembryomas, choriocarcinomas, teratomas, or mixed form tumors). Exemplary teratomas are immature teratomas, mature teratomas, solid teratomas, and cystic teratomas (*e.g*., dermoid cysts such as mature cystic teratomas, and dermoid cysts with malignant transformation). Some teratomas are monodermal and highly specialized, such as struma ovarii, carcinoid, struma ovarii and carcinoid, or others (*e.g*., malignant neuroectodermal and ependymomas).

In some embodiments, the hyperplasia is restenosis. Thus, there is provided a method of treating restenosis in an individual comprising administering to the individual an effective amount of a composition comprising nanoparticles comprising an mTOR inhibitor (such as a limus drug) and an albumin, wherein the individual is selected for treatment on the basis of having an mTOR-activating aberration. In some embodiments, there is provided a method of treating restenosis in an individual comprising: (a) assessing an mTOR-activating aberration in the individual; and (b) administering (for example intravenously) to the individual an effective amount of a composition comprising nanoparticles comprising an mTOR inhibitor (such as a limus drug) and an albumin, wherein the individual is selected for treatment based on having the mTOR-activating aberration. In some embodiments, there is provided a method of selecting an individual having restenosis for treatment with a composition comprising nanoparticles comprising an mTOR inhibitor (such as a limus drug) and an albumin, wherein the method comprises (a) assessing an mTOR-activating aberration in the individual; and (b) selecting or recommending the individual for treatment based on the individual having the mTOR-activating aberration. In some embodiments, there is provided a method of selecting an individual having restenosis for treatment with a composition comprising nanoparticles comprising an mTOR inhibitor (such as a limus drug) and an albumin, wherein the method comprises (a) assessing an mTOR-activating aberration in the individual; (b) selecting or recommending the individual for treatment based on the individual having the mTOR-activating aberration; and (c) administering an effective amount of the composition comprising the mTOR inhibitor (such as a limus drug) and the albumin to the selected individual. In some embodiments, there is provided a method of treating restenosis carcinoma (such as mTOR-inhibitor-sensitive restenosis) in an individual comprising administering to the individual an effective amount of a composition comprising nanoparticles comprising an mTOR inhibitor (such as a limus drug) and an albumin, wherein the individual has an mTOR-activating aberration. In some embodiments, the composition comprising nanoparticles comprises a limus drug and an albumin, wherein the limus drug in the nanoparticles is associated (*e.g*., coated) with the albumin. In some embodiments, the composition comprising nanoparticles comprises a limus drug and an albumin, wherein the nanoparticles have an average particle size of no greater than about 150 nm (such as no greater than about 120 nm). In some embodiments, the composition comprising nanoparticles comprises sirolimus and human serum albumin, wherein the nanoparticles comprise sirolimus associated (*e.g.*, coated) with human serum albumin, wherein the nanoparticles have an average particle size of no greater than about 150 nm (such as no greater than about 120 nm, for example about 100 nm), and wherein the weight ratio of human albumin and sirolimus in the composition is about 9:1 or less (such as about 9:1 or about 8:1). In some embodiments, the composition comprising nanoparticles comprises *Nab*-sirolimus. In some embodiments, the mTOR-activating aberration comprises a mutation of an mTOR-associated gene. In some embodiments, the mTOR-activating aberration comprises a copy number variation of an mTOR-associated gene. In some embodiments, the mTOR-activating aberration comprises an aberrant expression level of an mTOR-associated gene. In some embodiments, the mTOR-activating aberration comprises an aberrant activity level of an mTOR-associated gene. In some embodiments, the mTOR-activating aberration leads to activation of mTORC1 (including for example activation of mTORC1 but not mTORC2). In some embodiments, the mTOR-activating aberration leads to activation of mTORC2 (including for example activation of mTORC2 but not mTORC1). In some embodiments, the mTOR-activating aberration leads to activation of both mTORC1 and mTORC2. In some embodiments, the mTOR-activating aberration is an aberration in at least one mTOR-associated gene selected from the group consisting of AKT1, FLT3, MTOR, PIK3CA, PIK3CG, TSC1, TSC2, RHEB, SIK11, NF1, NF2, PTEN, TP53, FGFR4, KRAS, NRAS, and BAP1. In some embodiments, the mTOR-activating aberration is assessed by gene sequencing. In some embodiments, the gene sequencing is based on sequencing of DNA in a tumor sample. In some embodiments, the gene sequencing is based on sequencing of circulating DNA or cell-free DNA isolated from a blood sample. In some embodiments, the mutational status of TFE3 is further used as a basis for selecting the individual. In some embodiments, the mutational status of TFE3 comprises translocation of TFE3. In some embodiments, the mTOR-activating aberration comprises an aberrant phosphorylation level of the protein encoded by the mTOR-associated gene. In some embodiments, the mTOR-activating aberration comprises an aberrant phosphorylation level of a protein encoded by an mTOR-associated gene selected from the group consisting of AKT, S6K, S6, 4EBP1, and SPARC. In some embodiments, the aberrant phosphorylation level is determined by immunohistochemistry.

In some embodiments, the restenosis is in the coronary artery. In some embodiments, the restenosis is in a peripheral blood vessel, such as the popliteal artery in the leg, the pudendal artery in the pelvis, and/or the carotid artery in the neck. In some embodiments, the restenosis follows an endovascular procedure or a vascular trauma, including, but not limited to, vascular surgery, cardiac surgery, antheroectomy, coronary artery bypass graft procedures, stent surgery, and angioplasty. In some embodiments, the restenosis is an in-stent restenosis. In some embodiments, the restenosis is a post-angioplasty restenosis. In some embodiments, the restenosis results from vascular diseases, including atherosclerosis, vascular stenosis or atrophy, cerebral vascular stenotic diseases, and the like. In some embodiments, the restenosis comprises a reduction in the percent diameter stenosis of at least about any of 10%, 20%, 30%, 40%, 50%, 60%, 70%, 80%, 90% or more. In some embodiments, the restenosis is binary restenosis.

In some embodiments, the method leads to retention of an expanded luminal diameter or cross-section area of a blood vessel following an endovascular procedure. In some embodiments, the luminal diameter or cross-section area of the blood vessel is retained at least about 50% (including for example at least about any of 60%, 70%, 80%, 90% or 100%) of the luminal diameter or cross-section area of the blood vessel after the endovascular procedure. In some embodiments, the method inhibits and/or reduces abnormal cell proliferation in the blood vessel. In some embodiments, the method inhibits at least about 10% (including for example at least about any of 20%, 30%, 40%, 60%, 70%, 80%, 90%, or 100%) abnormal cell proliferation. In some embodiments, the method relieves one or more of the symptoms associated with the restenosis. In some embodiments, the method delays the restenosis. In some embodiments, the method prevents the restenosis.

In some embodiments, the hyperplasia is pulmonary hypertension. Thus, there is provided a method of treating pulmonary hypertension in an individual comprising administering to the individual an effective amount of a composition comprising nanoparticles comprising an mTOR inhibitor (such as a limus drug) and an albumin, wherein the individual is selected for treatment on the basis of having an mTOR-activating aberration. In some embodiments, there is provided a method of treating pulmonary hypertension in an individual comprising: (a) assessing an mTOR-activating aberration in the individual; and (b) administering (for example intravenously) to the individual an effective amount of a composition comprising nanoparticles comprising an mTOR inhibitor (such as a limus drug) and an albumin, wherein the individual is selected for treatment based on having the mTOR-activating aberration. In some embodiments, there is provided a method of selecting an individual having pulmonary hypertension for treatment with a composition comprising nanoparticles comprising an mTOR inhibitor (such as a limus drug) and an albumin, wherein the method comprises (a) assessing an mTOR-activating aberration in the individual; and (b) selecting or recommending the individual for treatment based on the individual having the mTOR-activating aberration. In some embodiments, there is provided a method of selecting an individual having pulmonary hypertension for treatment with a composition comprising nanoparticles comprising an mTOR inhibitor (such as a limus drug) and an albumin, wherein the method comprises (a) assessing an mTOR-activating aberration in the individual; (b) selecting or recommending the individual for treatment based on the individual having the mTOR-activating aberration; and (c) administering an effective amount of the composition comprising the mTOR inhibitor (such as a limus drug) and the albumin to the selected individual. In some embodiments, there is provided a method of treating pulmonary hypertension (such as an mTOR-inhibitor-sensitive pulmonary hypertension) in an individual comprising administering to the individual an effective amount of a composition comprising nanoparticles comprising an mTOR inhibitor (such as a limus drug) and an albumin, wherein the individual has an mTOR-activating aberration. In some embodiments, the composition comprising nanoparticles comprises a limus drug and an albumin, wherein the limus drug in the nanoparticles is associated (*e.g*., coated) with the albumin. In some embodiments, the composition comprising nanoparticles comprises a limus drug and an albumin, wherein the nanoparticles have an average particle size of no greater than about 150 nm (such as no greater than about 120 nm). In some embodiments, the composition comprising nanoparticles comprises sirolimus and human serum albumin, wherein the nanoparticles comprise sirolimus associated (*e.g*., coated) with human serum albumin, wherein the nanoparticles have an average particle size of no greater than about 150 nm (such as no greater than about 120 nm, for example about 100 nm), and wherein the weight ratio of human albumin and sirolimus in the composition is about 9: 1 or less (such as about 9:1 or about 8:1). In some embodiments, the composition comprising nanoparticles comprises *Nab*-sirolimus. In some embodiments, the mTOR-activating aberration comprises a mutation of an mTOR-associated gene. In some embodiments, the mTOR-activating aberration comprises a copy number variation of an mTOR-associated gene. In some embodiments, the mTOR-activating aberration comprises an aberrant expression level of an mTOR-associated gene. In some embodiments, the mTOR-activating aberration comprises an aberrant activity level of an mTOR-associated gene. In some embodiments, the mTOR-activating aberration leads to activation of mTORC1 (including for example activation of mTORC1 but not mTORC2). In some embodiments, the mTOR-activating aberration leads to activation of mTORC2 (including for example activation of mTORC2 but not mTORC1). In some embodiments, the mTOR-activating aberration leads to activation of both mTORC1 and mTORC2. In some embodiments, the mTOR-activating aberration is an aberration in at least one mTOR-associated gene selected from the group consisting of AKT1, FLT3, MTOR, PIK3CA, PIK3CG, TSC1, TSC2, RHEB, STK11, NF1, NF2, PTEN, TP53, FGFR4, KRAS, NRAS, and BAP1. In some embodiments, the mTOR-activating aberration is assessed by gene sequencing. In some embodiments, the gene sequencing is based on sequencing of DNA in a tumor sample. In some embodiments, the gene sequencing is based on sequencing of circulating DNA or cell-free DNA isolated from a blood sample. In some embodiments, the mutational status of TFE3 is further used as a basis for selecting the individual. In some embodiments, the mutational status of TFE3 comprises translocation of TFE3. In some embodiments, the mTOR-activating aberration comprises an aberrant phosphorylation level of the protein encoded by the mTOR-associated gene. In some embodiments, the mTOR-activating aberration comprises an aberrant phosphorylation level of a protein encoded by an mTOR-associated gene selected from the group consisting of AKT, S6K, S6, 4EBP1, and SPARC. In some embodiments, the aberrant phosphorylation level is determined by immunohistochemistry.

In some embodiments, the pulmonary hypertension is pulmonary arterial hypertension (PAH). In some embodiments, the PAH is idiopathic PAH. In some embodiments, the PAH is familial PAH. In some variations, the PAH is associated with persistent pulmonary hypertension of a newborn. In some embodiments, the PAH is associated with pulmonary veno-occlusive disease. In some embodiments, the PAH is associated with pulmonary capillary hemangiomatosis. In some embodiments, the pulmonary hypertension is pulmonary venous hypertension. In some embodiments, the pulmonary hypertension is pulmonary hypertension associated with disorders of the respiratory system and/or hypoxia. In some embodiments, the pulmonary hypertension is pulmonary hypertension due to chronic thrombotic and/or embolic disease. In some embodiments, the pulmonary hypertension is miscellaneous pulmonary hypertension. In some embodiments, the miscellaneous pulmonary hypertension is associated with sarcoidosis, eosiniphilic granuloma, histicytosis X, lymphangiolomyiomatosis, or compression of pulmonary vessels (*e.g*., adenopath, tumor, or fibrosing medianstinitis). In some embodiments, the pulmonary hypertension is associated with chronic obstructive pulmonary disease (COPD). In some embodiments, the pulmonary hypertension is associated with pulmonary fibrosis. In some embodiments, the pulmonary hypertension is early stage pulmonary hypertension or advanced pulmonary hypertension. In some embodiments, the pulmonary hypertension is severe progressive pulmonary arterial hypertension.

In some embodiments, the method reduces pulmonary pressure. In some embodiments, the pulmonary pressure is reduced by at least about 10% (including for example at least about any of 20%, 30%, 40%, 60%, 70%, 80%, 90%, or 100%). In some embodiments, the method inhibits and/or reduces abnormal cell proliferation in the pulmonary artery. In some embodiments, the method inhibits at least about 10% (including for example at least about any of 20%, 30%, 40%, 60%, 70%, 80%, 90%, or 100%) abnormal cell proliferation. In some embodiments, the method relieves one or more of the symptoms associated with the pulmonary hypertension. In some embodiments, the method delays the pulmonary hypertension. In some embodiments, the method prevents the pulmonary hypertension.

In some embodiments according to any of the methods for treating restenosis or pulmonary hypertension as described above, the method inhibits negative remodeling in a blood vessel in the individual. In some embodiments, the blood vessel is an artery. In some embodiments, the artery is a coronary artery or a peripheral artery. In some embodiments, the artery is a pulmonary artery. Negative remodeling includes the physiologic or pathologic response of a blood vessel to a stimulus resulting in a reduction of vessel diameter and lumen diameter. Such a stimulus could be provided by, for example, a change in blood flow or an angioplasty procedure. In some embodiments, the administration of the mTOR inhibitor nanoparticle composition leads to an increase of vessel diameter by about any of 10%, 20%, 30%, 40%, 60%, 70%, 80%, 95%, or more, compared to the diameter of a vessel of without the injection. Negative remodeling can be quantified, for example, angiographically as the percent diameter stenosis at the lesion site (or disease site). Another method of determining the degree of remodeling involves measuring in-lesion external elastic lamina area using intravascular ultrasound (IVUS). IVUS is a technique that can image the external elastic lamina as well as the vascular lumen. In some embodiments, the negative remodeling is associated with a vascular interventional procedure, such as angioplasty, stenting, or atherectomy. The nanoparticle composition can therefore be injected during or after the vascular interventional procedure.

In some embodiments according to any of the methods for treating restenosis or pulmonary hypertension as described above, the method inhibits vascular fibrosis (such as medial fibrosis or adventitia fibrosis) in a blood vessel in the individual. In some embodiments, the blood vessel is an artery. In some embodiments, the artery is a coronary artery or a peripheral artery. In some embodiments, the artery is a pulmonary artery.

Vascular fibrosis as used herein refers to the extensive fibrous (connective) tissue formation in the blood vessel, and includes, for example, medial fibrosis or adventitial fibrosis. Vascular fibrosis is frequently associated with abundant deposition of extracellular matrix and proliferation of myofibroblasts and fibroblasts. The method described herein therefore in some embodiments inhibits fibrous tissue formation in the blood vessel, for example inhibits about any of 10%, 20%, 30%, 40%, 50%, 60%, 70%, 80%, or 90% fibrous tissue formation compared to a vessel without the injection. In some embodiments, the method inhibits deposition of extracellular matrix in the blood vessel, for example inhibits about any of 10%, 20%, 30%, 40%, 50%, 60%, 70%, 80%, or 90% deposition of extracellular matrix compared to a vessel without the injection. In some embodiments, the method inhibits proliferation of myofibroblast in the blood vessel, for example inhibits about any of 10%, 20%, 30%, 40%, 50%, 60%, 70%, 80%, or 90% proliferation of myofibroblast compared to a vessel without the injection. In some embodiments, the method inhibits proliferation of fibroblast in the blood vessel, for example inhibits about any of 10%, 20%, 30%, 40%, 50%, 60%, 70%, 80%, or 90% proliferation of fibroblast compared to a vessel without the injection. In some embodiments, the vascular fibrosis is associated with a vascular interventional procedure, such as angioplasty, stenting, or atherectomy.

The methods provided herein can be used to treat an individual (*e.g*., human) who has been diagnosed with or is suspected of having a hyperplasia (such as cancer, restenosis or pulmonary hypertension). In some embodiments, the individual is human. In some embodiments, the individual is at least about any of 20, 25, 30, 35, 40, 45, 50, 55, 60, 65, 70, 75, 80, or 85 years old. In some embodiments, the individual is male. In some embodiments, the individual is female. In some embodiments, the individual has undergone a resection of the hyperplastic tissue (such as tumor). In some embodiments, the individual has refused surgery. In some embodiments, the individual is medically inoperable. In some of embodiments, the individual is genetically or otherwise predisposed (*e.g*., having a risk factor) to developing a hyperplasia (such as cancer, restenosis or pulmonary hypertension). These risk factors include, but are not limited to, age, sex, race, diet, history of previous disease, presence of precursor disease, genetic considerations, and environmental exposure. In some embodiments, the individuals at risk for the hyperplasia (such as cancer, restenosis, or pulmonary hypertension) include, *e.g.*, those having relatives who have experienced the hyperplasia (such as cancer, restenosis, or pulmonary hypertension), and those whose risk is determined by analysis of genetic or biochemical markers.

The methods provided herein may be practiced in an adjuvant setting. In some embodiments, the method is practiced in a neoadjuvant setting, i.e., the method may be carried out before the primary/definitive therapy. In some embodiments, the method is used to treat an individual who has previously been treated. In some embodiments, the individual is resistant, non-responsive, partially responsive, initially responsive, or refractory to a prior therapy. In some embodiments, the individual has progressed on the prior therapy at the time of treatment. In some embodiments, the individual is unsuitable to continue with the prior therapy, for example, due to failure to respond and/or due to toxicity. In some embodiments, the individual has not previously been treated. In some embodiments, the method is used as a first line therapy. In some embodiments, the method is used as a second line therapy.

The methods described herein for treating hyperplasia can be used in monotherapy as well as in combination therapy with another agent. In some embodiments, the composition comprising nanoparticles comprising the mTOR inhibitor (such as a limus drug) and the albumin is administered as a single agent. In some embodiments, the method further comprises administering to the individual an effective amount of at least another therapeutic agent. The other therapeutic agent may be a chemotherapeutic agent or an antibody. In some embodiments, the other therapeutic agent is selected from the group consisting of an alkylating agent, an anthracycline antibiotic, a DNA crosslinking agent, an antimetabolite, an indolequinone, a taxane, or a platinum-based agent.

Also provided are pharmaceutical compositions comprising nanoparticles comprising an mTOR inhibitor (such as limus drug, for example sirolimus) for use in any of the methods of treating an individual having a hyperplasia (such as cancer, restenosis, or pulmonary hypertension) described herein. In some embodiments, the compositions comprise nanoparticles comprising an mTOR inhibitor (such as limus drug, for example sirolimus) and an albumin (such as human serum albumin).

### Biomarkers

The present invention uses biomarkers to select individuals for treatment with mTOR inhibitor nanoparticle compositions. Deviations from the normal sequence, expression level, and/or activity level of the biomarkers described herein may be used as the basis for selecting the individual for the treatment.

"Biomarker" as used herein may refer to a molecule (typically protein, nucleic acid, carbohydrate, or lipid) that is encoded by or expressed in a hyperplastic cell (such as a cancer cell, or an abnormally proliferative cell in pulmonary hypertension or restenosis), which is useful for the diagnosis, prognosis, and/or preferential targeting of the mTOR inhibitor nanoparticle compositions to the hyperplastic cell. The biomarkers described herein include mTOR-associated genes, molecules encoded by mTOR-associated genes, or derivatives of mTOR-associated genes or molecules encoded by mTOR-associated genes, such as nucleic acids (DNA or RNA), proteins, or naturally modified nucleic acids or proteins thereof corresponding to the mTOR-associated genes. Aberrations in the sequence, expression level and/or activity level of the biomarkers are correlated with an mTOR signaling level above the normal mTOR signaling level in the hyperplastic cells.

### mTOR signaling pathway

The mTOR signaling pathway is mediated by multiple upstream proteins which sense various sources of signals and relay the signals to the mTOR complex. The mTOR complex integrates the upstream signals and regulates cell growth and proliferation by activating or inhibiting downstream effector proteins. The mTOR signaling pathway has been described. See, for example, Laplante et al. Journal of cell science 122.20 (2009): 3589-3594.

The mTOR complex is a multi-subunit protein complex comprising the mTOR protein, a 289-kDa serine-threonine kinase, as the catalytic subunit. There are at least two structurally and functionally distinct mTOR complexes, mTOR complex 1 (mTORC1) and mTOR complex 2 (mTORC2), each comprising a distinct set of protein components. mTORC1 and mTORC2 are known to have distinct biochemical properties, including affinity to mTOR inhibitors, and signaling properties (such as upstream and downstream interacting partners). For example, rapamycin (or a rapalog) binds to FK506-binding protein of 12 kDa (FKBP12), which interacts with the FKBP12-rapamycin binding domain (FRB) of mTOR, thus inhibiting mTORC1 functions. mTORC2 have been characterized as rapamycin-insensitive, i.e. at low concentrations that are sufficient for rapamycin (or a rapalog) to fully inhibit mTORC1, rapamycin (or the rapalog) has insignificant amount of inhibition (such as less than about 1%) on the activity of mTORC2. At concentrations at which rapamycin (or a rapalog) inhibits the activity of mTORC2 by a significant amount (such as at least about any of 10%, 20%, 30%, 40%, 50%, 60%, 70%, 80%, 90% or more), rapamycin (or the rapalog) may be toxic to the individual being treated.

mTORC1 comprises at least five proteins, including the mTOR protein, regulatory-associated protein of mTOR (RAPTOR); mammalian lethal with Sec13 protein 8 (mLST8, also known as GβL); proline-rich AKT substrate 40 kDa (PRAS40;); and DEP-domain-containing mTOR-interacting protein (DEPTOR). Signals integrated by mTORC1 include growth factors, energy status, oxygen level and amino acids. An important axis of sensing the upstream signals and regulating the mTORC1 activity involves TSC1/2 and RHEB (Ras homolog enriched in brain). TSC1/2 is a heterodimeric protein complex composed of TSC1 and TSC2, which functions as a GTPase-activating protein (GAP) for the small GTPase RHEB. While RHEB can stimulate mTORC1 activity through direct interaction, TSC1/2 can convert RHEB into its inactive GDP-bound state and thereby negatively regulates mTORC1 activity. Additionally, TSC1/2-independent signaling pathways exist to mediate the upstream signals and to regulate mTORC1 activity.

Different sources of upstream signals are relayed to mTORC1 through a variety of signaling pathways. For example, growth factors stimulate mTORC1 through activation of the insulin and Ras signaling pathways. The insulin signaling pathway is initiated by insulin (such as IGF-1) binding to its cell-surface receptor, which stimulates the tyrosine kinase activity of the insulin receptor, and phosphorylates the insulin receptor substrate 1 (IRS1). The phosphorylated IRS-1 activates PI3K to produce phosphatidylinositol (3,4,5)-triphosphate (PtdIns(3,4,5)P₃, or PIP₃). PTEN (phosphatase and tensin homolog) negatively regulates intracellular levels of PIP₃ by dephosphorylating PIP₃ into PIP₂ (PtdIns(4,5)P₂), and thereby inhibiting the insulin signaling pathway. PIP₃ recruits AKT (also known as Protein kinase B, or PKB) to the plasma membrane, and activates AKT by phosphorylation through PDK1 (protein kinase 3-phosphoinositide dependent protein kinase-1). Activated AKT in turn phosphorylates TSC2, leading to inactivation of TSC1/2 and thus the activation of mTORC1. Alternatively, AKT activation can activate mTORC1 by promoting phosphorylation and dissociation of PRAS40 from mTORC1 in a TSC1/2-independent manner.

Growth factor binding to cell-surface receptors may also be signaled to mTORC1 through the Ras signaling pathway. For example, binding of extracellular ligands (such as EGF) can activate a tyrosine kinase receptor (such as an EGFR), leading to phosphorylation of the cytoplasmic domain of the receptor, which recruits docking proteins, such as GRB2, and activation of the guanine nucleotide exchange factor SOS. Activated SOS promotes removal of GDP from Ras, and allows Ras to bind to GTP and become activated. Neurofibromin (NF)-1 is a negative regulator of the Ras pathway by stimulating GTPase activity of Ras. NF-2 is another negative regulator of Ras signaling, acting downstream of the Grb2-SOS complex. Activated Ras activates the downstream protein kinase RAF, which phosphorylates and activates MEK. MEK phosphorylates and activates MAPK (mitogen-activated protein kinase, also known as ERK or extracellular signal-regulated kinases). ERK1/2 can phosphorylate TSC2 directly, or activate p90 ribosomal S6 kinase 1 (RSK1), which in turn phosphorylates TSC2, thereby leading to inactivation of TSC1/2 and activation of mTORC1.

AMP-activated protein kinase (AMPK) is a key sensor for intracellular energy status and a regulator of mTORC1. Among different activation mechanisms in the AMPK pathway, STK11 (serine/threonine kinase 11, also known as LBK1) can serve as a primary upstream kinase of AMPK, which activates AMPK upon energy depletion. Activated AMPK phosphorylates TSC2, which activates the TSC1/2 GAP activity, inactivates Rheb, and thereby reduces mTORC1 activation. AMPK can also directly phosphorylate RAPTOR, which inhibits mTORC1 activity.

Similarly, hypoxia (low oxygen level) can be signaled to mTORC1 through activation of AMPK. Alternatively, hypoxia can activate TSC1/2 through transcriptional regulation of DNA damage response 1 (REDD1). Hypoxia can also reduce mTORC1 signaling by disrupting RHEB-mTOR interaction through PML (promyelocytic leukemia tumor suppressor) or BNIP3 (BCL2/adenovirus E1B 19kDa protein-interacting protein 3).

The amino acids positively regulate mTORC1 activity, and signaling of amino acid deprivation to the mTORC1 can be independent of TSC1/2. RAG proteins, including RAGA, RAGB, RAGC, and RAGD, a family of small GTPases, may bind to RAPTOR in an amino-acid sensitive manner and promote activation of mTORC1.

Additional upstream signals that regulate mTORC1 activity include, but are not limited to, genotoxic stress, inflammation, Wnt ligand and phosphatidic acid (PA). For example, pro-inflammatory cytokines, such as TNFα, activate IκB kinase-β (IKKβ), which inactivates TSC1, leading to mTORC1 activation. Activation of the Wnt pathway may inhibit glycogen synthase kinase 3 (GSK3), which phosphorylates TSC2 and activates TSC1/2, thereby reducing mTORC1 activity.

mTORC2 comprises at least six proteins, including the mTOR protein, rapamycin-insensitive companion of mTOR (RICTOR); mammalian stress-activated protein kinase interacting protein (mSIN1); protein observed with Rictor-1 (PROTOR-1); mLST8, and DEPTOR. mTORC2 is involved in activation of AKT at residue Ser473 and the downstream phosphorylation of some AKT substrates. mTORC2 also regulates cytoskeletal organization, for example, by promoting protein kinase Cα (PKCα) phosphorylation, phosphorylation of paxillin, and the GTP loading of RhoA and RAC1.

The outputs of the mTOR signaling pathway include diverse molecular, cellular and physiological effects. For example, activation of mTORC1 leads to many downstream activities, including promoting biosynthesis of proteins, lipids and organelles (such as mitochondria), and inhibition of autophagy. For example, mTORC1 promotes protein synthesis by phosphorylating the eukaryotic initiation factor 4E (eIF3E)-binding protein 1 (4EBP1) and the p70 ribosomal S6 kinase I (S6K1). Phosphorylated 4EBP1 (p-4EBP1) prevents its binding to eIF4E and enables eIF4E to promote cap-dependent translation. Phosphorylation of S6Kl activates the kinase activity of S6K1, which promotes mRNA biogenesis, cap-dependent translation and elongation, and the translation of ribosomal proteins by regulating the activity of many protein targets, such as S6K1 aly/REF-like target (SKAR), programmed cell death 4 (PDCD4), eukaryotic elongation factor 2 kinase (eEF2K) and ribosomal protein S6. Activated mTORC1 may also phosphorylate and repress ULK1 and ATG13, which represses autophagy. Activation of mTORC2 may lead to activation of the forkhead box protein O1 (FoxO1) and FoxO3a transcription factors, which control the expression of genes involved in stress resistance, metabolism, cell cycle arrest and apoptosis.

### mTOR-associated genes

The biomarkers and the mTOR-activating aberrations described herein are related to mTOR-associated genes. As used herein, "mTOR-associated genes" encode for molecules, such as proteins, that participate in the mTOR signaling pathway. mTOR-associated genes contemplated by the present invention include, but are not limited to, the genes described in the section "mTOR signaling pathway". mTOR-associated genes may function as part of the mTORC1 and/or mTORC2 complex, or mediate the upstream signals to regulate the mTORC1 and/or mTORC2 complex. In some embodiments, the mTOR-associated gene is selected from MTOR, TSC1, TSC2, RHEB, AKT (such as AKT1), PI3K (such as PIK3CA and PIK3CG), PTEN, NF1, NF2, STK11, TP53, FGFR4, BAP1, RAS, SOS, GRB2, IRS1, PDK1, RAF, MEK, ERK1, ERK2, RSK1, GSK3, REDD1, BNIP3, PML, AMPK, RAPTOR, DEPTOR, mLST8, PRAS40, VPS34, RAGA, RAGB, RAGC, RAGD, PAXILLIN, RHOA, RAC1, mSIN1, RICTOR (such as RICTOR-1), PROTOR-1, PKCα, PLD, IKKβ, and combinations thereof. In some embodiments, the mTOR-associated gene is selected from AKT1, FLT3, MTOR, PIK3CA, PIK3CG, TSC1, TSC2, RHEB, STK11, NF1, NF2, PTEN, TP53, FGFR4, KRAS, NRAS, BAP1, and combinations thereof. Exemplary reference (i.e. wildtype) sequences of some mTOR-associated genes and molecules encoded by the mTOR-associated genes (such as RNA and protein) are described below.

### mTOR

mTOR is also known as serine/threonine-protein kinase mTOR, FK506-binding protein 12-rapamycin complex-associated protein 1, FKBP12-rapamycin complex-associated protein, mammalian target of rapamycin, mechanistic target of rapamycin, rapamycin and FKBP12 target 1, rapamycin target protein 1, FRAP, FRAP1, FRAP2, RAFT1, and RAPT1. In some embodiments, the nucleic acid sequence of a wildtype MTOR gene is identified by the Genbank accession number NC_000001.11 from nucleotide 11106531 to nucleotide 11262557 of the reverse strand of chromosome 1 according to the GRCh38.p2 assembly of the human genome. The wildtype MTOR gene comprises 59 exons, and a mutation of the MTOR gene may occur in any one or any combination of the 59 exons, or in any intron or noncoding regions of the MTOR gene.

In some embodiments, the amino acid sequence of a wildtype mTOR protein is identified by the Genbank accession number NP_004949.1. The wildtype mTOR protein comprises various domains, including HEAT repeats, the FAT domain, the FKBP12-rapamyicn binding (FRB) domain, the serine/threonine kinase catalytic domain, and the carboxy-terminal FATC domain. A mutation of the mTOR protein may occur in any one or any combination of the protein domains.

In some embodiments, the nucleic acid sequence of a cDNA encoding a wildtype mTOR protein is identified by the Genbank accession number NM_004958.3.

### AKT

AKT is also known as the protein kinase B (PKB), and the human genome encodes three AKT family members, Akt1, Akt2, and Akt3. The present application contemplates mTOR-activating aberration in any member of the AKT family. In some embodiments, the mTOR-associated gene is AKT1.

AKT1 is also known as the RAC-alpha serine/threonine protein kinase, protein kinase B, protein kinase B alpha, PKB alpha, proto-oncogene c-Akt, AKT, RAC, CWS6, PRKBA, and RAC-alpha. In some embodiments, the nucleic acid sequence of a wildtype AKT1 gene is identified by the Genbank accession number NC_000014.9, from nucleotide 104769349 to nucleotide 104795743 of the reverse strand of chromosome 14 according to the GRCh38.p2 assembly of the human genome. The wildtype AKT1 gene comprises 17 exons. A mutation of the AKT1 gene may occur in any one or any combination of the 17 exons, or in any intron or noncoding regions of the AKT1 gene.

In some embodiments, the amino acid sequence of a wildtype AKT1 protein is identified by the Genbank accession number NP_001014431.1. The wildtype AKT1 protein comprise various domains, including a PH domain, a protein kinase domain, and an AGC-kinase C-terminal domain. A mutation of the AKT1 protein may occur in any one or any combination of the protein domains.

In some embodiments, the nucleic acid sequence of a cDNA encoding a wildtype AKT1 protein is identified by the Genbank accession number NM_001014431.1. In some embodiments, the nucleic acid sequence of a cDNA encoding a wildtype AKT1 protein is identified by the Genbank accession number NM_001014432.1. In some embodiments, the nucleic acid sequence of a cDNA encoding a wildtype AKT1 protein is identified by the Genbank accession number NM_005163.2.

### PI2K

PI3Ks are a family of related lipid kinases capable of phosphorylating the 3 position hydroxyl group of the inositol ring of phosphatidylinositol. There are four classes of PI3Ks, including Class I, Class II, Class III and Class IV. Class IA PI3K is composed of a heterodimer between a p110 catalytic subunit and a p85 regulatory subunit. The p85 regulatory subunit has five variants, designated p85α, p55α, p50α, p85β, and p55γ. In the human genome, while p85α, p55α and p50α are splice variants encoded by the same gene (PIK3R1), p85β is encoded by the gene PIK3R2 and p55α is encoded by the gene PIK3R3. The p110 catalytic subunit has three variants designated p110α, p110β, and p110δ, which are encoded by three separate genes. The gene PIK3CA encodes p110α, the gene PIK3CB encodes p110β, and the gene PIK3CD encodes p110δ in the human genome. Similar to Class IA PI3K, the Class IB PI3K is composed of a catalytic subunit and a regulatory subunit. While Class IA PI3K is activated by receptor tyrosine kinases (RTKs), Class IB PI3K is activated by G-protein-coupled receptors (GPCRs). The only known class IB PI3K catalytic subunit is p110γ encoded by the gene PIK3CG. There are two known regulatory subunits for p110γ, including p101 and p84/p87PIKAP. The present application contemplates mTOR-activating aberration in any class, member, complex, subunit, variant, or combination of variants of PI3K. In some embodiments, the mTOR-associated gene is PIK3CA. In some embodiments, the mTOR-associated gene is PIK3CG.

PIK3CA is also known as the phosphatidylinositol 4,5-bisphosphate 3-kinase catalytic subunit alpha isoform, PI3-kinase subunit alpha, PI3K-alpha, PtdIns-3-kinase subunit alpha, phosphatidylinositol 4,5-bisphosphate 3-kinase 110 kDa catalytic subunit alpha, PtdIns-3-kinase subunit p110-alpha, p110alpha, MCM, CWS5, MCAP, PI2K, CLOVE, and MCMTC. In some embodiments, the nucleic acid sequence of a wildtype PIK3CA gene is identified by the Genbank accession number NC_000003.12, from nucleotide 179148114 to nucleotide 179240084 of the forward strand of chromosome 3 according to the GRCh38.p2 assembly of the human genome. The wildtype PIK3CA gene comprises 23 exons. A mutation of the PIK3CA gene may occur in any one or any combination of the 23 exons, or in any intron or noncoding regions of the PIK3CA gene.

In some embodiments, the amino acid sequence of a wildtype PIK3CA protein is identified by the Genbank accession number NP_006209.2. The wildtype PIK3CA protein comprise various domains, including a PI3K-ABD domain, a PI3K-RBD domain, a C2-PI3K-type domain, a PIK helical domain and a PI3K/PI4K domain. A mutation of the PIK3CA protein may occur in any one or any combination of the protein domains.

In some embodiments, the nucleic acid sequence of a cDNA encoding a wildtype PIK3CA protein is identified by the Genbank accession number NM_006218.2.

PIK3CG is also known as phosphatidylinositol-4,5-bisphosphate 3-kinase, catalytic subunit gamma; PI3K, PIK3, PI3CG; PI3Ky; p110γ, and p120-PI3K. In some embodiments, the nucleic acid sequence of a wildtype PIK3CG gene is identified by the Genbank accession number NC_000007.14, from nucleotide 106865278 to nucleotide 106908978 of the forward strand of chromosome 7 according to the GRCh38.p2 assembly of the human genome. The wildtype PIK3CG gene comprises 14 exons. A mutation of the PIK3CG gene may occur in any one or any combination of the 14 exons, or in any intron or noncoding regions of the PIK3CG gene.

In some embodiments, the amino acid sequence of a wildtype PIK3CG protein is identified by the Genbank accession number NP_002640.2. The wildtype PIK3CG protein comprise various domains, including a PI3K-ABD domain, a PI3K-RBD domain, a C2-PI3K-type domain, a PIK helical domain and a PI3K/PI4K domain. A mutation of the PIK3CG protein may occur in any one or any combination of the protein domains.

In some embodiments, the nucleic acid sequence of a cDNA encoding a wildtype PIK3CG protein is identified by the Genbank accession number NM_001282426.1. In some embodiments, the nucleic acid sequence of a cDNA encoding a wildtype PIK3CG protein is identified by the Genbank accession number NM_002649.3. In some embodiments, the nucleic acid sequence of a cDNA encoding a wildtype PIK.3CG protein is identified by the Genbank accession number NM_001282427.1.

### TSC1

TSC1 is also known as Hamartin, Tuberous sclerosis 1 protein, TSC, KIAA0243, and LAM. TSC1 protein functions as part of a complex with TSC2 by negatively regulating mTORC1 signaling. In some embodiments, the nucleic acid sequence of a wildtype TSC1 gene is identified by the Genbank accession number NC_000009.12, from nucleotide 132891348 to nucleotide 132945370 on the reverse strand of chromosome 9 according to the GRCh38.p2 assembly of the human genome. The wildtype TSC1 gene comprises 25 exons. A mutation of the TSC1 gene may occur in any one or any combination of the 25 exons, or in any intron or noncoding regions of the TSC1 gene.

In some embodiments, the amino acid sequence of a wildtype TSC1 protein is identified by the Genbank accession number NP_000359.1. In some embodiments, the amino acid sequence of a wildtype TSC1 protein is identified by the Genbank accession number NP_ 001155898.1. In some embodiments, the amino acid sequence of a wildtype TSC1 protein is identified by the Genbank accession number NP_001155899.1.

In some embodiments, the nucleic acid sequence of a cDNA encoding a wildtype TSC1 protein is identified by the Genbank accession number NM_000368.4. In some embodiments, the nucleic acid sequence of a cDNA encoding a wildtype TSC1 protein is identified by the Genbank accession number NM_001162.426.1. In some embodiments, the nucleic acid sequence of a cDNA encoding a wildtype TSC1 protein is identified by the Genbank accession number NM_001162427.1.

### TSC2

TSC2 is also known as Tuberin, Tuberous sclerosis 2 protein, protein phosphatase 1 regulatory subunit 160, TSC4, PPP1R160, and LAM. TSC2 protein functions as part of a complex with TSC1 by negatively regulating mTORCT1 signaling. In some embodiments, the nucleic acid sequence of a wildtype TSC2 gene is identified by the Genbank accession number NC_000016.10, from nucleotide 2047936 to nucleotide 2088712 on the forward strand of chromosome 16 according to the GRCh38.p2 assembly of the human genome. The wildtype TSC2 gene comprises 42 exons. A mutation of the TSC2 gene may occur in any one or any combination of the 42 exons, or in any intron or noncoding regions of the TSC2 gene.

In some embodiments, the amino acid sequence of a wildtype TSC2 protein is identified by the Genbank accession number NP_000539.2. In some embodiments, the amino acid sequence of a wildtype TSC2 protein is identified by the Genbank accession number NP_001070651.1. In some embodiments, the amino acid sequence of a wildtype TSC2 protein is identified by the Genbank accession number NP_001107854.1.

In some embodiments, the nucleic acid sequence of a cDNA encoding a wildtype TSC2 protein is identified by the Genbank accession number NM_000548.3. In some embodiments, the nucleic acid sequence of a cDNA encoding a wildtype TSC2 protein is identified by the Genbank accession number NM _001077183.1. In some embodiments, the nucleic acid sequence of a cDNA encoding a wildtype TSC2 protein is identified by the Genbank accession number NM_001114382.1.

### RHEB

RHEB is a member of the small GTPase superfamily that shuttles between a GDP-bound inactive form and a GTP-bound active from to regulate mTORC1 signaling. The human genome also has three pseudogenes of RHEB, including RHEBP1 on chromosome 10. Additionally, the RHEBL1 (Ras homolog enriched in brain like-1) gene encodes a homolog of RHEB, which is also a downstream target of the TSC1/2 complex and promotes signal transduction through mTOR. The present application contemplates mTOR-activating aberrations in all RHEB-related genes, including RHEB, RHEB pseudogenes, and RHEBL1. In some embodiments, the mTOR-associated gene is RHEB.

RHEB is also known as the Ras homolog enriched in brain, GTP-binding protein Rheb and RHEB2. In some embodiments, the nucleic acid sequence of a wildtype RHEB gene is identified by the Genbank accession number NC_000007.14 from nucleotide 151466012 to nucleotide 151519924 of the reverse strand of chromosome 7 according to the GRCh38.p2 assembly of the human genome. The wildtype RHEB gene comprises 9 exons. A mutation of the RHEB gene may occur in any one or any combination of the 9 exons, or in any intron or noncoding regions of the RHEB gene.

In some embodiments, the amino acid sequence of a wildtype RHEB protein is identified by the Genbank accession number NP_ 005605.1. In some embodiments, the nucleic acid sequence of a cDNA encoding a wildtype RHEB protein is identified by the Genbank accession number NM_005614.3.

### STK11

STK11 is also known as the serine/threonine-protein kinase STK11, liver kinase B1, renal carcinoma antigen NY-REN-19, PJS, LKB1, and hLKB1. In some embodiments, the nucleic acid sequence of a wildtype STK11 gene is identified by the Genbank accession number NC_000019.10 from nucleotide 1205799 to nucleotide 1228435 of the forward strand of chromosome 19 according to the GRCh38.p2 assembly of the human genome. The wildtype STK11 gene comprises 13 exons. A mutation of the STK11 gene may occur in any one or any combination of the 13 exons, or in any intron or noncoding regions of the STK11 gene.

In some embodiments, the amino acid sequence of a wildtype STK11 protein is identified by the Genbank accession number NP_000446.1. In some embodiments, the nucleic acid sequence of a cDNA encoding a wildtype STK11 protein is identified by the Genbank accession number NM_000455.4.

### NFl

NF1 is also known as the neurofibromatosis-related protein, neurofibromin 1, WSS, NFNS, and VRNF. In some embodiments, the nucleic acid sequence of a wildtype NF1 gene is identified by the Genbank accession number NC_000017.11 from nucleotide 31007873 to nucleotide 31377677 of the forward strand of chromosome 17 according to the GRCh38.p2 assembly of the human genome. The wildtype NF1 gene comprises 73 exons. A mutation of the NF1 gene may occur in any one or any combination of the 73 exons, or in any intron or noncoding regions of the NF1 gene.

In some embodiments, the amino acid sequence of a wildtype NF1 protein is identified by the Genbank accession number NP_001035957.1. In some embodiments, the amino acid sequence of a wildtype NF1 protein is identified by the Genbank accession number NP_000258.1. In some embodiments, the amino acid sequence of a wildtype NF1 protein is identified by the Genbank accession number NP_001121619.1. In some embodiments, the wildtype NF1 is a naturally truncated NF1 protein lacking the C-terminal 1534 amino acids from the full-length NF1 protein. The NF1 protein comprises a Ras-GAP domain and a CRAL-TRIO domain. A mutation of the NF1 protein may occur in either one or both of the protein domains.

In some embodiments, the nucleic acid sequence of a cDNA encoding a wildtype NF1 protein is identified by the Genbank accession number NM_001042492.2. In some embodiments, the nucleic acid sequence of a cDNA encoding a wildtype NF1 protein is identified by the Genbank accession number NM_000267.3. In some embodiments, the nucleic acid sequence of a cDNA encoding a wildtype NF1 protein is identified by the Genbank accession number NM_001128147.2. In some embodiments, the wildtype mRNA encoding NF1 protein is subject to RNA editing (CGA>UGA→Arg1306Term), resulting in premature translation termination and producing a naturally truncated NF1 protein.

### NF2

NF2 is also known as Merlin, Moesin-ezrin-radixin-like protein, neurofibromin-2, Schwannomerlin, Schwannomin, SCH, CAN, and BANF. In some embodiments, the nucleic acid sequence of a wildtype NF2 gene is identified by the Genbank accession number NC_ 000022.11 from nucleotide 29603556 to nucleotide 29698600 of the forward strand of chromosome 22 according to the GRCh38.p2 assembly of the human genome. The wildtype NF2 gene comprises 18 exons. A mutation of the NF2 gene may occur in any one or any combination of the 18 exons, or in any intron or noncoding regions of the NF2 gene.

In some embodiments, the amino acid sequence of a wildtype NF2 protein is identified by the Genbank accession number NP_000259.1. In some embodiments, the amino acid sequence of a wildtype NF2 protein is identified by the Genbank accession number NP_057502.2. In some embodiments, the amino acid sequence of a wildtype NF2 protein is identified by the Genbank accession number NP_861546.1. In some embodiments, the amino acid sequence of a wildtype NF2 protein is identified by the Genbank accession number NP_861966.1. In some embodiments, the amino acid sequence of a wildtype NF2 protein is identified by the Genbank accession number NP_861967.1. In some embodiments, the amino acid sequence of a wildtype NF2 protein is identified by the Genbank accession number NP_861968.1. In some embodiments, the amino acid sequence of a wildtype NF2 protein is identified by the Genbank accession number NP_861969.1. In some embodiments, the amino acid sequence of a wildtype NF2 protein is identified by the Genbank accession number NP_861970.1. In some embodiments, the amino acid sequence of a wildtype NF2 protein is identified by the Genbank accession number NP_861971.1.

In some embodiments, the nucleic acid sequence of a cDNA encoding a wildtype NF2 protein is identified by the Genbank accession number NM_000268.3. In some embodiments, the nucleic acid sequence of a cDNA encoding a wildtype NF2 protein is identified by the Genbank accession number NM_016418.5. In some embodiments, the nucleic acid sequence of a cDNA encoding a wildtype NF2 protein is identified by the Genbank accession number NM_181825.2. In some embodiments, the nucleic acid sequence of a cDNA encoding a wildtype NF2 protein is identified by the Genbank accession number NM_181828.2. In some embodiments, the nucleic acid sequence of a cDNA encoding a wildtype NF2 protein is identified by the Genbank accession number NM_181829.2. In some embodiments, the nucleic acid sequence of a cDNA encoding a wildtype NF2 protein is identified by the Genbank accession number NM_181830.2. In some embodiments, the nucleic acid sequence of a cDNA encoding a wildtype NF2 protein is identified by the Genbank accession number NM_181831.2. In some embodiments, the nucleic acid sequence of a cDNA encoding a wildtype NF2 protein is identified by the Genbank accession number NM _181832.2. In some embodiments, the nucleic acid sequence of a cDNA encoding a wildtype NF2 protein is identified by the Genbank accession number NM_181833.2.

### PTEN

PTEN is also known as the phosphatidylinositol 3,4,5-triphosphate 3-phosphtase and dual-specificity phosphatase PTEN, mutated in multiple advanced cancers 1, phosphatase and tensin homolog, MMAC1, TEP1, BZS, DEC, CWS1, GLM2, MHAM, and PTEN1. In some embodiments, the nucleic acid sequence of a wildtype PTEN gene is identified by the Genbank accession number NC_000010.11 from nucleotide 87863438 to nucleotide 87971930 of the forward strand of chromosome 10 according to the GRCh38.p2 assembly of the human genome. The wildtype PTEN gene comprises 16 exons. A mutation of the PTEN gene may occur in any one or any combination of the 16 exons, or in any intron or noncoding regions of the PTEN gene.

In some embodiments, the amino acid sequence of a wildtype PTEN protein is identified by the Genbank accession number NP_000305.3. In some embodiments, the amino acid sequence of a wildtype PTEN protein is identified by the Genbank accession number NP_001291646.2. In some embodiments, the amino acid sequence of a wildtype PTEN protein is identified by the Genbank accession number NP_00129164 7.1. The wildtype PTEN protein comprises a phosphatase tensin-type domain, and a C2 tensin-type domain. A mutation in the PTEN protein may occur in either one or both protein domains.

In some embodiments, the nucleic acid sequence of a cDNA encoding a wildtype PTEN protein is identified by the Genbank accession number NM_000114.6. In some embodiments, the nucleic acid sequence of a cDNA encoding a wildtype PTEN protein is identified by the Genbank accession number NM_001304717.2. In some embodiments, the nucleic acid sequence of a cDNA encoding a wildtype PTEN protein is identified by the Genbank accession number NM_001304718.1.

### Genes that crosstalk with the mTOR pathway

The mTOR-associated genes that are contemplated by the present application also include genes in pathways that crosstalk with the mTOR pathway, thereby modulating the activity of the mTOR signaling pathway (e.g., mediated through mTORC1 and/or mTORC2). For example, TP53, FGFR4, BAP1, FLT3, KRAS and NRAS are described below as nonlimiting examples of genes that may crosstalk with the mTOR pathway.

TP53, also known as tumor protein p53, P53, BCC7, LFS1 or TRP53, is a tumor suppressor protein that responds to diverse cellular stresses to regulate expression of target genes, thereby inducing cell cycle arrest, apoptosis, senescence, DNA repair, or changes in metabolism. TP53 crosstalks with the mTOR signaling pathway by inhibiting mTOR activity. In some embodiments, the nucleic acid sequence of a wildtype TP53 gene is identified by the Genbank accession number NC_000017.11 from nucleotide 7668402 to nucleotide 7687550 of the complement strand of chromosome 17 according to the GRCh38.p2 assembly of the human genome. The wildtype TP53 gene comprises 12 exons. A mutation of the TP53 gene may occur in any one or any combination of the 12 exons, or in any intron or noncoding regions of the TP53 gene. The wildtype protein encoded by TP53 includes multiple isoforms, such as isoforms a-1. A mutation may affect any of the of TP53 isoforms. In some embodiments, the amino acid sequence of a wildtype TP53 protein is identified by the Genbank accession number NP_000537.3. In some embodiments, the nucleic acid sequence of a cDNA encoding a wildtype TP53 protein is identified by the Genbank accession number NM_000546.5.

FGFR4 is also known as fibroblast growth factor receptor 4, TKF, JTK2, and CD334. FGFR4 is a member of the fibroblast growth factor receptor family. The extracellular domain of the protein encoded by FGFR4 interacts with fibroblast growth factors, and initiates a cascade of downstream signals that are involved in mitogenesis and differentiation. FGFR4 crosstalks with the mTOR signaling pathway. For example, RAS is known as a common regulator of FGFR4 and mTOR. In some embodiments, the nucleic acid sequence of a wildtype FGFR4 gene is identified by the Genbank accession number NC_000005.10 from nucleotide 177086872 to nucleotide 177098142 of the forward strand of chromosome 5 according to the GRCh38.p2 assembly of the human genome. The wildtype FGFR4 gene comprises 19 exons. A mutation of the FGFR4 gene may occur in any one or any combination of the 19 exons, or in any intron or noncoding regions of the FGFR4 gene. In some embodiments, the amino acid sequence of a wildtype TP53 protein is identified by the Genbank accession number NP_002002.3. In some embodiments, the nucleic acid sequence of a cDNA encoding a wildtype FGFR4 protein is identified by the Genbank accession number NM_002011.4.

BAP1 is also known as BRCA1 associated protein-1, UCHL2, hucep-6 or HUCEP-13. BAP1 belongs to the ubiquitin C-terminal hydrolase subfamily of deubiquitinating enzymes that are involved in the removal of ubiquitin from proteins. The encoded enzyme binds to the breast cancer type 1 susceptibility protein (BRCA1) via the RING finger domain of the latter and acts as a tumor suppressor. In addition, the enzyme may be involved in regulation of transcription, regulation of cell cycle and growth, response to DNA damage and chromatin dynamics. In some embodiments, the nucleic acid sequence of a wildtype BAP1 gene is identified by the Genbank accession number NC_000003.12 from nucleotide 52401004 to nucleotide 52410105 of the complement strand of chromosome 3 according to the GRCh38.p2 assembly of the human genome. The wildtype BAPl gene comprises 17 exons. A mutation of the BAP1 gene may occur in any one or any combination of the 17 exons, or in any intron or noncoding regions of the BAP1 gene. In some embodiments, the amino acid sequence of a wildtype BAP1 protein is identified by the Genbank accession number NP_004647.1. In some embodiments, the nucleic acid sequence of a cDNA encoding a wildtype BAP1 protein is identified by the Genbank accession number NM_004656.3.

FLT3 is also known as fins-related tyrosine kinase 3, FLK2, STK1, CD135 or FLK-2. FLT3 encodes a class III receptor tyrosine kinase. In some embodiments, the nucleic acid sequence of a wildtype FLT3 gene is identified by the Genbank accession number NC_ 000013.11 from nucleotide 28003274 to nucleotide 28100592, of the complement strand of chromosome 13 according to the GRCh38.p2 assembly of the human genome. The wildtype FLT3 gene comprises 27 exons. A mutation of the FLT3 gene may occur in any one or any combination of the 27 exons, or in any intron or noncoding regions of the FLT3 gene. In some embodiments, an amino acid encoding a FLT3 protein is identified by Genbank accession number NP_004110.2. In some embodiments, the nucleic acid sequence of a cDNA encoding a wildtype NRAS protein is identified by Genbank accession number NM_004119.2.

KRAS is also known as Kirsten rat sarcoma viral oncogene homology, NS, NS3, CFC2, KRAS1, KRAS2, RASK2, KI-RAS, C-K-RAS, K-RAS2A, K-RAS2B, K-RAS4A, or K-RAS4B. In some embodiments, the nucleic acid sequence of a wildtype KRAS gene is identified by the Genbank accession number NC_000012.12 from nucleotide 25204789 to nucleotide 25250931 of the complement strand of chromosome 12 according to the GRCh38.p2 assembly of the human genome. The wildtype KRAS gene comprises 6 exons. A mutation of the KRAS gene may occur in any one or any combination of the 6 exons, or in any intron or noncoding regions of the KRAS gene. In some embodiments, an amino acid encoding a KRAS protein is identified by Genbank accession number NP_004976.2. In other embodiments, an amino acid encoding a KRAS protein is identified by Genbank accession number NP_203524.1. In some embodiments, the nucleic acid sequence of a cDNA encoding a wildtype KRAS protein is identified by Genbank accession number NM_004985.3. In other embodiments, the nucleic acid sequence of a cDNA encoding a wildtype KRAS protein is identified by Genbank accession number NM_033360.2.

NRAS is also known as neuroblastoma RAS viral (v-ras) oncogene homolog, NS6, CMNS, NCMS, ALPS4, N-ras or NRAS 1. In some embodiments, the nucleic acid sequence of a wildtype NRAS gene is identified by the Genbank accession number NC_000001.11 from nucleotide 114704464 to nucleotide114716894, of the complement strand of chromosome 1 according to the GRCh38.p2 assembly of the human genome. The wildtype NRAS gene comprises 7 exons. A mutation of the NRAS gene may occur in any one or any combination of the 7 exons, or in any intron or noncoding regions of the NRAS gene. In some embodiments, an amino acid encoding a NRAS protein is identified by Genbank accession number NP_002515.1. In some embodiments, the nucleic acid sequence of a cDNA encoding a wildtype NRAS protein is identified by Genbank accession number NM_002524.4.

### mTOR-activating aberrations

The present application contemplates mTOR-activating aberrations in any one or more mTOR-associated genes described above, including deviations from the reference sequences (i.e. genetic aberrations), abnormal expression levels and/or abnormal activity levels of the one or more mTOR-associated genes. The present application encompasses treatments and methods based on the status of any one or more of the mTOR-activating aberrations disclosed herein.

The mTOR-activating aberrations described herein are associated with an increased (i.e. hyperactivated) mTOR signaling level or activity level. The mTOR signaling level or mTOR activity level described in the present application may include mTOR signaling in response to any one or any combination of the upstream signals described above, and may include mTOR signaling through mTORC1 and/or mTORC2, which may lead to measurable changes in any one or combinations of downstream molecular, cellular or physiological processes (such as protein synthesis, autophagy, metabolism, cell cycle arrest, apoptosis etc.). In some embodiments, the mTOR-activating aberration hyperactivates the mTOR activity by at least about any one of 10%, 20%, 30%, 40%, 60%, 70%, 80%, 90%, 100%, 200%, 500% or more above the level of mTOR activity without the mTOR-activating aberration. In some embodiments, the hyperactivated mTOR activity is mediated by mTORC1 only. In some embodiments, the hyperactivated mTOR activity is mediated by mTORC2 only. In some embodiments, the hyperactivated mTOR activity is mediated by both mTORC1 and mTORC2.

Methods of determining mTOR activity are known in the art. See, for example, Brian CG et al., Cancer Discovery, 2014, 4:554-563. The mTOR activity may be measured by quantifying any one of the downstream outputs (e.g. at the molecular, cellular, and/or physiological level) of the mTOR signaling pathway as described above. For example, the mTOR activity through mTORC1 may be measured by determining the level of phosphorylated 4EBP1 (e.g. P-S65-4EBP1), and/or the level of phosphorylated S6K1 (e.g. P-T389-S6K1), and/or the level of phosphorylated AKT1 (e.g. P-S473-AKT1). The mTOR activity through mTORC2 may be measured by determining the level of phosphorylated FoxO1 and/or FoxO3a. The level of a phosphorylated protein may be determined using any method known in the art, such as Western blot assays using antibodies that specifically recognize the phosphorylated protein of interest.

Candidate mTOR-activating aberrations may be identified through a variety of methods, for example, by literature search or by experimental methods known in the art, including, but not limited to, gene expression profiling experiments (e.g. RNA sequencing or microarray experiments), quantitative proteomics experiments, and gene sequencing experiments. For example, gene expression profiling experiments and quantitative proteomics experiments conducted on a sample collected from an individual having hyperplasia (such as cancer, restenosis or pulmonary hypertension) compared to a control sample may provide a list of genes and gene products (such as RNA, protein, and phosphorylated protein) that are present at aberrant levels. In some instances, gene sequencing (such as exome sequencing) experiments conducted on a sample collected from an individual having hyperplasia (such as cancer, restenosis or pulmonary hypertension) compared to a control sample may provide a list of genetic aberrations. Statistical association studies (such as genome-wide association studies) may be performed on experimental data collected from a population of individuals having hyperplasia to associate aberrations (such as aberrant levels or genetic aberrations) identified in the experiments with hyperplasia. In some embodiments, targeted sequencing experiments (such as the ONCOPANEL^{™} test) are conducted to provide a list of genetic aberrations in an individual having hyperplasia (such as cancer, restenosis, or pulmonary hypertension).

The ONCOPANEL^{™} test can be used to survey exonic DNA sequences of cancer related genes and intronic regions for detection of genetic aberrations, including somatic mutations, copy number variations and structural rearrangements in DNA from various sources of samples (such as a tumor biopsy or blood sample), thereby providing a candidate list of genetic aberrations that may be mTOR-activating aberrations. In some embodiments, the mTOR-associated gene aberration is a genetic aberration or an aberrant level (such as expression level or activity level) in a gene selected from the ONCOPANEL^{™} test. See, for example, Wagle N. et al. Cancer discovery 2.1 (2012): 82-93.

An exemplary version of ONCOPANEL^{™} test includes 300 cancer genes and 113 introns across 35 genes. The 300 genes included in the exemplary ONCOPANEL^{™} test are: ABL1, AKT1, AKT2, AKT3, ALK, ALOX12B, APC, AR, ARAF, ARID1A, ARID1B, ARID2, ASXL1, ATM, ATRX, AURKA, AURKB, AXL, B2M, BAP1, BCL2, BCL2L1, BCL2L12, BCL6, BCOR, BCORL1, BLM, BMPRIA, BRAF, BRCA1, BRCA2, BRD4, BRIP1, BUB1B, CADM2, CARD11, CBL, CBLB, CCND1, CCND2, CCND3, CCNE1, CD274, CD58, CD79B, CDC73, CDH1, CDK1, CDK2, CDK4, CDK5, CDK6, CDK9, CDKN1A, CDKN1B, CDKN1C, CDKN2A, CDKN2B, CDKN2C, CEBPA, CHEK2, CIITA, CREBBP, CRKL, CRLF2, CRTC1, CRTC2, CSF1R, CSF3R, CTNNB1, CUX1, CYLD, DDB2, DDR2, DEPDC5, DICER1, DIS3, DMD, DNMT3A, EED, EGFR, EP300, EPHA3, EPHA5, EPHA7, ERBB2, ERBB3, ERBB4, ERCC2, ERCC3, ERCC4, ERCC5, ESR1, ETV1, ETV4, ETV5, ETV6, EWSR1, EXT1, EXT2, EZH2, FAM46C, FANCA, FANCC, FANCD2, FANCE, FANCF, FANCG, FAS, FBXW7, FGFR1, FGFR2, FGFR3, FGFR4, FH, FKBP9, FLCN, FLT1, FLT3, FLT4, FUS, GATA3, GATA4, GATA6, GLI1, GLI2, GLI3, GNA11, GNAQ, GNAS, GNB2L1, GPC3, GSTM5, H3F3A, HNF1A, HRAS, ID3, IDH1, IDH2, IGF1R, IKZF1, IKZF3, INSIG1, JAK2, JAK3, KCNIP1, KDM5C, KDM6A, KDM6B, KDR, KEAP1, KIT, KRAS, LINC00894, LMO1, LMO2, LMO3, MAP2Kl, MAP2K4, MAP3K1, MAPK1, MCL1, MDM2, MDM4, MECOM, MEF2B, MEN1, MET, MITF, MLH1, MLL (KMT2A), MLL2 (KTM2D), MPL, MSH2, MSH6, MTOR, MUTYH, MYB, MYBL1, MYC, MYCL1 (MYCL), MYCN, MYD88, NBN, NEGR1, NF1, NF2, NFE2L2, NFKBIA, NFKBIZ, NKX2-1, NOTCH1, NOTCH2, NPM1, NPRL2, NPRL3, NRAS, NTRK1, NTRK2, NTRK3, PALB2, PARK2, PAX5, PBRM1, PDCD1LG2, PDGFRA, PDGFRB, PHF6, PHOX2B, PIK3C2B, PIK3CA, PIK3R1, PIM1, PMS1, PMS2, PNRC1, PRAME, PRDM1, PRF1, PRKAR1A, PRKCI, PRKCZ, PRKDC, PRPF40B, PRPF8, PSMD13, PTCH1, PTEN, PTK2, PTPN11, PTPRD, QKI, RAD21, RAF1, RARA, RB1, RBL2, RECQL4, REL, RET, RFWD2, RHEB, RHPN2, ROS1, RPL26, RUNX1, SBDS, SDHA, SDHAF2, SDHB, SDHC, SDHD, SETBP1, SETD2, SF1, SF3B1, SH2B3, SLITRK6, SMAD2, SMAD4, SMARCA4, SMARCB1, SMC1A, SMC3, SMO, SOCS1, SOX2, SOX9, SQSTM1, SRC, SRSF2, STAG1, STAG2, STAT3, STAT6, STK11, SUFU, SUZ12, SYK, TCF3, TCF7L1, TCF7L2, TERC, TERT, TET2, TLR4, TNFAIP3, TP53, TSC1, TSC2, U2AF1, VHL, WRN, WT1, XPA, XPC, XPO1, ZNF217, ZNF708, ZRSR2. The intronic regions surveyed in the exemplary ONCOPANEL^{™} test are tiled on specific introns of ABL1, AKT3, ALK, BCL2, BCL6, BRAF, CIITA, EGFR, ERG, ETV1, EWSR1, FGFR1, FGFR2, FGFR3, FUS, IGH, IGL, JAK2, MLL, MYC, NPM1, NTRK1, PAX5, PDGFRA, PDGFRB, PPARG, RAF1, RARA, RET, ROS1, SS18, TRA, TRB, TRG, TMPRSS2. mTOR-activating aberrations (such as genetic aberration and aberrant levels) of any of the genes included in any embodiment or version of the ONCOPANEL^{™} test, including, but not limited to the genes and intronic regions listed above, are contemplated by the present application to serve as a basis for selecting an individual for treatment with the mTOR inhibitor nanoparticle compositions.

Whether a candidate genetic aberration or aberrant level is an mTOR-activating aberration can be determined with methods known in the art. Genetic experiments in cells (such as cell lines) or animal models may be performed to ascertain that the hyperplasia-associated aberrations identified from all aberrations observed in the experiments are mTOR-activating aberrations. For example, a genetic aberration may be cloned and engineered in a cell line or animal model, and the mTOR activity of the engineered cell line or animal model may be measured and compared with corresponding cell line or animal model that do not have the genetic aberration. An increase in the mTOR activity in such experiment may indicate that the genetic aberration is a candidate mTOR-activating aberration, which may be tested in a clinical study.

### Genetic aberrations

Genetic aberrations of one or more mTOR-associated genes may comprise a change to the nucleic acid (such as DNA and RNA) or protein sequence (i.e. mutation) or an epigenetic feature associated with an mTOR-associated gene, including, but not limited to, coding, noncoding, regulatory, enhancer, silencer, promoter, intron, exon, and untranslated regions of the mTOR-associated gene.

The genetic aberration may be a germline mutation (including chromosomal rearrangement), or a somatic mutation (including chromosomal rearrangement). In some embodiments, the genetic aberration is present in all tissues, including normal tissue and the hyperplasia tissue, of the individual. In some embodiments, the genetic aberration is present only in the hyperplasia tissue (such as tumor tissue, or abnormally proliferative cells in pulmonary hypertension or restenosis) of the individual. In some embodiments, the genetic aberration is present only in a fraction of the hyperplasia tissue.

In some embodiments, the mTOR-activating aberration comprises a mutation of an mTOR-associated gene, including, but not limited to, deletion, frame shift, insertion, indel, missense mutation, nonsense mutation, point mutation, single nucleotide variation (SNV), silent mutation, splice site mutation, splice variant, and translocation. In some embodiments, the mutation may be a loss of function mutation for a negative regulator of the mTOR signaling pathway or a gain of function mutation of a positive regulator of the mTOR signaling pathway.

In some embodiments, the genetic aberration comprises a copy number variation of an mTOR-associated gene. Normally, there are two copies of each mTOR-associated gene per genome. In some embodiments, the copy number of the mTOR-associated gene is amplified by the genetic aberration, resulting in at least about any of 3, 4, 5, 6, 7, 8, or more copies of the mTOR-associated gene in the genome. In some embodiments, the genetic aberration of the mTOR-associated gene results in loss of one or both copies of the mTOR-associated gene in the genome. In some embodiments, the copy number variation of the mTOR-associated gene is loss of heterozygosity of the mTOR-associated gene. In some embodiments, the copy number variation of the mTOR-associated gene is deletion of the mTOR-associated gene. In some embodiments, the copy number variation of the mTOR-associated gene is caused by structural rearrangement of the genome, including deletions, duplications, inversion, and translocation of a chromosome or a fragment thereof.

In some embodiments, the genetic aberration comprises an aberrant epigenetic feature associated with an mTOR-associated gene, including, but not limited to, DNA methylation, hydroxymethylation, aberrant histone binding, chromatin remodeling, and the like. In some embodiments, the promotor of the mTOR-associated gene is hypermethylated in the individual, for example by at least about any of 10%, 20%, 30%, 40%, 50%, 60%, 70%, 80%, 90%, or more compared to a control level (such as a clinically accepted normal level in a standardized test).

In some embodiments, the mTOR-activating aberration is a genetic aberration (such as a mutation or a copy number variation) in any one of the mTOR-associated genes described above. In some embodiments, the mTOR-activating aberration is a mutation or a copy number variation in one or more genes selected from AKT1, FLT3, MTOR, PIK3CA, PIK3CG, TSC1, TSC2, RHEB, STK11, NF1, NF2, PTEN, TP53, FGFR4, KRAS, NRAS, and BAP1.

Genetic aberrations in mTOR-associated genes have been identified in various human cancers, including hereditary cancers and sporadic cancers. For example, germline inactivating mutations in TSC1/2 cause tuberous sclerosis, and patients with this condition are present with lesions that include skin and brain hamartomas, renal angiomyolipomas, and renal cell carcinoma (RCC) (Krymskaya VP et al. 2011 FASEB Journal 25(6): 1922-1933). PTEN hamartoma tumor syndrome (PHTS) is linked to inactivating germline PTEN mutations and is associated with a spectrum of clinical manifestations, including breast cancer, endometrial cancer, follicular thyroid cancer, hamartomas, and RCC (Legendre C. et al. 2003 Transplantation proceedings 35(3 Suppl): 151 S-153S). In addition, sporadic kidney cancer has also been shown to harbor somatic mutations in several genes in the PI3K-Akt-mTOR pathway (e.g. AKT1, MTOR, PIK3CA, PTEN, RHEB, TSC1, TSC2) (Power LA, 1990 Am. J. Hosp. Pharm. 475.5: 1033-1049; Badesch DB et al. 2010 Chest 137(2): 376-3871; Kim JC & Steinberg GD, 2001, The Journal of urology, 165(3): 745-756; McKiernan J. et al. 2010, J. Urol. 183(Suppl 4)). Of the top 50 significantly mutated genes identified by the Cancer Genome Atlas in clear cell renal cell carcinoma, the mutation rate is about 17% for gene mutations that converge on mTORC1 activation (Cancer Genome Atlas Research Network. "Comprehensive molecular characterization of clear cell renal cell carcinoma." 2013 Nature 499: 43-49). Genetic aberrations in mTOR-associated genes have been found to confer sensitivity in individuals having cancer to treatment with a limus drug. See, for example, Wagle et al., N. Eng. J. Med. 2014, 371:1426-33; Iyer et al., Science 2012, 338: 221; Wagle et al. Cancer Discovery 2014, 4:546-553; Grabiner et al., Cancer Discovery 2014, 4:554-563; Dickson et al. Int J. Cancer 2013, 132(7): 1711-1717, and Lim et al, J Clin. Oncol. 33, 2015 suppl; abstr 11010. Genetic aberrations of mTOR-associated genes described by the above references are incorporated herein. Exemplary genetic *aberrations* in some mTOR-associated genes are described below, and it is understood that the present application is not limited to the exemplary genetic aberrations described herein.

In some embodiments, the mTOR-activating aberration comprises a genetic aberration in MTOR. In some embodiments, the genetic aberration comprises an activating mutation of MTOR. In some embodiments, the activating mutation of MTOR is at one or more positions (such as about any one of 1, 2, 3, 4, 5, 6, or more positions) in the protein sequence of MTOR selected from the group consisting of N269, L1357, N1421, L1433, A1459, L1460, C1483, E1519, K1771, E1799, F1888, 11973, T1977, V2006, E2014, 12017, N2206, L2209, A2210, S2215, L2216, R2217, L2220, Q2223, A2226, E2419, L2431, 12500, R2505, and D2512. In some embodiments, the activating mutation of MTOR is one or more missense mutations (such as about any one of 1, 2, 3, 4, 5, 6, or more mutations) selected from the group consisting of N269S, L1357F, N1421D, L1433S, A1459P, L1460P, C1483F, C1483R, C1483W, C1483Y, E1519T, K1771R, E1799K, F18881, F1888I L, 11973F, T1977R, T1977K, V2006I, E2014K, I2017T, N2206S, L2209V, A2210P, S2215Y, S2215F, S2215P, L2216P, R2217W, L2220F, Q2223K, A2226S, E2419K, L2431P, I2500M, R2505P, and D2512H. In some embodiments, the activating mutation of MTOR disrupts binding of MTOR with RHEB. In some embodiments, the activating mutation of MTOR disrupts binding of MTOR with DEPTOR.

In some embodiments, the mTOR-activating aberration comprises a genetic aberration in TSC1 or TSC2. In some embodiments, the genetic aberration comprises a loss of heterozygosity of TSC1 or TSC2. In some embodiments, the genetic aberration comprises a loss of function mutation in TSC1 or TSC2. In some embodiments, the loss of function mutation is a frameshift mutation or a nonsense mutation in TSC1 or TSC2. In some embodiments, the loss of function mutation is a frameshift mutation c.1907_1908del in TSC1. In some embodiments, the loss of function mutation is a splice variant of TSC1: c.1019+1G>A. In some embodiments, the loss of function mutation is the nonsense mutation c.1073G>A in TSC2, and/or p.Trp103^{*} in TSC1. In some embodiments, the loss of function mutation comprises a missense mutation in TSC1 or in TSC2. In some embodiments, the missense mutation is in position A256 of TSC1, and/or position Y719 of TSC2. In some embodiments, the missense mutation comprises A256V in TSClor Y719H in TSC2.

In some embodiments, the mTOR-activating aberration comprises a genetic aberration in RHEB. In some embodiments, the genetic aberration comprises a loss of function mutation in RHEB. In some embodiments, the loss of function mutation is at one or more positions in the protein sequence of RHEB selected from Y35 and E139. In some embodiments, the loss of function mutation in RHEB is selected from Y35N, Y35C, Y35H and E139K.

In some embodiments, the mTOR-activating aberration comprises a genetic aberration in NF1. In some embodiments, the genetic aberration comprises a loss of function mutation in NF1. In some embodiments, the loss of function mutation in NF1 is a missense mutation at position D1644 in NF1. In some embodiments, the missense mutation is D1644A in NF1.

In some embodiments, the mTOR-activating aberration comprises a genetic aberration in NF2. In some embodiments, the genetic aberration comprises a loss of function mutation in NF2. In some embodiments, the loss of function mutation in NF2 is a nonsense mutation. In some embodiments, the nonsense mutation in NF2 is c.863C>G.

In some embodiments, the mTOR-activating aberration comprises a genetic aberration in PTEN. In some embodiments, the genetic aberration comprises a deletion of PTEN in the genome.

In some embodiments, the mTOR-activating aberration comprises a genetic aberration in PI3K. In some embodiments, the genetic aberration comprises a loss of function mutation in PIK3CA or PIK3CG. In some embodiments, the loss of function mutation comprises a missense mutation at a position in PIK3CA selected from the group consisting of E542, 1844, and H1047. In some embodiments, the loss of function mutation comprises a missense in PIK3CA selected from the group consisting of E542K, I844V, and H1047R.

In some embodiments, the mTOR-activating aberration comprises a genetic aberration in AKT1. In some embodiments, the genetic aberration comprises an activating mutation in AKT1. In some embodiments, the activating mutation is a missense mutation in position H238 in AKT1. In some embodiments, the missense mutation is H238Y in AKT1.

In some embodiments, the mTOR-activating aberration comprises a genetic aberration in TP53. In some embodiments, the genetic aberration comprises a loss of function mutation in TP53. In some embodiments, the loss of function mutation is a frameshift mutation in TP53, such as A39fs*5.

In some embodiments, the mTOR-activating aberration comprises a genetic aberration in KRAS. In some embodiments, the mTOR-activating aberration comprises a mutation in exon 2 or exon 3 of the KRAS gene. In some embodiments, the mTOR-activating aberration comprises a KRAS mutation at one or more of the positions selected from the group consisting of G12, G13, S17, P34, Q61, K117 or A146 of the KRAS amino acid sequence. In some embodiments, the mTOR-activating aberration comprises a KRAS mutation selected from the group consisting of G12C, G12S, G12R, G12F, G12L, G12N, G12A, G12D, G12V, G13R, G13C, G13S, G13A, G13D, G13V, G13P, S17G, P34S, Q61K, Q61L, Q61R, Q61H, K117N, A146P, A146T and A146V.

The genetic aberrations of the mTOR-associated genes may be assessed based on a sample, such as a sample from the individual and/or reference sample. In some embodiments, the sample is a tissue sample or nucleic acids extracted from a tissue sample. In some embodiments, the sample is a cell sample (for example a CTC sample) or nucleic acids extracted from a cell sample. In some embodiments, the sample is a tumor biopsy. In some embodiments, the sample is a tumor sample or nucleic acids extracted from a tumor sample. In some embodiments, the sample is a biopsy sample or nucleic acids extracted from the biopsy sample. In some embodiments, the sample is a Formaldehyde Fixed-Paraffin Embedded (FFPE) sample or nucleic acids extracted from the FFPE sample. In some embodiments, the sample is a blood sample. In some embodiments, cell-free DNA is isolated from the blood sample. In some embodiments, the biological sample is a plasma sample or nucleic acids extracted from the plasma sample.

The genetic aberrations of the mTOR-associated gene may be determined by any method known in the art. See, for example, Dickson et al. Int. J. Cancer, 2013, 132(7): 1711-1717; Wagle N. Cancer Discovery, 2014, 4:546-553; and Cancer Genome Atlas Research Network. Nature 2013, 499: 43-49. Exemplary methods include, but are not limited to, genomic DNA sequencing, bisulfite sequencing or other DNA sequencing-based methods using Sanger sequencing or next generation sequencing platforms; polymerase chain reaction assays; in situ hybridization assays; and DNA microarrays. The epigenetic features (such as DNA methylation, histone binding, or chromatin modifications) of one or more mTOR-associated genes from a sample isolated from the individual may be compared with the epigenetic features of the one or more mTOR-associated genes from a control sample. The nucleic acid molecules extracted from the sample can be sequenced or analyzed for the presence of the mTOR-activating genetic aberrations relative to a reference sequence, such as the wildtype sequences of AKT1, MTOR, PIK3CA, PIK3CG, TSC1, TSC2, RHEB, STK11, NF1, NF2, PTEN, TP53, FGFR4, KRAS, NRAS, and/or BAP1 described in the section "mTOR-associated genes".

In some embodiments, the genetic aberration of an mTOR-associated gene is assessed using cell-free DNA sequencing methods. In some embodiments, the genetic aberration of an mTOR-associated gene is assessed using next-generation sequencing. In some embodiments, the genetic aberration of an mTOR-associated gene isolated from a blood sample is assessed using next-generation sequencing. In some embodiments, the genetic aberration of an mTOR-associated gene is assessed using exome sequencing. In some embodiments, the genetic aberration of an mTOR-associated gene is assessed using fluorescence in-situ hybridization analysis. In some embodiments, the genetic aberration of an mTOR-associated gene is assessed prior to initiation of the methods of treatment described herein. In some embodiments, the genetic aberration of an mTOR-associated gene is assessed after initiation of the methods of treatment described herein. In some embodiments, the genetic aberration of an mTOR-associated gene is assessed prior to and after initiation of the methods of treatment described herein.

### Aberrant levels

An aberrant level of an mTOR-associated gene may refer to an aberrant expression level or an aberrant activity level.

Aberrant expression level of an mTOR-associated gene comprises an increase or decrease in the level of a molecule encoded by the mTOR-associated gene compared to the control level. The molecule encoded by the mTOR-associated gene may include RNA transcript(s) (such as mRNA), protein isoform(s), phosphorylated and/or dephosphorylated states of the protein isoform(s), ubiquitinated and/or de-ubiquitinated states of the protein isoform(s), membrane localized (e.g. myristoylated, palmitoylated, and the like) states of the protein isoform(s), other post-translationally modified states of the protein isoform(s), or any combination thereof.

Aberrant activity level of an mTOR-associated gene comprises enhancement or repression of a molecule encoded by any downstream target gene of the mTOR-associated gene, including epigenetic regulation, transcriptional regulation, translational regulation, post-translational regulation, or any combination thereof of the downstream target gene. Additionally, activity of an mTOR-associated gene comprises downstream cellular and/or physiological effects in response to the mTOR-activating aberration, including, but not limited to, protein synthesis, cell growth, proliferation, signal transduction, mitochondria metabolism, mitochondria biogenesis, stress response, cell cycle arrest, autophagy, microtubule organization, and lipid metabolism.

Aberrant levels of mTOR-associated genes (including gene products encoded by mTOR-associated genes) have been associated with hyperplasia, including cancer, restenosis and pulmonary hypertension. For example, mTOR expression was shown to increase as a function of the disease stage in progression from superficial disease to invasive bladder cancer, as evident by activation of pS6-kinase, which was activated in 54 of 70 cases (77%) of T2 muscle-invasive bladder tumors (Seager CM et al, (2009) Cancer Prev. Res. (Phila) 2, 1008-1014). The mTOR signaling pathway is also known to be hyperactivated in pulmonary arterial hypertension.

The levels (such as expression levels and/or activity levels) of an mTOR-associated gene in an individual may be determined based on a sample (e.g., sample from the individual or reference sample). In some embodiments, the sample is from a tissue, organ, cell, or tumor. In some embodiments, the sample is a biological sample. In some embodiments, the biological sample is a biological fluid sample or a biological tissue sample. In further embodiments, the biological fluid sample is a bodily fluid. In some embodiments, the sample is a hyperplasia (such as tumor) tissue, normal tissue adjacent to said hyperplasia (such as tumor) tissue, normal tissue distal to said hyperplasia (such as tumor) tissue, blood sample, or other biological sample. In some embodiments, the sample is a fixed sample. Fixed samples include, but are not limited to, a formalin fixed sample, a paraffin-embedded sample, or a frozen sample. In some embodiments, the sample is a biopsy containing hyperplasia (such as cancer) cells. In a further embodiment, the biopsy is a fine needle aspiration of hyperplasia (such as cancer) cells. In a further embodiment, the biopsy is laparoscopy obtained hyperplasia (such as cancer) cells. In some embodiments, the biopsied cells are centrifuged into a pellet, fixed, and embedded in paraffin. In some embodiments, the biopsied cells are flash frozen. In some embodiments, the biopsied cells are mixed with an antibody that recognizes a molecule encoded by the mTOR-associated gene. In some embodiments, a biopsy is taken to determine whether an individual has hyperplasia (such as cancer, pulmonary hypertension or restenosis) and is then used as a sample. In some embodiments, the sample comprises surgically obtained hyperplasia (such as cancer) cells. In some embodiments, samples may be obtained at different times than when the determining of expression levels of mTOR-associated gene occurs.

In some embodiments, the sample comprises a circulating metastatic cancer cell. In some embodiments, the sample is obtained by sorting circulating tumor cells (CTCs) from blood. In a further embodiment, the CTCs have detached from a primary tumor and circulate in a bodily fluid. In yet a further embodiment, the CTCs have detached from a primary tumor and circulate in the bloodstream. In a further embodiment, the CTCs are an indication of metastasis.

In some embodiments, the level of a protein encoded by an mTOR-associated gene is determined to assess the aberrant expression level of the mTOR-associated gene. In some embodiments, the level of a protein encoded by a downstream target gene of an mTOR-associated gene is determined to assess the aberrant activity level of the mTOR-associated gene. In some embodiments, protein level is determined using one or more antibodies specific for one or more epitopes of the individual protein or proteolytic fragments thereof. Detection methodologies suitable for use in the practice of the invention include, but are not limited to, immunohistochemistry, enzyme linked immunosorbent assays (ELISAs), Western blotting, mass spectroscopy, and immuno-PCR. In some embodiments, levels of protein(s) encoded by the mTOR-associated gene and/or downstream target gene(s) thereof in a sample are normalized (such as divided) by the level of a housekeeping protein (such as glyceraldehyde 3-phosphate dehydrogenase, or GAPDH) in the same sample.

In some embodiments, the level of an mRNA encoded by an mTOR-associated gene is determined to assess the aberrant expression level of the mTOR-associated gene. In some embodiments, the level of an mRNA encoded by a downstream target gene of an mTOR-associated gene is determined to assess the aberrant activity level of the mTOR-associated gene. In some embodiments, a reverse-transcription (RT) polymerase chain reaction (PCR) assay (including a quantitative RT-PCR assay) is used to determine the mRNA levels. In some embodiments, a gene chip or next-generation sequencing methods (such as RNA (cDNA) sequencing or exome sequencing) are used to determine the levels of RNA (such as mRNA) encoded by the mTOR-associated gene and/or downstream target genes thereof. In some embodiments, an mRNA level of the mTOR-associated gene and/or downstream target genes thereof in a sample are normalized (such as divided) by the mRNA level of a housekeeping gene (such as GAPDH) in the same sample.

The levels of an mTOR-associated gene may be a high level or a low level as compared to a control or reference. In some embodiments, wherein the mTOR-associated gene is a positive regulator of the mTOR activity (such as mTORC1 and/or mTORC2 activity), the aberrant level of the mTOR associated gene is a high level compared to the control. In some embodiments, wherein the mTOR-associated gene is a negative regulator of the mTOR activity (such as mTORC1 and/or mTORC2 activity), the aberrant level of the mTOR associated gene is a low level compared to the control.

In some embodiments, the level of the mTOR -associated gene in an individual is compared to the level of the mTOR-associated gene in a control sample. In some embodiments, the level of the mTOR-associated gene in an individual is compared to the level of the mTOR-associated gene in multiple control samples. In some embodiments, multiple control samples are used to generate a statistic that is used to classify the level of the mTOR-associated gene in an individual with hyperplasia (such as cancer, restenosis, or pulmonary hypertension).

The classification or ranking of the level (i.e., high or low) of the mTOR-associated gene may be determined relative to a statistical distribution of control levels. In some embodiments, the classification or ranking is relative to a control sample, such as a normal tissue (*e.g*. peripheral blood mononuclear cells), or a normal epithelial cell sample (*e.g.* a buccal swap or a skin punch) obtained from the individual. In some embodiments, the level of the mTOR-associated gene is classified or ranked relative to a statistical distribution of control levels. In some embodiments, the level of the mTOR-associated gene is classified or ranked relative to the level from a control sample obtained from the individual.

Control samples can be obtained using the same sources and methods as non-control samples. In some embodiments, the control sample is obtained from a different individual (for example an individual not having the hyperplasia, such as cancer, restenosis, or pulmonary hypertension; an individual having a benign or less advanced form of a disease corresponding to the hyperplasia; and/or an individual sharing similar ethnic, age, and gender). In some embodiments when the sample is a tumor tissue sample, the control sample may be a non-cancerous sample from the same individual. In some embodiments, multiple control samples (for example from different individuals) are used to determine a range of levels of the inT'OR-associated genes in a particular tissue, organ, or cell population.

In some embodiments, the control sample is a cultured tissue or cell that has been determined to be a proper control. In some embodiments, the control is a cell that does not have the mTOR-activating aberration. In some embodiments, a clinically accepted normal level in a standardized test is used as a control level for determining the aberrant level of the mTOR-associated gene. In some embodiments, the level of the mTOR-associated gene or downstream target genes thereof in the individual is classified as high, medium or low according to a scoring system, such as an immunohistochemistry-based scoring system.

In some embodiments, the level of the mTOR-associated gene is determined by measuring the level of the mTOR-associated gene in an individual and comparing to a control or reference (*e.g*., the median level for the given patient population or level of a second individual). For example, if the level of the mTOR-associated gene for the single individual is determined to be above the median level of the patient population, that individual is determined to have high expression level of the mTOR-associated gene. Alternatively, if the level of the mTOR-associated gene for the single individual is determined to be below the median level of the patient population, that individual is determined to have low expression level of the mTOR-associated gene. In some embodiments, the individual is compared to a second individual and/or a patient population which is responsive to the treatment. In some embodiments, the individual is compared to a second individual and/or a patient population which is not responsive to the treatment. In some embodiments, the levels are determined by measuring the level of a nucleic acid encoded by the mTOR-associated gene and/or a downstream target gene thereof. For example, if the level of a molecule (such as an mRNA or a protein) encoded by the mTOR-associated gene for the single individual is determined to be above the median level of the patient population, that individual is determined to have a high level of the molecule (such as mRNA or protein) encoded by the mTOR-associated gene. Alternatively, if the level of a molecule (such as an mRNA or a protein) encoded by the mTOR-associated gene for the single individual is determined to be below the median level of the patient population, that individual is determined to have a low level of the molecule (such as mRNA or protein) encoded by the mTOR-associated gene.

In some embodiments, the control level of an mTOR-associated gene is determined by obtaining a statistical distribution of the levels of mTOR-associated gene. In some embodiments, the level of the mTOR-associated gene is classified or ranked relative to control levels or a statistical distribution of control levels.

In some embodiments, bioinformatics methods are used for the determination and classification of the levels of the mTOR-associated gene, including the levels of downstream target genes of the mTOR-associated gene as a measure of the activity level of the mTOR-associated gene. Numerous bioinformatics approaches have been developed to assess gene set expression profiles using gene expression profiling data. Methods include but are not limited to those described in Segal, E. et al. Nat. Genet. 34:66-176 (2003); Segal, E. et al. Nat. Genet. 36:1090-1098 (2004); Barry, W. T. et al. Bioinformatics 21:1943-1949 (2005); Tian, L. et al. Proc Nat'l Acad Sci USA 102:13544-13549 (2005); Novak B A and Jain A N. Bioinformatics 22:233-41 (2006); Maglietta R et al. Bioinformatics 23:2063-72 (2007); Bussemaker H J, BMC Bioinformatics 8 Suppl 6:S6 (2007).

In some embodiments, the control level is a pre-determined threshold level. In some embodiments, mRNA level is determined, and a low level is an mRNA level less than about any of 1, 0.9, 0.8, 0.7, 0.6, 0.5, 0.4, 0.3, 0.2, 0.1, 0.05, 0.02, 0.01, 0.005, 0.002, 0.001 or less time that of what is considered as clinically normal or of the level obtained from a control. In some embodiments, a high level is an mRNA level more than about 1.1, 1.2, 1.3, 1.5, 1.7, 2, 2.2, 2.5, 2.7, 3, 5, 7, 10, 20, 50, 70, 100, 200, 500, 1000 times or more than 1000 times that of what is considered as clinically normal or of the level obtained from a control.

In some embodiments, protein expression level is determined, for example by Western blot or an enzyme-linked immunosorbent assay (ELISA). For example, the criteria for low or high levels can be made based on the total intensity of a band on a protein gel corresponding to the protein encoded by the mTOR-associated gene that is blotted by an antibody that specifically recognizes the protein encoded by the mTOR-associated gene, and normalized (such as divided) by a band on the same protein gel of the same sample corresponding to a housekeeping protein (such as GAPDH) that is blotted by an antibody that specifically recognizes the housekeeping protein (such as GAPDH). In some embodiments, the protein level is low if the protein level is less than about any of 1, 0.9, 0.8, 0.7, 0.6, 0.5, 0.4, 0.3, 0.2, 0.1, 0.05, 0.02, 0.01, 0.005, 0.002, 0.001 or less time of what is considered as clinically normal or of the level obtained from a control. In some embodiments, the protein level is high if the protein level is more than about any of 1.1, 1.2, 1.3, 1.5, 1.7, 2, 2.2, 2.5, 2.7, 3, 5, 7, 10, 20, 50, or 100 times or more than 100 times of what is considered as clinically normal or of the level obtained from a control.

In some embodiments, protein expression level is determined, for example by immunohistochemistry. For example, the criteria for low or high levels can be made based on the number of positive staining cells and/or the intensity of the staining, for example by using an antibody that specifically recognizes the protein encoded by the mTOR-associated gene. In some embodiments, the level is low if less than about 1%, 5%, 10%, 15%, 20%, 25%, 30%, 35%, 40%, 45%, or 50% cells have positive staining. In some embodiments, the level is low if the staining is 1%, 5%, 10%*,* 15%, 20%, 25%, 30%, 35%, 40%, 45%, or 50% less intense than a positive control staining. In some embodiments, the level is high if more than about 40%, 45%, 50%, 55%, 60%, 65%, 70%, 75%, 80%, 85%, or 90%, cells have positive staining. In some embodiments, the level is high if the staining is as intense as positive control staining. In some embodiments, the level is high if the staining is 80%, 85%, or 90% as intense as positive control staining.

In some embodiments, the scoring is based on an "H-score" as described in US Pat. Pub. No. 2013/0005678. An H-score is obtained by the formula: 3 x percentage of strongly staining cells + 2 x percentage of moderately staining cells + percentage of weakly staining cells, giving a range of 0 to 300.

In some embodiments, strong staining, moderate staining, and weak staining are calibrated levels of staining, wherein a range is established and the intensity of staining is binned within the range. In some embodiments, strong staining is staining above the 75th percentile of the intensity range, moderate staining is staining from the 25th to the 75th percentile of the intensity range, and low staining is staining is staining below the 25th percentile of the intensity range. In some aspects one skilled in the art, and familiar with a particular staining technique, adjusts the bin size and defines the staining categories.

In some embodiments, the label high staining is assigned where greater than 50% of the cells stained exhibited strong reactivity, the label no staining is assigned where no staining was observed in less than 50% of the cells stained, and the label low staining is assigned for all of other cases.

In some embodiments, the assessment and/or scoring of the genetic aberration or the level of the mTOR-associated gene in a sample, patient, etc., is performed by one or more experienced clinicians, i.e., those who are experienced with the mTOR-associated gene expression and the mTOR-associated gene product staining patterns. For example, in some embodiments, the clinician(s) is blinded to clinical characteristics and outcome for the samples, patients, etc. being assessed and scored.

### Aberrant Phosphorylation level

In some embodiments, the mTOR-activating aberration (*e.g*. aberrant expression level or aberrant activity level) comprises an aberrant protein phosphorylation level. In some embodiments, the aberrant phosphorylation level is in a protein encoded by an mTOR-associated gene selected from the group consisting of AKT, TSC2, mTOR, PRAS40, S6K, S6, 4EBP1, and SPARC. Exemplary phosphorylated species of mTOR-associated genes that may serve as relevant biomarkers include, but are not limited to, AKT S473 phosphorylation, PRAS40 T246 phosphorylation, mTOR S2448 phosphorylation, 4EBP1 T36 phosphorylation, S6K T389 phosphorylation, 4EBP1 T70 phosphorylation, and S6 S235 phosphorylation. In some embodiments, the individual is selected for treatment if the protein in the individual is phosphorylated. In some embodiments, the individual is selected for treatment if the protein in the individual is not phosphorylated. In some embodiments, the individual is selected for treatment based on the phosphorylation level of one or more proteins encoded by one or more mTOR-associated genes. In some embodiments, the phosphorylation status of the protein is determined by immunohistochemistry.

Aberrant phosphorylation levels of proteins encoded by mTOR-associated genes have been associated with hyperplasia, including cancer, restenosis and pulmonary hypertension. For example, high levels (74%) of phosphorylated mTOR expression were found in human bladder cancer tissue array, and phosphorylated mTOR intensity was associated with reduced survival (Hansel DE et al, (2010) Am. J. Pathol. 176: 3062-3072).

In some embodiments, the level of protein phosphorylation of one or more mTOR-associated genes is determined. The phosphorylation status of a protein may be assessed from a variety of sample sources. In some embodiments, the sample is a tumor biopsy. The phosphorylation status of a protein may be assessed via a variety of methods. In some embodiments, the phosphorylation status is assessed using immunohistochemistry. The phosphorylation status of a protein may be site specific. The phosphorylation status of a protein may be compared to a control sample. The control sample may be any one of the control samples described in the section above for methods that comprise determination of expression level or activity level of mTOR-associated genes. In some embodiments, the phosphorylation status is assessed prior to initiation of the methods of treatment described herein. In some embodiments, the phosphorylation status is assessed after initiation of the methods of treatment described herein. In some embodiments, the phosphorylation status is assessed prior to and after initiation of the methods of treatment described herein.

Further provided herein are methods of directing treatment of a hyperplasia (such as cancer, restenosis, or pulmonary hypertension) by delivering a sample to a diagnostic lab for determination of the level of an mTOR-associated gene; providing a control sample with a known level of the mTOR-associated gene; providing an antibody to a molecule encoded by the mTOR-associated gene or an antibody to a molecule encoded by a downstream target gene of the mTOR-associated gene; individually contacting the sample and control sample with the antibody, and/or detecting a relative amount of antibody binding, wherein the level of the sample is used to provide a conclusion that a patient should receive a treatment with any one of the methods described herein.

Also provided herein are methods of directing treatment of a hyperplasia (such as cancer, restenosis, or pulmonary hypertension), further comprising reviewing or analyzing data relating to the status (such as presence/absence or level) of an mTOR-activating aberration in a sample; and providing a conclusion to an individual, such as a health care provider or a health care manager, about the likelihood or suitability of the individual to respond to a treatment, the conclusion being based on the review or analysis of data. In one aspect of the invention a conclusion is the transmission of the data over a network.

### Resistance biomarkers

Genetic aberrations and aberrant levels of certain genes may be associated with resistance to the treatment methods described herein. In some embodiments, the individual having an aberration (such as genetic aberration or aberrant level) in a resistance biomarker is excluded from the methods of treatment using the mTOR inhibitor nanoparticles as described herein. In some embodiments, the status of the resistance biomarkers combined with the status of one or more of the mTOR-activating aberrations are used as the basis for selecting an individual for any one of the methods of treatment using mTOR inhibitor nanoparticles as described herein.

For example, TFE3, also known as transcription factor binding to IGHM enhancer 3, TFEA, RCCP2, RCCX1, or bHLHe33, is a transcription factor that specifically recognizes and binds MUE3-type E-box sequences in the promoters of genes. TFE3 promotes expression of genes downstream of transforming growth factor beta (TGF-beta) signaling. Translocation of TFE3 has been associated with renal cell carcinomas and other cancers. In some embodiments, the nucleic acid sequence of a wildtype TFE3 gene is identified by the Genbank accession number NC_ 000023.11 from nucleotide 49028726 to nucleotide 49043517 of the complement strand of chromosome X according to the GRCh38.p2 assembly of the human genome. Exemplary translocations of TFE3 that may be associated with resistance to treatment using the mTOR inhibitor nanoparticles as described herein include, but are not limited to, Xp11 translocation, such as t(X; 1)(p11.2; q21), t(X; 1)(p11.2; p34), (X; 17)(p11.2; q25.3), and inv(X)(p11.2; q12). Translocation of the TFE3 locus can be assessed using immunohistochemical methods or fluorescence in situ hybridization (FISH).

### Other methods of treatment

One aspect of the present application provides methods and compositions for treating non-muscle invasive bladder cancer (NMIBC, such as BCG-refractory NMIBC), peripheral artery disease (PAD, such as restenotic symptomatic lesions after revascularization of the above or below the knee femoropopliteal arteries) and pulmonary arterial hypertension (PAH, such as severe progressive PAH on maximal currently available background therapy) in an individual in need thereof comprising administering to the individual an effective amount of a composition comprising nanoparticles comprising an mTOR inhibitor (such as limus drug, for example sirolimus) and an albumin. The individual receiving the treatment may or may not have an mTOR-activating aberration as described above. In some embodiments, the individual is selected for the treatment based on having an mTOR-activating aberration as described above. In some embodiments, the status of any of the mTOR-activating aberrations as described above is not used as the basis for selecting the individual for the treatment.

In some embodiments, there is provided a method of treating a non-muscle invasive bladder cancer (NMIBC, such as BCG-refractory or recurrent NMIBC) in an individual (such as human) comprising administering to the individual an effective amount of a composition comprising nanoparticles comprising a limus drug and an albumin, wherein the composition is intravesicularly administered at a dose of about 100 mg. In some embodiments, there is provided a method of treating a non-muscle invasive bladder cancer (NMIBC, such as BCG-refractory or recurrent NMIBC) in an individual (such as human) comprising administering to the individual an effective amount of a composition comprising nanoparticles comprising a limus drug and an albumin, wherein the composition is administered at a dose of about 100 mg, and wherein the composition is administered weekly (*e.g.* for about 6 weeks). In some embodiments, there is provided a method of treating a non-muscle invasive bladder cancer (NMIBC, such as BCG-refractory or recurrent NMIBC) in an individual (such as human) comprising administering to the individual an effective amount of a composition comprising nanoparticles comprising a limus drug and an albumin, wherein the composition is administered at a dose of about 100 mg, wherein the composition is administered weekly (*e.g*. for about 6 weeks), and wherein the dose is administered intravesically. In some embodiments, there is provided a method of treating a non-muscle invasive bladder cancer (NMIBC, such as BCG-refractory or recurrent NMIBC) in an individual (such as human) comprising administering to the individual an effective amount of a composition comprising nanoparticles comprising a limus drug and an albumin, wherein the composition is administered at a dose of about 100 mg, wherein the composition is administered weekly (*e.g*. for about 6 weeks), and wherein the dose is administered intravesically by sterile urethral catheterization following resection of visible tumors during cystoscopy. In some embodiments, the composition is kept in the bladder for about 2 hours before voiding. In some embodiments, the individual is administered a maintenance dose of the composition after about 6 weeks, wherein the maintenance dose is administered monthly. In some embodiments, the composition is administered as a single agent. In some embodiments, the composition is administered in combination with a second agent. In some embodiments, the second agent is a chemotherapy agent selected from the group consisting of mitomycin C, cisplatin, gemcitabine, valrubicin, and docetaxel. In some embodiments, the second agent is gemcitabine. In some embodiments, the second agent and the nanoparticle composition are administered sequentially. In some embodiments, the second agent and the nanoparticle composition are administered simultaneously. In some embodiments, the second agent and the nanoparticle composition are administered concurrently. In some embodiments, the nanoparticles in the composition have an average particle size of no greater than about 150 nm (such as no greater than about 120 nm). In some embodiments, the nanoparticles in the composition comprise a limus drug associated (*e.g*., coated) with albumin, wherein the nanoparticles have an average particle size of no greater than about 150 nm (such as no greater than about 120 nm). In some embodiments, the nanoparticles in the composition comprise sirolimus associated (*e.g*., coated) with human albumin, wherein the nanoparticles have an average particle size of no greater than about 150 nm (such as no greater than about 120 nm, for example about 100 nm), wherein the weight ratio of human albumin and sirolimus in the composition is about 9:1 or less (such as about 9:1 or about 8:1). In some embodiments, the composition comprises *Nab*-sirolimus. In some embodiments, the composition is *Nab*-sirolimus.

In some embodiments, there is provided a method of treating a non-muscle invasive bladder cancer (NMIBC, such as BCG-refractory or recurrent NMIBC) in an individual (such as human) comprising administering to the individual an effective amount of a composition comprising nanoparticles comprising a limus drug and an albumin, wherein the composition is intravesicularly administered at a dose of about 100 mg, and wherein the composition is administered twice per week (*e.g*. for about 6 weeks). In some embodiments, there is provided a method of treating a non-muscle invasive bladder cancer (NMIBC, such as BCG-refractory or recurrent NMIBC) in an individual (such as human) comprising administering to the individual an effective amount of a composition comprising nanoparticles comprising a limus drug and an albumin, wherein the composition is administered at a dose of about 100 mg, wherein the composition is administered twice per week (*e.g.* for about 6 weeks), and wherein the dose is administered intravesically. In some embodiments, there is provided a method of treating a non-muscle invasive bladder cancer (NMIBC, such as BCG-refractory or recurrent NMIBC) in an individual (such as human) comprising administering to the individual an effective amount of a composition comprising nanoparticles comprising a limus drug and an albumin, wherein the composition is administered at a dose of about 100 mg, wherein the composition is administered twice per week (*e.g*. for about 6 weeks), and wherein the dose is administered intravesically by sterile urethral catheterization following resection of visible tumors during cystoscopy. In some embodiments, the composition is kept in the bladder for about 2 hours before voiding. In some embodiments, the individual is administered a maintenance dose of the composition after about 6 weeks, wherein the maintenance dose is administered monthly. In some embodiments, the composition is administered as a single agent. In some embodiments, the composition is administered in combination with a second agent. In some embodiments, the second agent is a chemotherapy agent selected from the group consisting of mitomycin C, cisplatin, gemcitabine, valrubicin, and docetaxel. In some embodiments, the second agent is gemcitabine. In some embodiments, the second agent and the nanoparticle composition are administered sequentially. In some embodiments, the second agent and the nanoparticle composition are administered simultaneously. In some embodiments, the second agent and the nanoparticle composition are administered concurrently. In some embodiments, the nanoparticles in the composition have an average particle size of no greater than about 150 nm (such as no greater than about 120 nm). In some embodiments, the nanoparticles in the composition comprise a limus drug associated (*e.g.,* coated) with albumin, wherein the nanoparticles have an average particle size of no greater than about 150 nm (such as no greater than about 120 nm). In some embodiments, the nanoparticles in the composition comprise sirolimus associated (*e.g*., coated) with human albumin, wherein the nanoparticles have an average particle size of no greater than about 150 nm (such as no greater than about 120 nm, for example about 100 nm), wherein the weight ratio of human albumin and sirolimus in the composition is about 9:1 or less (such as about 9: 1 or about 8:1). In some embodiments, the composition comprises *Nab*-sirolimus. In some embodiments, the composition is *Nab*-sirolimus.

In some embodiments, there is provided a method of treating a non-muscle invasive bladder cancer (NMIBC, such as BCG-refractory or recurrent NMIBC) in an individual (such as human) comprising administering to the individual an effective amount of a composition comprising nanoparticles comprising a limus drug and an albumin, wherein the composition is intravesicularly administered at a dose of about 300 mg. In some embodiments, there is provided a method of treating a non-muscle invasive bladder cancer (NMIBC, such as BCG-refractory or recurrent NMIBC) in an individual (such as human) comprising administering to the individual an effective amount of a composition comprising nanoparticles comprising a limus drug and an albumin, wherein the composition is administered at a dose of about 300 mg, and wherein the composition is administered weekly (*e.g*. for about 6 weeks). In some embodiments, there is provided a method of treating a non-muscle invasive bladder cancer (NMIBC, such as BCG-refractory or recurrent NMIBC) in an individual (such as human) comprising administering to the individual an effective amount of a composition comprising nanoparticles comprising a limus drug and an albumin, wherein the composition is administered at a dose of about 300 mg, wherein the composition is administered weekly (*e.g*. for about 6 weeks), and wherein the dose is administered intravesically. In some embodiments, there is provided a method of treating a non-muscle invasive bladder cancer (NMIBC, such as BCG-refractory or recurrent NMIBC) in an individual (such as human) comprising administering to the individual an effective amount of a composition comprising nanoparticles comprising a limus drug and an albumin, wherein the composition is administered at a dose of about 300 mg, wherein the composition is administered weekly (*e.g*. for about 6 weeks), and wherein the dose is administered intravesically by sterile urethral catheterization following resection of visible tumors during cystoscopy. In some embodiments, the composition is kept in the bladder for about 2 hours before voiding. In some embodiments, the individual is administered a maintenance dose of the composition after about 6 weeks, wherein the maintenance dose is administered monthly. In some embodiments, the composition is administered as a single agent. In some embodiments, the composition is administered in combination with a second agent. In some embodiments, the second agent is a chemotherapy agent selected from the group consisting of mitomycin C, cisplatin, gemcitabine, valrubicin, and docetaxel. In some embodiments, the second agent is gemcitabine. In some embodiments, the second agent and the nanoparticle composition are administered sequentially. In some embodiments, the second agent and the nanoparticle composition are administered simultaneously. In some embodiments, the second agent and the nanoparticle composition are administered concurrently. In some embodiments, the nanoparticles in the composition have an average particle size of no greater than about 150 nm (such as no greater than about 120 nm). In some embodiments, the nanoparticles in the composition comprise a limus drug associated (*e.g.,* coated) with albumin, wherein the nanoparticles have an average particle size of no greater than about 150 nm (such as no greater than about 120 nm). In some embodiments, the nanoparticles in the composition comprise sirolimus associated (*e.g*., coated) with human albumin, wherein the nanoparticles have an average particle size of no greater than about 150 nm (such as no greater than about 120 nm, for example about 100 nm), wherein the weight ratio of human albumin and sirolimus in the composition is about 9:1 or less (such as about 9:1 or about 8:1). In some embodiments, the composition comprises *Nab*-sirolimus. In some embodiments, the composition is *Nab*-sirolimus.

In some embodiments, there is provided a method of treating a non-muscle invasive bladder cancer (NMIBC, such as BCG-refractory or recurrent NMIBC) in an individual (such as human) comprising administering to the individual an effective amount of a composition comprising nanoparticles comprising a limus drug and an albumin, wherein the composition is intravesicularly administered at a dose of about 200 mg. In some embodiments, there is provided a method of treating a non-muscle invasive bladder cancer (NMIBC, such as BCG-refractory or recurrent NMIBC) in an individual (such as human) comprising administering to the individual an effective amount of a composition comprising nanoparticles comprising a limus drug and an albumin, wherein the composition is administered at a dose of about 200 mg, and wherein the composition is administered twice per week (*e.g*. for about 6 weeks). In some embodiments, there is provided a method of treating a non-muscle invasive bladder cancer (NMIBC, such as BCG-refractory or recurrent NMIBC) in an individual (such as human) comprising administering to the individual an effective amount of a composition comprising nanoparticles comprising a limus drug and an albumin, wherein the composition is administered at a dose of about 200 mg, wherein the composition is administered twice per week (*e.g.* for about 6 weeks), and wherein the dose is administered intravesically. In some embodiments, there is provided a method of treating a non-muscle invasive bladder cancer (NMIBC, such as BCG-refractory or recurrent NMIBC) in an individual (such as human) comprising administering to the individual an effective amount of a composition comprising nanoparticles comprising a limus drug and an albumin, wherein the composition is administered at a dose of about 200 mg, wherein the composition is administered twice per week (*e.g*. for about 6 weeks), and wherein the dose is administered intravesically by sterile urethral catheterization following resection of visible tumors during cystoscopy. In some embodiments, the composition is kept in the bladder for about 2 hours before voiding. In some embodiments, the individual is administered a maintenance dose of the composition after about 6 weeks, wherein the maintenance dose is administered monthly. In some embodiments, the composition is administered as a single agent. In some embodiments, the composition is administered in combination with a second agent. In some embodiments, the second agent is a chemotherapy agent selected from the group consisting of mitomycin C, cisplatin, gemcitabine, valrubicin, and docetaxel. In some embodiments, the second agent is gemcitabine. In some embodiments, the second agent and the nanoparticle composition are administered sequentially. In some embodiments, the second agent and the nanoparticle composition are administered simultaneously. In some embodiments, the second agent and the nanoparticle composition are administered concurrently. In some embodiments, the nanoparticles in the composition have an average particle size of no greater than about 150 nm (such as no greater than about 120 nm). In some embodiments, the nanoparticles in the composition comprise a limus drug associated (*e.g*., coated) with albumin, wherein the nanoparticles have an average particle size of no greater than about 150 nm (such as no greater than about 120 nm). In some embodiments, the nanoparticles in the composition comprise sirolimus associated (*e.g*., coated) with human albumin, wherein the nanoparticles have an average particle size of no greater than about 150 nm (such as no greater than about 120 nm, for example about 100 nm), wherein the weight ratio of human albumin and sirolimus in the composition is about 9: 1 or less (such as about 9:1 or about 8:1). In some embodiments, the composition comprises *Nab*-sirolimus. In some embodiments, the composition is *Nab*-sirolimus.

In some embodiments, there is provided a method of treating a non-muscle invasive bladder cancer (NMIBC, such as BCG-refractory or recurrent NMIBC) in an individual (such as human) comprising administering to the individual an effective amount of a composition comprising nanoparticles comprising a limus drug and an albumin, wherein the composition is intravesicularly administered at a dose of about 400 mg. In some embodiments, there is provided a method of treating a non-muscle invasive bladder cancer (NMIBC, such as BCG-refractory or recurrent NMIBC) in an individual (such as human) comprising administering to the individual an effective amount of a composition comprising nanoparticles comprising a limus drug and an albumin, wherein the composition is administered at a dose of about 400 mg, and wherein the composition is administered weekly (*e.g*. for about 6 weeks). In some embodiments, there is provided a method of treating a non-muscle invasive bladder cancer (NMIBC, such as BCG-refractory or recurrent NMIBC) in an individual (such as human) comprising administering to the individual an effective amount of a composition comprising nanoparticles comprising a limus drug and an albumin, wherein the composition is administered at a dose of about 400 mg, wherein the composition is administered weekly (*e.g.* for about 6 weeks), and wherein the dose is administered intravesically. In some embodiments, there is provided a method of treating a non-muscle invasive bladder cancer (NMIBC, such as BCG-refractory or recurrent NMIBC) in an individual (such as human) comprising administering to the individual an effective amount of a composition comprising nanoparticles comprising a limus drug and an albumin, wherein the composition is administered at a dose of about 400 mg, wherein the composition is administered weekly (*e.g*. for about 6 weeks), and wherein the dose is administered intravesically by sterile urethral catheterization following resection of visible tumors during cystoscopy. In some embodiments, the composition is kept in the bladder for about 2 hours before voiding. In some embodiments, the individual is administered a maintenance dose of the composition after about 6 weeks, wherein the maintenance dose is administered monthly. In some embodiments, the composition is administered as a single agent. In some embodiments, the composition is administered in combination with a second agent. In some embodiments, the second agent is a chemotherapy agent selected from the group consisting of mitomycin C, cisplatin, gemcitabine, valrubicin, and docetaxel. In some embodiments, the second agent is gemcitabine. In some embodiments, the second agent and the nanoparticle composition are administered sequentially. In some embodiments, the second agent and the nanoparticle composition are administered simultaneously. In some embodiments, the second agent and the nanoparticle composition are administered concurrently. In some embodiments, the nanoparticles in the composition have an average particle size of no greater than about 150 nm (such as no greater than about 120 nm). In some embodiments, the nanoparticles in the composition comprise a limus drug associated (*e.g*., coated) with albumin, wherein the nanoparticles have an average particle size of no greater than about 150 nm (such as no greater than about 120 nm). In some embodiments, the nanoparticles in the composition comprise sirolimus associated (*e.g*., coated) with human albumin, wherein the nanoparticles have an average particle size of no greater than about 150 nm (such as no greater than about 120 nm, for example about 100 nm), wherein the weight ratio of human albumin and sirolimus in the composition is about 9:1 or less (such as about 9: 1 or about 8:1). In some embodiments, the composition comprises *Nab*-sirolimus. In some embodiments, the composition is *Nab*-sirolimus.

In some embodiments, there is provided a method of treating a non-muscle invasive bladder cancer (NMIBC, such as BCG-refractory or recurrent NMIBC) in an individual (such as human) comprising administering to the individual an effective amount of a composition comprising *Nab*-sirolimus, wherein the composition is intravesicularly administered at a dose of about 100 mg. In some embodiments, there is provided a method of treating a non-muscle invasive bladder cancer (NMIBC, such as BCG-refractory or recurrent NMIBC) in an individual (such as human) comprising administering to the individual an effective amount of a composition comprising *Nab*-sirolimus, wherein the composition is administered at a dose of about 100 mg, and wherein the composition is administered weekly (*e.g*. for about 6 weeks). In some embodiments, there is provided a method of treating a non-muscle invasive bladder cancer (NMIBC, such as BCG-refractory or recurrent NMIBC) in an individual (such as human) comprising administering to the individual an effective amount of a composition comprising Nab-sirolimus, wherein the composition is administered at a dose of about 100 mg, wherein the composition is administered weekly (*e.g.* for about 6 weeks), and wherein the dose is administered intravesically. In some embodiments, there is provided a method of treating a non-muscle invasive bladder cancer (NMIBC, such as BCG-refractory or recurrent NMIBC) in an individual (such as human) comprising administering to the individual an effective amount of a composition comprising *Nab*-sirolimus, wherein the composition is administered at a dose of about 100 mg, wherein the composition is administered weekly (*e.g*. for about 6 weeks), and wherein the dose is administered intravesically by sterile urethral catheterization following resection of visible tumors during cystoscopy. In some embodiments, the composition is kept in the bladder for about 2 hours before voiding. In some embodiments, the individual is administered a maintenance dose of the composition after about 6 weeks, wherein the maintenance dose is administered monthly. In some embodiments, the composition is administered as a single agent. In some embodiments, the composition is administered in combination with a second agent. In some embodiments, the second agent is a chemotherapy agent selected from the group consisting of mitomycin C, cisplatin, gemcitabine, valrubicin, and docetaxel. In some embodiments, the second agent is gemcitabine. In some embodiments, the second agent and the nanoparticle composition are administered sequentially. In some embodiments, the second agent and the nanoparticle composition are administered simultaneously. In some embodiments, the second agent and the nanoparticle composition are administered concurrently.

In some embodiments, there is provided a method of treating a non-muscle invasive bladder cancer (NMIBC, such as BCG-refractory or recurrent NMIBC) in an individual (such as human) comprising administering to the individual an effective amount of a composition comprising *Nab*-sirolimus, wherein the composition is intravesicularly administered at a dose of about 100 mg, and wherein the composition is administered twice per week (*e.g*. for about 6 weeks). In some embodiments, there is provided a method of treating a non-muscle invasive bladder cancer (NMIBC, such as BCG-refractory or recurrent NMIBC) in an individual (such as human) comprising administering to the individual an effective amount of a composition comprising *Nab*-sirolimus, wherein the composition is administered at a dose of about 100 mg, wherein the composition is administered twice per week (*e.g*. for about 6 weeks), and wherein the dose is administered intravesically. In some embodiments, there is provided a method of treating a non-muscle invasive bladder cancer (NMIBC, such as BCG-refractory or recurrent NMIBC) in an individual (such as human) comprising administering to the individual an effective amount of a composition comprising nanoparticles comprising *Nab*-sirolimus, wherein the composition is administered at a dose of about 100 mg, wherein the composition is administered twice per week (*e.g*. for about 6 weeks), and wherein the dose is administered intravesically by sterile urethral catheterization following resection of visible tumors during cystoscopy. In some embodiments, the composition is kept in the bladder for about 2 hours before voiding. In some embodiments, the individual is administered a maintenance dose of the composition after about 6 weeks, wherein the maintenance dose is administered monthly. In some embodiments, the composition is administered as a single agent. In some embodiments, the composition is administered in combination with a second agent. In some embodiments, the second agent is a chemotherapy agent selected from the group consisting of mitomycin C, cisplatin, gemcitabine, valrubicin, and docetaxel. In some embodiments, the second agent is gemcitabine. In some embodiments, the second agent and the nanoparticle composition are administered sequentially. In some embodiments, the second agent and the nanoparticle composition are administered simultaneously. In some embodiments, the second agent and the nanoparticle composition are administered concurrently.

In some embodiments, there is provided a method of treating a non-muscle invasive bladder cancer (NMIBC, such as BCG-refractory or recurrent NMIBC) in an individual (such as human) comprising administering to the individual an effective amount of a composition comprising *Nab*-sirolimus, wherein the composition is intravesicularly administered at a dose of about 300 mg. In some embodiments, there is provided a method of treating a non-muscle invasive bladder cancer (NMIBC, such as BCG-refractory or recurrent NMIBC) in an individual (such as human) comprising administering to the individual an effective amount of a composition comprising *Nab*-sirolimus, wherein the composition is administered at a dose of about 300 mg, and wherein the composition is administered weekly (*e.g*. for about 6 weeks). In some embodiments, there is provided a method of treating a non-muscle invasive bladder cancer (NMIBC, such as BCG-refractory or recurrent NMIBC) in an individual (such as human) comprising administering to the individual an effective amount of a composition comprising *Nab*-sirolimus, wherein the composition is administered at a dose of about 300 mg, wherein the composition is administered weekly (*e.g*. for about 6 weeks), and wherein the dose is administered intravesically. In some embodiments, there is provided a method of treating a non-muscle invasive bladder cancer (NMIBC, such as BCG-refractory or recurrent NMIBC) in an individual (such as human) comprising administering to the individual an effective amount of a composition comprising *Nab*-sirolimus, wherein the composition is administered at a dose of about 300 mg, wherein the composition is administered weekly (*e.g*. for about 6 weeks), and wherein the dose is administered intravesically by sterile urethral catheterization following resection of visible tumors during cystoscopy. In some embodiments, the composition is kept in the bladder for about 2 hours before voiding. In some embodiments, the individual is administered a maintenance dose of the composition after about 6 weeks, wherein the maintenance dose is administered monthly. In some embodiments, the composition is administered as a single agent. In some embodiments, the composition is administered in combination with a second agent. In some embodiments, the second agent is a chemotherapy agent selected from the group consisting of mitomycin C, cisplatin, gemcitabine, valrubicin, and docetaxel. In some embodiments, the second agent is gemcitabine. In some embodiments, the second agent and the nanoparticle composition are administered sequentially. In some embodiments, the second agent and the nanoparticle composition are administered simultaneously. In some embodiments, the second agent and the nanoparticle composition are administered concurrently.

In some embodiments, there is provided a method of treating a non-muscle invasive bladder cancer (NMIBC, such as BCG-refractory or recurrent NMIBC) in an individual (such as human) comprising administering to the individual an effective amount of a composition comprising *Nab*-sirolimus, wherein the composition is intravesicularly administered at a dose of about 200 mg. In some embodiments, there is provided a method of treating a non-muscle invasive bladder cancer (NMIBC, such as BCG-refractory or recurrent NMIBC) in an individual (such as human) comprising administering to the individual an effective amount of a composition comprising *Nab-*sirolimus, wherein the composition is administered at a dose of about 200 mg, and wherein the composition is administered twice per week (*e.g*. for about 6 weeks). In some embodiments, there is provided a method of treating a non-muscle invasive bladder cancer (NMIBC, such as BCG-refractory or recurrent NMIBC) in an individual (such as human) comprising administering to the individual an effective amount of a composition comprising *Nab*-sirolimus, wherein the composition is administered at a dose of about 200 mg, wherein the composition is administered twice per week (*e.g*. for about 6 weeks), and wherein the dose is administered intravesically. In some embodiments, there is provided a method of treating a non-muscle invasive bladder cancer (NMIBC, such as BCG-refractory or recurrent NMIBC) in an individual (such as human) comprising administering to the individual an effective amount of a composition comprising *Nab*-sirolimus, wherein the composition is administered at a dose of about 200 mg, wherein the composition is administered twice per week (*e.g.* for about 6 weeks), and wherein the dose is administered intravesically by sterile urethral catheterization following resection of visible tumors during cystoscopy. In some embodiments, the composition is kept in the bladder for about 2 hours before voiding. In some embodiments, the individual is administered a maintenance dose of the composition after about 6 weeks, wherein the maintenance dose is administered monthly. In some embodiments, the composition is administered as a single agent. In some embodiments, the composition is administered in combination with a second agent. In some embodiments, the second agent is a chemotherapy agent selected from the group consisting of mitomycin C, cisplatin, gemcitabine, valrubicin, and docetaxel. In some embodiments, the second agent is gemcitabine.

In some embodiments, there is provided a method of treating a non-muscle invasive bladder cancer (NMIBC, such as BCG-refractory or recurrent NMIBC) in an individual (such as human) comprising administering to the individual an effective amount of a composition comprising *Nab*-sirolimus, wherein the composition is intravesicularly administered at a dose of about 400 mg. In some embodiments, there is provided a method of treating a non-muscle invasive bladder cancer (NMIBC, such as BCG-refractory or recurrent NMIBC) in an individual (such as human) comprising administering to the individual an effective amount of a composition comprising *Nab-*sirolimus, wherein the composition is administered at a dose of about 400 mg, and wherein the composition is administered weekly (*e.g*. for about 6 weeks). In some embodiments, there is provided a method of treating a non-muscle invasive bladder cancer (NMIBC, such as BCG-refractory or recurrent NMIBC) in an individual (such as human) comprising administering to the individual an effective amount of a composition comprising *Nab*-sirolimus, wherein the composition is administered at a dose of about 400 mg, wherein the composition is administered weekly (*e.g*. for about 6 weeks), and wherein the dose is administered intravesically. In some embodiments, there is provided a method of treating a non-muscle invasive bladder cancer (NMIBC, such as BCG-refractory or recurrent NMIBC) in an individual (such as human) comprising administering to the individual an effective amount of a composition comprising *Nab*-sirolimus, wherein the composition is administered at a dose of about 400 mg, wherein the composition is administered weekly (*e.g*. for about 6 weeks), and wherein the dose is administered intravesically by sterile urethral catheterization following resection of visible tumors during cystoscopy. In some embodiments, the composition is kept in the bladder for about 2 hours before voiding. In some embodiments, the individual is administered a maintenance dose of the composition after about 6 weeks, wherein the maintenance dose is administered monthly. In some embodiments, the composition is administered as a single agent. In some embodiments, the composition is administered in combination with a second agent. In some embodiments, the second agent is a chemotherapy agent selected from the group consisting of mitomycin C, cisplatin, gemcitabine, valrubicin, and docetaxel. In some embodiments, the second agent and the nanoparticle composition are administered sequentially. In some embodiments, the second agent and the nanoparticle composition are administered simultaneously. In some embodiments, the second agent and the nanoparticle composition are administered concurrently.

In some embodiments, there is provided a method of treating a non-muscle invasive bladder cancer (NMIBC, such as BCG-refractory or recurrent NMIBC) in an individual (such as human) comprising intravesicularly administering to the individual an effective amount of a composition comprising *Nab*-sirolimus, and administering to the individual an effective amount of gemcitabine. In some embodiments, there is provided a method of treating a non-muscle invasive bladder cancer (NMIBC, such as BCG-refractory or recurrent NMIBC) in an individual (such as human) comprising administering to the individual an effective amount of a composition comprising *Nab*-sirolimus, and administering to the individual an effective amount of gemcitabine, wherein the composition is intravesicularly administered at a dose of no more than about 400 mg. In some embodiments, there is provided a method of treating a non-muscle invasive bladder cancer (NMIBC, such as BCG-refractory or recurrent NMIBC) in an individual (such as human) comprising administering to the individual an effective amount of a composition comprising *Nab*-sirolimus, and administering to the individual an effective amount of gemcitabine, wherein the composition is administered at a dose of no more than about 400 mg, and wherein the composition is administered weekly (*e.g*. for about 6 weeks). In some embodiments, there is provided a method of treating a non-muscle invasive bladder cancer (NMIBC, such as BCG-refractory or recurrent NMIBC) in an individual (such as human) comprising administering to the individual an effective amount of a composition comprising *Nab*-sirolimus, and administering to the individual an effective amount of gemcitabine, wherein the composition is administered at a dose of no more than about 400 mg, wherein the composition is administered weekly (*e.g*. for about 6 weeks), and wherein the dose is administered intravesically. In some embodiments, there is provided a method of treating a non-muscle invasive bladder cancer (NMIBC, such as BCG-refractory or recurrent NMIBC) in an individual (such as human) comprising administering to the individual an effective amount of a composition comprising *Nab*-sirolimus, and administering to the individual an effective amount of gemcitabine, wherein the composition is administered at a dose of no more than about 400 mg, wherein the composition is administered weekly (*e.g*. for about 6 weeks), and wherein the dose is administered intravesically by sterile urethral catheterization following resection of visible tumors during cystoscopy. In some embodiments, the composition is kept in the bladder for about 2 hours before voiding. In some embodiments, the individual is administered a maintenance dose of the composition after about 6 weeks, wherein the maintenance dose is administered monthly. In some embodiments, gemcitabine is administered intravenously. In some embodiments, gemcitabine is administered at a dose of no more than about 1250 mg/m² or no more than about 1000 mg/m². In some embodiments, each dose of gemcitabine is administered over about 30 minutes. In some embodiments, gemcitabine is administered once weekly for two out of each three-week cycle. In some embodiments, gemcitabine is administered on days 1 and 8 of each 21-day cycle. In some embodiments, gemcitabine is administered once weekly for each three out four-week cycle. In some embodiments, gemcitabine is administered on days 1, 8, and 15 of each 28-day cycle. In some embodiments, gemcitabine is administered once weekly for the first 7 weeks, then one week rest, then once weekly for three out of each four-week cycle. In some embodiments, gemcitabine and the *Nab*-sirolimus composition are administered sequentially. In some embodiments, the second agent and the *Nab*-sirolimus composition are administered simultaneously. In some embodiments, the second agent and the *Nab*-sirolimus composition are administered concurrently.

In some embodiments, there is provided a method of treating a peripheral artery disease (such as restenotic symptomatic lesions after revascularization of the above or below the knee femoropopliteal arteries) in an individual (such as human) comprising administering to the individual an effective amount of a composition comprising nanoparticles comprising a limus drug and an albumin, wherein the composition is administered intra-adventitially at a dose of about 40 µg/cm of desired vessel treatment length. In some embodiments, there is provided a method of treating a peripheral artery disease (such as restenotic symptomatic lesions after revascularization of the above or below the knee femoropopliteal arteries) in an individual (such as human) comprising administering to the individual an effective amount of a composition comprising nanoparticles comprising a limus drug and an albumin, wherein the composition is administered intra-adventitially at a dose of about 40 µg/cm of desired vessel treatment length, and wherein the composition is administered to the adventitia using a micro-infusion catheter (such as a Bullfrog^{®} micro-infusion catheter). In some embodiments, the method improves luminal diameter of the blood vessel. In some embodiments, the method improves outcomes of femoropopliteal revascularization after balloon angioplasty and provisional stenting of the popliteal and contiguous peripheral arteries. In some embodiments, the individual has a de novo atherosclerotic lesion greater than about 70% in the popliteal artery, allowing lesion extension into contiguous arteries that totals up to 15 cm in length, and with a reference vessel diameter of about 3 mm to about 8 mm. In some embodiments, the nanoparticles in the composition have an average particle size of no greater than about 150 nm (such as no greater than about 120 nm). In some embodiments, the nanoparticles in the composition comprise a limus drug associated (*e.g*., coated) with albumin, wherein the nanoparticles have an average particle size of no greater than about 150 nm (such as no greater than about 120 nm). In some embodiments, the nanoparticles in the composition comprise sirolimus associated (*e.g*., coated) with human albumin, wherein the nanoparticles have an average particle size of no greater than about 150 nm (such as no greater than about 120 nm, for example about 100 nm), wherein the weight ratio of human albumin and sirolimus in the composition is about 9:1 or less (such as about 9:1 or about 8:1). In some embodiments, the composition comprises *Nab*-sirolimus. In some embodiments, the composition is *Nab*-sirolimus.

In some embodiments, there is provided a method of treating a peripheral artery disease (such as restenotic symptomatic lesions after revascularization of the above or below the knee femoropopliteal arteries) in an individual (such as human) comprising administering to the individual an effective amount of a composition comprising nanoparticles comprising a limus drug and an albumin, wherein the composition is administered intra-adventitially at a dose of about 100 µg/cm of desired vessel treatment length. In some embodiments, there is provided a method of treating a peripheral artery disease (such as restenotic symptomatic lesions after revascularization of the above or below the knee femoropopliteal arteries) in an individual (such as human) comprising administering to the individual an effective amount of a composition comprising nanoparticles comprising a limus drug and an albumin, wherein the composition is administered intra-adventitially at a dose of about 100 µg/cm of desired vessel treatment length, and wherein the composition is administered to the adventitia using a micro-infusion catheter (such as a Bullfrog^{®} micro-infusion catheter). In some embodiments, the method improves luminal diameter of the blood vessel. In some embodiments, the method improves outcomes of femoropopliteal revascularization after balloon angioplasty and provisional stenting of the popliteal and contiguous peripheral arteries. In some embodiments, the individual has a de novo atherosclerotic lesion greater than about 70% in the popliteal artery, allowing lesion extension into contiguous arteries that totals up to 15 cm in length, and with a reference vessel diameter of about 3 mm to about 8 mm. In some embodiments, the nanoparticles in the composition have an average particle size of no greater than about 150 nm (such as no greater than about 120 nm). In some embodiments, the nanoparticles in the composition comprise a limus drug associated (*e.g*., coated) with albumin, wherein the nanoparticles have an average particle size of no greater than about 150 nm (such as no greater than about 120 nm). In some embodiments, the nanoparticles in the composition comprise sirolimus associated (e.g., coated) with human albumin, wherein the nanoparticles have an average particle size of no greater than about 150 nm (such as no greater than about 120 nm, for example about 100 nm), wherein the weight ratio of human albumin and sirolimus in the composition is about 9:1 or less (such as about 9:1 or about 8:1). In some embodiments, the composition comprises *Nab*-sirolimus. In some embodiments, the composition is *Nab*-sirolimus.

In some embodiments, there is provided a method of treating a peripheral artery disease (such as restenotic symptomatic lesions after revascularization of the above or below the knee femoropopliteal arteries) in an individual (such as human) comprising administering to the individual an effective amount of a composition comprising *Nab*-sirolimus, wherein the composition is administered intra-adventitially at a dose of about 40 µg/cm of desired vessel treatment length. In some embodiments, there is provided a method of treating a peripheral artery disease (such as restenotic symptomatic lesions after revascularization of the above or below the knee femoropopliteal arteries) in an individual (such as human) comprising administering to the individual an effective amount of a composition comprising *Nab-*sirolimus, wherein the composition is administered intra-adventitially at a dose of about 40 µg/cm of desired vessel treatment length, and wherein the composition is administered to the adventitia using a micro-infusion catheter (such as a Bullfrog^{®} micro-infusion catheter). In some embodiments, the method improves luminal diameter of the blood vessel. In some embodiments, the method improves outcomes of femoropopliteal revascularization after balloon angioplasty and provisional stenting of the popliteal and contiguous peripheral arteries. In some embodiments, the individual has a de novo atherosclerotic lesion greater than about 70% in the popliteal artery, allowing lesion extension into contiguous arteries that totals up to 15 cm in length, and with a reference vessel diameter of about 3 mm to about 8 mm.

In some embodiments, there is provided a method of treating a peripheral artery disease (such as restenotic symptomatic lesions after revascularization of the above or below the knee femoropopliteal arteries) in an individual (such as human) comprising administering to the individual an effective amount of a composition comprising *Nab*-sirolimus, wherein the composition is administered intra-adventitially at a dose of about 100 µg/cm of desired vessel treatment length. In some embodiments, there is provided a method of treating a peripheral artery disease (such as restenotic symptomatic lesions after revascularization of the above or below the knee femoropopliteal arteries) in an individual (such as human) comprising administering to the individual an effective amount of a composition comprising *Nab*-sirolimus, wherein the composition is administered intra-adventitially at a dose of about 100 µg/cm of desired vessel treatment length, and wherein the composition is administered to the adventitia using a micro-infusion catheter (such as a Bullfrog^{®} micro-infusion catheter). In some embodiments, the method improves luminal diameter of the blood vessel. In some embodiments, the method improves outcomes of femoropopliteal revascularization after balloon angioplasty and provisional stenting of the popliteal and contiguous peripheral arteries. In some embodiments, the individual has a de novo atherosclerotic lesion greater than about 70% in the popliteal artery, allowing lesion extension into contiguous arteries that totals up to 15 cm in length, and with a reference vessel diameter of about 3 mm to about 8 mm.

In some embodiments, there is provided a method of treating a pulmonary arterial hypertension (PAH, such as severe progressive PAH on maximal currently available background therapy) in an individual (such as human) comprising administering to the individual an effective amount of a composition comprising nanoparticles comprising a limus drug and an albumin, wherein the composition is administered at a dose of about 20 mg/m². In some embodiments, a pulmonary arterial hypertension (PAH, such as severe progressive PAH on maximal currently available background therapy) in an individual (such as human) comprising administering to the individual an effective amount of a composition comprising nanoparticles comprising a limus drug and an albumin, wherein the composition is administered at a dose of about 20 mg/m², and wherein the composition is administered weekly. In some embodiments, a pulmonary arterial hypertension (PAH, such as severe progressive PAH on maximal currently available background therapy) in an individual (such as human) comprising administering to the individual an effective amount of a composition comprising nanoparticles comprising a limus drug and an albumin, wherein the composition is administered at a dose of about 20 mg/m², and wherein the composition is administered weekly, and wherein the dose is administered by intravenous infusion. In some embodiments, the individual is treated for about 16 months to about 24 months. In some embodiments, the currently available background therapy comprises at least two drugs including an oral agent comprising an endothelin receptor antagonist, a phosphodiesterase type 5 inhibitor, or a prostacyclin analogue. In some embodiments, the nanoparticles in the composition have an average particle size of no greater than about 150 nm (such as no greater than about 120 nm). In some embodiments, the nanoparticles in the composition comprise a limus drug associated (*e.g*., coated) with albumin, wherein the nanoparticles have an average particle size of no greater than about 150 nm (such as no greater than about 120 nm). In some embodiments, the nanoparticles in the composition comprise sirolimus associated (*e.g*., coated) with human albumin, wherein the nanoparticles have an average particle size of no greater than about 150 nm (such as no greater than about 120 nm, for example about 100 nm), wherein the weight ratio of human albumin and sirolimus in the composition is about 9:1 or less (such as about 9:1 or about 8:1). In some embodiments, the composition comprises *Nab*-sirolimus. In some embodiments, the composition is *Nab*-sirolimus.

In some embodiments, there is provided a method of treating a pulmonary arterial hypertension (PAH, such as severe progressive PAH on maximal currently available background therapy) in an individual (such as human) comprising administering to the individual an effective amount of a composition comprising nanoparticles comprising a limus drug and an albumin, wherein the composition is administered at a dose of about 45 mg/m². In some embodiments, a pulmonary arterial hypertension (PAH, such as severe progressive PAH on maximal currently available background therapy) in an individual (such as human) comprising administering to the individual an effective amount of a composition comprising nanoparticles comprising a limus drug and an albumin, wherein the composition is administered at a dose of about 45 mg/m², and wherein the composition is administered weekly. In some embodiments, a pulmonary arterial hypertension (PAH, such as severe progressive PAH on maximal currently available background therapy) in an individual (such as human) comprising administering to the individual an effective amount of a composition comprising nanoparticles comprising a limus drug and an albumin, wherein the composition is administered at a dose of about 45 mg/m², and wherein the composition is administered weekly, and wherein the dose is administered by intravenous infusion. In some embodiments, the individual is treated for about 16 months to about 24 months. In some embodiments, the currently available background therapy comprises at least two drugs including an oral agent comprising an endothelin receptor antagonist, a phosphodiesterase type 5 inhibitor, or a prostacyclin analogue. In some embodiments, the nanoparticles in the composition have an average particle size of no greater than about 150 nm (such as no greater than about 120 nm). In some embodiments, the nanoparticles in the composition comprise a limus drug associated (*e.g*., coated) with albumin, wherein the nanoparticles have an average particle size of no greater than about 150 nm (such as no greater than about 120 nm). In some embodiments, the nanoparticles in the composition comprise sirolimus associated (e.g., coated) with human albumin, wherein the nanoparticles have an average particle size of no greater than about 150 nm (such as no greater than about 120 nm, for example about 100 nm), wherein the weight ratio of human albumin and sirolimus in the composition is about 9:1 or less (such as about 9:1 or about 8:1). In some embodiments, the composition comprises *Nab*-sirolimus. In some embodiments, the composition is *Nab*-sirolimus.

In some embodiments, there is provided a method of treating a pulmonary arterial hypertension (PAH, such as severe progressive PAH on maximal currently available background therapy) in an individual (such as human) comprising administering to the individual an effective amount of a composition comprising nanoparticles comprising a limus drug and an albumin, wherein the composition is administered at a dose of about 75 mg/m². In some embodiments, a pulmonary arterial hypertension (PAH, such as severe progressive PAH on maximal currently available background therapy) in an individual (such as human) comprising administering to the individual an effective amount of a composition comprising nanoparticles comprising a limus drug and an albumin, wherein the composition is administered at a dose of about 75 mg/m², and wherein the composition is administered weekly. In some embodiments, a pulmonary arterial hypertension (PAH, such as severe progressive PAH on maximal currently available background therapy) in an individual (such as human) comprising administering to the individual an effective amount of a composition comprising nanoparticles comprising a limus drug and an albumin, wherein the composition is administered at a dose of about 75 mg/m², and wherein the composition is administered weekly, and wherein the dose is administered by intravenous infusion. In some embodiments, the individual is treated for about 16 months to about 24 months. In some embodiments, the currently available background therapy comprises at least two drugs including an oral agent comprising an endothelin receptor antagonist, a phosphodiesterase type 5 inhibitor, or a prostacyclin analogue. In some embodiments, the nanoparticles in the composition have an average particle size of no greater than about 150 nm (such as no greater than about 120 nm). In some embodiments, the nanoparticles in the composition comprise a limus drug associated (*e.g.,* coated) with albumin, wherein the nanoparticles have an average particle size of no greater than about 150 nm (such as no greater than about 120 nm). In some embodiments, the nanoparticles in the composition comprise sirolimus associated (*e.g*., coated) with human albumin, wherein the nanoparticles have an average particle size of no greater than about 150 nm (such as no greater than about 120 nm, for example about 100 nm), wherein the weight ratio of human albumin and sirolimus in the composition is about 9:1 or less (such as about 9:1 or about 8:1). In some embodiments, the composition comprises *Nab*-sirolimus. In some embodiments, the composition is *Nab*-sirolimus.

In some embodiments, there is provided a method of treating a pulmonary arterial hypertension (PAH, such as severe progressive PAH on maximal currently available background therapy) in an individual (such as human) comprising administering to the individual an effective amount of a composition comprising *Nab*-sirolimus, wherein the composition is administered at a dose of about 20 mg/m². In some embodiments, a pulmonary arterial hypertension (PAH, such as severe progressive PAH on maximal currently available background therapy) in an individual (such as human) comprising administering to the individual an effective amount of a composition comprising *Nab*-sirolimus, wherein the composition is administered at a dose of about 20 mg/m², and wherein the composition is administered weekly. In some embodiments, a pulmonary arterial hypertension (PAH, such as severe progressive PAH on maximal currently available background therapy) in an individual (such as human) comprising administering to the individual an effective amount of a composition comprising *Nab*-sirolimus, wherein the composition is administered at a dose of about 20 mg/m², and wherein the composition is administered weekly, and wherein the dose is administered by intravenous infusion. In some embodiments, the individual is treated for about 16 months to about 24 months. In some embodiments, the currently available background therapy comprises at least two drugs including an oral agent comprising an endothelin receptor antagonist, a phosphodiesterase type 5 inhibitor, or a prostacyclin analogue.

In some embodiments, there is provided a method of treating a pulmonary arterial hypertension (PAH, such as severe progressive PAH on maximal currently available background therapy) in an individual (such as human) comprising administering to the individual an effective amount of a composition comprising *Nab*-sirolimus, wherein the composition is administered at a dose of about 45 mg/m². In some embodiments, a pulmonary arterial hypertension (PAH, such as severe progressive PAH on maximal currently available background therapy) in an individual (such as human) comprising administering to the individual an effective amount of a composition comprising *Nab-*sirolimus, wherein the composition is administered at a dose of about 45 mg/m², and wherein the composition is administered weekly. In some embodiments, a pulmonary arterial hypertension (PAH, such as severe progressive PAH on maximal currently available background therapy) in an individual (such as human) comprising administering to the individual an effective amount of a composition comprising *Nab*-sirolimus, wherein the composition is administered at a dose of about 45 mg/m², and wherein the composition is administered weekly, and wherein the dose is administered by intravenous infusion. In some embodiments, the individual is treated for about 16 months to about 24 months. In some embodiments, the currently available background therapy comprises at least two drugs including an oral agent comprising an endothelin receptor antagonist, a phosphodiesterase type 5 inhibitor, or a prostacyclin analogue.

In some embodiments, there is provided a method of treating a pulmonary arterial hypertension (PAH, such as severe progressive PAH on maximal currently available background therapy) in an individual (such as human) comprising administering to the individual an effective amount of a composition comprising *Nab*-sirolimus, wherein the composition is administered at a dose of about 75 mg/m². In some embodiments, a pulmonary arterial hypertension (PAH, such as severe progressive PAH on maximal currently available background therapy) in an individual (such as human) comprising administering to the individual an effective amount of a composition comprising *Nab*-sirolimus, wherein the composition is administered at a dose of about 75 mg/m², and wherein the composition is administered weekly. In some embodiments, a pulmonary arterial hypertension (PAH, such as severe progressive PAH on maximal currently available background therapy) in an individual (such as human) comprising administering to the individual an effective amount of a composition comprising *Nab*-sirolimus, wherein the composition is administered at a dose of about 75 mg/m², and wherein the composition is administered weekly, and wherein the dose is administered by intravenous infusion. In some embodiments, the individual is treated for about 16 months to about 24 months. In some embodiments, the currently available background therapy comprises at least two drugs including an oral agent comprising an endothelin receptor antagonist, a phosphodiesterase type 5 inhibitor, or a prostacyclin analogue.

The methods provided herein may be practiced in an adjuvant setting. In some embodiments, the method is practiced in a neoadjuvant setting, i.e., the method may be carried out before the primary/definitive therapy. In some embodiments, the method is used to treat an individual who has previously been treated. In some embodiments, the individual has not previously been treated. In some embodiments, the method is used as a first line therapy. In some embodiments, the method is used as a second line therapy.

In some embodiments, the individual has not been previously treated with an mTOR inhibitor. In some embodiments, the individual has not been previously treated with a limus drug. In some embodiments, the individual has been treated for NMIBC, PAD or PAH previously. In some embodiments, the individual is resistant to treatment of NMIBC, PAD or PAH with other agents (such as non-nanoparticle formulations of mTOR inhibitors). In some embodiments, the individual is initially responsive to treatment of NMIBC, PAD or PAH with other agents but has progressed after treatment. In some embodiments, the individual has been treated previously with chemotherapy, radiation, or surgery.

Also provided are pharmaceutical compositions comprising nanoparticles comprising an mTOR inhibitor (such as limus drug, for example sirolimus) for use in any of the methods of treating NMIBC (such as BCG refractory or recurrent BCG), PAD (such as restenotic symptomatic lesions after revascularization of the above or below the knee femoropopliteal arteries) or PAH (such as severe progressive PAH on maximal currently available background therapy) described herein. In some embodiments, the compositions comprise nanoparticles comprising an mTOR inhibitor (such as limus drug, for example sirolimus) and albumin (such as human albumin).

### Methods of treating pediatric solid tumors

One aspect of the present application provides methods and compositions for treating pediatric solid tumors using a composition comprising nanoparticles comprising an mTOR inhibitor (such as limus drug, for example sirolimus) and albumin. The individual receiving the treatment may or may not have an mTOR-activating aberration as described above. In some embodiments, the individual is selected for the treatment based on having an mTOR-activating aberration as described above. In some embodiments, the status of any of the mTOR-activating aberrations as described above is not used as the basis for selecting the individual for the treatment.

In some embodiments, there is provided a method of treating solid tumor (such as recurrent or refractory solid tumor) in a human individual comprising administering to the individual an effective amount of a composition comprising nanoparticles comprising an mTOR inhibitor (such as limus drug, for example sirolimus) and albumin, wherein the individual is no more than about 21 years old (such as no more than about 18 years old). In some embodiments, the composition comprising nanoparticles comprises a limus drug and an albumin, wherein the limus drug in the nanoparticles is associated (*e.g*., coated) with the albumin. In some embodiments, the composition comprising nanoparticles comprises a limus drug and an albumin, wherein the nanoparticles have an average particle size of no greater than about 150 nm (such as no greater than about 120 nm). In some embodiments, the composition comprising nanoparticles comprises sirolimus and human serum albumin, wherein the nanoparticles comprise sirolimus associated (*e.g.,* coated) with human serum albumin, wherein the nanoparticles have an average particle size of no greater than about 150 nm (such as no greater than about 120 nm, for example about 100 nm), and wherein the weight ratio of human albumin and sirolimus in the composition is about 9:1 or less (such as about 9:1 or about 8:1). In some embodiments, the composition comprising nanoparticles comprises *Nab*-sirolimus. In some embodiments, the composition comprising nanoparticles is *Nab*-sirolimus. In some embodiments, the individual is no more than about any of 17, 16, 15, 14, 13, 12, 11, 10, 9, 8, 7, 6, 5, 4, 3, 2, or 1 year old. In some embodiments, the individual is about 9 to about 15 years old. In some embodiments, the individual is about 5 to about 9 years old. In some embodiments, the individual is about 1 to about 5 years old. In some embodiments, the individual is no more than about 1 year old, such as about 6 months old to about 1 year old, less than about 6 months old, or less than about 3 months old. In some embodiments, the method further comprises administering to the individual an effective amount of a second agent, such as a chemotherapy agent, for example vincristine, or irinotecan and temozolomide. In some embodiments, the second agent and the nanoparticle composition are administered sequentially. In some embodiments, the second agent and the nanoparticle composition are administered simultaneously. In some embodiments, the second agent and the nanoparticle composition are administered concurrently.

In some embodiments, the solid tumor is sarcoma. In some embodiments, the solid tumor is carcinoma (such as adenocarcinoma). In some embodiments, the solid tumor is an abdominal tumor, a soft tissue tumor, a bone tumor, or an eye tumor. In some embodiments, the solid tumor is a brain tumor. In some embodiments, the solid tumor is melanoma. In some embodiments, the method further comprises a step of selecting the individual for treatment based on the expression level of S6K1 and/or 4EBP1. In some embodiments, the method further comprises a step of determining the expression level of S6K1 and/or 4EBP1 in the individual. In some embodiments, the solid tumor is selected from the group consisting of neuroblastoma, soft tissue tumor (such as rhabdomyosarcoma), bone tumor (such as osteosarcoma, Ewing's sarcoma), CNS tumor (such as meduloblastoma, glioma), renal tumor, hepatic tumor (such as hepatoblastoma and hepatocellular carcinoma), and vascular tumors (such as Kaposi' sarcoma, angiosarcoma, Tufted angioma, and kaposiform hemangioendothelioma).

In some embodiments, the solid tumor is a soft tissue sarcoma, such as rhabdomyosarcoma. Thus, for example, in some embodiments, there is provided a method of treating a soft tissue sarcoma in a human individual, comprising administering to the individual an effective amount of a composition comprising nanoparticles comprising an mTOR inhibitor (such as limus drug, for example sirolimus) and albumin, wherein the individual is no more than about 21 years old (such as no more than about 18 years old). In some embodiments, there is provided a method of treating rhabdomyosarcoma in a human individual, comprising administering to the individual an effective amount of a composition comprising nanoparticles comprising an mTOR inhibitor (such as limus drug, for example sirolimus) and albumin, wherein the individual is no more than about 21 years old (such as no more than about 18 years old). In some embodiments, the composition comprising nanoparticles comprises a limus drug and an albumin, wherein the limus drug in the nanoparticles is associated (e.g., coated) with the albumin. In some embodiments, the composition comprising nanoparticles comprises a limus drug and an albumin, wherein the nanoparticles have an average particle size of no greater than about 150 nm (such as no greater than about 120 nm). In some embodiments, the composition comprising nanoparticles comprises sirolimus and human serum albumin, wherein the nanoparticles comprise sirolimus associated (*e.g*., coated) with human serum albumin, wherein the nanoparticles have an average particle size of no greater than about 150 nm (such as no greater than about 120 nm, for example about 100 nm), and wherein the weight ratio of human albumin and sirolimus in the composition is about 9:1 or less (such as about 9:1 or about 8:1). In some embodiments, the composition comprising nanoparticles comprises *Nab*-sirolimus. In some embodiments, the composition comprising nanoparticles is *Nab*-sirolimus. In some embodiments, the individual is no more than about any of 17, 16, 15, 14, 13, 12, 11, 10, 9, 8, 7, 6, 5, 4, 3, 2, or 1 year old. In some embodiments, the individual is about 9 to about 15 years old. In some embodiments, the individual is about 5 to about 9 years old. In some embodiments, the individual is about 1 to about 5 years old. In some embodiments, the individual is no more than about 1 year old, such as about 6 months old to about 1 year old, less than about 6 months old, or less than about 3 months old. In some embodiments, the method further comprises administering to the individual an effective amount of a second agent, such as a chemotherapy agent, for example irinotecan and temozolomide. In some embodiments, the second agent and the nanoparticle composition are administered sequentially. In some embodiments, the second agent and the nanoparticle composition are administered simultaneously. In some embodiments, the second agent and the nanoparticle composition are administered concurrently.

Rhabdomyosarcoma (RMS) is a cancer of the connective tissue that can arise from mesenchymal cells (i.e., skeletal muscle progenitor cells). RMS can also be found attached to muscle tissue, wrapped around intestines, or in any anatomic location. Most RMS occurs in areas naturally lacking in skeletal muscle, such as the head, neck, or genitourinary tract. Its two most common forms are embryonal RMS and alveolar RMS. Embryonal RMA is more common in infants and younger children, and the cancer cells resemble those of a typical 6-to-8-week embryo. Alveolar RMS is more common in older children and teenagers, and the cancer cells resemble those of a 10-to-12-week embryo. Alveolar RMS can occur in the large muscles of the trunk and legs.

In Stage 1 RMS, the tumor has started in a favorable site, *e.g*., the orbit of the eye, the head and neck area, a genital or urinary site (except the bladder and prostate), or in the bile ducts. A Stage 1 RMS tumor can be any size and may have grown into nearby areas and/or spread to nearby lymph nodes. A Stage 1 RMS tumor has not spread to distant sites. In Stage 2 RMS, the tumor has started in an unfavorable site, *e.g*., bladder or prostate, arm or leg, a parameningeal site, or any other site listed in Stage 1. The tumor is about 2 inches or smaller across and has not spread to nearby lymph nodes or distant sites. In Stage 3 RMS, the tumor has started in an unfavorable site, and is either < 2 inches across but has spread to nearby lymph nodes or is > 2 inches across and may or may not have spread to the lymph nodes. In either case, the cancer has not spread to distant sites. In Stage 4, the cancer can have started at any site and can be of any size, but it has spread to distant sites such as the bone marrow, lungs, liver, bones, or bone marrow.

The prognosis for a child or adolescent with rhabdomyosarcoma is related to, but not limited to, the age of the patient, site of origin, tumor size (widest diameter), resectability, presence of metastases, number of metastatic sites or tissues involved, presence or absence of regional lymph node involvement, histopathologic subtype (alveolar vs. embryonal) as well as unique biological characteristics of rhabdomyosarcoma tumor cells. Rhabdomyosarcoma is usually curable in most children with localized disease, with more than 70% surviving 5 years after diagnosis. Relapses are uncommon after 5 years of disease-free survival, with a 9% late-event rate at 10 years. Relapses, however, are more common for patients who have gross residual disease in unfavorable sites following initial surgery and those who have metastatic disease at diagnosis.

Thus, in some embodiments, the solid tumor is embryonal rhabdomyosarcoma. In some embodiments, the solid tumor is alveolar RMS (for example alveolar in the large muscles of the trunk and/or legs). In some embodiments, the individual has Stage 1 rhabdomyosarcoma. In some embodiments, the individual has Stage 2 rhabdomyosarcoma. In some embodiments, the individual has Stage 3 rhabdomyosarcoma. In some embodiments, the individual has Stage 4 rhabdomyosarcoma. In some embodiments, the individual having rhabdomyosarcoma is about 6 months to about 7 years old, for example about 6 months to about 5 years old. In some embodiments, the individual having rhabdomyosarcoma is about 9 to about 15 years old, for example about 11 to about 15 years old. In some embodiments, the individual has had a prior treatment, and has had a treatment free period for 3, 4, or 5 years or more.

In some embodiments, the solid tumor is neuroblastoma. For example, in some embodiments, there is provided a method of treating neuroblastoma in a human individual, comprising administering to the individual an effective amount of a composition comprising nanoparticles comprising an mTOR inhibitor (such as limus drug, for example sirolimus) and albumin, wherein the individual is no more than about 21 years old (such as no more than about 18 years old). In some embodiments, the composition comprising nanoparticles comprises a limus drug and an albumin, wherein the limus drug in the nanoparticles is associated (e.g., coated) with the albumin. In some embodiments, the composition comprising nanoparticles comprises a limus drug and an albumin, wherein the nanoparticles have an average particle size of no greater than about 150 nm (such as no greater than about 120 nm). In some embodiments, the composition comprising nanoparticles comprises sirolimus and human serum albumin, wherein the nanoparticles comprise sirolimus associated (*e.g*., coated) with human serum albumin, wherein the nanoparticles have an average particle size of no greater than about 150 nm (such as no greater than about 120 nm, for example about 100 nm), and wherein the weight ratio of human albumin and sirolimus in the composition is about 9:1 or less (such as about 9:1 or about 8:1). In some embodiments, the composition comprising nanoparticles comprises *Nab-*sirolimus. In some embodiments, the composition comprising nanoparticles is *Nab*-sirolimus. In some embodiments, the individual is no more than about any of 17, 16, 15, 14, 13, 12, 11, 10, 9, 8, 7, 6, 5, 4, 3, 2, or 1 year old. In some embodiments, the individual is about 9 to about 15 years old. In some embodiments, the individual is about 5 to about 9 years old. In some embodiments, the individual is about 1 to about 5 years old. In some embodiments, the individual is no more than about 1 year old, such as about 6 months old to about 1 year old, less than about 6 months old, or less than about 3 months old. In some embodiments, the method further comprises administering to the individual an effective amount of a second agent, such as a chemotherapy agent, for example, irinotecan and temozolomide. In some embodiments, the second agent and the nanoparticle composition are administered sequentially. In some embodiments, the second agent and the nanoparticle composition are administered simultaneously. In some embodiments, the second agent and the nanoparticle composition are administered concurrently.

Neuroblastoma is the most common extracranial solid tumor cancer in childhood and the most common cancer in infancy. Neuroblastoma has an incidence rate of about 650 cases per year in the United States. Neuroblastoma is a neuroendocrine tumor that arises from any neural crest element of the sympathetic nervous system. It frequently originates in one of the adrenal glands, but it can also develop in nerve tissues in the head, neck, chest, and abdomen. In Stage 1 neuroblastoma, the tumor is in only one area and all of the tumor that can be seen can be removed during surgery. In Stage 2A, the tumor is in only one area, but all of the tumor that can be seen cannot be removed during surgery. In Stage 2B, the tumor is in only one area, all of the tumor that can be seen may be completely removed during surgery, and cancer cells are found in the lymph nodes near the tumor. In Stage 3, the tumor cannot be completely removed during surgery, has spread from one side of the body to the other, and may have also spread to nearby lymph nodes. In Stage 4, the tumor has spread to distant lymph nodes, the skin, bone marrow, bone, liver, or the other parts of the body. Stage 4S is diagnosed in infants less than 12 months old with localized primary tumor as defined in Stage 1 or 2, with dissemination limited to liver, skin, or bone marrow. Between 20%-50% of high-risk neuroblastoma cases do not respond adequately to induction high-dose chemotherapy and are progressive or refractory. Relapse after completion of frontline therapy is also common. Growth reduction, thyroid function disorders, learning difficulties, and greater risk of secondary cancers affect survivors of high-risk disease.

Thus, in some embodiments, the solid tumor is Stage I neuroblastoma. In some embodiments, the solid tumor is Stage 2A neuroblastoma. In some embodiments, the solid tumor is Stage I neuroblastoma. In some embodiments, the solid tumor is Stage 3 neuroblastoma. In some embodiments, the solid tumor is Stage I neuroblastoma. In some embodiments, the solid tumor is Stage 4S neuroblastoma. In some embodiments, the individual has neuroblastoma and has had a prior therapy (such as a prior high-dose chemotherapy). In some embodiments, the individual has neuroblastoma and has had a prior therapy (such as a prior high-dose chemotherapy) and is progressive or refractory to the prior therapy.

In some embodiments, the solid tumor is a bone tumor, such as osteosarcoma or Ewing's sarcoma. For example, in some embodiments, there is provided a method of treating osteosarcoma in a human individual, comprising administering to the individual an effective amount of a composition comprising nanoparticles comprising an mTOR inhibitor (such as limus drug, for example sirolimus) and albumin, wherein the individual is no more than about 21 years old (such as no more than about 18 years old). In some embodiments, there is provided a method of treating Ewing's sarcoma in a human individual, comprising administering to the individual an effective amount of a composition comprising nanoparticles comprising an mTOR inhibitor (such as limus drug, for example sirolimus) and albumin, wherein the individual is no more than about 21 years old (such as no more than about 18 years old). In some embodiments, the composition comprising nanoparticles comprises a limus drug and an albumin, wherein the limus drug in the nanoparticles is associated (*e.g*., coated) with the albumin. In some embodiments, the composition comprising nanoparticles comprises a limus drug and an albumin, wherein the nanoparticles have an average particle size of no greater than about 150 nm (such as no greater than about 120 nm). In some embodiments, the composition comprising nanoparticles comprises sirolimus and human serum albumin, wherein the nanoparticles comprise sirolimus associated (*e.g*., coated) with human serum albumin, wherein the nanoparticles have an average particle size of no greater than about 150 nm (such as no greater than about 120 nm, for example about 100 nm), and wherein the weight ratio of human albumin and sirolimus in the composition is about 9:1 or less (such as about 9:1 or about 8:1). In some embodiments, the composition comprising nanoparticles comprises *Nab*-sirolimus. In some embodiments, the composition comprising nanoparticles is *Nab*-sirolimus. In some embodiments, the individual is no more than about any of 17, 16, 15, 14, 13, 12, 11, 10, 9, 8, 7, 6, 5, 4, 3, 2, or 1 year old. In some embodiments, the individual is about 9 to about 15 years old. In some embodiments, the individual is about 5 to about 9 years old. In some embodiments, the individual is about 1 to about 5 years old. In some embodiments, the individual is no more than about 1 year old, such as about 6 months old to about 1 year old, less than about 6 months old, or less than about 3 months old. In some embodiments, the method further comprises administering to the individual an effective amount of a second agent, such as a chemotherapy agent, for example, irinotecan and temozolomide. In some embodiments, the second agent and the nanoparticle composition are administered sequentially. In some embodiments, the second agent and the nanoparticle composition are administered simultaneously. In some embodiments, the second agent and the nanoparticle composition are administered concurrently.

Osteosarcoma (OS) is a malignant neoplasm arising from primitive transformed cells of mesenchymal origin that exhibit osteoblastic differentiation and produce malignant osteoid (i.e., the unmineralized, organic portion of the bone matrix that forms prior to the maturation of bone tissue). OS is the eighth most common form of childhood cancer, comprising 2.4% of all malignancies in pediatric patients. OS originates more frequently in the growing part of tubular long bones, with 42% occurring in the femur, 19% in the tibia, and 10%in the humerus. 8% of cases occur in the jaw, and another 8% occurs in the pelvis. OS is more prevalent in males than in females, and more prevalent in African-American and Hispanic children than in Caucasian children.

Osteosarcoma can be localized, metastatic, or recurrent. In localized OS, the cancer cells have not spread beyond the bone or nearby tissue win which the cancer began. In metastatic OS, the cancer cells have spread from the tissue of origin to other sites in the body (*e.g*., lungs, other bones). Recurrent OS refers to cases in which the cancer has recurred after treatment. The OS can come back in the tissues where it was first identified, or it may recur in another part of the body (*e.g*., the lung). Another way to describe the extent of OS is via the "TNM" system, in which the "T" refer to the size and location of the tumor, the "N" refers to whether the cancer has spread to the lymph nodes, and "M" refers to whether the cancer has metastasized to other parts of the body (Ritter et al. (2010) "Osteosarcoma." *Ann Oncol.* 21: vii320-vii325).

With treatment, the 5-year survival rates for patients with localized osteosarcoma can be in the range of 60%-80%. OS is more likely to be cures if the tumor is resectable. If metastases are present when the osteosarcoma is first diagnosed, the 5-year survival rate can be in the range or about 15%-30%. The survival rate can be higher if the cancer has spread only to the lungs or if all the tumors can be resected. Other factors that have been linked with an improved prognosis include, but are not limited to, age (younger), sex (female), tumor on arm or leg, tumor(s) being completely resectable, normal blood alkaline phosphatase and LDH levels, and good response to chemotherapy.

In some embodiments, the osteosarcoma is localized. In some embodiments, the osteosarcoma is resectable. In some embodiments, the osteosarcoma is metastatic. In some embodiments, the osteosarcoma is recurrent. In some embodiments, the individual has TX, T0, T1, T2, or T3 osteosarcoma. In some embodiments, the individual has NX, N0, or N1 osteosarcoma. In some embodiments, the individual has MX, M0, M1, M1a, or M1b osteosarcoma. In some embodiments, the individual has GX, G1, G2, G3, or G4 osteosarcoma. In some embodiments, the individual has Stage IA osteosarcoma (T1, N0, M0, G1-G2). In some embodiments, the individual has Stage IB osteosarcoma (T2, N0, M0, G1-G2). In some embodiments, the individual has Stage IIA osteosarcoma (T1, N0, M0, G3-G4). In some embodiments, the individual has Stage IIB osteosarcoma (T2, No, M0, G3-G4). In some embodiments, the individual has Stage III osteosarcoma (T3, N0, M0, any G). In some embodiments, the individual has Stage IVA osteosarcoma (any T, N0, M1a, any G). In some embodiments, the individual has Stage IVB (any T, N1, any M; or any T, any N, M1b, any G). In some embodiments, the individual having the osteosarcoma is a male. In some embodiments, the individual having the osteosarcoma is an African-American or Hispanic individual.

In some embodiments, the individual has Ewing's sarcoma. In some embodiments, the individual has localized Ewing's sarcoma. In some embodiments, the individual has metastatic Ewing's sarcoma. In some embodiments, the individual has Stage 1 Ewing's sarcoma. In some embodiments, the individual has Stage 2 Ewing's sarcoma. In some embodiments, the individual has Stage 3 Ewing's sarcoma. In some embodiments, the individual has Stage 4 Ewing's sarcoma. In some embodiments, the individual has recurrent Ewing's sarcoma.

In some embodiments, the solid tumor is a central nervous system (CNS) tumor, such as medulloblastoma, or glioma. For example, in some embodiments, there is provided a method of treating medulloblastoma in a human individual, comprising administering to the individual an effective amount of a composition comprising nanoparticles comprising an mTOR inhibitor (such as limus drug, for example sirolimus) and albumin, wherein the individual is no more than about 21 years old (such as no more than about 18 years old). In some embodiments, there is provided a method of treating glioma in a human individual, comprising administering to the individual an effective amount of a composition comprising nanoparticles comprising an mTOR inhibitor (such as limus drug, for example sirolimus) and albumin, wherein the individual is no more than about 21 years old (such as no more than about 18 years old). In some embodiments, the composition comprising nanoparticles comprises a limus drug and an albumin, wherein the limus drug in the nanoparticles is associated (*e.g*., coated) with the albumin. In some embodiments, the composition comprising nanoparticles comprises a limus drug and an albumin, wherein the nanoparticles have an average particle size of no greater than about 150 nm (such as no greater than about 120 nm). In some embodiments, the composition comprising nanoparticles comprises sirolimus and human serum albumin, wherein the nanoparticles comprise sirolimus associated (*e.g*., coated) with human serum albumin, wherein the nanoparticles have an average particle size of no greater than about 150 nm (such as no greater than about 120 nm, for example about 100 nm), and wherein the weight ratio of human albumin and sirolimus in the composition is about 9:1 or less (such as about 9:1 or about 8:1). In some embodiments, the composition comprising nanoparticles comprises *Nab*-sirolimus. In some embodiments, the composition comprising nanoparticles is *Nab*-sirolimus. In some embodiments, the individual is no more than about any of 17, 16, 15, 14, 13, 12, 11, 10, 9, 8, 7, 6, 5, 4, 3, 2, or 1 year old. In some embodiments, the individual is about 9 to about 15 years old. In some embodiments, the individual is about 5 to about 9 years old. In some embodiments, the individual is about 1 to about 5 years old. In some embodiments, the individual is no more than about 1 year old, such as about 6 months old to about 1 year old, less than about 6 months old, or less than about 3 months old. In some embodiments, the method further comprises administering to the individual an effective amount of a second agent, such as a chemotherapy agent, for example, irinotecan and temozolomide. In some embodiments, the second agent and the nanoparticle composition are administered sequentially. In some embodiments, the second agent and the nanoparticle composition are administered simultaneously. In some embodiments, the second agent and the nanoparticle composition are administered concurrently.

In some embodiments, the solid tumor is a renal tumor. For example, in some embodiments, there is provided a method of treating renal tumor in a human individual, comprising administering to the individual an effective amount of a composition comprising nanoparticles comprising an mTOR inhibitor (such as limus drug, for example sirolimus) and albumin, wherein the individual is no more than about 21 years old (such as no more than about 18 years old). In some embodiments, the composition comprising nanoparticles comprises a limus drug and an albumin, wherein the limus drug in the nanoparticles is associated (*e.g*., coated) with the albumin. In some embodiments, the composition comprising nanoparticles comprises a limus drug and an albumin, wherein the nanoparticles have an average particle size of no greater than about 150 nm (such as no greater than about 120 nm). In some embodiments, the composition comprising nanoparticles comprises sirolimus and human serum albumin, wherein the nanoparticles comprise sirolimus associated (*e.g.*, coated) with human serum albumin, wherein the nanoparticles have an average particle size of no greater than about 150 nm (such as no greater than about 120 nm, for example about 100 nm), and wherein the weight ratio of human albumin and sirolimus in the composition is about 9:1 or less (such as about 9:1 or about 8:1). In some embodiments, the composition comprising nanoparticles comprises *Nab-*sirolimus. In some embodiments, the composition comprising nanoparticles is *N*ab-sirolimus. In some embodiments, the individual is no more than about any of 17, 16, 15, 14, 13, 12, 11, 10, 9, 8, 7, 6, 5, 4, 3, 2, or 1 year old. In some embodiments, the individual is about 9 to about 15 years old. In some embodiments, the individual is about 5 to about 9 years old. In some embodiments, the individual is about 1 to about 5 years old. In some embodiments, the individual is no more than about 1 year old, such as about 6 months old to about 1 year old, less than about 6 months old, or less than about 3 months old. In some embodiments, the method further comprises administering to the individual an effective amount of a second agent, such as a chemotherapy agent, for example, irinotecan and temozolomide. In some embodiments, the second agent and the nanoparticle composition are administered sequentially. In some embodiments, the second agent and the nanoparticle composition are administered simultaneously. In some embodiments, the second agent and the nanoparticle composition are administered concurrently.

In some embodiments, the solid tumor is a hepatic tumor, such as hepatoblastoma, or hepatocellular carcinoma. For example, in some embodiments, there is provided a method of treating hepatoblastoma in a human individual, comprising administering to the individual an effective amount of a composition comprising nanoparticles comprising an mTOR inhibitor (such as limus drug, for example sirolimus) and albumin, wherein the individual is no more than about 21 years old (such as no more than about 18 years old). In some embodiments, there is provided a method of treating hepatocellular carcinoma in a human individual, comprising administering to the individual an effective amount of a composition comprising nanoparticles comprising an mTOR inhibitor (such as limus drug, for example sirolimus) and albumin, wherein the individual is no more than about 21 years old (such as no more than about 18 years old). In some embodiments, the composition comprising nanoparticles comprises a limus drug and an albumin, wherein the limus drug in the nanoparticles is associated (*e.g*., coated) with the albumin. In some embodiments, the composition comprising nanoparticles comprises a limus drug and an albumin, wherein the nanoparticles have an average particle size of no greater than about 150 nm (such as no greater than about 120 nm). In some embodiments, the composition comprising nanoparticles comprises sirolimus and human serum albumin, wherein the nanoparticles comprise sirolimus associated (*e.g*., coated) with human serum albumin, wherein the nanoparticles have an average particle size of no greater than about 150 nm (such as no greater than about 120 nm, for example about 100 nm), and wherein the weight ratio of human albumin and sirolimus in the composition is about 9:1 or less (such as about 9:1 or about 8:1). In some embodiments, the composition comprising nanoparticles comprises *Nab*-sirolimus. In some embodiments, the composition comprising nanoparticles is *Nab*-sirolimus. In some embodiments, the individual is no more than about any of 17, 16, 15, 14, 13, 12, 11, 10, 9, 8, 7, 6, 5, 4, 3, 2, or 1 year old. In some embodiments, the individual is about 9 to about 15 years old. In some embodiments, the individual is about 5 to about 9 years old. In some embodiments, the individual is about 1 to about 5 years old. In some embodiments, the individual is no more than about 1 year old, such as about 6 months old to about 1 year old, less than about 6 months old, or less than about 3 months old. In some embodiments, the method further comprises administering to the individual an effective amount of a second agent, such as a chemotherapy agent, for example, irinotecan and temozolomide. In some embodiments, the second agent and the nanoparticle composition are administered sequentially. In some embodiments, the second agent and the nanoparticle composition are administered simultaneously. In some embodiments, the second agent and the nanoparticle composition are administered concurrently.

In some embodiments, there is provided a method of treating solid tumor (such as recurrent or refractory solid tumor) in a human individual comprising administering to the individual an effective amount of a composition comprising nanoparticles comprising an mTOR inhibitor (such as limus drug, for example sirolimus) and albumin, and administering to the individual an effective amount of irinotecan and temozolomide, wherein the individual is no more than about 21 years old (such as no more than about 18 years old). In some embodiments, the composition comprising nanoparticles comprises a limus drug and an albumin, wherein the limus drug in the nanoparticles is associated (*e.g*., coated) with the albumin. In some embodiments, the composition comprising nanoparticles comprises a limus drug and an albumin, wherein the nanoparticles have an average particle size of no greater than about 150 nm (such as no greater than about 120 nm). In some embodiments, the composition comprising nanoparticles comprises sirolimus and human serum albumin, wherein the nanoparticles comprise sirolimus associated (*e.g*., coated) with human serum albumin, wherein the nanoparticles have an average particle size of no greater than about 150 nm (such as no greater than about 120 nm, for example about 100 nm), and wherein the weight ratio of human albumin and sirolimus in the composition is about 9:1 or less (such as about 9:1 or about 8:1). In some embodiments, the composition comprising nanoparticles comprises *Nab*-sirolimus. In some embodiments, the composition comprising nanoparticles is *Nab*-sirolimus. In some embodiments, the individual is no more than about any of 17, 16, 15, 14, 13, 12, 11, 10, 9, 8, 7, 6, 5, 4, 3, 2, or 1 year old. In some embodiments, the individual is about 9 to about 15 years old. In some embodiments, the individual is about 5 to about 9 years old. In some embodiments, the individual is about 1 to about 5 years old. In some embodiments, the individual is no more than about 1 year old, such as about 6 months old to about 1 year old, less than about 6 months old, or less than about 3 months old. In some embodiments, irinotecan, temozolomide and the nanoparticle composition are administered sequentially. In some embodiments, irinotecan, temozolomide and the nanoparticle composition are administered simultaneously. In some embodiments, irinotecan, temozolomide and the nanoparticle composition are administered concurrently. In some embodiments, the solid tumor is selected from the group consisting of neuroblastoma, soft tissue tumor (*e.g*., rhabdomyosarcoma), bone tumor (*e.g*., osteosarcoma, Ewing's sarcoma), and CNS tumor (*e.g.,* meduloblastoma, glioma), renal tumor, hepatic tumor (*e.g.*, hepatoblastoma and hepatocellular carcinoma). In some embodiments, irinotecan is administered at a dose of about 90 mg/m². In some embodiments, irinotecan is administered orally. In some embodiments, irinotecan is administered once daily for first five days in a 3-week treatment cycle. In some embodiments, temozolomide is administered at a dose of about 125 mg/m². In some embodiments, temozolomide is administered orally. In some embodiments, temozolomide is administered once daily for first five days in a 3-week treatment cycle. In some embodiments, the nanoparticle composition is administered about 1 hour after irinotecan administration. In some embodiments, irinotecan is administered one hour after administration of temozolomide. In some embodiments, a diarrheal prophylaxis, such as cefixime, is administered, for example, about 2 days prior to the first dose of irinotecan, during irinotecan administration, and about 3 days after the last does of irinotecan of each cycle. In some embodiments, the method is repeated, such as for about 35 cycles.

In some embodiments, the solid tumor is a vascular tumor, such as high-risk vascular tumor, for example, Kaposi' sarcoma, angiosarcoma, Tufted angioma, and kaposiform hemangioendothelioma. For example, in some embodiments, there is provided a method of treating Kaposi' sarcoma in a human individual, comprising administering to the individual an effective amount of a composition comprising nanoparticles comprising an mTOR inhibitor (such as limus drug, for example sirolimus) and albumin, wherein the individual is no more than about 21 years old (such as no more than about 18 years old). In some embodiments, there is provided a method of treating angiosarcoma in a human individual, comprising administering to the individual an effective amount of a composition comprising nanoparticles comprising an mTOR inhibitor (such as limus drug, for example sirolimus) and albumin, wherein the individual is no more than about 21 years old (such as no more than about 18 years old). In some embodiments, there is provided a method of treating Tufted angioma in a human individual, comprising administering to the individual an effective amount of a composition comprising nanoparticles comprising an mTOR inhibitor (such as limus drug, for example sirolimus) and albumin, wherein the individual is no more than about 21 years old (such as no more than about 18 years old). In some embodiments, there is provided a method of treating kaposiform hemangioendothelioma in a human individual, comprising administering to the individual an effective amount of a composition comprising nanoparticles comprising an mTOR inhibitor (such as limus drug, for example sirolimus) and albumin, wherein the individual is no more than about 21 years old (such as no more than about 18 years old). In some embodiments, the composition comprising nanoparticles comprises a limus drug and an albumin, wherein the limus drug in the nanoparticles is associated (*e.g.*, coated) with the albumin. In some embodiments, the composition comprising nanoparticles comprises a limus drug and an albumin, wherein the nanoparticles have an average particle size of no greater than about 150 nm (such as no greater than about 120 nm). In some embodiments, the composition comprising nanoparticles comprises sirolimus and human serum albumin, wherein the nanoparticles comprise sirolimus associated (*e.g*., coated) with human serum albumin, wherein the nanoparticles have an average particle size of no greater than about 150 nm (such as no greater than about 120 nm, for example about 100 nm), and wherein the weight ratio of human albumin and sirolimus in the composition is about 9:1 or less (such as about 9:1 or about 8:1). In some embodiments, the composition comprising nanoparticles comprises *Nab*-sirolimus. In some embodiments, the composition comprising nanoparticles is *Nab*-sirolimus. In some embodiments, the individual is no more than about any of 17, 16, 15, 14, 13, 12, 11, 10, 9, 8, 7, 6, 5, 4, 3, 2, or 1 year old. In some embodiments, the individual is about 9 to about 15 years old. In some embodiments, the individual is about 5 to about 9 years old. In some embodiments, the individual is about 1 to about 5 years old. In some embodiments, the individual is no more than about 1 year old, such as about 6 months old to about 1 year old, less than about 6 months old, or less than about 3 months old. In some embodiments, the method further comprises administering to the individual an effective amount of a second agent, such as a chemotherapy agent, such as vincristine. In some embodiments, the second agent and the nanoparticle composition are administered sequentially. In some embodiments, the second agent and the nanoparticle composition are administered simultaneously. In some embodiments, the second agent and the nanoparticle composition are administered concurrently.

Nab--rapamycin can be used for treatment of vascular tumors, such as Kaposi' sarcoma and angiosarcoma. Additionally, Tufted angioma and kaposiform hemangioendothelioma (KHE) are rare vascular tumors occurring during infancy or early childhood. The incidence of KHE is estimated at 0.07/100,000 children per year. Over 70 percent of KHE develop the Kasabach-Merritt phenomenon (KMP) - characterized by profound thrombocytopenia and consumption coagulopathy. Vincristine is often used as first-line treatment for KHE. A combination of vincristine and *Nab*-sirolimus (such as ABI-009) may be used for treatment of these high risk vascular tumors.

In some embodiments, there is provided a method of treating vascular tumor (such as Kaposi' sarcoma, angiosarcoma, Tufted angioma, and kaposiform hemangioendothelioma) in a human individual, comprising administering to the individual an effective amount of a composition comprising *Nab*-sirolimus, and administering to the individual an effective amount of vincristine, wherein the individual is no more than about 21 years old (such as no more than about 18 years old). In some embodiments, the *Nab*-sirolimus composition is administered intravenously. In some embodiments, the *Nab-*sirolimus composition is administered weekly. In some embodiments, the vincristine is administered intravenously. In some embodiments, vincristine and the *Nab*-sirolimus composition are administered sequentially. In some embodiments, vincristine and the *Nab*-sirolimus composition are administered simultaneously. In some embodiments, vincristine and the *Nab*-sirolimus composition are administered concurrently.

In some embodiments, the solid tumor is an early stage solid tumor, such as Stage 0, Stage I, or Stage II. In some embodiments, the solid tumor is a late stage cancer, such as Stage III or Stage TV. In some embodiments, the solid tumor is at stage IIIb or Stage IV.

In some embodiments, the individual is no more than about any of 17, 16, 15, 14, 13, 12, 11, 10, 9, 8, 7, 6, 5, 4, 3, 2, or 1 year old. In some embodiments, the individual is about 9 to about 15 years old. In some embodiments, the individual is about 5 to about 9 years old. In some embodiments, the individual is about 1 to about 5 years old. In some embodiments, the individual is no more than about 1 year old, such as about 6 months old to about 1 year old, less than about 6 months old, or less than about 3 months old. The methods described herein thus in some embodiments also encompasses selecting a human individual for treatment based on the age of the individual (such as the ages indicated above).

In some embodiments, the solid tumor is early stage cancer, non-metastatic cancer, primary cancer, advanced cancer, locally advanced cancer, metastatic cancer, cancer in remission, or recurrent cancer. In some embodiments, the solid tumor is localized resectable, localized unresectable, or unresectable. In some embodiments, the solid tumor is a progressive solid tumor. In some embodiments, the solid tumor is substantially refractory to hormone therapy. The methods provided herein can be practiced in an adjuvant setting. Alternatively, the methods can be practiced in a neoadjuvant setting. In some embodiments, the method is a first line therapy. In some embodiments, the method is a second line therapy.

In some embodiments, the method further comprises a step of selecting the patient for treatment based on the status of one or more biomarkers, such as any one of the biomarkers described in the section "Methods of Treatment Based on Status of an mTOR-activating Aberration". In some embodiments, the selecting is based on the expression level of S6K1 and/or 4EBP1. In some embodiments, the expression level of S6K1 and/or 4EBP1 is assessed by immunohistochemistry. Thus, for example, in some embodiments, a) determining the expression level of S6K1 and/or 4EBP1 in the individual, wherein the individual is no more than about 21 years old (such as no more than about 18 years old), and b) administering an effective amount of a composition comprising nanoparticles comprising an mTOR inhibitor (such as limus drug, for example sirolimus) and albumin to the individual. In some embodiments, there is provided a method of treating solid tumor in a human individual, the method comprising administering an effective amount of a composition comprising nanoparticles comprising an mTOR inhibitor (such as limus drug, for example sirolimus) and albumin to the individual, wherein the individual is no more than about 21 years old (such as no more than about 18 years old), and wherein said individual is selected for treatment based on the expression level of S6K1 and/or 4EBP1 in the individual. In some embodiments, the composition comprising nanoparticles comprises a limus drug and an albumin, wherein the limus drug in the nanoparticles is associated (*e.g*., coated) with the albumin. In some embodiments, the composition comprising nanoparticles comprises a limus drug and an albumin, wherein the nanoparticles have an average particle size of no greater than about 150 nm (such as no greater than about 120 nm). In some embodiments, the composition comprising nanoparticles comprises sirolimus and human serum albumin, wherein the nanoparticles comprise sirolimus associated (*e.g*., coated) with human serum albumin, wherein the nanoparticles have an average particle size of no greater than about 150 nm (such as no greater than about 120 nm, for example about 100 nm), and wherein the weight ratio of human albumin and sirolimus in the composition is about 9:1 or less (such as about 9:1 or about 8:1). In some embodiments, the composition comprising nanoparticles comprises *Nab*-sirolimus. In some embodiments, the composition comprising nanoparticles is *Nab*-sirolimus. In some embodiments, the individual is no more than about any of 17, 16, 15, 14, 13, 12, 11, 10, 9, 8, 7, 6, 5, 4, 3, 2, or 1 year old. In some embodiments, the individual is about 9 to about 15 years old. In some embodiments, the individual is about 5 to about 9 years old. In some embodiments, the individual is about 1 to about 5 years old. In some embodiments, the individual is no more than about 1 year old, such as about 6 months old to about 1 year old, less than about 6 months old, or less than about 3 months old. In some embodiments, the method further comprises administering to the individual an effective amount of a second agent, such as a chemotherapy agent, for example, vincristine, or irinotecan and temozolomide. In some embodiments, the second agent and the nanoparticle composition are administered sequentially. In some embodiments, the second agent and the nanoparticle composition are administered simultaneously. In some embodiments, the second agent and the nanoparticle composition are administered concurrently. In some embodiments, the method further comprises a step of selecting the individual for treatment based on the expression level of S6K1 and/or 4EBP1. In some embodiments, the method further comprises a step of determining the expression level of S6K1 and/or 4EBP1 in the individual. In some embodiments, the solid tumor is selected from the group consisting of neuroblastoma, soft tissue tumor (*e.g*., rhabdomyosarcoma), bone tumor (*e.g.,* osteosarcoma, Ewing's sarcoma), CNS tumor (*e.g*., meduloblastoma, glioma), renal tumor, hepatic tumor (*e.g*., hepatoblastoma and hepatocellular carcinoma), and vascular tumors (*e.g.,* Kaposi' sarcoma, angiosarcoma, Tufted angioma, and kaposiform hemangioendothelioma).

In some embodiments, the individual has been previously treated for the solid tumor (also referred to as the "prior therapy"). Thus, for example, in some embodiments, there is provided a method of treating a solid tumor in a human individual, comprising administering to the individual an effective amount of a composition comprising nanoparticles comprising an mTOR inhibitor (such as limus drug, for example sirolimus) and albumin, wherein the individual is no more than about 21 years old (such as no more than about 18 years old), and wherein the individual has been previously treated for the solid tumor. In some embodiments, there is provided a method of treating a sarcoma (such as a soft tissue sarcoma, for example rhabdomyosarcoma) in a human individual, comprising administering to the individual an effective amount of a composition comprising nanoparticles comprising an mTOR inhibitor (such as limus drug, for example sirolimus) and albumin, wherein the individual is no more than about 21 years old (such as no more than about 18 years old), and wherein the individual has been previously treated for the sarcoma. In some embodiments, there is provided a method of treating neuroblastoma in a human individual, comprising administering to the individual an effective amount of a composition comprising nanoparticles comprising an mTOR inhibitor (such as limus drug, for example sirolimus) and albumin, wherein the individual is no more than about 21 years old (such as no more than about 18 years old), and wherein the individual has been previously treated for neuroblastoma. In some embodiments, there is provided a method of treating bone tumor (such as osteosarcoma, or Ewing's sarcoma) in a human individual, comprising administering to the individual an effective amount of a composition comprising nanoparticles comprising an mTOR inhibitor (such as limus drug, for example sirolimus) and albumin, wherein the individual is no more than about 21 years old (such as no more than about 18 years old), and wherein the individual has been previously treated for bone tumor (such as osteosarcoma, or Ewing's sarcoma). In some embodiments, there is provided a method of treating CNS tumor (such as meduloblastoma or glioma) in a human individual, comprising administering to the individual an effective amount of a composition comprising nanoparticles comprising an mTOR inhibitor (such as limus drug, for example sirolimus) and albumin, wherein the individual is no more than about 21 years old (such as no more than about 18 years old), and wherein the individual has been previously treated for CNS tumor (such as meduloblastoma or glioma). In some embodiments, there is provided a method of treating renal tumor in a human individual, comprising administering to the individual an effective amount of a composition comprising nanoparticles comprising an mTOR inhibitor (such as limus drug, for example sirolimus) and albumin, wherein the individual is no more than about 21 years old (such as no more than about 18 years old), and wherein the individual has been previously treated for renal tumor. In some embodiments, there is provided a method of treating hepatic tumor (such as hepatoblastoma or hepatocellular carcinoma) in a human individual, comprising administering to the individual an effective amount of a composition comprising nanoparticles comprising an mTOR inhibitor (such as limus drug, for example sirolimus) and albumin, wherein the individual is no more than about 21 years old (such as no more than about 18 years old), and wherein the individual has been previously treated for hepatic tumor (such as hepatoblastoma or hepatocellular carcinoma). In some embodiments, there is provided a method of treating vascular tumor (such as Kaposi' sarcoma, angiosarcoma, Tufted angioma, or kaposiform hemangioendothelioma) in a human individual, comprising administering to the individual an effective amount of a composition comprising nanoparticles comprising an mTOR inhibitor (such as limus drug, for example sirolimus) and albumin, wherein the individual is no more than about 21 years old (such as no more than about 18 years old), and wherein the individual has been previously treated for vascular tumor (such as Kaposi' sarcoma, angiosarcoma, Tufted angioma, or kaposiform hemangioendothelioma). In some embodiments, there is provided a method of treating vascular tumor (such as Kaposi' sarcoma, angiosarcoma, Tufted angioma, or kaposiform hemangioendothelioma) in a human individual, comprising administering to the individual an effective amount of a composition comprising nanoparticles comprising an mTOR inhibitor (such as limus drug, for example sirolimus) and albumin, and administering to the individual an effective amount of vincristine, wherein the individual is no more than about 21 years old (such as no more than about 18 years old), and wherein the individual has been previously treated for vascular tumor (such as Kaposi' sarcoma, angiosarcoma, Tufted angioma, or kaposiform hemangioendothelioma). In some embodiments, the composition comprising nanoparticles comprises a limus drug and an albumin, wherein the limus drug in the nanoparticles is associated (*e.g*., coated) with the albumin. In some embodiments, the composition comprising nanoparticles comprises a limus drug and an albumin, wherein the nanoparticles have an average particle size of no greater than about 150 nm (such as no greater than about 120 nm). In some embodiments, the composition comprising nanoparticles comprises sirolimus and human serum albumin, wherein the nanoparticles comprise sirolimus associated (*e.g*., coated) with human serum albumin, wherein the nanoparticles have an average particle size of no greater than about 150 nm (such as no greater than about 120 nm, for example about 100 nm), and wherein the weight ratio of human albumin and sirolimus in the composition is about 9:1 or less (such as about 9:1 or about 8:1). In some embodiments, the composition comprising nanoparticles comprises *Nab*-sirolimus. In some embodiments, the composition comprising nanoparticles is *Nab*-sirolimus. In some embodiments, the individual is no more than about any of 17, 16, 15, 14, 13, 12, 11, 10, 9, 8, 7, 6, 5, 4, 3, 2, or 1 year old. In some embodiments, the individual is about 9 to about 15 years old. In some embodiments, the individual is about 5 to about 9 years old. In some embodiments, the individual is about 1 to about 5 years old. In some embodiments, the individual is no more than about 1 year old, such as about 6 months old to about 1 year old, less than about 6 months old, or less than about 3 months old. In some embodiments, the method further comprises administering to the individual an effective amount of a second agent, such as a chemotherapy agent for example, vincristine, or irinotecan and temozolomide. In some embodiments, the second agent and the nanoparticle composition are administered sequentially. In some embodiments, the second agent and the nanoparticle composition are administered simultaneously. In some embodiments, the second agent and the nanoparticle composition are administered concurrently.

In some embodiments, the individual has progressed on the prior therapy at the time of treatment. For example, the individual has progressed within any of about 1, 2, 3, 4, 5, 6, 7, 8, 9, 10, 11, or 12 months upon treatment with the prior therapy. In some embodiments, the individual is resistant or refractory to the prior therapy. In some embodiments, the individual is unsuitable to continue with the prior therapy (for example due to failure to respond and/or due to toxicity). In some embodiments, the individual has failed to respond to the prior therapy. In some embodiments, the individual is non-responsive to the prior therapy. In some embodiments, the individual is partially responsive to the prior therapy. In some embodiments, the individual exhibits a less desirable degree of responsiveness. In some embodiments, the individual exhibits enhanced responsiveness. In some embodiments, the individual has recurrent solid tumor, i.e., the individual is initially responsive to the treatment with the prior therapy, but develops solid tumor after about any of about 2, 3, 4, 5, 6, 7, 8, 9, 10, 11, 12, 24, 36, 48, or 60 months upon the cessation of the prior therapy.

In some embodiments, the prior therapy has stopped (for example for at least 1, 2, 3, 4, 5, 6, 7, 8, 9, 10, 11, 12, 24, 36, 48, or 60 months) when initiating the methods of the present invention. In some embodiments, the prior therapy has not stopped when initialing the methods of the present invention.

In some embodiments, the method further comprises a step of selecting patients for treatment based on the status of a prior therapy. For example, in some embodiments, there is provided a method of treating a solid tumor in a human individual who has been treated with a prior therapy, the method comprising: a) determining whether the individual has progressed on the prior therapy (such as mTOR inhibitor -based therapy), wherein the individual is no more than about 21 years old (such as no more than about 18 years old), and b) administering an effective amount of a composition comprising nanoparticles comprising an mTOR inhibitor (such as limus drug, for example sirolimus) and albumin to the individual. In some embodiments, there is provided a method of treating a solid tumor in a human individual who has been treated with a prior therapy, the method comprising: a) selecting the individual who is not responsive to the prior therapy (such as mTOR inhibitor -based therapy), wherein the individual is no more than about 21 years old (such as no more than about 18 years old), and b) administering an effective amount of a composition comprising nanoparticles comprising an mTOR inhibitor (such as limus drug, for example sirolimus) and albumin to the individual. In some embodiments, there is provided a method of treating solid tumor in a human individual who has been treated with a prior therapy (such as mTOR inhibitor -based therapy), the method comprising administering an effective amount of a composition comprising nanoparticles comprising an mTOR inhibitor (such as limus drug, for example sirolimus) and albumin to the individual, wherein the individual is no more than about 21 years old (such as no more than about 18 years old), and wherein said individual is selected for treatment based on the determination that the individual has progressed on the prior therapy. In some embodiments, there is provided a method of treating a solid tumor in a human individual who has been treated with a prior therapy (such as mTOR inhibitor-based therapy), the method comprising administering an effective amount of a composition comprising nanoparticles comprising an mTOR inhibitor (such as limus drug, for example sirolimus) and albumin to the individual, wherein the individual is no more than about 21 years old (such as no more than about 18 years old), and wherein said individual is selected on the basis of the non-responsiveness to the prior therapy. In some embodiments, the composition comprising nanoparticles comprises a limus drug and an albumin, wherein the limus drug in the nanoparticles is associated (*e.g*., coated) with the albumin. In some embodiments, the composition comprising nanoparticles comprises a limus drug and an albumin, wherein the nanoparticles have an average particle size of no greater than about 150 nm (such as no greater than about 120 nm). In some embodiments, the composition comprising nanoparticles comprises sirolimus and human serum albumin, wherein the nanoparticles comprise sirolimus associated (*e.g*., coated) with human serum albumin, wherein the nanoparticles have an average particle size of no greater than about 150 nm (such as no greater than about 120 nm, for example about 100 nm), and wherein the weight ratio of human albumin and sirolimus in the composition is about 9:1 or less (such as about 9:1 or about 8:1). In some embodiments, the composition comprising nanoparticles comprises *Nab*-sirolimus. In some embodiments, the composition comprising nanoparticles is *Nab*-sirolimus. In some embodiments, the individual is no more than about any of 17, 16, 15, 14, 13, 12, 11, 10, 9, 8, 7, 6, 5, 4, 3, 2, or 1 year old. In some embodiments, the individual is about 9 to about 15 years old. In some embodiments, the individual is about 5 to about 9 years old. In some embodiments, the individual is about 1 to about 5 years old. In some embodiments, the individual is no more than about 1 year old, such as about 6 months old to about 1 year old, less than about 6 months old, or less than about 3 months old. In some embodiments, the method further comprises administering to the individual an effective amount of a second agent, such as a chemotherapy agent, for example, vincristine, or irinotecan and temozolomide. In some embodiments, the second agent and the nanoparticle composition are administered sequentially. In some embodiments, the second agent and the nanoparticle composition are administered simultaneously. In some embodiments, the second agent and the nanoparticle composition are administered concurrently. In some embodiments, the method further comprises a step of selecting the individual for treatment based on the expression level of S6K1 and/or 4EBP1. In some embodiments, the method further comprises a step of determining the expression level of S6K1 and/or 4EBP1 in the individual. In some embodiments, the solid tumor is selected from the group consisting of neuroblastoma, soft tissue tumor (*e.g*., rhabdomyosarcoma), bone tumor (*e.g*., osteosarcoma, Ewing's sarcoma), CNS tumor (*e.g*., meduloblastoma, glioma), renal tumor, hepatic tumor (*e.g.*, hepatoblastoma and hepatocellular carcinoma), and vascular tumors (*e.g.,* Kaposi' sarcoma, angiosarcoma, Tufted angioma, and kaposiform hemangioendothelioma).

In some embodiments, there is provided a method of treating a solid tumor in a human individual who has been treated with a prior therapy (such as mTOR inhibitor-based therapy), the method comprising: a) determining whether the individual is suitable for continued treatment with the prior therapy (for example due to lack of responsiveness and/or toxicity), wherein the individual is no more than about 21 years old (such as no more than about 18 years old); and b) administering an effective amount of a composition comprising nanoparticles comprising an mTOR inhibitor (such as limus drug, for example sirolimus) and albumin to the individual. In some embodiments, there is provided a method of treating a solid tumor in a human individual who has been treated with a prior therapy (such as mTOR-inhibitor-based therapy), the method comprising administering an effective amount of a composition comprising nanoparticles comprising an mTOR inhibitor (such as limus drug, for example sirolimus) and albumin to the individual, wherein the individual is no more than about 21 years old (such as no more than about 18 years old), and wherein said individual is selected based on the determination that the individual is unsuitable for continued treatment with the prior therapy (for example due to lack of responsiveness and/or toxicity). A human individual can also be unsuitable for continued treatment with the prior therapy if the individual exhibits a less than desirable responsiveness or exhibits undesirable symptoms associated with the prior therapy. In some embodiments, the composition comprising nanoparticles comprises a limus drug and an albumin, wherein the limus drug in the nanoparticles is associated (*e.g.*, coated) with the albumin. In some embodiments, the composition comprising nanoparticles comprises a limus drug and an albumin, wherein the nanoparticles have an average particle size of no greater than about 150 nm (such as no greater than about 120 nm). In some embodiments, the composition comprising nanoparticles comprises sirolimus and human serum albumin, wherein the nanoparticles comprise sirolimus associated (*e.g.,* coated) with human serum albumin, wherein the nanoparticles have an average particle size of no greater than about 150 nm (such as no greater than about 120 nm, for example about 100 nm), and wherein the weight ratio of human albumin and sirolimus in the composition is about 9:1 or less (such as about 9:1 or about 8:1). In some embodiments, the composition comprising nanoparticles comprises *Nab*-sirolimus. In some embodiments, the composition comprising nanoparticles is *Nab*-sirolimus. In some embodiments, the individual is no more than about any of 17, 16, 15, 14, 13, 12, 11, 10, 9, 8, 7, 6, 5, 4, 3, 2, or 1 year old. In some embodiments, the individual is about 9 to about 15 years old. In some embodiments, the individual is about 5 to about 9 years old. In some embodiments, the individual is about 1 to about 5 years old. In some embodiments, the individual is no more than about 1 year old, such as about 6 months old to about 1 year old, less than about 6 months old, or less than about 3 months old. In some embodiments, the method further comprises administering to the individual an effective amount of a second agent, such as a chemotherapy agent, for example, vincristine, or irinotecan and temozolomide. In some embodiments, the second agent and the nanoparticle composition are administered sequentially. In some embodiments, the second agent and the nanoparticle composition are administered simultaneously. In some embodiments, the second agent and the nanoparticle composition are administered concurrently. In some embodiments, the method further comprises a step of selecting the individual for treatment based on the expression level of S6K1 and/or 4EBP1. In some embodiments, the method further comprises a step of determining the expression level of S6K1 and/or 4EBP1 in the individual. In some embodiments, the solid tumor is selected from the group consisting of neuroblastoma, soft tissue tumor (*e.g*., rhabdomyosarcoma), bone tumor (*e.g*., osteosarcoma, Ewing's sarcoma), CNS tumor (*e.g*., meduloblastoma, glioma), renal tumor, hepatic tumor *(*e.g*.,* hepatoblastoma and hepatocellular carcinoma), and vascular tumors (*e.g*., Kaposi' sarcoma, angiosarcoma, Tufted angioma, and kaposiform hemangioendothelioma). In some embodiments, the composition comprising nanoparticles comprises a limus drug and an albumin, wherein the limus drug in the nanoparticles is associated (*e.g*., coated) with the albumin. In some embodiments, the composition comprising nanoparticles comprises a limus drug and an albumin, wherein the nanoparticles have an average particle size of no greater than about 150 nm (such as no greater than about 120 nm). In some embodiments, the composition comprising nanoparticles comprises sirolimus and human serum albumin, wherein the nanoparticles comprise sirolimus associated (*e.g.,* coated) with human serum albumin, wherein the nanoparticles have an average particle size of no greater than about 150 nm (such as no greater than about 120 nm, for example about 100 nm), and wherein the weight ratio of human albumin and sirolimus in the composition is about 9:1 or less (such as about 9:1 or about 8:1). In some embodiments, the composition comprising nanoparticles comprises Nab-sirolimus. In some embodiments, the composition comprising nanoparticles is *Nab*-sirolionus. In some embodiments, the individual is no more than about any of 17, 16, 15, 14, 13, 12, 11, 10, 9, 8, 7, 6, 5, 4, 3, 2, or 1 year old. In some embodiments, the individual is about 9 to about 15 years old. In some embodiments, the individual is about 5 to about 9 years old. In some embodiments, the individual is about 1 to about 5 years old. In some embodiments, the individual is no more than about 1 year old, such as about 6 months old to about 1 year old, less than about 6 months old, or less than about 3 months old. In some embodiments, the method further comprises administering to the individual an effective amount of a second agent, such as a chemotherapy agent, for example, vincristine, or irinotecan and temozolomide. In some embodiments, the second agent and the nanoparticle composition are administered sequentially. In some embodiments, the second agent and the nanoparticle composition are administered simultaneously. In some embodiments, the second agent and the nanoparticle composition are administered concurrently. In some embodiments, the method further comprises a step of selecting the individual for treatment based on the expression level of S6K1 and/or 4EBP1. In some embodiments, the method further comprises a step of determining the expression level of S6K1 and/or 4EBP1 in the individual. In some embodiments, the solid tumor is selected from the group consisting of neuroblastoma, soft tissue tumor *(e.g.,* rhabdomyosarcoma), bone tumor *(e.g.,* osteosarcoma, Ewing's sarcoma), CNS tumor (e.g., meduloblastoma, glioma), renal tumor, hepatic tumor (e.g., hepatoblastoma and hepatocellular carcinoma), and vascular tumors (e.g., Kaposi' sarcoma, angiosarcoma, Tufted angioma, and kaposiform hemangioendothelioma).

In some embodiments, the prior therapy comprises administration of an mTOR-inhibitor ("mTOR-inhibitor-based therapy"), such as limus drug, for example sirolimus. In some embodiments, the prior therapy comprises the administration of Cosmegen (Dactinomycin, also known as actinomycin-D), Vincasar PFS (Vincristine Sulfate), cyclophosphamide, Doxorubicin Hydrochloride (Adriamycin PFS or Adriamycin RDF), carboplatin, cisplatin, etoposide, teniposide, cyclosporin, dacarbazine, epirubicin, gemcitabine, ifosfamide, methotrexate, topotecan, and/or dactinomycin. In some embodiments, the prior therapy comprises surgery.

In some embodiments, the method described herein comprises administering inTOR-inhibitor (such as limus drug, for example sirolimus) nanoparticle composition in conjunction with one or more of the same agent(s) used in the prior therapy. In some embodiments, the method described herein comprises administering mTOR-inhibitor (such as limus drug, for example sirolimus) nanoparticle composition in conjunction with the agent(s) that is not used in the prior therapy.

In some embodiments, the method comprises administering to the individual an effective amount of a composition comprising nanoparticles comprising a mTOR-inhibitor (such as limus drug, for example sirolimus) and an albumin, wherein the individual is no more than about 21 years old (such as no more than about 18 years old), and wherein the individual has progressed on a prior therapy (such as mTOR-inhibitor-based therapy). In some embodiments, the method comprises administering to the individual an effective amount of a composition comprising nanoparticles comprising a limus drug and an albumin, wherein the limus drug in the nanoparticles is associated (*e.g.*, coated) with the albumin, wherein the individual is no more than about 21 years old (such as no more than about 18 years old), and wherein the individual has progressed on a prior therapy (such as mTOR-inhibitor-based therapy). In some embodiments, there is provided a method of treating a solid tumor in a human individual, comprising administering to the individual an effective amount of a composition comprising nanoparticles comprising a limus drug and an albumin, wherein the nanoparticles have an average particle size of no greater than about 150 nm (such as no greater than about 120 nm) wherein the individual is no more than about 21 years old (such as no more than about 18 years old), and wherein the individual has progressed on a prior therapy (such as mTOR-inhibitor-based therapy). In some embodiments, there is provided a method of treating a solid tumor in a human individual, comprising administering to the individual an effective amount of a composition comprising nanoparticles comprising sirolimus and human serum albumin, wherein the nanoparticles comprise sirolimus associated (*e.g.*, coated) with human serum albumin, wherein the nanoparticles have an average particle size of no greater than about 150 nm (such as no greater than about 120 nm, for example about 100 nm), and wherein the weight ratio of human albumin and sirolimus in the composition is about 9:1 or less (such as about 9:1 or about 8:1), wherein the individual is no more than about 21 years old (such as no more than about 18 years old), and wherein the individual has progressed on a prior therapy (such as mTOR-inhibitor-based therapy). In some embodiments, there is provided a method of treating a solid tumor in a human individual, comprising administering to the individual an effective amount of a composition comprising *Nab*-sirolimus, wherein the individual is no more than about 21 years old (such as no more than about 18 years old), and wherein the individual has progressed on a prior therapy (such as mTOR-inhibitor-based therapy). In some embodiments, the individual is no more than about any of 17, 16, 15, 14, 13, 12, 11, 10, 9, 8, 7, 6, 5, 4, 3, 2, or 1 year old. In some embodiments, the individual is about 9 to about 15 years old. In some embodiments, the individual is about 5 to about 9 years old. In some embodiments, the individual is about 1 to about 5 years old. In some embodiments, the individual is no more than about 1 year old, such as about 6 months old to about 1 year old, less than about 6 months old, or less than about 3 months old. In some embodiments, the method further comprises administering to the individual an effective amount of a second agent, such as a chemotherapy agent, for example, vincristine, or irinotecan and temozolomide. In some embodiments, the second agent and the nanoparticle composition are administered sequentially. In some embodiments, the second agent and the nanoparticle composition are administered simultaneously. In some embodiments, the second agent and the nanoparticle composition are administered concurrently. In some embodiments, the method further comprises a step of selecting the individual for treatment based on the expression level of S6K1 and/or 4EBP1. In some embodiments, the method further comprises a step of determining the expression level of S6K1 and/or 4EBP1 in the individual. In some embodiments, the solid tumor is selected from the group consisting of neuroblastoma, soft tissue tumor (*e.g.*, rhabdomyosarcoma), bone tumor (*e.g*., osteosarcoma, Ewing's sarcoma), CNS tumor (*e.g.,* meduloblastoma, glioma), renal tumor, hepatic tumor *(e.g.,* hepatoblastoma and hepatocellular carcinoma), and vascular tumors (*e.g*., Kaposi' sarcoma, angiosarcoma, Tufted angioma, and kaposiform hemangioendothelioma).

In some embodiments, the method comprises administering to the individual an effective amount of a composition comprising nanoparticles comprising a mTOR-inhibitor (such as limus drug, for example sirolimus) and an albumin, wherein the individual is no more than about 21 years old (such as no more than about 18 years old), and wherein the individual is resistant or refractory to a prior therapy (such as mTOR-inhibitor-based therapy). In some embodiments, the method comprises administering to the individual an effective amount of a composition comprising nanoparticles comprising a limus drug and an albumin, wherein the limus drug in the nanoparticles is associated (*e.g*., coated) with the albumin, wherein the individual is no more than about 21 years old (such as no more than about 18 years old), and wherein the individual is resistant or refractory to a prior therapy (such as mTOR-inhibitor-based therapy). In some embodiments, there is provided a method of treating a solid tumor in a human individual, comprising administering to the individual an effective amount of a composition comprising nanoparticles comprising a limus drug and an albumin, wherein the nanoparticles have an average particle size of no greater than about 150 nm (such as no greater than about 120 nm) wherein the individual is no more than about 21 years old (such as no more than about 18 years old), and wherein the individual is resistant or refractory to a prior therapy (such as mTOR-inhibitor-based therapy). In some embodiments, there is provided a method of treating a solid tumor in a human individual, comprising administering to the individual an effective amount of a composition comprising nanoparticles comprising sirolimus and human serum albumin, wherein the nanoparticles comprise sirolimus associated (*e.g*., coated) with human serum albumin, wherein the nanoparticles have an average particle size of no greater than about 150 nm (such as no greater than about 120 nm, for example about 100 nm), and wherein the weight ratio of human albumin and sirolimus in the composition is about 9:1 or less (such as about 9:1 or about 8:1), wherein the individual is no more than about 21 years old (such as no more than about 18 years old), and wherein the individual is resistant or refractory to a prior therapy (such as mTOR-inhibitor-based therapy). In some embodiments, there is provided a method of treating a solid tumor in a human individual, comprising administering to the individual an effective amount of a composition comprising *Nab*-sirolimus, wherein the individual is no more than about 21 years old (such as no more than about 18 years old), and wherein the individual is resistant or refractory to a prior therapy (such as mTOR-inhibitor-based therapy). In some embodiments, the individual is no more than about any of 17, 16, 15, 14, 13, 12, 11, 10, 9, 8, 7, 6, 5, 4, 3, 2, or 1 year old. In some embodiments, the individual is about 9 to about 15 years old. In some embodiments, the individual is about 5 to about 9 years old. In some embodiments, the individual is about 1 to about 5 years old. In some embodiments, the individual is no more than about 1 year old, such as about 6 months old to about 1 year old, less than about 6 months old, or less than about 3 months old. In some embodiments, the method further comprises administering to the individual an effective amount of a second agent, such as a chemotherapy agent, for example, vincristine, or irinotecan and temozolomide. In some embodiments, the second agent and the nanoparticle composition are administered sequentially. In some embodiments, the second agent and the nanoparticle composition are administered simultaneously. In some embodiments, the second agent and the nanoparticle composition are administered concurrently. In some embodiments, the method further comprises a step of selecting the individual for treatment based on the expression level of S6K1 and/or 4EBP1. In some embodiments, the method further comprises a step of determining the expression level of S6K1 and/or 4EBP1 in the individual. In some embodiments, the solid tumor is selected from the group consisting of neuroblastoma, soft tissue tumor (*e.g*., rhabdomyosarcoma), bone tumor *(e.g.,* osteosarcoma, Ewing's sarcoma), CNS tumor (*e.g.,* meduloblastoma, glioma), renal tumor, hepatic tumor (*e.g.*, hepatoblastoma and hepatocellular carcinoma), and vascular tumors (e.g., Kaposi' sarcoma, angiosarcoma, Tufted angioma, and kaposiform hemangioendothelioma).

In some embodiments, the method comprises administering to the individual an effective amount of a composition comprising nanoparticles comprising a mTOR-inhibitor (such as limus drug, for example sirolimus) and an albumin, wherein the individual is no more than about 21 years old (such as no more than about 18 years old), and wherein the individual has failed to respond to a prior therapy (such as mTOR-inhibitor-based therapy). In some embodiments, the method comprises administering to the individual an effective amount of a composition comprising nanoparticles comprising a limus drug and an albumin, wherein the limus drug in the nanoparticles is associated (*e.g*., coated) with the albumin, wherein the individual is no more than about 21 years old (such as no more than about 18 years old), and wherein the individual has failed to respond to a prior therapy (such as mTOR-inhibitor-based therapy). In some embodiments, there is provided a method of treating a solid tumor in a human individual, comprising administering to the individual an effective amount of a composition comprising nanoparticles comprising a limus drug and an albumin, wherein the nanoparticles have an average particle size of no greater than about 150 nm (such as no greater than about 120 nm) wherein the individual is no more than about 21 years old (such as no more than about 18 years old), and wherein the individual has failed to respond to a prior therapy (such as mTOR-inhibitor-based therapy). In some embodiments, there is provided a method of treating a solid tumor in a human individual, comprising administering to the individual an effective amount of a composition comprising nanoparticles comprising sirolimus and human serum albumin, wherein the nanoparticles comprise sirolimus associated (*e.g.*, coated) with human serum albumin, wherein the nanoparticles have an average particle size of no greater than about 150 nm (such as no greater than about 120 nm, for example about 100 nm), and wherein the weight ratio of human albumin and sirolimus in the composition is about 9:1 or less (such as about 9:1 or about 8:1), wherein the individual is no more than about 21 years old (such as no more than about 18 years old), and wherein the individual has failed to respond to a prior therapy (such as mTOR-inhibitor-based therapy). In some embodiments, there is provided a method of treating a solid tumor in a human individual, comprising administering to the individual an effective amount of a composition comprising *Nab*-sirolimus, wherein the individual is no more than about 21 years old (such as no more than about 18 years old), and wherein the individual has failed to respond to a prior therapy (such as mTOR-inhibitor-based therapy). In some embodiments, the individual is no more than about any of 17, 16, 15, 14, 13, 12, 11, 10, 9, 8, 7, 6, 5, 4, 3, 2, or 1 year old. In some embodiments, the individual is about 9 to about 15 years old. In some embodiments, the individual is about 5 to about 9 years old. In some embodiments, the individual is about 1 to about 5 years old. In some embodiments, the individual is no more than about 1 year old, such as about 6 months old to about 1 year old, less than about 6 months old, or less than about 3 months old. In some embodiments, the method further comprises administering to the individual an effective amount of a second agent, such as a chemotherapy agent, for example, vincristine, or irinotecan and temozolomide. In some embodiments, the second agent and the nanoparticle composition are administered sequentially. In some embodiments, the second agent and the nanoparticle composition are administered simultaneously. In some embodiments, the second agent and the nanoparticle composition are administered concurrently. In some embodiments, the method further comprises a step of selecting the individual for treatment based on the expression level of S6K1 and/or 4EBP1. In some embodiments, the method further comprises a step of determining the expression level of S6K1 and/or 4EBP1 in the individual. In some embodiments, the solid tumor is selected from the group consisting of neuroblastoma, soft tissue tumor (*e.g*., rhabdomyosarcoma), bone tumor (*e.g*., osteosarcoma, Ewing's sarcoma), CNS tumor (*e.g.,* meduloblastoma, glioma), renal tumor, hepatic tumor *(e.g.,* hepatoblastoma and hepatocellular carcinoma), and vascular tumors (*e.g*., Kaposi' sarcoma, angiosarcoma, Tufted angioma, and kaposiform hemangioendothelioma).

In some embodiments, the method comprises administering to the individual an effective amount of a composition comprising nanoparticles comprising a mTOR-inhibitor (such as limus drug, for example sirolimus) and an albumin, wherein the individual is no more than about 21 years old (such as no more than about 18 years old), and wherein the individual exhibits a less desirable degree of responsiveness to a prior therapy (such as mTOR-inhibitor-based therapy). In some embodiments, the method comprises administering to the individual an effective amount of a composition comprising nanoparticles comprising a limus drug and an albumin, wherein the limus drug in the nanoparticles is associated (*e.g.,* coated) with the albumin, wherein the individual is no more than about 21 years old (such as no more than about 18 years old), and wherein the individual exhibits a less desirable degree of responsiveness to a prior therapy (such as mTOR-inhibitor-based therapy). In some embodiments, there is provided a method of treating a solid tumor in a human individual, comprising administering to the individual an effective amount of a composition comprising nanoparticles comprising a limus drug and an albumin, wherein the nanoparticles have an average particle size of no greater than about 150 nm (such as no greater than about 120 nm) wherein the individual is no more than about 21 years old (such as no more than about 18 years old), and wherein the individual exhibits a less desirable degree of responsiveness to a prior therapy (such as mTOR-inhibitor-based therapy). In some embodiments, there is provided a method of treating a solid tumor in a human individual, comprising administering to the individual an effective amount of a composition comprising nanoparticles comprising sirolimus and human serum albumin, wherein the nanoparticles comprise sirolimus associated (*e.g*., coated) with human serum albumin, wherein the nanoparticles have an average particle size of no greater than about 150 nm (such as no greater than about 120 nm, for example about 100 nm), and wherein the weight ratio of human albumin and sirolimus in the composition is about 9:1 or less (such as about 9:1 or about 8:1), wherein the individual is no more than about 21 years old (such as no more than about 18 years old), and wherein the individual exhibits a less desirable degree of responsiveness to a prior therapy (such as mTOR-inhibitor-based therapy). In some embodiments, there is provided a method of treating a solid tumor in a human individual, comprising administering to the individual an effective amount of a composition comprising *Nab*-sirolimus, wherein the individual is no more than about 21 years old (such as no more than about 18 years old), and wherein the individual exhibits a less desirable degree of responsiveness to a prior therapy (such as mTOR-inhibitor-based therapy). In some embodiments, the individual is no more than about any of 17, 16, 15, 14, 13, 12, 11, 10, 9, 8, 7, 6, 5, 4, 3, 2, or 1 year old. In some embodiments, the individual is about 9 to about 15 years old. In some embodiments, the individual is about 5 to about 9 years old. In some embodiments, the individual is about 1 to about 5 years old. In some embodiments, the individual is no more than about 1 year old, such as about 6 months old to about 1 year old, less than about 6 months old, or less than about 3 months old. In some embodiments, the method further comprises administering to the individual an effective amount of a second agent, such as a chemotherapy agent, for example, vincristine, or irinotecan and temozolomide. In some embodiments, the second agent and the nanoparticle composition are administered sequentially. In some embodiments, the second agent and the nanoparticle composition are administered simultaneously. In some embodiments, the second agent and the nanoparticle composition are administered concurrently. In some embodiments, the method further comprises a step of selecting the individual for treatment based on the expression level of S6K1 and/or 4EBP1. In some embodiments, the method further comprises a step of determining the expression level of S6K1 and/or 4EBP1 in the individual. In some embodiments, the solid tumor is selected from the group consisting of neuroblastoma, soft tissue tumor (*e.g*., rhabdomyosarcoma), bone tumor (*e.g*., osteosarcoma, Ewing's sarcoma), CNS tumor (*e.g*., meduloblastoma, glioma), renal tumor, hepatic tumor (*e.g*., hepatoblastoma and hepatocellular carcinoma), and vascular tumors (*e.g*., Kaposi' sarcoma, angiosarcoma, Tufted angioma, and kaposiform hemangioendothelioma).

In some embodiments, the method comprises administering to the individual an effective amount of a composition comprising nanoparticles comprising a mTOR-inhibitor (such as limus drug, for example sirolimus) and an albumin, wherein the individual is no more than about 21 years old (such as no more than about 18 years old), and wherein the individual has recurrent solid tumor (for example, the individual develops solid tumor after about any of about 2, 3, 4, 5, 6, 7, 8, 9, 10, 11, 12, 24, 36, 48, or 60 months upon the cessation of a prior therapy). In some embodiments, the method comprises administering to the individual an effective amount of a composition comprising nanoparticles comprising a limus drug and an albumin, wherein the limus drug in the nanoparticles is associated (*e.g*., coated) with the albumin, wherein the individual is no more than about 21 years old (such as no more than about 18 years old), and wherein the individual has recurrent solid tumor (for example, the individual develops solid tumor after about any of about 2, 3, 4, 5, 6, 7, 8, 9, 10, 11, 12, 24, 36, 48, or 60 months upon the cessation of a prior therapy). In some embodiments, there is provided a method of treating a solid tumor in a human individual, comprising administering to the individual an effective amount of a composition comprising nanoparticles comprising a limus drug and an albumin, wherein the nanoparticles have an average particle size of no greater than about 150 nm (such as no greater than about 120 nm) wherein the individual is no more than about 21 years old (such as no more than about 18 years old), and wherein the individual has recurrent solid tumor (for example, the individual develops solid tumor after about any of about 2, 3, 4, 5, 6, 7, 8, 9, 10, 11, 12, 24, 36, 48, or 60 months upon the cessation of a prior therapy). In some embodiments, there is provided a method of treating a solid tumor in a human individual, comprising administering to the individual an effective amount of a composition comprising nanoparticles comprising sirolimus and human serum albumin, wherein the nanoparticles comprise sirolimus associated (*e.g*., coated) with human serum albumin, wherein the nanoparticles have an average particle size of no greater than about 150 nm (such as no greater than about 120 nm, for example about 100 nm), and wherein the weight ratio of human albumin and sirolimus in the composition is about 9:1 or less (such as about 9:1 or about 8:1), wherein the individual is no more than about 21 years old (such as no more than about 18 years old), and wherein the individual has recurrent solid tumor (for example, the individual develops solid tumor after about any of about 2, 3, 4, 5, 6, 7, 8, 9, 10, 11, 12, 24, 36, 48, or 60 months upon the cessation of a prior therapy). In some embodiments, there is provided a method of treating a solid tumor in a human individual, comprising administering to the individual an effective amount of a composition comprising *Nab*-sirolimus, wherein the individual is no more than about 21 years old (such as no more than about 18 years old), and wherein the individual has recurrent solid tumor (for example, the individual develops solid tumor after about any of about 2, 3, 4, 5, 6, 7, 8, 9, 10, 11, 12, 24, 36, 48, or 60 months upon the cessation of a prior therapy). In some embodiments, the individual is no more than about any of 17, 16, 15, 14, 13, 12, 11, 10, 9, 8, 7, 6, 5, 4, 3, 2, or 1 year old. In some embodiments, the individual is about 9 to about 15 years old. In some embodiments, the individual is about 5 to about 9 years old. In some embodiments, the individual is about 1 to about 5 years old. In some embodiments, the individual is no more than about 1 year old, such as about 6 months old to about 1 year old, less than about 6 months old, or less than about 3 months old. In some embodiments, the method further comprises administering to the individual an effective amount of a second agent, such as a chemotherapy agent, for example, vincristine, or irinotecan and temozolomide. In some embodiments, the second agent and the nanoparticle composition are administered sequentially. In some embodiments, the second agent and the nanoparticle composition are administered simultaneously. In some embodiments, the second agent and the nanoparticle composition are administered concurrently. In some embodiments, the method further comprises a step of selecting the individual for treatment based on the expression level of S6K1 and/or 4EBP1. In some embodiments, the method further comprises a step of determining the expression level of S6K1 and/or 4EBP1 in the individual. In some embodiments, the solid tumor is selected from the group consisting of neuroblastoma, soft tissue tumor (*e.g*., rhabdomyosarcoma), bone tumor (*e.g*., osteosarcoma, Ewing's sarcoma), CNS tumor (*e.g*., meduloblastoma, glioma), renal tumor, hepatic tumor (*e.g*., hepatoblastoma and hepatocellular carcinoma), and vascular tumors (*e.g*., Kaposi' sarcoma, angiosarcoma, Tufted angioma, and kaposiform hemangioendothelioma).

In some embodiments, the method comprises administering to the individual an effective amount of a composition comprising nanoparticles comprising a mTOR-inhibitor (such as limus drug, for example sirolimus) and an albumin, wherein the individual is no more than about 21 years old (such as no more than about 18 years old), and wherein a prior therapy (such as a mTOR-inhibitor-based therapy) has stopped (for example, for at least 1, 2, 3, 4, 5, 6, 7, 8, 9, or 10 months) when initiating the administration of the composition to the individual. In some embodiments, the method comprises administering to the individual an effective amount of a composition comprising nanoparticles comprising a limus drug and an albumin, wherein the limus drug in the nanoparticles is associated (*e.g*., coated) with the albumin, wherein the individual is no more than about 21 years old (such as no more than about 18 years old), and wherein a prior therapy (such as a mTOR-inhibitor-based therapy) has stopped (for example, for at least 1, 2, 3, 4, 5, 6, 7, 8, 9, or 10 months) when initiating the administration of the composition to the individual. In some embodiments, there is provided a method of treating a solid tumor in a human individual, comprising administering to the individual an effective amount of a composition comprising nanoparticles comprising a limus drug and an albumin, wherein the nanoparticles have an average particle size of no greater than about 150 nm (such as no greater than about 120 nm) wherein the individual is no more than about 21 years old (such as no more than about 18 years old), and wherein a prior therapy (such as a mTOR-inhibitor-based therapy) has stopped (for example, for at least 1, 2, 3, 4, 5, 6, 7, 8, 9, or 10 months) when initiating the administration of the composition to the individual. In some embodiments, there is provided a method of treating a solid tumor in a human individual, comprising administering to the individual an effective amount of a composition comprising nanoparticles comprising sirolimus and human serum albumin, wherein the nanoparticles comprise sirolimus associated (*e.g*., coated) with human serum albumin, wherein the nanoparticles have an average particle size of no greater than about 150 nm (such as no greater than about 120 nm, for example about 100 nm), and wherein the weight ratio of human albumin and sirolimus in the composition is about 9:1 or less (such as about 9:1 or about 8:1), wherein the individual is no more than about 21 years old (such as no more than about 18 years old), and wherein a prior therapy (such as a mTOR-inhibitor-based therapy) has stopped (for example, for at least 1, 2, 3, 4, 5, 6, 7, 8, 9, or 10 months) when initiating the administration of the composition to the individual. In some embodiments, there is provided a method of treating a solid tumor in a human individual, comprising administering to the individual an effective amount of a composition comprising *Nab*-sirolimus, wherein the individual is no more than about 21 years old (such as no more than about 18 years old), and wherein a prior therapy (such as a mTOR-inhibitor-based therapy) has stopped (for example, for at least 1, 2, 3, 4, 5, 6, 7, 8, 9, or 10 months) when initiating the administration of the composition to the individual. In some embodiments, the individual is no more than about any of 17, 16, 15, 14, 13, 12, 11, 10, 9, 8, 7, 6, 5, 4, 3, 2, or 1 year old. In some embodiments, the individual is about 9 to about 15 years old. In some embodiments, the individual is about 5 to about 9 years old. In some embodiments, the individual is about 1 to about 5 years old. In some embodiments, the individual is no more than about 1 year old, such as about 6 months old to about 1 year old, less than about 6 months old, or less than about 3 months old. In some embodiments, the method further comprises administering to the individual an effective amount of a second agent, such as a chemotherapy agent, for example, vincristine, or irinotecan and temozolomide. In some embodiments, the second agent and the nanoparticle composition are administered sequentially. In some embodiments, the second agent and the nanoparticle composition are administered simultaneously. In some embodiments, the second agent and the nanoparticle composition are administered concurrently. In some embodiments, the method further comprises a step of selecting the individual for treatment based on the expression level of S6K1 and/or 4EBP1. In some embodiments, the method further comprises a step of determining the expression level of S6K1 and/or 4EBP1 in the individual. In some embodiments, the solid tumor is selected from the group consisting of neuroblastoma, soft tissue tumor (*e.g*., rhabdomyosarcoma), bone tumor (*e.g*., osteosarcoma, Ewing's sarcoma), CNS tumor (*e.g*., meduloblastoma, glioma), renal tumor, hepatic tumor (*e.g*., hepatoblastoma and hepatocellular carcinoma), and vascular tumors (*e.g*., Kaposi' sarcoma, angiosarcoma, Tufted angioma, and kaposiform hemangioendothelioma).

In some embodiments, the individual is resistant to treatment of solid tumor with mTOR inhibitor-based therapy (*e.g*., mTOR inhibitor monotherapy or combination therapy) and has progressed after treatment (*e.g*., the solid tumor has been refractory). In some embodiments, the individual is initially responsive to treatment of solid tumor with mTOR inhibitor-based therapy (*e.g*., mTOR inhibitor monotherapy or combination therapy) but has progressed after treatment. In some embodiments, the individual is human. In some embodiments, the individual has a family history of solid tumor (*e.g*., at least 2 first-degree relatives affected with solid tumor without accumulation of other cancers or familial diseases). In some embodiments, the individual has one or more hereditary pediatric solid tumor symptoms. For neuroblastoma, symptoms can depend on the location of the primary tumor. Symptoms of neuroblastoma can include, but are not limited to, *e.g*., bulging eyes, dark circles around eyes, bone pain, swollen stomach, fatigue, painless, constipation, anemia, bluish lumps under the skin in infants, weakness or paralysis, edema, and lump in the abdomen, neck, or chest. For retinoblastoma, symptoms can include, but are not limited to, *e.g*., crossed eyes, double vision, visual disturbances, strabismus, eye pain and redness, and differing iris colors in each eye. For osteosarcoma, symptoms include, but are not limited to, *e.g*., bone pain than may become worse during exercise or at might, joint tenderness or inflammation, bone fractures due to bone weakness, limited range of motion, fatigue and anemia. For rhabdomyosarcoma, symptoms can range widely depending on the location of the tumor. Such symptoms can include, but are not limited to, *e.g*., nosebleed, symptoms similar to a sinus infection, earaches, discharge from the ear canal, bulged or crossed eyes, difficult urination, bleeding from the vagina, mass growing from the vagina or around the testicles, abdominal pain and vomiting, and mass or lump in the arm or leg. In some embodiments, the individual is a male. In some embodiments, the individual is a female. In some embodiments, the individual has a single lesion at presentation. In some embodiments, the individual has multiple lesions at presentation.

In some embodiments, the individual is a human who exhibits one or more symptoms associated with a solid tumor. In some embodiments, the individual is at an early stage of solid tumor. In some embodiments, the individual is at an advanced stage of solid tumor. In some embodiments, the individual has non-metastatic solid tumor. In some embodiments, the individual has primary solid tumor. In some of embodiments, the individual is genetically or otherwise predisposed (*e.g*., having a risk factor) to developing solid tumor. These risk factors include, but are not limited to, age, sex, race, diet, genetic considerations, family history, inherited conditions (*e.g*., Li-Fraumeni syndrome, neurofibromatosis type 1, Beckwith-Widemann syndrome, Rothmund-Thompson syndrome, Bloom syndrome, Werner syndrome, t:ostello syndrome, Noonan syndrome), certain diseases (*e.g*., Paget disease, bone disease), prenatal exposure (*e.g*., to tobacco or certain medications) and environmental exposure (*e.g*., to ionizing radiation).

The methods described herein are useful for various aspects of solid tumor treatment as discussed below. These methods in some embodiments further comprise administering to the individual an effective amount of vincristine, or a combination of irinotecan and temozolomide.

In some embodiments, there is provided a method of inhibiting solid tumor cell proliferation in a human individual, comprising administering to the individual an effective amount of a composition comprising nanoparticles comprising an mTOR-inhibitor and an albumin, wherein the individual is no more than about 21 years old (such as no more than about 18 years old). In some embodiments, at least about 10% (including for example at least about any of 20%, 30%, 40%, 60%, 70%, 80%, 90%, or 100%) cell proliferation is inhibited. In some embodiments, the mTOR-inhibitor is sirolimus. In some embodiments, the mTOR-inhibitor in the nanoparticle in the composition is administered by intravenous administration. In some embodiments, the solid tumor is selected from the group consisting of neuroblastoma, soft tissue tumor (*e.g*., rhabdomyosarcoma), bone tumor (*e.g*., osteosarcoma, Ewing's sarcoma), CNS tumor (*e.g*., meduloblastoma, glioma), renal tumor, hepatic tumor (*e.g*., hepatoblastoma and hepatocellular carcinoma), and vascular tumors (*e.g*., Kaposi' sarcoma, angiosarcoma, Tufted angioma, and kaposiform hemangioendothelioma).

In some embodiments, there is provided a method of inhibiting solid tumor metastasis in a human individual, comprising administering to the individual an effective amount of a composition comprising nanoparticles comprising a mTOR-inhibitor (such as limus drug, for example sirolimus) and an albumin, wherein the individual is no more than about 21 years old (such as no more than about 18 years old). In some embodiments, at least about 10% (including for example at least about any of 20%, 30%, 40%, 60%, 70%, 80%, 90%, or 100%) metastasis is inhibited. In some embodiments, a method of inhibiting metastasis to one or more lymph nodes is provided. In some embodiments, the mTOR-inhibitor is sirolimus. In some embodiments, the mTOR-inhibitor in the nanoparticle in the composition is administered by intravenous administration. In some embodiments, the solid tumor is selected from the group consisting of neuroblastoma, soft tissue tumor (*e.g*., rhabdomyosarcoma), bone tumor (*e.g*., osteosarcoma, Ewing's sarcoma), CNS tumor (*e.g*., meduloblastoma, glioma), renal tumor, hepatic tumor (*e.g*., hepatoblastoma and hepatocellular carcinoma), and vascular tumors (*e.g*., Kaposi' sarcoma, angiosarcoma, Tufted angioma, and kaposiform hemangioendothelioma).

In some embodiments, there is provided a method of inhibiting solid tumor metastasis in a human individual, comprising administering to the individual an effective amount of a composition comprising nanoparticles comprising a mTOR-inhibitor (such as limus drug, for example sirolimus) and an albumin, wherein the individual is no more than about 21 years old (such as no more than about 18 years old), and wherein the individual is resistant or refractory to a mTOR-inhibitor-based therapy. In some embodiments, at least about 10% (including for example at least about any of 20%, 30%, 40%, 60%, 70%, 80%, 90%, or 100%) metastasis is inhibited. In some embodiments, a method of inhibiting metastasis to one or more lymph nodes is provided. In some embodiments, the mTOR-inhibitor is sirolimus. In some embodiments, the mTOR-inhibitor in the nanoparticle in the composition is administered by intravenous administration. In some embodiments, the solid tumor is selected from the group consisting of neuroblastoma, soft tissue tumor (*e.g*., rhabdomyosarcoma), bone tumor (*e.g*., osteosarcoma, Ewing's sarcoma), CNS tumor (*e.g*., meduloblastoma, glioma), renal tumor, hepatic tumor (*e.g*., hepatoblastoma and hepatocellular carcinoma), and vascular tumors *(e.g.,* Kaposi' sarcoma, angiosarcoma, Tufted angioma, and kaposiform hemangioendothelioma).

In some embodiments, there is provided a method of inhibiting solid tumor metastasis in a human individual, comprising administering to the individual an effective amount of a composition comprising nanoparticles comprising a mTOR-inhibitor (such as limus drug, for example sirolimus) and an albumin, wherein the individual is no more than about 21 years old (such as no more than about 18 years old), and wherein the individual has failed to respond to a mTOR-inhibitor-based therapy. In some embodiments, at least about 10% (including for example at least about any of 20%, 30%, 40%, 60%, 70%, 80%, 90%, or 100%) metastasis is inhibited. In some embodiments, a method of inhibiting metastasis to one or more lymph nodes is provided. In some embodiments, the mTOR-inhibitor is sirolimus. In some embodiments, the mTOR-inhibitor in the nanoparticle in the composition is administered by intravenous administration. In some embodiments, the solid tumor is selected from the group consisting of neuroblastoma, soft tissue tumor (*e.g*., rhabdomyosarcoma), bone tumor (*e.g*., osteosarcoma, Ewing's sarcoma), CNS tumor (*e.g*., meduloblastoma, glioma), renal tumor, hepatic tumor (*e.g*., hepatoblastoma and hepatocellular carcinoma), and vascular tumors (*e.g*., Kaposi' sarcoma, angiosarcoma, Tufted angioma, and kaposiform hemangioendothelioma).

In some embodiments, there is provided a method of inhibiting solid tumor metastasis in a human individual, comprising administering to the individual an effective amount of a composition comprising nanoparticles comprising a mTOR-inhibitor (such as limus drug, for example sirolimus) and an albumin, wherein the individual is no more than about 21 years old (such as no more than about 18 years old), and wherein the individual exhibits a less desirable degree of responsiveness to a mTOR-inhibitor-based therapy. In some embodiments, at least about 10% (including for example at least about any of 20%, 30%, 40%, 60%, 70%, 80%, 90%, or 100%) metastasis is inhibited. In some embodiments, a method of inhibiting metastasis to one or more lymph nodes is provided. In some embodiments, the mTOR-inhibitor is sirolimus. In some embodiments, the mTOR-inhibitor in the nanoparticle in the composition is administered by intravenous administration. In some embodiments, the solid tumor is selected from the group consisting of neuroblastoma, soft tissue tumor (*e.g*., rhabdomyosarcoma), bone tumor (*e.g*., osteosarcoma, Ewing's sarcoma), CNS tumor (*e.g*., meduloblastoma, glioma), renal tumor, hepatic tumor (*e.g*., hepatoblastoma and hepatocellular carcinoma), and vascular tumors (*e.g.,* Kaposi' sarcoma, angiosarcoma, Tufted angioma, and kaposiform hemangioendothelioma).

In some embodiments, there is provided a method of inhibiting solid tumor metastasis in a human individual, comprising administering to the individual an effective amount of a composition comprising nanoparticles comprising a mTOR-inhibitor (such as limus drug, for example sirolimus) and an albumin, wherein the individual is no more than about 21 years old (such as no more than about 18 years old), and wherein the individual has recurrent solid tumor (for example, the individual develops solid tumor after about any of about 2, 3, 4, 5, 6, 7, 8, 9, 10, 11, 12, 24, 36, 48, or 60 months upon the cessation of a mTOR-inhibitor-based therapy). In some embodiments, at least about 10% (including for example at least about any of 20%, 30%, 40%, 60%, 70%, 80%, 90%, or 100%) metastasis is inhibited. In some embodiments, a method of inhibiting metastasis to one or more lymph nodes is provided. In some embodiments, the mTOR-inhibitor is sirolimus. In some embodiments, the mTOR-inhibitor in the nanoparticle in the composition is administered by intravenous administration. In some embodiments, the solid tumor is selected from the group consisting of neuroblastoma, soft tissue tumor (*e.g*., rhabdomyosarcoma), bone tumor (*e.g.,* osteosarcoma, Ewing's sarcoma), CNS tumor (*e.g*., meduloblastoma, glioma), renal tumor, hepatic tumor (*e.g*., hepatoblastoma and hepatocellular carcinoma), and vascular tumors (*e.g*., Kaposi' sarcoma, angiosarcoma, Tufted angioma, and kaposiform hemangioendothelioma).

In some embodiments, there is provided a method of inhibiting solid tumor metastasis in a human individual, comprising administering to the individual an effective amount of a composition comprising nanoparticles comprising a mTOR-inhibitor (such as limus drug, for example sirolimus) and an albumin, wherein the individual is no more than about 21 years old (such as no more than about 18 years old), and wherein a mTOR-inhibitor-based therapy has stopped (for example, for at least 1, 2, 3, 4, 5, 6, 7, 8, 9, or 10 months) when initiating the administration of the effective amount of the composition comprising nanoparticles comprising a mTOR-inhibitor (such as limus drug, for example sirolimus) and an albumin to the individual. In some embodiments, at least about 10% (including for example at least about any of 20%, 30%, 40%, 60%, 70%, 80%, 90%, or 100%) metastasis is inhibited. In some embodiments, a method of inhibiting metastasis to one or more lymph nodes is provided. In some embodiments, the mTOR-inhibitor is sirolimus. In some embodiments, the mTOR-inhibitor in the nanoparticle in the composition is administered by intravenous administration. In some embodiments, the solid tumor is selected from the group consisting of neuroblastoma, soft tissue tumor (*e.g*., rhabdomyosarcoma), bone tumor (*e.g*., osteosarcoma, Ewing's sarcoma), CNS tumor (*e.g*., meduloblastoma, glioma), renal tumor, hepatic tumor (*e.g*., hepatoblastoma and hepatocellular carcinoma), and vascular tumors (*e.g*., Kaposi' sarcoma, angiosarcoma, Tufted angioma, and kaposiform hemangioendothelioma).

In some embodiments, there is provided a method of reducing (such as eradiating) preexisting tumor metastasis (such as metastasis to the lymph node) in a human individual, comprising administering to the individual an effective amount of a composition comprising nanoparticles comprising a mTOR-inhibitor (such as limus drug, for example sirolimus) and an albumin, wherein the individual is no more than about 21 years old (such as no more than about 18 years old). In some embodiments, at least about 10% (including for example at least about any of 20%, 30%, 40%, 60%, 70%, 80%, 90%, or 100%) metastasis is reduced. In some embodiments, method of reducing metastasis to lymph node is provided. In some embodiments, the mTOR-inhibitor is sirolimus. In some embodiments, the mTOR-inhibitor in the nanoparticle in the composition is administered by intravenous administration. In some embodiments, the solid tumor is selected from the group consisting of neuroblastoma, soft tissue tumor (*e.g*., rhabdomyosarcoma), bone tumor (*e.g*., osteosarcoma, Ewing's sarcoma), CNS tumor (*e.g.*, meduloblastoma, glioma), renal tumor, hepatic tumor (*e.g*., hepatoblastoma and hepatocellular carcinoma), and vascular tumors (*e.g*., Kaposi' sarcoma, angiosarcoma, Tufted angioma, and kaposiform hemangioendothelioma).

In some embodiments, there is provided a method of reducing (such as eradiating) preexisting tumor metastasis (such as metastasis to the lymph node) in a human individual, comprising administering to the individual an effective amount of a composition comprising nanoparticles comprising a mTOR-inhibitor (such as limus drug, for example sirolimus) and an albumin, wherein the individual is no more than about 21 years old (such as no more than about 18 years old), and wherein the individual is resistant or refractory to a mTOR-inhibitor-based therapy. In some embodiments, at least about 10% (including for example at least about any of 20%, 30%, 40%, 60%, 70%, 80%, 90%, or 100%) metastasis is reduced. In some embodiments, method of reducing metastasis to lymph node is provided. In some embodiments, the mTOR-inhibitor is sirolimus. In some embodiments, the mTOR-inhibitor in the nanoparticle in the composition is administered by intravenous administration. In some embodiments, the solid tumor is selected from the group consisting of neuroblastoma, soft tissue tumor (*e.g*., rhabdomyosarcoma), bone tumor (*e.g*., osteosarcoma, Ewing's sarcoma), CNS tumor (*e.g*., meduloblastoma, glioma), renal tumor, hepatic tumor (*e.g*., hepatoblastoma and hepatocellular carcinoma), and vascular tumors (*e.g*., Kaposi' sarcoma, angiosarcoma, Tufted angioma, and kaposiform hemangioendothelioma).

In some embodiments, there is provided a method of reducing (such as eradiating) preexisting tumor metastasis (such as metastasis to the lymph node) in a human individual, comprising administering to the individual an effective amount of a composition comprising nanoparticles comprising a mTOR-inhibitor (such as limus drug, for example sirolimus) and an albumin, wherein the individual is no more than about 21 years old (such as no more than about 18 years old), and wherein the individual has failed to respond to a mTOR-inhibitor-based therapy. In some embodiments, at least about 10% (including for example at least about any of 20%, 30%, 40%, 60%, 70%, 80%, 90%, or 100%) metastasis is reduced. In some embodiments, method of reducing metastasis to lymph node is provided. In some embodiments, the mTOR-inhibitor is sirolimus. In some embodiments, the mTOR-inhibitor in the nanoparticle in the composition is administered by intravenous administration. In some embodiments, the solid tumor is selected from the group consisting of neuroblastoma, soft tissue tumor (*e.g*., rhabdomyosarcoma), bone tumor (*e.g*., osteosarcoma, Ewing's sarcoma), CNS tumor (*e.g*., meduloblastoma, glioma), renal tumor, hepatic tumor (*e.g*., hepatoblastoma and hepatocellular carcinoma), and vascular tumors (*e.g*., Kaposi' sarcoma, angiosarcoma, Tufted angioma, and kaposiform hemangioendothelioma).

In some embodiments, there is provided a method of reducing (such as eradiating) preexisting tumor metastasis (such as metastasis to the lymph node) in a human individual, comprising administering to the individual an effective amount of a composition comprising nanoparticles comprising a mTOR-inhibitor (such as limus drug, for example sirolimus) and an albumin, wherein the individual is no more than about 21 years old (such as no more than about 18 years old), and wherein the individual exhibits a less desirable degree of responsiveness to a mTOR-inhibitor-based therapy. In some embodiments, at least about 10% (including for example at least about any of 20%, 30%, 40%, 60%, 70%, 80%, 90%, or 100%) metastasis is reduced. In some embodiments, method of reducing metastasis to lymph node is provided. In some embodiments, the mTOR-inhibitor is sirolimus. In some embodiments, the mTOR-inhibitor in the nanoparticle in the composition is administered by intravenous administration. In some embodiments, the solid tumor is selected from the group consisting of neuroblastoma, soft tissue tumor (*e.g*., rhabdomyosarcoma), bone tumor (*e.g*., osteosarcoma, Ewing's sarcoma), CNS tumor (*e.g*., meduloblastoma, glioma), renal tumor, hepatic tumor (*e.g*., hepatoblastoma and hepatocellular carcinoma), and vascular tumors (*e.g*., Kaposi' sarcoma, angiosarcoma, Tufted angioma, and kaposiform hemangioendothelioma).

In some embodiments, there is provided a method of reducing (such as eradiating) preexisting tumor metastasis (such as metastasis to the lymph node) in a human individual, comprising administering to the individual an effective amount of a composition comprising nanoparticles comprising a mTOR-inhibitor (such as limus drug, for example sirolimus) and an albumin, and wherein the individual is no more than about 21 years old (such as no more than about 18 years old), and wherein the individual has recurrent solid tumor (for example, the individual develops solid tumor after about any of about 2, 3, 4, 5, 6, 7, 8, 9, 10, 11, 12, 24, 36, 48, or 60 months upon the cessation of a mTOR-inhibitor-based therapy). In some embodiments, at least about 10% (including for example at least about any of 20%, 30%, 40%, 60%, 70%, 80%, 90%, or 100%) metastasis is reduced. In some embodiments, method of reducing metastasis to lymph node is provided. In some embodiments, the mTOR-inhibitor is sirolimus. In some embodiments, the mTOR-inhibitor in the nanoparticle in the composition is administered by intravenous administration. In some embodiments, the solid tumor is selected from the group consisting of neuroblastoma, soft tissue tumor (*e.g*., rhabdomyosarcoma), bone tumor (*e.g*., osteosarcoma, Ewing's sarcoma), CNS tumor (*e.g*., meduloblastoma, glioma), renal tumor, hepatic tumor (*e.g*., hepatoblastoma and hepatocellular carcinoma), and vascular tumors (*e.g*., Kaposi' sarcoma, angiosarcoma, Tufted angioma, and kaposiform hemangioendothelioma).

In some embodiments, there is provided a method of reducing (such as eradiating) preexisting tumor metastasis (such as metastasis to the lymph node) in a human individual, comprising administering to the individual an effective amount of a composition comprising nanoparticles comprising a mTOR-inhibitor (such as limus drug, for example sirolimus) and an albumin, wherein the individual is no more than about 21 years old (such as no more than about 18 years old), and wherein a mTOR-inhibitor-based therapy has stopped (for example for at least 1, 2, 3, 4, 5, 6, 7, 8, 9, or 10 months) when initiating the administration of the effective amount of the composition comprising nanoparticles comprising a mTOR-inhibitor (such as limus drug, for example sirolimus) and an albumin to the individual. In some embodiments, at least about 10% (including for example at least about any of 20%, 30%, 40%, 60%, 70%, 80%, 90%, or 100%) metastasis is reduced. In some embodiments, method of reducing metastasis to lymph node is provided. In some embodiments, the mTOR-inhibitor is sirolimus. In some embodiments, the mTOR-inhibitor in the nanoparticle in the composition is administered by intravenous administration. In some embodiments, the solid tumor is selected from the group consisting of neuroblastoma, soft tissue tumor (*e.g*., rhabdomyosarcoma), bone tumor (*e.g*., osteosarcoma, Ewing's sarcoma), CNS tumor (*e.g*., meduloblastoma, glioma), renal tumor, hepatic tumor (*e.g*., hepatoblastoma and hepatocellular carcinoma), and vascular tumors (*e.g*., Kaposi' sarcoma, angiosarcoma, Tufted angioma, and kaposiform hemangioendothelioma).

In some embodiments, there is provided a method of reducing incidence or burden of preexisting tumor metastasis (such as metastasis to the lymph node) in a human individual, comprising administering to the individual an effective amount of a composition comprising nanoparticles comprising a mTOR-inhibitor (such as limus drug, for example sirolimus) and an albumin, wherein the individual is no more than about 21 years old (such as no more than about 18 years old). In some embodiments, the mTOR-inhibitor is sirolimus. In some embodiments, the mTOR-inhibitor in the nanoparticle in the composition is administered by intravenous administration. In some embodiments, the solid tumor is selected from the group consisting of neuroblastoma, soft tissue tumor (*e.g*., rhabdomyosarcoma), bone tumor (*e.g*., osteosarcoma, Ewing's sarcoma), CNS tumor (*e.g*., meduloblastoma, glioma), renal tumor, hepatic tumor (*e.g*., hepatoblastoma and hepatocellular carcinoma), and vascular tumors (*e.g*., Kaposi' sarcoma, angiosarcoma, Tufted angioma, and kaposiform hemangioendothelioma).

In some embodiments, there is provided a method of reducing incidence or burden of preexisting tumor metastasis (such as metastasis to the lymph node) in a human individual, comprising administering to the individual an effective amount of a composition comprising nanoparticles comprising a mTOR-inhibitor (such as limus drug, for example sirolimus) and an albumin, wherein the individual is no more than about 21 years old (such as no more than about 18 years old), and wherein the individual is resistant or refractory to a mTOR-inhibitor-based therapy. In some embodiments, the mTOR-inhibitor is sirolimus. In some embodiments, the mTOR-inhibitor in the nanoparticle in the composition is administered by intravenous administration. In some embodiments, the solid tumor is selected from the group consisting of neuroblastoma, soft tissue tumor (*e.g*., rhabdomyosarcoma), bone tumor (*e.g.,* osteosarcoma, Ewing's sarcoma), CNS tumor (*e.g*., meduloblastoma, glioma), renal tumor, hepatic tumor (*e.g*., hepatoblastoma and hepatocellular carcinoma), and vascular tumors (*e.g*., Kaposi' sarcoma, angiosarcoma, Tufted angioma, and kaposiform hemangioendothelioma).

In some embodiments, there is provided a method of reducing incidence or burden of preexisting tumor metastasis (such as metastasis to the lymph node) in a human individual, comprising administering to the individual an effective amount of a composition comprising nanoparticles comprising a mTOR-inhibitor (such as limus drug, for example sirolimus) and an albumin, wherein the individual is no more than about 21 years old (such as no more than about 18 years old), and wherein the individual has failed to respond to a mTOR-inhibitor-based therapy. In some embodiments, the mTOR-inhibitor is sirolimus. In some embodiments, the mTOR-inhibitor in the nanoparticle in the composition is administered by intravenous administration. In some embodiments, the solid tumor is selected from the group consisting of neuroblastoma, soft tissue tumor (*e.g*., rhabdomyosarcoma), bone tumor (*e.g*., osteosarcoma, Ewing's sarcoma), CNS tumor (*e.g*., meduloblastoma, glioma), renal tumor, hepatic tumor (*e.g*., hepatoblastoma and hepatocellular carcinoma), and vascular tumors (*e.g*., Kaposi' sarcoma, angiosarcoma, Tufted angioma, and kaposiform hemangioendothelioma).

In some embodiments, there is provided a method of reducing incidence or burden of preexisting tumor metastasis (such as metastasis to the lymph node) in a human individual, comprising administering to the individual an effective amount of a composition comprising nanoparticles comprising a mTOR-inhibitor (such as limus drug, for example sirolimus) and an albumin, wherein the individual is no more than about 21 years old (such as no more than about 18 years old), and wherein the individual exhibits a less desirable degree of responsiveness to a mTOR-inhibitor-based therapy. In some embodiments, the mTOR-inhibitor is sirolimus. In some embodiments, the mTOR-inhibitor in the nanoparticle in the composition is administered by intravenous administration. In some embodiments, the solid tumor is selected from the group consisting of neuroblastoma, soft tissue tumor (*e.g*., rhabdomyosarcoma), bone tumor (*e.g*., osteosarcoma, Ewing's sarcoma), CNS tumor (*e.g*., meduloblastoma, glioma), renal tumor, hepatic tumor (*e.g*., hepatoblastoma and hepatocellular carcinoma), and vascular tumors (*e.g*., Kaposi' sarcoma, angiosarcoma, Tufted angioma, and kaposiform hemangioendothelioma).

In some embodiments, there is provided a method of reducing incidence or burden of preexisting tumor metastasis (such as metastasis to the lymph node) in a human individual, comprising administering to the individual an effective amount of a composition comprising nanoparticles comprising a mTOR-inhibitor (such as limus drug, for example sirolimus) and an albumin, wherein the individual is no more than about 21 years old (such as no more than about 18 years old), and wherein the individual has recurrent solid tumor (for example, the individual develops solid tumor after about any of about 2, 3, 4, 5, 6, 7, 8, 9, 10, 11, 12, 24, 36, 48, or 60 months upon the cessation of a mTOR-inhibitor-based therapy). In some embodiments, the mTOR-inhibitor is sirolimus. In some embodiments, the mTOR-inhibitor in the nanoparticle in the composition is administered by intravenous administration. In some embodiments, the solid tumor is selected from the group consisting of neuroblastoma, soft tissue tumor (*e.g*., rhabdomyosarcoma), bone tumor (*e.g*., osteosarcoma, Ewing's sarcoma), CNS tumor (*e.g*., meduloblastoma, glioma), renal tumor, hepatic tumor (*e.g*., hepatoblastoma and hepatocellular carcinoma), and vascular tumors (*e.g*., Kaposi' sarcoma, angiosarcoma, Tufted angioma, and kaposiform hemangioendothelioma).

In some embodiments, there is provided a method of reducing incidence or burden of preexisting solid tumor metastasis (such as metastasis to the lymph node) in a human individual, comprising administering to the individual an effective amount of a composition comprising nanoparticles comprising a mTOR-inhibitor (such as limus drug, for example sirolimus) and an albumin, and wherein a mTOR-inhibitor-based therapy has stopped (for example, for at least 1, 2, 3, 4, 5, 6, 7, 8, 9, or 10 months) when initiating the administration of the effective amount of the composition comprising nanoparticles comprising a mTOR-inhibitor (such as limus drug, for example sirolimus) and an albumin to the individual. In some embodiments, the mTOR-inhibitor is sirolimus. In some embodiments, the mTOR-inhibitor in the nanoparticle in the composition is administered by intravenous administration. In some embodiments, the solid tumor is selected from the group consisting of neuroblastoma, soft tissue tumor (*e.g*., rhabdomyosarcoma), bone tumor (*e.g*., osteosarcoma, Ewing's sarcoma), CNS tumor (*e.g*., meduloblastoma, glioma), renal tumor, hepatic tumor (*e.g*., hepatoblastoma and hepatocellular carcinoma), and vascular tumors (*e.g*., Kaposi' sarcoma, angiosarcoma, Tufted angioma, and kaposiform hemangioendothelioma).

In some embodiments, there is provided a method of reducing solid tumor size in a human individual, comprising administering to the individual an effective amount of a composition comprising nanoparticles comprising a mTOR-inhibitor (such as limus drug, for example sirolimus) and an albumin, wherein the individual is no more than about 21 years old (such as no more than about 18 years old). In some embodiments, the tumor size is reduced at least about 10% (including for example at least about any of 20%, 30%, 40%, 60%, 70%, 80%, 90%, or 100%). In some embodiments, the mTOR-inhibitor is sirolimus. In some embodiments, the mTOR-inhibitor in the nanoparticle in the composition is administered by intravenous administration. In some embodiments, the solid tumor is selected from the group consisting of neuroblastoma, soft tissue tumor (*e.g*., rhabdomyosarcoma), bone tumor (*e.g*., osteosarcoma, Ewing's sarcoma), CNS tumor (*e.g*., meduloblastoma, glioma), renal tumor, hepatic tumor (*e.g*., hepatoblastoma and hepatocellular carcinoma), and vascular tumors (*e.g*., Kaposi' sarcoma, angiosarcoma, Tufted angioma, and kaposiform hemangioendothelioma).

In some embodiments, there is provided a method of reducing tumor size in a human individual, comprising administering to the individual an effective amount of a composition comprising nanoparticles comprising a mTOR-inhibitor (such as limus drug, for example sirolimus) and an albumin, wherein the individual is no more than about 21 years old (such as no more than about 18 years old), and wherein the individual is resistant or refractory to a mTOR-inhibitor-based therapy. In some embodiments, the tumor size is reduced at least about 10% (including for example at least about any of 20%, 30%, 40%, 60%, 70%, 80%, 90%, or 100%). In some embodiments, the mTOR-inhibitor is sirolimus. In some embodiments, the mTOR-inhibitor in the nanoparticle in the composition is administered by intravenous administration. In some embodiments, the solid tumor is selected from the group consisting of neuroblastoma, soft tissue tumor (*e.g*., rhabdomyosarcoma), bone tumor (*e.g*., osteosarcoma, Ewing's sarcoma), CNS tumor (*e.g*., meduloblastoma, glioma), renal tumor, hepatic tumor (*e.g*., hepatoblastoma and hepatocellular carcinoma), and vascular tumors (*e.g*., Kaposi' sarcoma, angiosarcoma, Tufted angioma, and kaposiform hemangioendothelioma).

In some embodiments, there is provided a method of reducing solid tumor size in a human individual, comprising administering to the individual an effective amount of a composition comprising nanoparticles comprising a mTOR-inhibitor (such as limus drug, for example sirolimus) and an albumin, wherein the individual is no more than about 21 years old (such as no more than about 18 years old), and wherein the individual has failed to respond to a mTOR-inhibitor-based therapy. In some embodiments, the tumor size is reduced at least about 10% (including for example at least about any of 20%, 30%, 40%, 60%, 70%, 80%, 90%, or 100%). In some embodiments, the mTOR-inhibitor is sirolimus. In some embodiments, the mTOR-inhibitor in the nanoparticle in the composition is administered by intravenous administration. In some embodiments, the solid tumor is selected from the group consisting of neuroblastoma, soft tissue tumor (*e.g*., rhabdomyosarcoma), bone tumor (*e.g*., osteosarcoma, Ewing's sarcoma), CNS tumor (*e.g*., meduloblastoma, glioma), renal tumor, hepatic tumor (*e.g*., hepatoblastoma and hepatocellular carcinoma), and vascular tumors (*e.g*., Kaposi' sarcoma, angiosarcoma, Tufted angioma, and kaposiform hemangioendothelioma).

In some embodiments, there is provided a method of reducing solid tumor size in a human individual, comprising administering to the individual an effective amount of a composition comprising nanoparticles comprising a mTOR-inhibitor (such as limus drug, for example sirolimus) and an albumin, wherein the individual is no more than about 21 years old (such as no more than about 18 years old), and wherein the individual exhibits a less desirable degree of responsiveness to a mTOR-inhibitor-based therapy. In some embodiments, the tumor size is reduced at least about 10% (including for example at least about any of 20%, 30%, 40%, 60%, 70%, 80%, 90%, or 100%).

In some embodiments, there is provided a method of reducing solid tumor size in a human individual, comprising administering to the individual an effective amount of a composition comprising nanoparticles comprising a mTOR-inhibitor (such as limus drug, for example sirolimus) and an albumin, wherein the individual is no more than about 21 years old (such as no more than about 18 years old), and wherein the individual has recurrent solid tumor (for example, the individual develops solid tumor after about any of about 2, 3, 4, 5, 6, 7, 8, 9, 10, 11, 12, 24, 36, 48, or 60 months upon the cessation of a mTOR-inhibitor-based therapy). In some embodiments, the tumor size is reduced at least about 10% (including for example at least about any of 20%, 30%, 40%, 60%, 70%, 80%, 90%, or 100%). In some embodiments, the mTOR-inhibitor is sirolimus. In some embodiments, the mTOR-inhibitor in the nanoparticle in the composition is administered by intravenous administration. In some embodiments, the solid tumor is selected from the group consisting of neuroblastoma, soft tissue tumor (*e.g*., rhabdomyosarcoma), bone tumor (*e.g*., osteosarcoma, Ewing's sarcoma), CNS tumor (*e.g*., meduloblastoma, glioma), renal tumor, hepatic tumor (*e.g*., hepatoblastoma and hepatocellular carcinoma), and vascular tumors (*e.g*., Kaposi' sarcoma, angiosarcoma, Tufted angioma, and kaposiform hemangioendothelioma).

In some embodiments, there is provided a method of reducing solid tumor size in a human individual, comprising administering to the individual an effective amount of a composition comprising nanoparticles comprising a mTOR-inhibitor (such as limus drug, for example sirolimus) and an albumin, wherein the individual is no more than about 21 years old (such as no more than about 18 years old), and wherein a mTOR-inhibitor-based therapy has stopped (for example for at least 1, 2, 3, 4, 5, 6, 7, 8, 9, or 10 months) when initiating the administration of the effective amount of the composition comprising nanoparticles comprising a mTOR-inhibitor (such as limus drug, for example sirolimus) and an albumin to the individual. In some embodiments, the tumor size is reduced at least about 10% (including for example at least about any of 20%, 30%, 40%, 60%, 70%, 80%, 90%, or 100%). In some embodiments, the mTOR-inhibitor is sirolimus. In some embodiments, the mTOR-inhibitor in the nanoparticle in the composition is administered by intravenous administration. In some embodiments, the solid tumor is selected from the group consisting of neuroblastoma, soft tissue tumor (*e.g*., rhabdomyosarcoma), bone tumor (*e.g.,* osteosarcoma, Ewing's sarcoma), CNS tumor (*e.g*., meduloblastoma, glioma), renal tumor, hepatic tumor (*e.g*., hepatoblastoma and hepatocellular carcinoma), and vascular tumors (*e.g*., Kaposi' sarcoma, angiosarcoma, Tufted angioma, and kaposiform hemangioendothelioma).

In some embodiments, there is provided a method of prolonging time to disease progression of solid tumor (*e.g*., progression-free survival) in a human individual, comprising administering to the individual an effective amount of a composition comprising nanoparticles comprising a mTOR-inhibitor (such as limus drug, for example sirolimus) and an albumin, wherein the individual is no more than about 21 years old (such as no more than about 18 years old). In some embodiments, the method prolongs the time to disease progression by at least any of 1, 2, 3, 4, 5, 6, 7, 8, 9, 10, 11, 12 weeks. In some embodiments, the method prolongs the time to disease progression by at least any of 1.0, 1.2, 1.4, 1.6, 1.8, 2.0, 2.2, 2.4, 2.6, 2.8, 3.0, 3.2, 3.4, 3.6, 3.8, 4.0, 4.2, 4.4, 4.6, 4.8, 5.0, 5.2, 5.4, 5.6, 5.8, 6.0, 6.2, 6.4, 6.6, 6.8, 7.0, 7.2, 7.4, 7.6, 7.8, 8.0, 8.2, 8.4, 8.6, 8.8, 9.0, 9.2, 9.4, 9.6, 9.8, 10.0, 10.2, 10.4, 10.6, 10.8, 11.0, 11.2, 11.4, 11.6, 11.8, 12.0, 13, 14, 15, 16, 17, 18, 19, 20, 21, 22, 23, 24, 30, 36, 42, 48, 54, 60, 66, or 72 months. In some embodiments, the mTOR-inhibitor is sirolimus. In some embodiments, the mTOR-inhibitor in the nanoparticle in the composition is administered by intravenous administration. In some embodiments, the solid tumor is selected from the group consisting of neuroblastoma, soft tissue tumor (*e.g*., rhabdomyosarcoma), bone tumor (*e.g*., osteosarcoma, Ewing's sarcoma), CNS tumor (*e.g*., medulloblastoma, glioma), renal tumor, hepatic tumor (*e.g*., hepatoblastoma and hepatocellular carcinoma), and vascular tumors (*e.g*., Kaposi' sarcoma, angiosarcoma, Tufted angioma, and kaposiform hemangioendothelioma).

In some embodiments, there is provided a method of prolonging overall survival of a human individual having solid tumor, comprising administering to the individual an effective amount of a composition comprising nanoparticles comprising a mTOR-inhibitor (such as limus drug, for example sirolimus) and an albumin, wherein the individual is no more than about 21 years old (such as no more than about 18 years old). In some embodiments, the method prolongs the survival of the individual by at least any of 1.0, 1.2, 1.4, 1.6, 1.8, 2.0, 2.2, 2.4, 2.6, 2.8, 3.0, 3.2, 3.4, 3.6, 3.8, 4.0, 4.2, 4.4, 4.6, 4.8, 5.0, 5.2, 5.4, 5.6, 5.8, 6.0, 6.2, 6.4, 6.6, 6.8, 7.0, 7.2, 7.4, 7.6, 7.8, 8.0, 8.2, 8.4, 8.6, 8.8, 9.0, 9.2, 9.4, 9.6, 9.8, 10.0, 10.2, 10.4, 10.6, 10.8, 11.0, 11.2, 11.4, 11.6, 11.8, 12.0, 13, 14, 15, 16, 17, 18, 19, 20, 21, 22, 23, 24, 30, 36, 42, 48, 54, 60, 66, or 72 months. In some embodiments, the mTOR-inhibitor is sirolimus. In some embodiments, the mTOR-inhibitor in the nanoparticle in the composition is administered by intravenous administration. In some embodiments, the solid tumor is selected from the group consisting of neuroblastoma, soft tissue tumor (*e.g*., rhabdomyosarcoma), bone tumor (*e.g*., osteosarcoma, Ewing's sarcoma), CNS tumor (*e.g*., meduloblastoma, glioma), renal tumor, hepatic tumor (*e.g*., hepatoblastoma and hepatocellular carcinoma), and vascular tumors (*e.g*., Kaposi' sarcoma, angiosarcoma, Tufted angioma, and kaposiform hemangioendothelioma).

In some embodiments, there is provided a method of improving one or more clinical benefits of a human individual having a solid tumor, comprising administering to the individual an effective amount of a composition comprising nanoparticles comprising a mTOR-inhibitor (such as limus drug, for example sirolimus) and an albumin, wherein the individual is no more than about 21 years old (such as no more than about 18 years old). Clinical benefits includes, but are not limited to, improved/better quality of life, improved/better symptom control of the solid tumor, and increased weight gain. In some embodiments, the individual has improved quality of life, improved symptom control and increased weight gain. In some embodiments, the mTOR-inhibitor is sirolimus. In some embodiments, the mTOR-inhibitor in the nanoparticle in the composition is administered by intravenous administration. In some embodiments, the solid tumor is selected from the group consisting of neuroblastoma, soft tissue tumor (*e.g*., rhabdomyosarcoma), bone tumor (*e.g*., osteosarcoma, Ewing's sarcoma), CNS tumor (*e.g*., meduloblastoma, glioma), renal tumor, hepatic tumor (*e.g*., hepatoblastoma and hepatocellular carcinoma), and vascular tumors (*e.g*., Kaposi' sarcoma, angiosarcoma, Tufted angioma, and kaposiform hemangioendothelioma).

In some embodiments, there is provided a method of alleviating one or more symptoms in a human individual having a solid tumor, comprising administering to the individual an effective amount of a composition comprising nanoparticles comprising a mTOR-inhibitor (such as limus drug, for example sirolimus) and an albumin, wherein the individual is no more than about 21 years old (such as no more than about 18 years old). In some embodiments, the mTOR-inhibitor is sirolimus. In some embodiments, the mTOR-inhibitor in the nanoparticle in the composition is administered by intravenous administration. In some embodiments, the solid tumor is selected from the group consisting of neuroblastoma, soft tissue tumor (*e.g*., rhabdomyosarcoma), bone tumor (*e.g*., osteosarcoma, Ewing's sarcoma), CNS tumor (*e.g*., meduloblastoma, glioma), renal tumor, hepatic tumor (*e.g*., hepatoblastoma and hepatocellular carcinoma), and vascular tumors (*e.g*., Kaposi' sarcoma, angiosarcoma, Tufted angioma, and kaposiform hemangioendothelioma).

In some embodiments, there is provided a method of treating a solid tumor in a human individual comprising administering to the individual an effective amount of a composition comprising *Nab*-sirolimus, wherein the individual is no more than about 21 years old (such as no more than about 18 years old), and wherein the *Nab*-sirolimus is administered weekly for two out of three weeks at a dose ranging from about 20 mg/m² to about 55 mg/m² (for example, about 30 mg/m² to about 50 mg/m², e.g., about any one of 20 mg/m², 35 mg/m², 45 mg/m², or 55 mg/m²). In some embodiments, the *Nab*-sirolimus is administered by intravenous administration. In some embodiments, the solid tumor is selected from the group consisting of neuroblastoma, soft tissue tumor (*e.g*., rhabdomyosarcoma), bone tumor (*e.g*., osteosarcoma, Ewing's sarcoma), and CNS tumor (*e.g*., meduloblastoma, glioma), renal tumor, hepatic tumor (*e.g*., hepatoblastoma and hepatocellular carcinoma). In some embodiments, the individual is no more than about any of 17, 16, 15, 14, 13, 12, 11, 10, 9, 8, 7, 6, 5, 4, 3, 2, or 1 year old. In some embodiments, the individual is about 9 to about 15 years old. In some embodiments, the individual is about 5 to about 9 years old. In some embodiments, the individual is about 1 to about 5 years old. In some embodiments, the individual is no more than about 1 year old, such as about 6 months old to about 1 year old, less than about 6 months old, or less than about 3 months old. In some embodiments, the method further comprises a step of selecting the individual for treatment based on the expression level of S6K1 and/or 4EBP1. In some embodiments, the method further comprises a step of determining the expression level of S6K1 and/or 4EBP1 in the individual.

In some embodiments, there is provided a method of treating a solid tumor in a human individual comprising administering to the individual an effective amount of a composition comprising *Nab*-sirolimus, wherein the individual is no more than about 21 years old (such as no more than about 18 years old), wherein the *Nab*-sirolimus is administered weekly for two out of three weeks at a dose ranging from about 20 mg/m² to about 55 mg/m² (for example, about 30 mg/m² to about 50 mg/m², *e.g.,* about any one of 20 mg/m², 35 mg/m², 45 mg/m², or 55 mg/m²), and wherein the individual is resistant or refractory to a prior therapy (such as a mTOR-inhibitor-based therapy). In some embodiments, the *Nab*-sirolimus is administered by intravenous administration. In some embodiments, the solid tumor is selected from the group consisting of neuroblastoma, soft tissue tumor (*e.g*., rhabdomyosarcoma), bone tumor (*e.g*., osteosarcoma, Ewing's sarcoma), and CNS tumor (*e.g*., meduloblastoma, glioma), renal tumor, hepatic tumor (*e.g*., hepatoblastoma and hepatocellular carcinoma). In some embodiments, the individual is no more than about any of 17, 16, 15, 14, 13, 12, 11, 10, 9, 8, 7, 6, 5, 4, 3, 2, or 1 year old. In some embodiments, the individual is about 9 to about 15 years old. In some embodiments, the individual is about 5 to about 9 years old. In some embodiments, the individual is about 1 to about 5 years old. In some embodiments, the individual is no more than about 1 year old, such as about 6 months old to about 1 year old, less than about 6 months old, or less than about 3 months old. In some embodiments, the method further comprises a step of selecting the individual for treatment based on the expression level of S6K1 and/or 4EBP1. In some embodiments, the method further comprises a step of determining the expression level of S6K1 and/or 4EBP1 in the individual.

In some embodiments, there is provided a method of prolonging the survival of a human individual having a solid tumor comprising administering to the individual an effective amount of a composition comprising *Nab*-sirolimus, wherein the individual is no more than about 21 years old (such as no more than about 18 years old), and wherein the *Nab*-sirolimus is administered weekly for two out of three weeks at a dose ranging from about 20 mg/m² to about 55 mg/m² (for example, about 30 mg/m² to about 50 mg/m², e.g., about any one of 20 mg/m², 35 mg/m², 45 mg/m², or 55 mg/m²). In some embodiments, the *Nab*-sirolimus is administered by intravenous administration. In some embodiments, the solid tumor is selected from the group consisting of neuroblastoma, soft tissue tumor (*e.g*., rhabdomyosarcoma), bone tumor (*e.g*., osteosarcoma, Ewing's sarcoma), and CNS tumor (*e.g*., meduloblastoma, glioma), renal tumor, hepatic tumor (*e.g*., hepatoblastoma and hepatocellular carcinoma). In some embodiments, the individual is no more than about any of 17, 16, 15, 14, 13, 12, 11, 10, 9, 8, 7, 6, 5, 4, 3, 2, or 1 year old. In some embodiments, the individual is about 9 to about 15 years old. In some embodiments, the individual is about 5 to about 9 years old. In some embodiments, the individual is about 1 to about 5 years old. In some embodiments, the individual is no more than about 1 year old, such as about 6 months old to about 1 year old, less than about 6 months old, or less than about 3 months old. In some embodiments, the method further comprises a step of selecting the individual for treatment based on the expression level of S6K1 and/or 4EBP1. In some embodiments, the method further comprises a step of determining the expression level of S6K1 and/or 4EBP1 in the individual.

In some embodiments, there is provided a method of treating a solid tumor in a human individual comprising administering to the individual an effective amount of a composition comprising *Nab*-sirolimus, an effective amount of irinotecan, and an effective amount of temozolomide, wherein the individual is no more than about 21 years old (such as no more than about 18 years old), and wherein the *Nab*-sirolimus is administered weekly for two out of three weeks at a dose ranging from about 20 mg/m² to about 55 mg/m² (for example, about 30 mg/m² to about 50 mg/m², e.g., about any one of 20 mg/m², 35 mg/m², 45 mg/m², or 55 mg/m²). In some embodiments, the *Nab*-sirolimus is administered by intravenous administration. In some embodiments, the solid tumor is selected from the group consisting of neuroblastoma, soft tissue tumor (*e.g*., rhabdomyosarcoma), bone tumor (*e.g.,* osteosarcoma, Ewing's sarcoma), and CNS tumor (*e.g*., meduloblastoma, glioma), renal tumor, hepatic tumor (*e.g*., hepatoblastoma and hepatocellular carcinoma). In some embodiments, the individual is no more than about any of 17, 16, 15, 14, 13, 12, 11, 10, 9, 8, 7, 6, 5, 4, 3, 2, or 1 year old. In some embodiments, the individual is about 9 to about 15 years old. In some embodiments, the individual is about 5 to about 9 years old. In some embodiments, the individual is about 1 to about 5 years old. In some embodiments, the individual is no more than about 1 year old, such as about 6 months old to about 1 year old, less than about 6 months old, or less than about 3 months old. In some embodiments, the method further comprises a step of selecting the individual for treatment based on the expression level of S6K1 and/or 4EBP1. In some embodiments, the method further comprises a step of determining the expression level of S6K1 and/or 4EBP1 in the individual. In some embodiments, irinotecan is administered at a dose of about 90 mg/m². In some embodiments, irinotecan is administered orally. In some embodiments, irinotecan is administered once daily for first five days in a 3-week treatment cycle. In some embodiments, temozolomide is administered at a dose of about 125 mg/m². In some embodiments, temozolomide is administered orally. In some embodiments, temozolomide is administered once daily for first five days in a 3-week treatment cycle. In some embodiments, the nanoparticle composition is administered about 1 hour after irinotecan administration. In some embodiments, irinotecan is administered one hour after administration of temozolomide. In some embodiments, a diarrheal prophylaxis, such as cefixime, is administered, for example, about 2 days prior to the first dose of irinotecan, during irinotecan administration, and about 3 days after the last does of irinotecan of each cycle. In some embodiments, the method is repeated, such as for about 35 cycles.

In some embodiments, there is provided a method of treating a solid tumor in a human individual comprising administering to the individual an effective amount of a composition comprising *Nab*-sirolimus, an effective amount of irinotecan, and an effective amount of temozolomide, wherein the individual is no more than about 21 years old (such as no more than about 18 years old), wherein the *Nab*-sirolimus is administered weekly for two out of three weeks at a dose ranging from about 20 mg/m² to about 35 mg/m² (for example, about 30 mg/m² to about 50 mg/m², *e.g.,* about any one of 20 mg/m², 35 mg/m², 45 mg/m², or 55 mg/m²), and wherein the individual is resistant or refractory to a prior therapy (such as a mTOR-inhibitor-based therapy). In some embodiments, the *Nab*-sirolimus is administered by intravenous administration. In some embodiments, the solid tumor is selected from the group consisting of neuroblastoma, soft tissue tumor (*e.g*., rhabdomyosarcoma), bone tumor (*e.g*., osteosarcoma, Ewing's sarcoma), and CNS tumor (*e.g*., meduloblastoma, glioma), renal tumor, hepatic tumor (*e.g*., hepatoblastoma and hepatocellular carcinoma). In some embodiments, the individual is no more than about any of 17, 16, 15, 14, 13, 12, 11, 10, 9, 8, 7, 6, 5, 4, 3, 2, or 1 year old. In some embodiments, the individual is about 9 to about 15 years old. In some embodiments, the individual is about 5 to about 9 years old. In some embodiments, the individual is about 1 to about 5 years old. In some embodiments, the individual is no more than about 1 year old, such as about 6 months old to about 1 year old, less than about 6 months old, or less than about 3 months old. In some embodiments, the method further comprises a step of selecting the individual for treatment based on the expression level of S6K1 and/or 4EBP1. In some embodiments, the method further comprises a step of determining the expression level of S6K1 and/or 4EBP1 in the individual. In some embodiments, irinotecan is administered at a dose of about 90 mg/m². In some embodiments, irinotecan is administered orally. In some embodiments, irinotecan is administered once daily for first five days in a 3-week treatment cycle. In some embodiments, temozolomide is administered at a dose of about 125 mg/m². In some embodiments, temozolomide is administered orally. In some embodiments, temozolomide is administered once daily for first five days in a 3-week treatment cycle. In some embodiments, the nanoparticle composition is administered about 1 hour after irinotecan administration. In some embodiments, irinotecan is administered one hour after administration oftemozolomide. In some embodiments, a diarrheal prophylaxis, such as cefixime, is administered, for example, about 2 days prior to the first dose of irinotecan, during irinotecan administration, and about 3 days after the last does of irinotecan of each cycle. In some embodiments, the method is repeated, such as for about 35 cycles.

In some embodiments, there is provided a method of prolonging the survival of a human individual having a solid tumor comprising administering to the individual an effective amount of a composition comprising *Nab-*sirolimus, an effective amount of irinotecan, and an effective amount of temozolomide, wherein the individual is no more than about 21 years old (such as no more than about 18 years old), and wherein the *Nab*-sirolimus is administered weekly for two out of three weeks at a dose ranging from about 20 mg/m² to about 55 mg/m² (for example, about 30 mg/m² to about 50 mg/m², e.g., about any one of 20 mg/m², 35 mg/m², 45 mg/m², or 55 mg/m²). In some embodiments, the *Nab*-sirolimus is administered by intravenous administration. In some embodiments, the solid tumor is selected from the group consisting of neuroblastoma, soft tissue tumor (*e.g*., rhabdomyosarcoma), bone tumor (*e.g*., osteosarcoma, Ewing's sarcoma), and CNS tumor (*e.g*., meduloblastoma, glioma), renal tumor, hepatic tumor (*e.g*., hepatoblastoma and hepatocellular carcinoma). In some embodiments, the individual is no more than about any of 17, 16, 15, 14, 13, 12, 11, 10, 9, 8, 7, 6, 5, 4, 3, 2, or 1 year old. In some embodiments, the individual is about 9 to about 15 years old. In some embodiments, the individual is about 5 to about 9 years old. In some embodiments, the individual is about 1 to about 5 years old. In some embodiments, the individual is no more than about 1 year old, such as about 6 months old to about 1 year old, less than about 6 months old, or less than about 3 months old. In some embodiments, the method further comprises a step of selecting the individual for treatment based on the expression level of S6K1 and/or 4EBP1. In some embodiments, the method further comprises a step of determining the expression level of S6K1 and/or 4EBP1 in the individual. In some embodiments, irinotecan is administered at a dose of about 90 mg/m². In some embodiments, irinotecan is administered orally. In some embodiments, irinotecan is administered once daily for first five days in a 3-week treatment cycle. In some embodiments, temozolomide is administered at a dose of about 125 mg/m². In some embodiments, temozolomide is administered orally. In some embodiments, temozolomide is administered once daily for first five days in a 3-week treatment cycle. In some embodiments, the nanoparticle composition is administered about 1 hour after irinotecan administration. In some embodiments, irinotecan is administered one hour after administration of temozolomide. In some embodiments, a diarrheal prophylaxis, such as cefixime, is administered, for example, about 2 days prior to the first dose of irinotecan, during irinotecan administration, and about 3 days after the last does of irinotecan of each cycle. In some embodiments, the method is repeated, such as for about 35 cycles.

In some embodiments, there is provided a method of treating a vascular tumor (such as high-risk vascular tumor) in a human individual comprising administering to the individual an effective amount of a composition comprising *Nab*-sirolimus, and an effective amount of vincristine, wherein the individual is no more than about 21 years old (such as no more than about 18 years old). In some embodiments, the *Nab*-sirolimus is administered weekly for two out of three weeks at a dose ranging from about 20 mg/m² to about 55 mg/m² (for example, about 30 mg/m² to about 50 mg/m², e.g., about any one of 20 mg/m², 35 mg/m², 45 mg/m², or 55 mg/m²). In some embodiments, the *Nab*-sirolimus is administered by intravenous administration. In some embodiments, the individual is no more than about any of 17, 16, 15, 14, 13, 12, 11, 10, 9, 8, 7, 6, 5, 4, 3, 2, or 1 year old. In some embodiments, the individual is about 9 to about 15 years old. In some embodiments, the individual is about 5 to about 9 years old. In some embodiments, the individual is about 1 to about 5 years old. In some embodiments, the individual is no more than about 1 year old, such as about 6 months old to about 1 year old, less than about 6 months old, or less than about 3 months old. In some embodiments, the method further comprises a step of selecting the individual for treatment based on the expression level of S6K1 and/or 4EBP1. In some embodiments, the method further comprises a step of determining the expression level of S6K1 and/or 4EBP1 in the individual. In some embodiments, the vincristine is administered intravenously. In some embodiments, vincristine and the *Nab*-sirolimus composition are administered sequentially. In some embodiments, vincristine and the *Nab*-sirolimus composition are administered simultaneously. In some embodiments, vincristine and the *Nab*-sirolimus composition are administered concurrently. In some embodiments, the vascular tumor is selected from the group consisting of Kaposi' sarcoma, angiosarcoma, Tufted angioma, and kaposiform hemangioendothelioma.

Also provided are compositions (such as pharmaceutical compositions), medicine, kits, and unit dosages comprising nanoparticles comprising an mTOR inhibitor (such as limus drug, for example sirolimus) useful for any of the methods of treating pediatric solid tumors described above.

### Dosing and Method of Administering the Nanoparticle Compositions

The dose of the mTOR nanoparticles (such as a limus nanoparticle compositions) administered to an individual (such as a human) may vary with the particular composition, the mode of administration, and the type of hyperplasia (such as cancer, restenosis, or pulmonary hypertension) being treated. In some embodiments, the amount of the composition is effective to result in an objective response (such as a partial response or a complete response). In some embodiments, the amount of the mTOR inhibitor nanoparticle composition (such as a limus nanoparticle composition) is sufficient to result in a complete response in the individual. In some embodiments, the amount of the mTOR inhibitor nanoparticle composition (such as a limus nanoparticle composition) is sufficient to result in a partial response in the individual. In some embodiments, the amount of the mTOR inhibitor nanoparticle composition (such as a limus nanoparticle composition) administered (for example when administered alone) is sufficient to produce an overall response rate of more than about any of 20%, 30%, 40%, 50%, 60%, or 64% among a population of individuals treated with the mTOR inhibitor nanoparticle composition (such as a limus nanoparticle composition). Responses of an individual to the treatment of the methods described herein can be determined, for example, based on RECIST levels, cystoscopy (with or without biopsy), biopsy, cytology, and CT imaging.

In some embodiments, the amount of the mTOR inhibitor nanoparticle composition (such as a limus nanoparticle composition) is sufficient to produce a negative biopsy in the individual.

In some embodiments, the amount of the composition is sufficient to prolong progress-free survival of the individual. In some embodiments, the amount of the composition is sufficient to prolong overall survival of the individual. In some embodiments, the amount of the composition (for example when administered alone) is sufficient to produce clinical benefit of more than about any of 10%, 20%, 30%, 40%, 50%, 60%, 70%, 80% or more among a population of individuals treated with the mTOR inhibitor nanoparticle composition (such as a limus nanoparticle composition).

In some embodiments, the amount of the composition is an amount sufficient to decrease the size of a hyperplastic tissue (such as tumor), decrease the number of abnormally proliferative cells (such as cancer cells, or abnormally proliferative cells in pulmonary hypertension or restenosis), or decrease the growth rate of a hyperplastic tissue (such as tumor) by at least about any of 10%, 20%, 30%, 40%, 50%, 60%, 70%, 80%, 90%, 95% or 100% compared to the corresponding size or growth rate of the hyperplastic tissue (such as tumor) in the same subject prior to treatment or compared to the corresponding activity in other subjects not receiving the treatment. Standard methods can be used to measure the magnitude of this effect, such as in vitro assays with purified enzyme, cell-based assays, animal models, or human testing.

In some embodiments, the amount of the mTOR inhibitor (such as a limus drug, for example sirolimus) in the composition is below the level that induces a toxicological effect (i.e., an effect above a clinically acceptable level of toxicity) or is at a level where a potential side effect can be controlled or tolerated when the composition is administered to the individual.

In some embodiments, the amount of the composition is close to a maximum tolerated dose (MTD) of the composition following the same dosing regimen. In some embodiments, the amount of the composition is more than about any of 80%, 90%, 95%, or 98% of the MTD.

In some embodiments, the effective amounts of an mTOR inhibitor (*e.g.*, a limus drug) in the nanoparticle composition include, but are not limited to, at least about any of 25 mg/m², 30 mg/m², 50 mg/m², 60 mg/m², 75 mg/m², 80 mg/m², 90 mg/m², 100 mg/m², 120 mg/m², 125 mg/m², 150 mg/m², 160 mg/m², 175 mg/m², 180 mg/m², 200 mg/m², 210 mg/m², 220 mg/m², 250 mg/m², 260 mg/m², 300 mg/m², 350 mg/m², 400 mg/m², 500 mg/m², 540 mg/m², 750 mg/m², 1000 mg/m², or 1080 mg/m² of an mTOR inhibitor (e.g., sirolimus). In various embodiments, the composition includes less than about any of 350 mg/m², 300 mg/m², 250 mg/m², 200 mg/m², 150 mg/m², 120 mg/m², 100 mg/m², 90 mg/m², 50 mg/m², or 30 mg/m² of an mTOR inhibitor (*e.g*., sirolimus). In some embodiments, the amount of the mTOR inhibitor (*e.g.*, sirolimus) per administration is less than about any of 25 mg/m², 22 mg/m², 20 mg/m², 18 mg/m², 15 mg/m², 14 mg/m², 13 mg/m², 12 mg/m², 11 mg/m², 10 mg/m², 9 mg/m², 8 mg/m², 7 mg/m², 6 mg/m², 5 mg/m², 4 mg/m², 3 mg/m², 2 mg/m², or 1 mg/m². In some embodiments, the effective amount of an mTOR inhibitor (*e.g.*, sirolimus) in the composition is included in any of the following ranges: about 1 to about 5 mg/m², about 5 to about 10 mg/m², about 10 to about 25 mg/m², about 25 to about 50 mg/m², about 50 to about 75 mg/m², about 75 to about 100 mg/m², about 100 to about 125 mg/m², about 125 to about 150 mg/m², about 150 to about 175 mg/m², about 175 to about 200 mg/m², about 200 to about 225 mg/m², about 225 to about 250 mg/m², about 250 to about 300 mg/m², about 300 to about 350 mg/m², or about 350 to about 400 mg/m². In some embodiments, the effective amount of an mTOR inhibitor (*e.g*., sirolimus) in the composition is about 5 to about 300 mg/m², such as about 100 to about 150 mg/m², about 120 mg/m², about 130 mg/m², or about 140 mg/m².

In some embodiments of any of the above aspects, the effective amount of an mTOR inhibitor (*e.g.*, sirolimus) in the composition includes at least about any of 1 mg/kg, 2.5 mg/kg, 3.5 mg/kg, 5 mg/kg, 6.5 mg/kg, 7.5 mg/kg, 10 mg/kg, 15 mg/kg, 20 mg/kg, 25 mg/kg, 30 mg/kg, 35 mg/kg, 40 mg/kg, 45 mg/kg, 50 mg/kg, 55 mg/kg, or 60 mg/kg. In various embodiments, the effective amount of an mTOR inhibitor (*e.g.*, sirolimus) in the composition includes less than about any of 350 mg/kg, 300 mg/kg, 250 mg/kg, 200 mg/kg, 150 mg/kg, 100 mg/kg, 50 mg/kg, 25 mg/kg, 20 mg/kg, 10 mg/kg, 7.5 mg/kg, 6.5 mg/kg, 5 mg/kg, 3.5 mg/kg, 2.5 mg/kg, or 1 mg/kg of an mTOR inhibitor (*e.g*., sirolimus).

In some embodiments, the dosing frequencies for the administration of the nanoparticle compositions include, but are not limited to, daily, every two days, every three days, every four days, every five days, every six days, weekly without break, three out of four weeks, once every three weeks, once every two weeks, or two out of three weeks. In some embodiments, the composition is administered about once every 2 weeks, once every 3 weeks, once every 4 weeks, once every 6 weeks, or once every 8 weeks. In some embodiments, the composition is administered at least about any of 1x, 2x, 3x, 4x, 5x, 6x, or 7x (i.e., daily) a week. In some embodiments, the intervals between each administration are less than about any of 6 months, 3 months, 1 month, 20 days, 15, days, 14 days, 13 days, 12 days, 11 days, 10 days, 9 days, 8 days, 7 days, 6 days, 5 days, 4 days, 3 days, 2 days, or 1 day. In some embodiments, the intervals between each administration are more than about any of 1 month, 2 months, 3 months, 4 months, 5 months, 6 months, 8 months, or 12 months. In some embodiments, there is no break in the dosing schedule. In some embodiments, the interval between each administration is no more than about a week.

In some embodiments, the dosing frequency is once every two days for one time, two times, three times, four times, five times, six times, seven times, eight times, nine times, ten times, and eleven times. In some embodiments, the dosing frequency is once every two days for five times. In some embodiments, the mTOR inhibitor (*e.g*., sirolimus) is administered over a period of at least ten days, wherein the interval between each administration is no more than about two days, and wherein the dose of the mTOR inhibitor (*e.g*., sirolimus) at each administration is about 0.25 mg/m² to about 250 mg/m², about 0.25 mg/m² to about 150 mg/m², about 0.25 mg/m² to about 75 mg/m², such as about 0.25 mg/m² to about 25 mg/m², or about 25 mg/m² to about 50 mg/m².

The administration of the composition can be extended over an extended period of time, such as from about a month up to about seven years. In some embodiments, the composition is administered over a period of at least about any of 3, 4, 5, 6, 7, 8, 9, 10, 11, 12, 18, 24, 30, 36, 48, 60, 72, or 84 months.

In some embodiments, the dosage of an mTOR inhibitor (*e.g*., sirolimus) in a nanoparticle composition can be in the range of 5-400 mg/m² when given on a 3 week schedule, or 5-250 mg/m² (such as 80-150 mg/m², for example 100-120 mg/m²) when given on a weekly schedule. For example, the amount of an mTOR inhibitor (*e.g.*, sirolimus) is about 60 to about 300 mg/m² (*e.g*., about 260 mg/m²) on a three week schedule.

In some embodiments, the exemplary dosing schedules for the administration of the nanoparticle composition (*e.g*., sirolimus/albumin nanoparticle composition) include, but are not limited to, 100 mg/m², weekly, without break; 75 mg/m² weekly, 3 out of four weeks; 100 mg/m²,weekly, 2 out of 3 weeks; 100 mg/m²,weekly, 3 out of 4 weeks; 125 mg/m², weekly, 3 out of 4 weeks; 125 mg/m², weekly, 2 out of 3 weeks; 130 mg/m², weekly, without break; 175 mg/m², once every 2 weeks; 260 mg/m², once every 2 weeks; 260 mg/m², once every 3 weeks; 180-300 mg/m², every three weeks; 60-175 mg/m², weekly, without break; 20-150 mg/m² twice a week; 150-250 mg/m² twice a week, and 10-150 mg/m² weekly, 2 out of 3 weeks; and 10-150 mg/m² weekly, 3 out of 4 weeks. The dosing frequency of the composition may be adjusted over the course of the treatment based on the judgment of the administering physician.

In some embodiments, the individual is treated for at least about any of one, two, three, four, five, six, seven, eight, nine, or ten treatment cycles.

The compositions described herein allow infusion of the composition to an individual over an infusion time that is shorter than about 24 hours. For example, in some embodiments, the composition is administered over an infusion period of less than about any of 24 hours, 12 hours, 8 hours, 5 hours, 3 hours, 2 hours, 1 hour, 30 minutes, 20 minutes, or 10 minutes. In some embodiments, the composition is administered over an infusion period of about 30 minutes.

In some embodiments, the exemplary dose of the mTOR inhibitor (in some embodiments a limus drug, for example, sirolimus) in the nanoparticle composition include, but is not limited to, about any of 50 mg/m², 60 mg/m², 75 mg/m², 80 mg/m², 90 mg/m², 100 mg/m², 120 mg/m², 160 mg/m², 175 mg/m², 200 mg/m², 210 mg/m², 220 mg/m², 260 mg/m², and 300 mg/m². For example, the dosage of an mTOR inhibitor in a nanoparticle composition can be in the range of about 100-400 mg/m² when given on a 3 week schedule, or about 50-250 mg/m² when given on a weekly schedule.

The mTOR inhibitor nanoparticle composition (such as a limus nanoparticle composition) can be administered to an individual (such as human) via various routes, including, for example, intravenous, intra-arterial, intraperitoneal, intrapulmonary, oral, inhalation, intravesicular, intramuscular, intra-tracheal, subcutaneous, intraocular, intrathecal, transmucosal, and transdermal. In some embodiments, sustained continuous release formulation of the composition may be used. In some embodiments, the composition is administered intravenously. In some embodiments, the composition is administered intravesicularly. In some embodiments, the composition is administered intraarterially. In some embodiments, the composition is administered intraperitoneally. In some embodiments, the composition is administered subcutaneously.

In some embodiments when the limus nanoparticle composition is administered intravesicularly, the dosage of an mTOR inhibitor (such as a limus drug, *e.g*., sirolimus) in a nanoparticle composition can be in the range of about 30 mg to about 400 mg in volume of about 20 to about 150 ml, for example retained in the bladder for about 30 minutes to about 4 hours. In some embodiments, the nanoparticle composition is retained in the bladder for about 30 minutes to about 4 hours, including for example about 30 minutes to about 1 hour, about 1 hour to about 2 hours, about 2 hours to about 3 hours, or about 3 hours to about 4 hours.

In some embodiments, the dosage of an mTOR inhibitor (such as a limus drug, *e.g*., sirolimus) is about 100 mg to about 400 mg, for example about 100 mg, about 200 mg, about 300 mg, or about 400 mg. In some embodiments, the limus drug is administered at about 100 mg weekly, about 200 mg weekly, about 300 mg weekly, about 100 mg twice weekly, or about 200 mg twice weekly. In some embodiments, the administration is further followed by a monthly maintenance dose (which can be the same or different from the weekly doses).

In some embodiments when the limus nanoparticle composition is administered intravenously, the dosage of an mTOR inhibitor (such as a limus drug, *e.g.*, sirolimus) in a nanoparticle composition can be in the range of about 30 mg to about 400 mg. The compositions described herein allow infusion of the composition to an individual over an infusion time that is shorter than about 24 hours. For example, in some embodiments, the composition is administered over an infusion period of less than about any of 24 hours, 12 hours, 8 hours, 5 hours, 3 hours, 2 hours, 1 hour, 30 minutes, 20 minutes, or 10 minutes. In some embodiments, the composition is administered over an infusion period of about 30 minutes to about 40 minutes.

### Nanoparticle Compositions

The nanoparticle compositions described herein comprise nanoparticles comprising (in various embodiments consisting essentially of) an mTOR inhibitor (such as a limus drug, for example sirolimus). The nanoparticles may further comprise a carrier protein (*e.g*., an albumin such as human serum albumin or human albumin). Nanoparticles of poorly water soluble drugs have been disclosed in, for example, U.S. Pat. Nos. 5,916,596; 6,506,405; 6,749,868, 6,537,579, 7,820,788, and also in U.S. Pat. Pub. Nos. 2006/0263434, and 2007/0082838; PCT Patent Application WO08/137148, each of which is incorporated by reference in their entirety.

In some embodiments, the composition comprises nanoparticles with an average or mean diameter of no greater than about 1000 nanometers (nm), such as no greater than about (or less than about) any of 900, 800, 700, 600, 500, 400, 300, 200, or 100 nm. In some embodiments, the average or mean diameters of the nanoparticles is no greater than about 150 nm (such as no greater than about 120 nm). In some embodiments, the average or mean diameters of the nanoparticles is no greater than about 150 nm. In some embodiments, the average or mean diameters of the nanoparticles is no greater than about 100 nm. In some embodiments, the average or mean diameter of the nanoparticles is about 20 nm to about 400 nm. In some embodiments, the average or mean diameter of the nanoparticles is about 40 nm to about 200 nm. In some embodiments, the nanoparticles are sterile-filterable.

In some embodiments, the nanoparticles in the composition described herein have an average diameter of no greater than about 200 nm, including for example no greater than about any one of 190, 180, 170, 160, 150, 140, 130, 120, 110, 100, 90, 80, 70, or 60 nm. In some embodiments, at least about 50% (for example at least about any one of 60%, 70%, 80%, 90%, 95%, or 99%) of the nanoparticles in the composition have a diameter of no greater than about 200 nm, including for example no greater than about any one of 190, 180, 170, 160, 150, 140, 130, 120, 110, 100, 90, 80, 70, or 60 nm. In some embodiments, at least about 50% (for example at least any one of 60%, 70%, 80%, 90%, 95%, or 99%) of the nanoparticles in the composition fall within the range of about 20 nm to about 400 nm, including for example about 20 nm to about 200 nm, about 40 nm to about 200 nm, about 30 nm to about 180 nm, about 40 nm to about 150 nm, about 50 nm to about 120 nm, or about 60 nm to about 100 nm. In some embodiments, the average or mean diameter of the nanoparticles is about 10 nm to about 150 nm. In some embodiments, the average or mean diameter of the nanoparticles is about 40 nm to about 120 nm.

In some embodiments, the carrier protein (*e.g*., an albumin) has sulfhydryl groups that can form disulfide bonds. In some embodiments, at least about 5% (including for example at least about any one of 10%, 15%, 20%, 25%, 30%, 40%, 50%, 60%, 70%, 80%, or 90%) of carrier protein (*e.g.*, an albumin) in the nanoparticle portion of the composition are crosslinked (for example crosslinked through one or more disulfide bonds).

In some embodiments, the nanoparticles comprising the mTOR inhibitor (such as a limus drug, *e.g.*, sirolimus) are associated (*e.g.*, coated) with a carrier protein (*e.g.*, an albumin such as human albumin or human serum albumin). In some embodiments, the composition comprises an mTOR inhibitor (such as a limus drug, for example sirolimus) in both nanoparticle and non-nanoparticle forms (*e.g.*, in the form of solutions or in the form of soluble carrier protein/nanoparticle complexes), wherein at least about any one of 50%, 60%, 70%, 80%, 90%, 95%, or 99% of the mTOR inhibitor (such as a limus drug, e.g., sirolimus) in the composition are in nanoparticle form. In some embodiments, the mTOR inhibitor (such as a limus drug, *e.g.*, sirolimus) in the nanoparticles constitutes more than about any one of 50%, 60%, 70%, 80%, 90%, 95%, or 99% of the nanoparticles by weight. In some embodiments, the nanoparticles have a non-polymeric matrix. In some embodiments, the nanoparticles comprise a core of an mTOR inhibitor (such as a limus drug, for example sirolimus) that is substantially free of polymeric materials (such as polymeric matrix).

In some embodiments, the composition comprises a carrier protein (*e.g*., an albumin) in both nanoparticle and non-nanoparticle portions of the composition, wherein at least about any one of 50%, 60%, 70%, 80%, 90%, 95%, or 99% of the carrier protein (*e.g.*, an albumin) in the composition are in non-nanoparticle portion of the composition.

In some embodiments, the weight ratio of the albumin (such as human albumin or human serum albumin) and the mTOR inhibitor in the nanoparticle composition is about 18:1 or less, such as about 15:1 or less, for example about 10:1 or less, about 9:1 or less or about 8:1 or less. In some embodiments, the weight ratio of the albumin (such as human albumin or human serum albumin) and the mTOR inhibitor in the nanoparticle composition is about any of 18:1, 17:1, 16:1, 15:1, 14:1, 13:1, 12:1, 11:1, 10:1, 9:1, 8:1, 7:1, 6:1, 5:1, 4:1, 3:1, 2:1, or 1:1. In some embodiments, the weight ratio of the albumin to the mTOR inhibitor (such as a limus drug, for example sirolimus) in the nanoparticle portion of the composition is about any one of 1:1, 1:2, 1:3, 1:4, 1:5, 1:9, 1:10, 1:11, 1:12, 1:13, 1:14, 1:15, or less. In some embodiments, the weight ratio of the albumin (such as human albumin or human serum albumin) to the mTOR inhibitor (such as a limus drug, *e.g*., sirolimus) in the composition is any one of the following: about 1:1 to about 18:1, about 1:1 to about 15:1, about 1:1 to about 12:1, about 1:1 to about 10:1, about 1:1 to about 9: 1, about 1:1 to about 8: 1, about 1:1 to about 7:1, about 1:1 to about 6:1, about 1:1 to about 5:1, about 1:1 to about 4:1, about 1:1 to about 3:1, about 1:1 to about 2:1, about 2:1 to about 15:1, about 3:1 to about 13:1, about 4:1 to about 12:1, about 5:1 to about 10:1, about 6:1 to about 10:1, or about 8:1 to about 9:1.

In some embodiments, the nanoparticle composition comprises one or more of the above characteristics.

The nanoparticles described herein may be present in a dry formulation (such as lyophilized composition) or suspended in a biocompatible medium. Suitable biocompatible media include, but are not limited to, water, buffered aqueous media, saline, buffered saline, optionally buffered solutions of amino acids, optionally buffered solutions of proteins, optionally buffered solutions of sugars, optionally buffered solutions of vitamins, optionally buffered solutions of synthetic polymers, lipid-containing emulsions, and the like.

In some embodiments, the nanoparticle composition comprises an albumin, such as human albumin or human serum albumin. In some embodiments, the albumin is a recombinant albumin.

Human serum albumin (HSA) is a highly soluble globular protein of Mᵣ 65K and consists of 585 amino acids. HSA is the most abundant protein in the plasma and accounts for 70-80 % of the colloid osmotic pressure of human plasma. The amino acid sequence of HSA contains a total of 17 disulfide bridges, one free thiol (Cys 34), and a single tryptophan (Trp 214). Intravenous use of HSA solution has been indicated for the prevention and treatment of hypovolumic shock (see, *e.g*., Tullis, JAMA, 237: 355-360, 460-463, (1977)) and Houser et al., Surgery, Gynecology and Obstetrics, 150: 811-816 (1980)) and in conjunction with exchange transfusion in the treatment of neonatal hyperbilirubinemia (see, *e.g*., Finlayson, Seminars in Thrombosis and Hemostasis, 6, 85-120, (1980)). Other albumins are contemplated, such as bovine serum albumin. Use of such non-human albumins could be appropriate, for example, in the context of use of these compositions in non-human mammals, such as the veterinary (including domestic pets and agricultural context). Human serum albumin (HSA) has multiple hydrophobic binding sites (a total of eight for fatty acids, an endogenous ligand of HSA) and binds a diverse set of drugs, especially neutral and negatively charged hydrophobic compounds (Goodman et al., The Pharmacological Basis of Therapeutics, 9th ed, McGraw-Hill New York (1996)). Two high affinity binding sites have been proposed in subdomains IIA and IIIA of HSA, which are highly elongated hydrophobic pockets with charged lysine and arginine residues near the surface which function as attachment points for polar ligand features (see, *e.g.,* Fehske et al., Biochem. Pharmcol., 30, 687-92 (198a), Vorum, Dan. Med. Bull., 46, 379-99 (1999), Kragh-Hansen, Dan. Med. Bull., 1441, 131-40 (1990), Curry et al., Nat. Struct. Biol., 5, 827-35 (1998), Sugio et al., Protein. Eng., 12, 439-46 (1999), He et al., Nature, 358, 209-15 (199b), and Carter et al., Adv. Protein. Chem., 45, 153-203 (1994)). Sirolimus and propofol have been shown to bind HSA (see, *e.g.,* Paal et al., Eur. J. Biochem., 268(7), 2187-91 (200a), Purcell et al., Biochim. Biophys. Acta, 1478(a), 61-8 (2000), Altmayer et al., Arzneimittelforschung, 45, 1053-6 (1995), and Garrido et al., Rev. Esp. Anestestiol. Reanim., 41, 308-12 (1994)). In addition, docetaxel has been shown to bind to human plasma proteins (see, *e.g*., Urien et al., Invest. New Drugs, 14(b), 147-51 (1996)).

The carrier protein (*e.g*., an albumin such as human albumin or human serum albumin) in the composition generally serves as a carrier for the mTOR inhibitor, i.e., the albumin in the composition makes the mTOR inhibitor (such as a limus drug, *e.g*., sirolimus) more readily suspendable in an aqueous medium or helps maintain the suspension as compared to compositions not comprising a carrier protein. This can avoid the use of toxic solvents (or surfactants) for solubilizing the mTOR inhibitor, and thereby can reduce one or more side effects of administration of the mTOR inhibitor (such as a limus drug, *e.g*., sirolimus) into an individual (such as a human). Thus, in some embodiments, the composition described herein is substantially free (such as free) of surfactants, such as Cremophor (or polyoxyethylated castor oil, including Cremophor EL^{®} (BASF)). In some embodiments, the nanoparticle composition is substantially free (such as free) of surfactants. A composition is "substantially free of Cremophor" or "substantially free of surfactant" if the amount of Cremophor or surfactant in the composition is not sufficient to cause one or more side effect(s) in an individual when the nanoparticle composition is administered to the individual. In some embodiments, the nanoparticle composition contains less than about any one of 20%, 15%, 10%, 7.5%, 5%, 2.5%, or 1% organic solvent or surfactant. In some embodiments, the carrier protein is an albumin. In some embodiments, the albumin is human albumin or human serum albumin. In some embodiments, the albumin is recombinant albumin.

The amount of a carrier protein such as an albumin in the composition described herein will vary depending on other components in the composition. In some embodiments, the composition comprises a carrier protein such as an albumin in an amount that is sufficient to stabilize the mTOR inhibitor (such as a limus drug, *e.g.*, sirolimus) in an aqueous suspension, for example, in the form of a stable colloidal suspension (such as a stable suspension of nanoparticles). In some embodiments, the carrier protein such as an albumin is in an amount that reduces the sedimentation rate of the mTOR inhibitor (such as a limus drug, *e.g.*, sirolimus) in an aqueous medium. For particle-containing compositions, the amount of the carrier protein such as an albumin also depends on the size and density of nanoparticles of the mTOR inhibitor.

An mTOR inhibitor (such as a limus drug, for example sirolimus) is "stabilized" in an aqueous suspension if it remains suspended in an aqueous medium (such as without visible precipitation or sedimentation) for an extended period of time, such as for at least about any of 0.1, 0.2, 0.25, 0.5, 1, 2, 3, 4, 5, 6, 7, 8, 9, 10, 11, 12, 24, 36, 48, 60, or 72 hours. The suspension is generally, but not necessarily, suitable for administration to an individual (such as human). Stability of the suspension is generally (but not necessarily) evaluated at a storage temperature (such as room temperature (such as 20-25 °C) or refrigerated conditions (such as 4 °C)). For example, a suspension is stable at a storage temperature if it exhibits no flocculation or particle agglomeration visible to the naked eye or when viewed under the optical microscope at 1000 times, at about fifteen minutes after preparation of the suspension. Stability can also be evaluated under accelerated testing conditions, such as at a temperature that is higher than about 40 °C.

In some embodiments, the carrier protein (*e.g.*, an albumin) is present in an amount that is sufficient to stabilize the mTOR inhibitor (such as a limus drug, *e.g*., sirolimus) in an aqueous suspension at a certain concentration. For example, the concentration of the mTOR inhibitor (such as a limus drug, *e.g*., sirolimus) in the composition is about 0.1 to about 100 mg/ml, including for example any of about 0.1 to about 50 mg/ml, about 0.1 to about 20 mg/ml, about 1 to about 10 mg/ml, about 2 mg/ml to about 8 mg/ml, about 4 to about 6 mg/ml, or about 5 mg/ml. In some embodiments, the concentration of the mTOR inhibitor (such as a limus drug, e.g., sirolimus) is at least about any of 1.3 mg/ml, 1.5 mg/ml, 2 mg/ml, 3 mg/ml, 4 mg/ml, 5 mg/ml, 6 mg/ml, 7 mg/ml, 8 mg/ml, 9 mg/ml, 10 mg/ml, 15 mg/ml, 20 mg/ml, 25 mg/ml, 30 mg/ml, 40 mg/ml, and 50 mg/ml. In some embodiments, the carrier protein (*e.g*., an albumin) is present in an amount that avoids use of surfactants (such as Cremophor), so that the composition is free or substantially free of surfactant (such as Cremophor).

In some embodiments, the composition, in liquid form, comprises from about 0.1% to about 50% (w/v) (*e.g*. about 0.5% (w/v), about 5% (w/v), about 10% (w/v), about 15% (w/v), about 20% (w/v), about 30% (w/v), about 40% (w/v), or about 50% (w/v)) of carrier protein (*e.g.*, an albumin). In some embodiments, the composition, in liquid form, comprises about 0.5% to about 5% (w/v) of carrier protein (*e.g.*, an albumin).

In some embodiments, the weight ratio of a carrier protein (*e.g*., an albumin) to the mTOR inhibitor (such as a limus drug, *e.g*., sirolimus) in the nanoparticle composition is such that a sufficient amount of mTOR inhibitor binds to, or is transported by, the cell. While the weight ratio of a carrier protein (*e.g.*, an albumin) to mTOR inhibitor will have to be optimized for different carrier protein (*e.g.*, an albumin) and mTOR inhibitor combinations, generally the weight ratio of carrier protein (*e.g.*, an albumin), to mTOR inhibitor (such as a limus drug, *e.g.*, sirolimus) (w/w) is about 0.01:1 to about 100:1, about 0.02:1 to about 50:1, about 0.05:1 to about 20:1, about 0.1:1 to about 20:1, about 1:1 to about 18:1, about 2:1 to about 15:1, about 3:1 to about 12:1, about 4:1 to about 10:1, about 5:1 to about 9:1, or about 9:1. In some embodiments, the carrier protein (*e.g.*, an albumin) to mTOR inhibitor weight ratio is about any of 18:1 or less, 15:1 or less, 14:1 or less, 13:1 or less, 12:1 or less, 11:1 or less, 10:1 or less, 9:1 or less, 8:1 or less, 7:1 or less, 6:1 or less, 5:1 or less, 4:1 or less, and 3:1 or less. In some embodiments, the carrier protein is an albumin. In some embodiments, the weight ratio of the albumin (such as human albumin or human serum albumin) to the mTOR inhibitor in the composition is any one of the following: about 1:1 to about 18:1, about 1:1 to about 15:1, about 1:1 to about 12:1, about 1:1 to about 10:1, about 1:1 to about 9:1, about 1:1 to about 8:1, about 1:1 to about 7:1, about 1:1 to about 6:1, about 1:1 to about 5:1, about 1:1 to about 4:1, about 1:1 to about 3:1, about 1:1 to about 2:1, or about 1:1 to about 1:1.5.

In some embodiments, the carrier protein (*e.g*., an albumin) allows the composition to be administered to an individual (such as human) without significant side effects. In some embodiments, the carrier protein (*e.g*., an albumin such as human serum albumin or human albumin) is in an amount that is effective to reduce one or more side effects of administration of the mTOR inhibitor (such as a limus drug, *e.g*., sirolimus) to a human. The term "reducing one or more side effects" of administration of the mTOR inhibitor (such as a limus drug, *e.g*., sirolimus) refers to reduction, alleviation, elimination, or avoidance of one or more undesirable effects caused by the mTOR inhibitor, as well as side effects caused by delivery vehicles (such as solvents that render the limus drugs suitable for injection) used to deliver the mTOR inhibitor. Such side effects include, for example, myelosuppression, neurotoxicity, hypersensitivity, inflammation, venous irritation, phlebitis, pain, skin irritation, peripheral neuropathy, neutropenic fever, anaphylactic reaction, venous thrombosis, extravasation, and combinations thereof. These side effects, however, are merely exemplary and other side effects, or combination of side effects, associated with limus drugs (such as sirolimus) can be reduced.

In some embodiments, the nanoparticle compositions described herein comprises nanoparticles comprising an mTOR inhibitor (such as a limus drug, for example sirolimus) and an albumin (such as human albumin or human serum albumin), wherein the nanoparticles have an average diameter of no greater than about 150 nm. In some embodiments, the nanoparticle compositions described herein comprises nanoparticles comprising an mTOR inhibitor (such as a limus drug, for example sirolimus) and an albumin (such as human albumin or human serum albumin), wherein the nanoparticles have an average diameter of no greater than about 150 nm. In some embodiments, the nanoparticle compositions described herein comprises nanoparticles comprising an mTOR inhibitor (such as a limus drug, for example sirolimus) and an albumin (such as human albumin or human serum albumin), wherein the nanoparticles have an average diameter of no greater than about 150 nm (for example about 100 nm). In some embodiments, the nanoparticle compositions described herein comprises nanoparticles comprising sirolimus and human albumin (such as human serum albumin), wherein the nanoparticles have an average diameter of no greater than about 150 nm (for example about 100 nm).

In some embodiments, the nanoparticle compositions described herein comprises nanoparticles comprising an mTOR inhibitor (such as a limus drug, for example sirolimus) and an albumin (such as human albumin or human serum albumin), wherein the nanoparticles have an average diameter of no greater than about 150 nm, wherein the weight ratio of the albumin and the mTOR inhibitor in the composition is no greater than about 9:1 (such as about 9:1 or about 8:1). In some embodiments, the nanoparticle compositions described herein comprises nanoparticles comprising an mTOR inhibitor (such as a limus drug, for example sirolimus) and an albumin (such as human albumin or human serum albumin), wherein the nanoparticles have an average diameter of no greater than about 150 nm, wherein the weight ratio of the albumin and the mTOR inhibitor in the composition is no greater than about 9:1 (such as about 9:1 or about 8:1). In some embodiments, the nanoparticle compositions described herein comprises nanoparticles comprising an mTOR inhibitor (such as a limus drug, for example sirolimus) and an albumin (such as human albumin or human serum albumin), wherein the nanoparticles have an average diameter of about 150 nm, wherein the weight ratio of the albumin and the mTOR inhibitor in the composition is no greater than about 9:1 (such as about 9:1 or about 8:1). In some embodiments, the nanoparticle compositions described herein comprises nanoparticles comprising sirolimus and human albumin (such as human serum albumin), wherein the nanoparticles have an average diameter of no greater than about 150 nm (for example about 100 nm), wherein the weight ratio of albumin and sirolimus inhibitor in the composition is about 9:1 or about 8:1.

In some embodiments, the nanoparticle compositions described herein comprises nanoparticles comprising an mTOR inhibitor (such as a limus drug, for example sirolimus) associated (*e.g*., coated) with an albumin (such as human albumin or human serum albumin). In some embodiments, the nanoparticle compositions described herein comprises nanoparticles comprising an mTOR inhibitor (such as a limus drug, for example sirolimus) associated (*e.g*., coated) with an albumin (such as human albumin or human serum albumin), wherein the nanoparticles have an average diameter of no greater than about 150 nm. In some embodiments, the nanoparticle compositions described herein comprises nanoparticles comprising an mTOR inhibitor (such as a limus drug, for example sirolimus) associated (*e.g*., coated) with an albumin (such as human albumin or human serum albumin), wherein the nanoparticles have an average diameter of no greater than about 150 nm. In some embodiments, the nanoparticle compositions described herein comprises nanoparticles comprising an mTOR inhibitor (such as a limus drug, for example sirolimus) associated (*e.g*., coated) with an albumin (such as human albumin or human serum albumin), wherein the nanoparticles have an average diameter of no greater than about 150 nm (for example about 100 nm). In some embodiments, the nanoparticle compositions described herein comprises nanoparticles comprising sirolimus associated (*e.g*., coated) with human albumin (such as human serum albumin), wherein the nanoparticles have an average diameter of no greater than about 150 nm (for example about 100 nm).

In some embodiments, the nanoparticle compositions described herein comprise nanoparticles comprising an mTOR inhibitor (such as a limus drug, for example sirolimus) associated (*e.g.*, coated) with an albumin (such as human albumin or human serum albumin), wherein the weight ratio of the albumin and the mTOR inhibitor in the composition is no greater than about 9:1 (such as about 9:1 or about 8:1). In some embodiments, the nanoparticle compositions described herein comprises nanoparticles comprising an mTOR inhibitor (such as a limus drug, for example sirolimus) associated (*e.g*., coated) with an albumin (such as human albumin or human serum albumin), wherein the nanoparticles have an average diameter of no greater than about 150 nm, wherein the weight ratio of the albumin and the mTOR inhibitor in the composition is no greater than about 9:1 (such as about 9:1 or about 8:1). In some embodiments, the nanoparticle compositions described herein comprises nanoparticles comprising an mTOR inhibitor (such as a limus drug, for example sirolimus) associated (*e.g*., coated) with an albumin (such as human albumin or human serum albumin), wherein the nanoparticles have an average diameter of no greater than about 150 nm, wherein the weight ratio of the albumin and the mTOR inhibitor in the composition is no greater than about 9:1 (such as about 9:1 or about 8:1). In some embodiments, the nanoparticle compositions described herein comprises nanoparticles comprising an mTOR inhibitor (such as a limus drug, for example sirolimus) associated (*e.g.*, coated) with an albumin (such as human albumin or human serum albumin), wherein the nanoparticles have an average diameter of about 150 nm, wherein the weight ratio of the albumin and the mTOR inhibitor in the composition is no greater than about 9:1 (such as about 9:1 or about 8:1). In some embodiments, the nanoparticle compositions described herein comprises nanoparticles comprising sirolimus associated (*e.g*., coated) with human albumin (such as human serum albumin), wherein the nanoparticles have an average diameter of no greater than about 150 nm (for example about 100 nm), wherein the weight ratio of albumin and the sirolimus in the composition is about 9:1 or about 8:1.

In some embodiments, the nanoparticle compositions described herein comprises nanoparticles comprising an mTOR inhibitor (such as a limus drug, for example sirolimus) stabilized by an albumin (such as human albumin or human serum albumin). In some embodiments, the nanoparticle compositions described herein comprises nanoparticles comprising an mTOR inhibitor (such as a limus drug, for example sirolimus) stabilized by an albumin (such as human albumin or human serum albumin), wherein the nanoparticles have an average diameter of no greater than about 150 nm. In some embodiments, the nanoparticle compositions described herein comprises nanoparticles comprising an mTOR inhibitor (such as a limus drug, for example sirolimus) stabilized by an albumin (such as human albumin or human serum albumin), wherein the nanoparticles have an average diameter of no greater than about 150 nm. In some embodiments, the nanoparticle compositions described herein comprises nanoparticles comprising an mTOR inhibitor (such as a limus drug, for example sirolimus) stabilized by an albumin (such as human albumin or human serum albumin), wherein the nanoparticles have an average diameter of no greater than about 150 nm (for example about 100 nm). In some embodiments, the nanoparticle compositions described herein comprises nanoparticles comprising sirolimus stabilized by human albumin (such as human serum albumin), wherein the nanoparticles have an average diameter of no greater than about 150 nm (for example about 100 nm).

In some embodiments, the nanoparticle compositions described herein comprises nanoparticles comprising an mTOR inhibitor (such as a limus drug, for example sirolimus) stabilized by an albumin (such as human albumin or human serum albumin), wherein the weight ratio of the albumin and the mTOR inhibitor in the composition is no greater than about 9:1 (such as about 9:1 or about 8:1). In some embodiments, the nanoparticle compositions described herein comprises nanoparticles comprising an mTOR inhibitor (such as a limus drug, for example sirolimus) stabilized by an albumin (such as human albumin or human serum albumin), wherein the nanoparticles have an average diameter of no greater than about 150 nm, wherein the weight ratio of the albumin and the mTOR inhibitor in the composition is no greater than about 9:1 (such as about 9:1 or about 8:1). In some embodiments, the nanoparticle compositions described herein comprises nanoparticles comprising an mTOR inhibitor (such as a limus drug, for example sirolimus) stabilized by an albumin (such as human albumin or human serum albumin), wherein the nanoparticles have an average diameter of no greater than about 150 nm, wherein the weight ratio of the albumin and the mTOR inhibitor in the composition is no greater than about 9:1 (such as about 9:1 or about 8:1). In some embodiments, the nanoparticle compositions described herein comprises nanoparticles comprising an mTOR inhibitor (such as a limus drug, for example sirolimus) stabilized by an albumin (such as human albumin or human serum albumin), wherein the nanoparticles have an average diameter of about 150 nm, wherein the weight ratio of the albumin and the mTOR inhibitor in the composition is no greater than about 9:1 (such as about 9:1 or about 8:1). In some embodiments, the nanoparticle compositions described herein comprises nanoparticles comprising sirolimus stabilized by human albumin (such as human serum albumin), wherein the nanoparticles have an average diameter of no greater than about 150 nm (for example about 100 nm), wherein the weight ratio of albumin and the sirolimus in the composition is about 9:1 or about 8:1.

In some embodiments, the nanoparticle composition comprises *Nab*-sirolimus. In some embodiments, the nanoparticle composition is *Nab*-sirolimus. *Nab*-sirolimus is a formulation of sirolimus stabilized by human albumin USP, which can be dispersed in directly injectable physiological solution. The weight ratio of human albumin and sirolimus is about 8:1 to about 9:1. When dispersed in a suitable aqueous medium such as 0.9% sodium chloride injection or 5% dextrose injection, *Nab*-sirolimus forms a stable colloidal suspension of sirolimus. The mean particle size of the nanoparticles in the colloidal suspension is about 100 nanometers. Since HSA is freely soluble in water, *Nab*-sirolimus can be reconstituted in a wide range of concentrations ranging from dilute (0.1 mg/ml sirolimus) to concentrated (20 mg/ml sirolimus), including for example about 2 mg/ml to about 8 mg/ml, or about 5 mg/ml.

Methods of making nanoparticle compositions are known in the art. For example, nanoparticles containing mTOR inhibitor (such as a limus drug, *e.g*., sirolimus) and carrier protein (*e.g*., an albumin such as human serum albumin or human albumin) can be prepared under conditions of high shear forces (*e.g*., sonication, high pressure homogenization, or the like). These methods are disclosed in, for example, U.S. Pat. Nos. 5,916,596; 6,506,405; 6,749,868, 6,537,579 and 7,820,788 and also in U.S. Pat. Pub. Nos. 2007/0082838, 2006/0263434 and PCT Application WO08/137148.

Briefly, the mTOR inhibitor (such as a limus drug, *e.g*., sirolimus) is dissolved in an organic solvent, and the solution can be added to a carrier protein solution such as an albumin solution. The mixture is subjected to high pressure homogenization. The organic solvent can then be removed by evaporation. The dispersion obtained can be further lyophilized. Suitable organic solvent include, for example, ketones, esters, ethers, chlorinated solvents, and other solvents known in the art. For example, the organic solvent can be methylene chloride or chlorofom/ethanol (for example with a ratio of about any of 1:9, 1:8, 1:7, 1:6, 1:5, 1:4, 1:3, 1:2, 1:1, 2:1, 3:1, 4:1, 5:1, 6:1, 7:1, 8:1, or 9:1).

### mTOR inhibitor

The methods described herein in some embodiments comprise administration of nanoparticle compositions of mTOR inhibitors. "mTOR inhibitor" used herein refers to an inhibitor of mTOR. In some embodiments, the mTOR inhibitor is an inhibitor of mTORC1 (including for example, an inhibitor of mTORC1, but not an inhibitor of mTORC2 at a maximum tolerated dosage). In some embodiments, the mTOR inhibitor is an inhibitor of mTORC2 (including for example, an inhibitor of mTORC2, but not an inhibitor of mTORC1 at a maximum tolerated dosage). In some embodiments, the mTOR inhibitor is an inhibitor of both mTORC1 and mTORC2 (for example at a maximum tolerated dosage).

In some embodiments, the mTOR inhibitor is a limus drug, which includes sirolimus and its analogues. Examples of limus drugs include, but are not limited to, temsirolimus (CCI-779), everolimus (RAD001), ridaforolimus (AP-23573), deforolimus ( MK-8669), zotarolimus (ABT-578), pimecrolimus, and tacrolimus (FK-506). In some embodiments, the limus drug is selected from the group consisting of temsirolimus (CCI-779), everolimus (RAD001), ridaforolimus (AP-23573), deforolimus ( MK-8669), zotarolimus (ABT-578), pimecrolimus, and tacrolimus (FK-506).

In some embodiments, the mTOR inhibitor is sirolimus. Sirolimus is macrolide antibiotic that complexes with FKBP-12 and inhibits the mTOR pathway by binding mTORC1.

In some embodiments, the mTOR inhibitor is an mTOR kinase inhibitor. Examples of mTOR kinase inhibitors include, but are not limited to, CC-115 and CC-223.

In some embodiments, the mTOR inhibitor is selected from the group consisting of sirolimus (rapamycin), BEZ235 (NVP-BEZ235), everolimus (also known as RAD001, Zortress, Certican, and Afinitor), AZD8055, temsirolimus (also known as CCI-779 and Torisel), PI-103, Ku-0063794, INK 128, AZD2014, NVP-BGT226, PF-04691502, CH5132799, GDC-0980 (RG7422), Torin 1, WAY-600, WYE-125132, WYE-687, GSK2126458, PF-05212384 (PKI-587), PP-121, OSI-027, Palomid 529, PP242, XL765, GSK1059615, WYE-354, eforolimus (also known as ridaforolimus or deforolimus), CC-115 and CC-223.

BEZ235 (NVP-BEZ235) is an imidazoquilonine derivative that is an mTORC1 catalytic inhibitor (Roper J, et al. PLoS One, 2011, 6(9), e25132). Everolimus is the 40-O-(2-hydroxyethyl) derivative of rapamycin and binds the cyclophilin FKBP-12, and this complex also mTORC1. AZD8055 is a small molecule that inhibits the phosphorylation of mTORC1 (p70S6K and 4EBP1). Temsirolimus is a small molecule that forms a complex with the FK506-binding protein and prohibits the activation of mTOR when it resides in the mTORC1complex. PI-103 is a small molecule that inhibits the activation of the rapamycin-sensitive (mTORC1) complex (Knight et al. (2006) Cell. 125: 733-47). KU-0063794 is a small molecule that inhibits the phosphorylation of mTORC1 at Ser2448 in a dose-dependent and time-dependent manner. INK 128, AZD2014, NVP-BGT226, CH5132799, WYE-687, and are each small molecule inhibitors of mTORC1. PF-04691502 inhibits mTORC1 activity. GDC-0980 is an orally bioavailable small molecule that inhibits Class I PI3 Kinase and TORC1. Torin 1 is a potent small molecule inhibitor of mTOR. WAY-600 is a potent, ATP-competitive and selective inhibitor of mTOR. WYE-125132 is an ATP-competitive small molecule inhibitor of mTORC1. GSK2126458 is an inhibitor of mTORC1. PKI-587 is a highly potent dual inhibitor of PI3Kα, PI3Kγ and mTOR. PP-121 is a multi-target inhibitor of PDGFR, Hck, mTOR, VEGFR2, Src and Abl. OSI-027 is a selective and potent dual inhibitor of mTORC1 and mTORC2 with IC50 of 22 nM and 65 nM, respectively. Palomid 529 is a small molecule inhibitor of mTORC1 that lacks affinity for ABCB1/ABCG2 and has good brain penetration (Lin et al. (2013) Int J Cancer DOI: 10.1002/ijc.28126 (e-published ahead of print). PP242 is a selective mTOR inhibitor. XL765 is a dual inhibitor of mTOR/PI3k for mTOR, p110α, p110β, p110γ and p110δ. GSK1059615 is a novel and dual inhibitor of PI3Kα, PI3Kβ, PI3Kδ, PI3Ky and mTOR. WYE-354 inhibits mTORC1 in HEK293 cells (0.2 µM-5 µM) and in HUVEC cells (10 nM-1µM). WYE-354 is a potent, specific and ATP-competitive inhibitor of mTOR. Deforolimus (Ridaforolimus, AP23573, MK-8669) is a selective mTOR inhibitor.

### Other Components in the Nanoparticle Compositions

The nanoparticles described herein can be present in a composition that comprises other agents, excipients, or stabilizers. For example, to increase stability by increasing the negative zeta potential of nanoparticles, certain negatively charged components may be added. Such negatively charged components include, but are not limited to bile salts of bile acids consisting of glycocholic acid, cholic acid, chenodeoxycholic acid, taurocholic acid, glycochenodeoxycholic acid, taurochenodeoxycholic acid, litocholic acid, ursodeoxycholic acid, dehydrocholic acid and others; phospholipids including lecithin (egg yolk) based phospholipids which include the following phosphatidylcholines: palmitoyloleoylphosphatidylcholine, palmitoyllinoleoylphosphatidylcholine, stearoyllinoleoylphosphatidylcholine stearoyloleoylphosphatidylcholine, stearoylarachidoylphosphatidylcholine, and dipalmitoylphosphatidylcholine. Other phospholipids including L-α-dimyristoylphosphatidylcholine (DMPC), dioleoylphosphatidylcholine (DOPC), distearyolphosphatidylcholine (DSPC), hydrogenated soy phosphatidylcholine (HSPC), and other related compounds. Negatively charged surfactants or emulsifiers are also suitable as additives, *e.g*., sodium cholesteryl sulfate and the like.

In some embodiments, the composition is suitable for administration to a human. In some embodiments, the composition is suitable for administration to a mammal such as, in the veterinary context, domestic pets and agricultural animals. There are a wide variety of suitable formulations of the nanoparticle composition (see, *e.g.,* U.S. Pat. Nos. 5,916,596 and 6,096,331). The following formulations and methods are merely exemplary and are in no way limiting. Formulations suitable for oral administration can consist of (a) liquid solutions, such as an effective amount of the compound dissolved in diluents, such as water, saline, or orange juice, (b) capsules, sachets or tablets, each containing a predetermined amount of the active ingredient, as solids or granules, (c) suspensions in an appropriate liquid, and (d) suitable emulsions. Tablet forms can include one or more of lactose, mannitol, corn starch, potato starch, microcrystalline cellulose, acacia, gelatin, colloidal silicon dioxide, croscarmellose sodium, talc, magnesium stearate, stearic acid, and other excipients, colorants, diluents, buffering agents, moistening agents, preservatives, flavoring agents, and pharmacologically compatible excipients. Lozenge forms can comprise the active ingredient in a flavor, usually sucrose and acacia or tragacanth, as well as pastilles comprising the active ingredient in an inert base, such as gelatin and glycerin, or sucrose and acacia, emulsions, gels, and the like containing, in addition to the active ingredient, such excipients as are known in the art.

Examples of suitable carriers, excipients, and diluents include, but are not limited to, lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, saline solution, syrup, methylcellulose, methyl- and propylhydroxybenzoates, talc, magnesium stearate, and mineral oil. The formulations can additionally include lubricating agents, wetting agents, emulsifying and suspending agents, preserving agents, sweetening agents or flavoring agents.

Formulations suitable for parenteral administration include aqueous and non-aqueous, isotonic sterile injection solutions, which can contain anti-oxidants, buffers, bacteriostats, and solutes that render the formulation compatible with the blood of the intended recipient, and aqueous and non-aqueous sterile suspensions that can include suspending agents, solubilizers, thickening agents, stabilizers, and preservatives. The formulations can be presented in unit-dose or multi-dose sealed containers, such as ampules and vials, and can be stored in a freeze-dried (lyophilized) condition requiring only the addition of the sterile liquid excipient, for example, water, for injections, immediately prior to use. Extemporaneous injection solutions and suspensions can be prepared from sterile powders, granules, and tablets of the kind previously described. Injectable formulations are preferred.

In some embodiments, the composition is formulated to have a pH range of about 4.5 to about 9.0, including for example pH ranges of any of about 5.0 to about 8.0, about 6.5 to about 7.5, and about 6.5 to about 7.0. In some embodiments, the pH of the composition is formulated to no less than about 6, including for example no less than about any of 6.5, 7, or 8 (such as about 8). The composition can also be made to be isotonic with blood by the addition of a suitable tonicity modifier, such as glycerol.

### Kits, Medicines, and Compositions

The invention also provides kits, medicines, compositions, and unit dosage forms for use in any of the methods described herein.

In some embodiments, there is provided a kit comprising (a) a composition comprising nanoparticles comprising an mTOR inhibitor (such as a limus drug) and an albumin; and (b) an agent for assessing an mTOR-activating aberration. In some embodiments, the mTOR-activating aberration is in an mTOR-associated gene selected from AKT1, FLT3, MTOR, PIK3CA, PIK3CG, TSC1, TSC2, RHEB, STK11, NF1, NF2, PTEN, TP53, FGFR4, KRAS, NRAS, and BAP1. In some embodiments, the mTOR-activating aberration is in an mTOR-associated gene selected from the ONCOPANEL^{™} test. In some embodiments, the agent comprises a nucleic acid specific for the mTOR-associated gene. In some embodiments, the agent comprises an antibody that specifically recognizes a protein encoded by the mTOR-associated gene. In some embodiments, the kit further comprises instructions for use in accordance with any of the methods described herein including methods for treating, assessing responsiveness, monitoring, identifying individuals, and selecting patients for treatment of a hyperplasia (such as cancer, restenosis, or pulmonary hypertension) using the mTOR inhibitor nanoparticle composition based upon the status of the mTOR-activating aberration.

In some embodiments, the kit further comprises an agent for assessing the mutational status of a resistance biomarker, such as TFE3. In some embodiments, the kit further comprises instructions for using the mutational status of the resistance biomarker for selecting individuals for treatment of a hyperplasia (such as cancer, restenosis, or pulmonary hypertension) based on the mutational status of the resistance biomarker alone or in combination with at least one mTOR-activating aberration.

Kits of the invention may include one or more containers comprising the mTOR inhibitor (such as limus drug) nanoparticle compositions (or unit dosage forms and/or articles of manufacture), and one or more containers comprising the agent for assessing the mTOR-activating aberration.

In some embodiments, the kit comprises a second therapeutic agent. The nanoparticle compositions and the second therapeutic agent can be present in separate containers or in a single container. For example, the kit may comprise one distinct composition or two or more compositions wherein one composition comprises nanoparticles and one composition comprises the second therapeutic agent.

The kits of the invention are in suitable packaging. Suitable packaging include, but is not limited to, vials, bottles, jars, flexible packaging (*e.g*., seled Mylar or plastic bags), and the like. Kits may optionally provide additional components such as buffers and interpretative information. The present application thus also provides articles of manufacture, which include vials (such as sealed vials), bottles, jars, flexible packaging, and the like.

The instructions relating to the use of the nanoparticle compositions generally include information as to dosage, dosing schedule, and route of administration for the intended treatment. The containers may be unit doses, bulk packages (*e.g*., multi-dose packages) or subunit doses. For example, kits may be provided that contain sufficient dosages of the mTOR inhibitor (such as a limus drug, *e.g*., sirolimus) as disclosed herein to provide effective treatment of an individual for an extended period, such as any of a week, 8 days, 9 days, 10 days, 11 days, 12 days, 13 days, 2 weeks, 3 weeks, 4 weeks, 6 weeks, 8 weeks, 3 months, 4 months, 5 months, 7 months, 8 months, 9 months, or more. Kits may also include multiple unit doses of the mTOR inhibitor (such as a limus drug) and pharmaceutical compositions and instructions for use and packaged in quantities sufficient for storage and use in pharmacies, for example, hospital pharmacies and compounding pharmacies.

Also provided are medicines, compositions, and unit dosage forms useful for the methods described herein. In some embodiments, there is provided a medicine (or composition) for use in treating a hyperplasia (such as cancer, pulmonary hypertension, or restenosis) comprising nanoparticles comprising an mTOR inhibitor (such as a limus drug) and an albumin (such as human serum albumin).

Those skilled in the art will recognize that several embodiments are possible within the scope and spirit of this invention. The invention will now be described in greater detail by reference to the following non-limiting examples. The following examples further illustrate the invention but, of course, should not be construed as in any way limiting its scope.

### EXAMPLES

The invention can be further understood by reference to the following examples, which are provided by way of illustration and are not meant to be limiting.

### Example 1: Clinical pilot study of Nab-sirolimus in mTOR pathway aberrant malignancies

A single-arm phase II clinical trial is designed to assess the efficacy of*Nab*-sirolimus (also referred to as ABI-009) in patients with relevant mTOR pathway aberrations, particularly those with gene alterations that would confer sensitivity to mTOR inhibitors. The gene alterations are identified through clinical next-generation sequencing experiments. The primary goal of the study is to assess the response rate of *Nab*-sirolimus in advanced cancers with mTOR-activating aberrations. The secondary goals are (1) to estimate time to progression and overall survival of the selected patients; and (2) to estimate adverse events profile of *Nab-*sirolimus in the selected patients. Additionally, correlative research is performed to assess the rate of individual mTOR-activating aberrations and assess the association between the individual mTOR-activating aberrations and clinical outcome both across disease indications and within disease indications.

A single group of individuals are enrolled in the clinical study. Prior to registration, individuals are assessed in a CLIA certified lab for mTOR-activating aberrations in at least one mTOR-associated gene selected from AKT1, FLT3, MTOR, PIK3CA, PIK3CG, TSC1, TSC2, RHEB, STK11, NF1, NF2, PTEN, TP53, FGFR4, KRAS, NRAS, and BAP1, for example, in the primary tumor. Individuals having at least one mTOR-activating aberration and meeting all inclusion criteria are selected for the treatment. An archival paraffin embedded (PPFE) tissue sample from the primary tumor is obtained from each individual. The selected individuals are administered *Nab*-sirolimus intravenously at a dosage of 75 mg/m² on days 1, 8, and 15 of a 28-day cycle, or about 100 mg/m² on days 1, and 8 of a 21-day cycle. The *Nab*-sirolimus is infused over about 30 minutes during each administration. The individuals continue to receive *Nab-*sirolimus treatment and are actively monitored until the occurrence of disease progression and/or unacceptable adverse events, or until the individual refuses to receive the treatment. If multiple adverse events are observed, the dose of *Nab*-sirolimus may be interrupted or reduced to allow management of drug-related toxicities. For example, the dose of *Nab*-sirolimus may be first reduced to 56 mg/m² IV on days 1, 8, and 15 of a 28-day cycle, and then for a second time reduced to 45 mg/m² IV on days 1, 8, and 15 of a 28-day cycle. Only two dose reductions are allowed per individual. Ancillary treatments, such as antiemetics, growth factors (G-CSF), bisphosphonates or denosumab for pre-existing, painful bone metastases, blood and blood products, warfarin or LMWH, and/or loperamide for diarrhea may be permitted at physician's discretion. The individuals must return to the consenting institution for treatment and evaluation at least every 28 days (or every about 25 to about 31 days) during the treatment.

Various biological samples are collected from each individual during the course of the study (*e.g.,* before treatment, on-treatment, and post-treatment), and the biological samples are used to assess the mutational status and level of relevant biomarkers. On-treatment biological samples may be collected from the individual, for example, on Day 1 of cycle 1, Day 1 (± 3 days) of cycle 2, and Day 1 (± 3 days) of Cycle 3 and then every 2 cycles afterward. A blood sample is collected from each individual before and after the treatment. A cell-free plasma DNA sample is prepared from each blood sample for assessment of circulating DNA. The cell-free plasma DNA samples are analyzed using next-generation sequencing methods to assess the prevalence of the mTOR-activating aberrations (such as mutations) identified in the primary tumor sample over time as a measure of response to the treatment. Additionally, fresh or archival (such as PPFE) tumor biopsy samples are collected from each individual before the treatment, and optionally during the course of the treatment (i.e. on-treatment). The on-treatment tumor biopsy samples are used to assess pharmacodynamics effects of *Nab*-sirolimus in the individuals. Post-treatment tumor biopsy samples are collected from each individual at the time of disease progression after response to the treatment to assess mechanisms of resistance, including secondary mutations, genomic amplifications, or gene deletion events. Exome sequencing experiments using the ONCOPANEL^{™} test (CLIA certified) of approximately 300 genes are performed to assess mutations in mTOR pathway genes, including, but not limited to, PIK3CA, TSC1, TSC2, AKT, PTEN, MTOR, and RHEB. Additionally, mTOR-activating aberrations (such as sequences and levels of biomarkers, including, but not limited to, AKT1, FLT3, MTOR, PIK3CA, PIK3CG, TSC1, TSC2, RHEB, STK11, NF1, NF2, PTEN, TP53, FGFR4, KRAS, NRAS, and BAP1), and level of phosphorylated AKT (i.e. p-AKT), 4EBP1 (i.e. p-4EBP1), S6K (i.e. p-S6K), S6 (i.e. p-S6), and SPARC (i.e. p-SPARC) are evaluated using the tumor biopsy samples. Proliferation markers (such as Ki-67) and apoptosis markers (such as PARP) may be assessed using immunohistochemistry methods. FISH (fluorescence in-situ hybridization) analysis of translocations in TFE3 is performed. The assessment results are used to evaluate correlation of the mTOR-activating aberrations to clinical response to the treatment, and to test the correlation between mTOR-activating aberrations identified in tumor biopsy samples and circulating DNA.

The primary endpoint of this study is the proportion of confirmed responses. In solid tumors, a confirmed response is defined to be either a CR or PR noted as the objective status on two consecutive evaluations at least 8 weeks apart. For lymphoma, response is assessed using International Workshop Response Criteria (Cheson et al 1999). Confirmed response will be evaluated using all cycles of treatment. An exact binomial confidence interval for the true confirmed response proportion is calculated. Secondary endpoints of this study include survival time, time to disease progression, and adverse events. The distribution of survival time and the distribution of time to disease progression are estimated using the method of Kaplan-Meier. For all primary and secondary endpoints, statistical analysis is carried out for the overall patient population and within each disease group.

Correlative research is performed to determine association of the treatment with quality of life and individual mTOR-activating aberrations, both for the overall group of patients and within each disease group. Quality of life is assessed prior to review of treatment response and discussions of patient's general health since last treatment evaluation. Quality of life is measured using the EORTC QLQ-C30, a 30-item patient-report questionnaire about patient ability to function, symptoms related to the cancer and its treatment, overall health and quality of life, and perceived financial impact of the cancer and its treatment. Scale score trajectories of the quality of life over time are examined using stream plots and mean plots with standard deviation error bars. Changes from baseline at each cycle is statistically tested using paired t-tests, and standardized response means is interpreted after applying Middel's (2002) adjustment using Cohen's (1988) cutoffs: <0.20=trivial; 0.20-<0.50=small; 0.5-<0.8=moderate; and ≥0.8=large. Rate of individual mTOR-activating aberrations is described, and association with confirmed response is investigated using a Fisher's exact test. Associations with time to progression and overall survival are investigated using log-rank tests. One-sided p-values ≤ 0.10 are considered statistically significant throughout.

Eligible individuals must meet all of the following inclusion criteria: (a) have histological confirmation of pancreatic neuroendocrine cancer, endometrial cancer, ovarian cancer, breast cancer, renal cell carcinoma, LAM, prostate cancer, lymphoma, or bladder cancer; (b) have advanced stage cancer; (c) have at least one mTOR pathway aberration confirmed in a CLIA certified lab, and the mTOR pathway aberration may include, but is not limited to, genetic aberrations in AKT1, FLT3, MTOR, PIK3CA, PIK3CG, TSC1, TSC2, RHEB, STK11, NF1, NF2, PTEN (*e.g*. PTEN deletion), TP53, FGFR4, KRAS, NRAS and BAP1; (d) have none of the following treatments: (1) chemotherapy within 4 weeks before treatment with *Nab*-sirolimus; (2) hormonal therapy within 4 weeks before treatment with *Nab*-sirolimus; (3) radiotherapy within 4 weeks before treatment with *Nab*-sirolimus; (4) treatment with nitrosoureas, mitomycin, or extensive radiotherapy within 6 weeks before treatment with *Nab*-sirolimus; (5) immunosuppressive agents within 3 weeks before treatment with *Nab*-sirolimus (except corticosteroids used as antiemetics); (6) use of prior mTOR pathway inhibitor therapy; (d) have the following laboratory values obtained no more than 14 days prior to registration: (1) absolute neutrophil count (ANC) ≥1500/mm³, platelet count ≥ 100,000/mm³ (≥ 75,000/mm³ for patients diagnosed with lymphoma); (2) Hemoglobin ≥ 9.0 g/dL; (3) Total bilirubin ≤ 1.5 x institutional upper limit of normal (ULN); (4) Aspartate transaminase (AST); Alanine Aminotransferase (ALT) ≤ 3 x ULN, or ≤ 5 X ULN if subject has tumor involvement in the liver; (5) Serum cholesterol ≤ 350 mg/dL; (6) Serum triglyceride ≤ 300 mg/dL; (7) Serum creatinine ≤ 1.5 x ULN; (e) have previously failed, unable to tolerate, or refused other available active therapies; (f) have adequate coagulation function as defined by either of the following criteria: (1) INR ≤ 1.5 X ULN; (2) For subjects receiving warfarin or LMWH, the subjects must, in the investigator's opinion, be clinically stable with no evidence of active bleeding while receiving anticoagulant therapy.

Exclusion criteria are: (a) pregnant or nursing women, or women of child-bearing potential, who are biologically able to conceive, or men who are able to father a child, not employing two forms of highly effective contraception; (b) patients with a history of interstitial lung disease and/or pneumonia; (c) receiving any concomitant antitumor therapy or inhibitors of CYP3A4; (d) history of allergic reactions attributed to compounds of similar chemical or biologic composition including macrolide (*e.g*. azithromycin, clarithromycin, dirithromycin, and erythromycin) and ketolide antibiotics; (e) major surgery (*e.g*., intra-thoracic, intra-abdominal or intra-pelvic) ≤ 4 weeks prior to registration or failure to recover from side effects of such surgery with the exceptions of port placements, nephrectomy, tumor biopsies, and minor surgeries; (f) concurrent use of any other approved or investigational anticancer agents which would be considered as a treatment for the primary neoplasm; (g) uncontrolled diabetes mellitus as defined by HbA1c > 8% despite adequate therapy; (h) unstable coronary artery disease or myocardial infarction during preceding 6 months; and (i) hypertension uncontrolled by medication.

### Example 2: Evaluation of drugs in combination with Nab-sirolimus for anti-tumor activity in a UMUC3 (human bladder cancer) cell line mouse xenograft model

The anti-tumor efficacy of a panel of drugs, including mitomycin C, cisplatin, gemcitabine, valrubicin, and docetaxel, in combination with *Nab*-sirolimus (such as ABI-009) were evaluated and compared in a UMUC3 cell xenograft model in athymic nude mice.

The human bladder cancer (adenocarcinoma) cell line UMUC3 was prepared as follows. A frozen (liquid nitrogen) aliquot of the UMUC3 cell line (obtained from ATCC) was thawed out, dispersed into a 75 cm² flask containing DMEM media supplemented with 10% fetal bovine calf serum (FBS) and incubated at 37^{O}C in humidified atmosphere of 5% CO₂. As cells became 80% confluent, the cultures were expanded to 150 cm² flasks. The cultures were further expanded until sufficient cells were available for injection into mice (10 x 10⁶ cells per mouse).

Tumors were established from the UMUC3 cells as follows. Female athymic nude mice were obtained and housed in filter-topped cages supplied with autoclaved bedding. Animal handling procedures were under laminar flow hood. Each mouse was ear tagged for individual identification, and the body weight of each mouse was recorded. UMUC3 cells (10 x 10⁶ cells per flank in 0.1 mL PBS with 20% Matrigel) were injected subcutaneously into the right flank of each mouse to implant the tumor. Tumor measurements were recorded three times per week (such as on Mondays, Wednesdays and Fridays) until tumors became approximately 60 to 160 mm³ total. The mice were randomized for treatment once the average tumor volumes reached approximately 100 mm³. Prior to the treatment, body weights and tumor measurements of all mice were recorded.

In the Part A single-agent dose finding study, athymic mice bearing UMUC3 human bladder cancer xenografts were treated for 3 weeks with single agent rapamycin (3 mg/kg, qdx5, oral), everolimus (3 mg/kg, qdx5, oral), ABI-009 (7.5, 20, and 40 mg/kg, twice weekly, IV via tail vein), mitomycin C (0.5 mg/kg, twice weekly, IP), cisplatin (1.5 mg/kg, twice weekly, IP), gemcitabine (12.5 mg/kg, twice weekly, IP), valrubicin (20 mg/kg, twice weekly, IP), and docetaxel (2 mg/kg, twice weekly, IP). Animals were monitored for tumor volume and body weight.

In the Part B combination study, athymic mice bearing UMUC3 human bladder cancer xenografts were treated until study end with ABI-009 (3 mg/kg, twice weekly, IV via tail vein), mitomycin C (0.5 mg/kg, twice weekly, IP), cisplatin (3 mg/kg, twice weekly, IP), gemcitabine (30 mg/kg, twice weekly, IP), valrubicin (20 mg/kg, twice weekly, IP), and docetaxel (3 mg/kg, twice weekly, IP) either as single agent or in combination (ABI-009 plus chemotherapeutic agent). Animals were monitored for tumor volume and body weight.

In each combination of drugs being administered comprising *Nab*-sirolimus and a second drug (such as MMC, Cis, GEM, Val, and Doc), the second drug was administered immediately before *Nab*-sirolimus. The mice were monitored during the course of the treatment by recording body weights three times per week (such as on Mondays, Wednesdays and Fridays), recording signs of distress daily, and recording tumor measurements three times per week (such as on Mondays, Wednesdays and Fridays). Measurements of tumor sizes and body weights were continued for 2 weeks following completion of the dosing regimen, or until the mouse was sacrificed when the tumor size of the mouse is more than 2000 mm³.

### Results

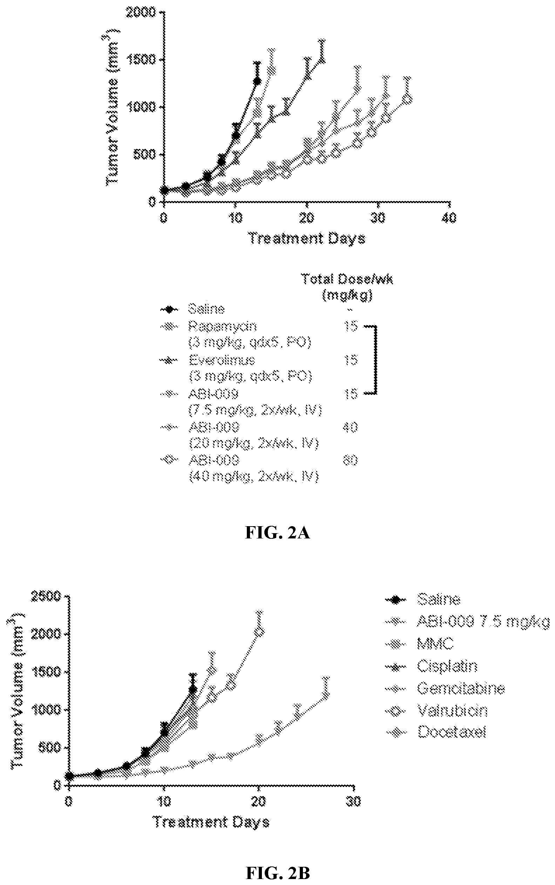

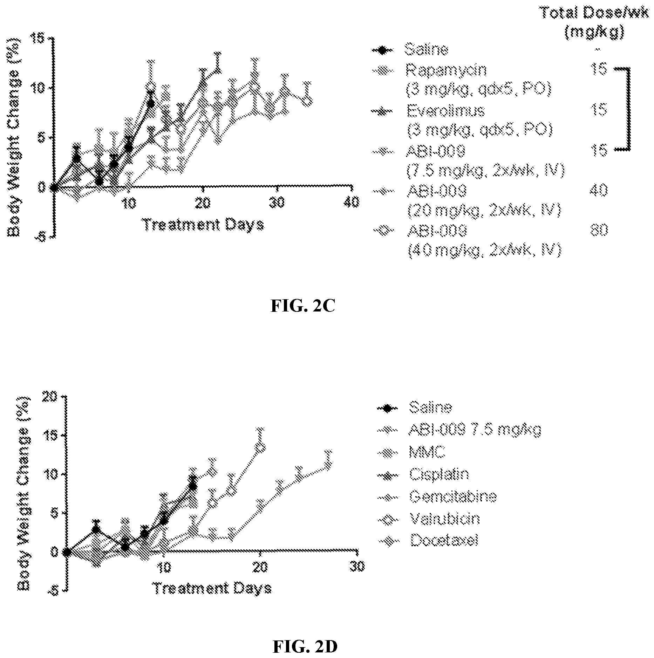

In the single-agent dose-finding stage (Part A) of the nonclinical study, all treatments were well tolerated, with no significant body weight loss in any group. No statistically significant difference in body weight was observed in any treatment group compared to saline control. All groups gained weight in the study duration (FIGs. 2C, 2D).

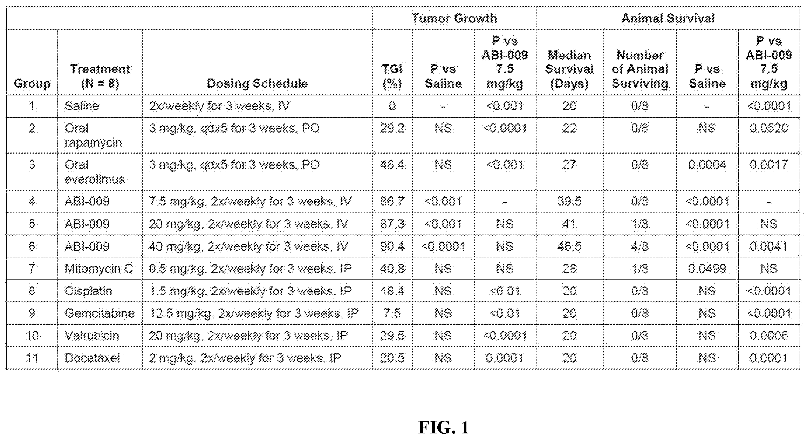

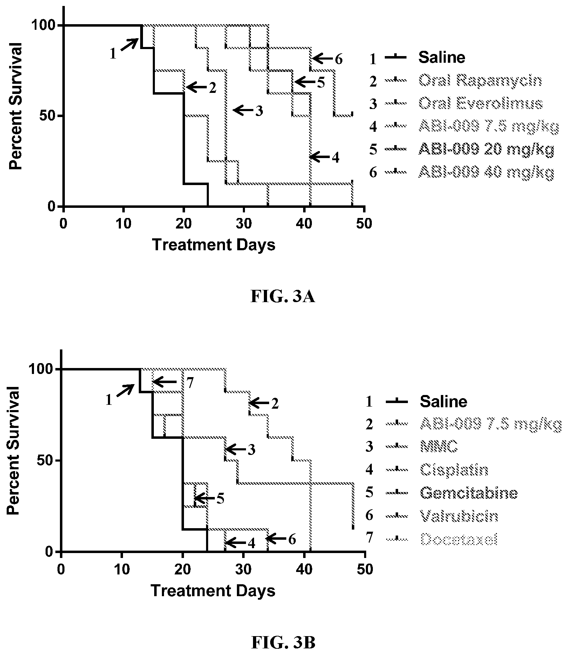

Only ABI-009 treated groups showed significant tumor growth inhibition compared with saline control, and antitumor activity of ABI-009 increased with higher doses (FIGs. 1 and 2A-2B). Further, ABI-009 at 7.5 mg/kg twice weekly IV demonstrated significantly greater antitumor activity compared with equal weekly dosing of oral rapamycin and oral everolimus (P < 0.001 and P < 0.0001, respectively) (FIG. 1 and FIG. 2A). Correspondingly, ABI-009 treated groups showed prolonged survival as demonstrated by longer median survival compared with oral rapamycin, oral everolimus, and other chemotherapy groups (FIG. 1 and FIG. 3A). Proper dose for other chemotherapeutic agents to use in the Part B combination study were identified (FIGs. 1, 2A-2B, and 3A-3B).

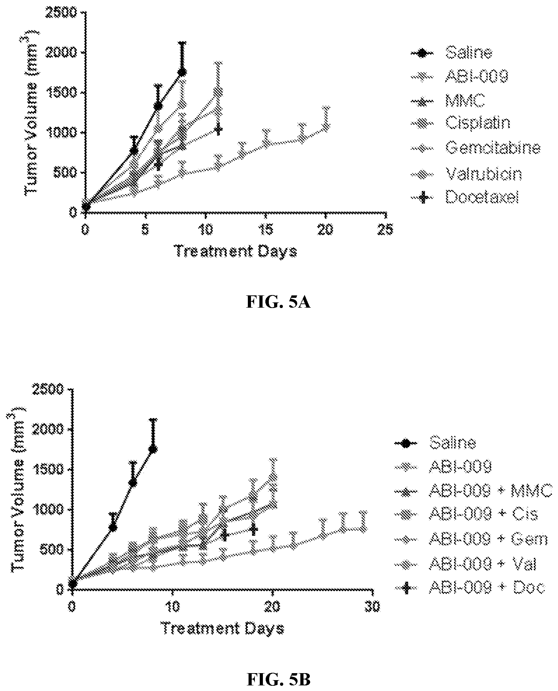

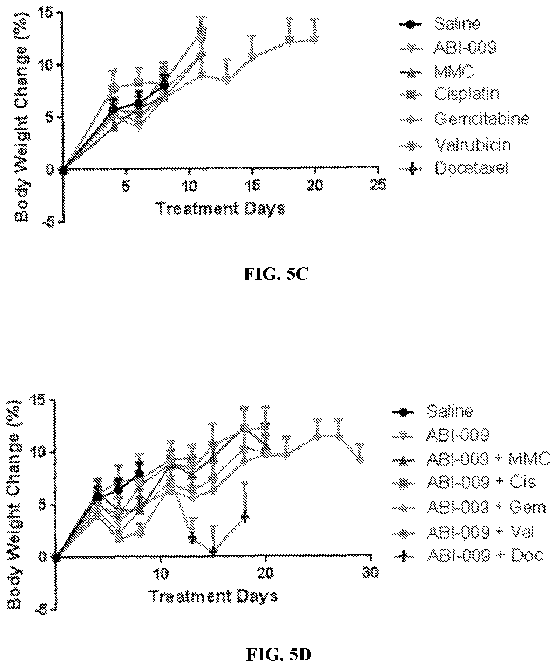

For the combination treatment stage (Part B), ABI-009 as a single agent or in combination with other chemotherapeutic agents were overall well tolerated, with no significant body weight loss in any group. All groups gained weight in the study duration (FIGs. 5C and 5D).

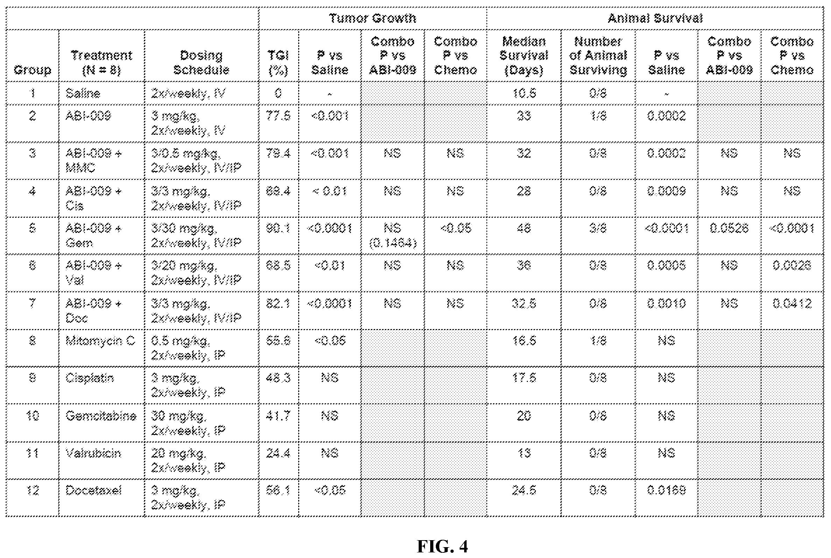

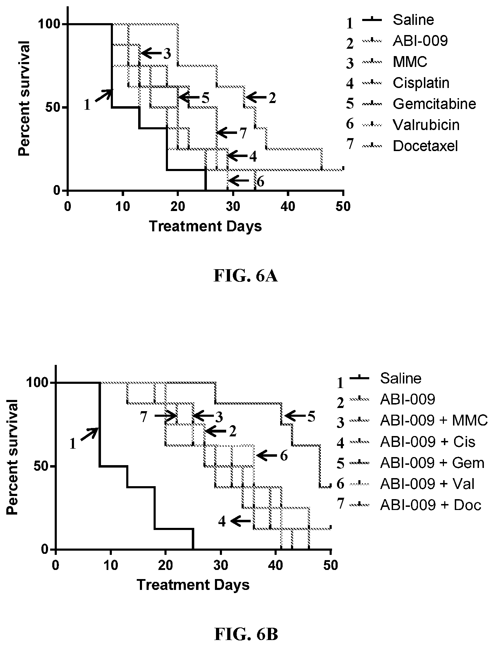

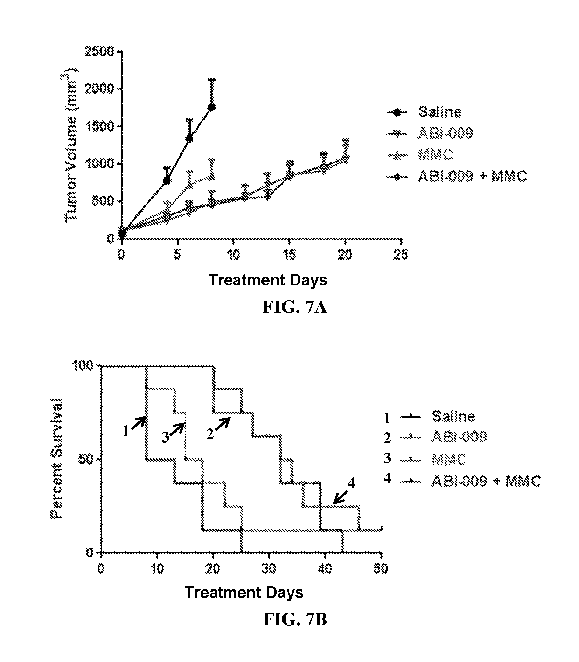

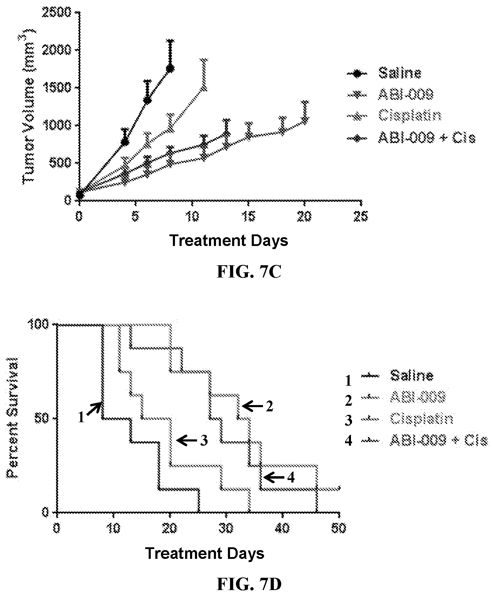

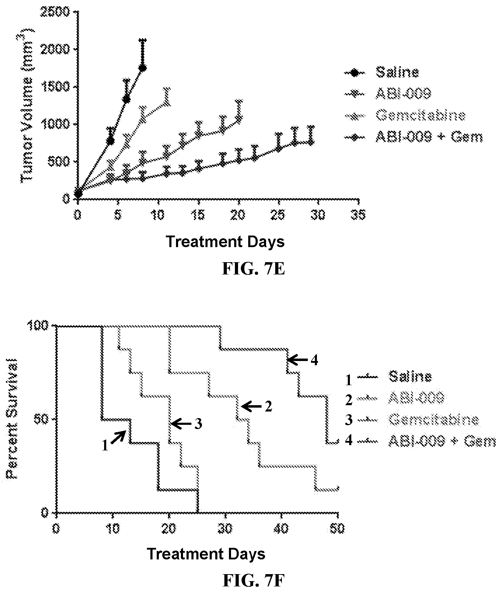

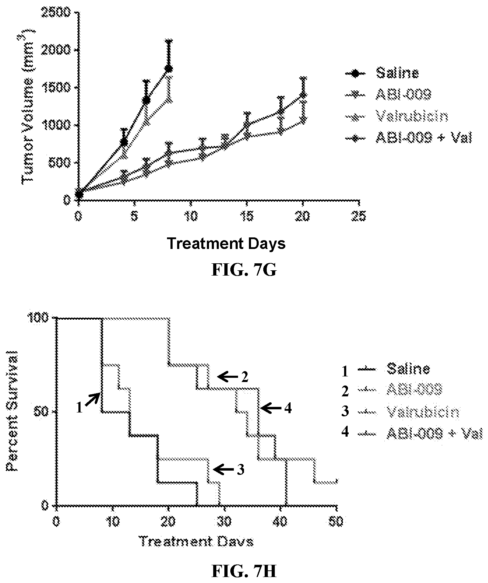

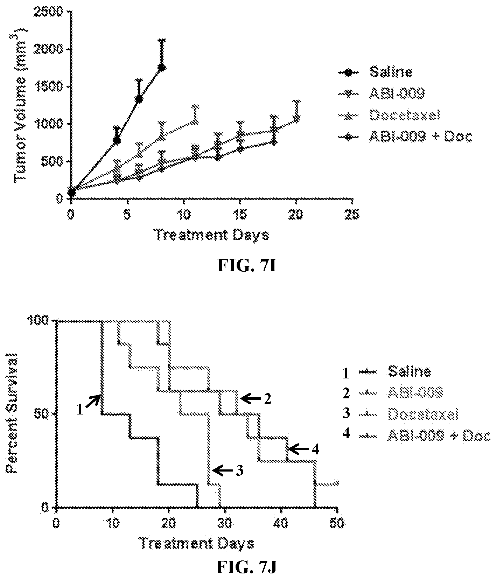

ABI-009 as a single agent or in combination with chemotherapeutic agents currently in clinical use to treat NMIBC demonstrated significant antitumor activity compared with saline control as well as significantly prolonged animal survival (FIGs. 4, 5A-5B, 6A-B, 7A-7J). Single agent gemcitabine showed only modest effects in tumor growth inhibition and animal survival, none of which were significantly improved over control.

Although ABI-009 dose in Part B was substantially reduced (-60%) from that in Part A (3 mg/kg vs 7.5 mg/kg), ABI-009 as a single agent still displayed robust antitumor activity (TGI: 77.5%). As a result, none of the ABI-009 combination groups showed significant improvement of antitumor activity when compared with ABI-009 alone, however ABI-009/gemcitabine combination showed a numerical trend of enhanced antitumor activity versus ABI-009 alone (TGI: 90.1% vs 77.5%) (FIGs. 4, 5B, 7A, 7C, 7E, 7G, and 7I). Importantly, out of all combinations, only ABI-009/gemcitabine demonstrated substantially longer survival compared with ABI-009 alone (median survival: 48 vs 33 days, P = 0.0526, Log-rank test), with more animals surviving till the study end (Treatment Day 50: 3/8 vs 1/8) (FIGs. 4, 6B, 7B, 7D, 7F, 7H, and 7J).

On the other hand, when ABI-009 combination groups were compared with the corresponding chemotherapeutic agents alone, only ABI-009/gemcitabine combination showed a significant improvement of antitumor activity (TGI: 90.1% vs 41.7% for single agent gemcitabine, P < 0.05) (FIGs. 4, 5B, and 7E). Correspondingly, ABI-009/gemcitabine combination also demonstrated a significantly longer animal survival over gemcitabine alone (median survival: 48 vs 20 days, P ≤ 0.0001, Log-rank test) (FIGs. 4, 6B, and 7F).

### Conclusion

In conclusion, ABI-009 administered IV as a single agent or in combination with other chemotherapies were well tolerated with no significant body weight loss. ABI-009 administered IV demonstrated significantly greater antitumor activity and prolonged survival compared with equal weekly dosing of oral rapamycin and oral everolimus. The combination study demonstrated that ABI-009/gemcitabine combination was the best among all combination options tested in the UMUC3 bladder cancer xenograft model, with better antitumor activity than either ABI-009 or gemcitabine as a single agent. Importantly, animal survival in the ABI-009/gemcitabine group was prolonged compared with either ABI-009 or gemcitabine as a single agent.

### Example 3: Phase I/II clinical studies of Nab-sirolimus in NMIBC

Patients with BCG-refractory or recurrent non-muscle invasive bladder cancer (NMIBC) are enrolled in a phase I/II clinical study to assess the safety, pharmacokinetics (PK), pharmacodynamics, and efficacy of intravesical *Nab*-sirolimus (also referred to as ABI-009), as a single agent or in combination with other chemotherapy agents.

Patients receive intravesical *Nab*-sirolimus by sterile urethral catheterization following resection of visible tumors during cystoscopy. In the phase 1 study, up to 30 patients are enrolled in 5 cohorts for 6 weeks of treatment (up to 6 patients per cohort): 100 mg/week, 100 mg 2x/week (total weekly dose 200 mg), 300 mg/week, 200 mg 2x/week (total weekly dose 400 mg), and 400 mg/week. For each treatment, *Nab*-sirolimus are reconstituted with 100 ml 0.9% sodium chloride. Patients are instructed to keep the drug in the bladder for 2 hours before voiding. If a National Cancer Institute Common Toxicity Criteria (NCI CTC) v4.0 Grade 2 local toxicity develops, treatment are delayed for 1 dose and resume if the toxicity resolves to Grade 1 or less. A dose-limiting toxicity (DLT) is considered to be any Grade 3 or 4 event, and a patient experiencing a DLT is immediately removed from the trial. Dose escalation follows the 3+3 rule to establish the maximum delivery dose (MDD). Six weeks after the last weekly or 2x weekly dose, patients undergo a cystoscopy and biopsy. Per standard criteria in NMIBC, a complete response (CR) is defined as a cancer-negative biopsy at the 6-week post-treatment cystoscopy.

If a patient has a CR, the patient receives additional monthly maintenance instillations at the maximum dose that particular patient received. Cystoscopic examinations are performed every 3 months, and the patient receives therapy until disease progression for a maximum of 1 year from the start of therapy. Systemic and local bladder toxicities are monitored throughout treatment and maintenance therapy.

The phase II study is initiated if no unacceptable toxicities are detected for intravesical *Nab*-sirolimus in the initial phase 1 portion of the study to determine efficacy and obtain additional safety data in patients with BCG-refractory or recurrent NMIBC. The primary endpoint is to evaluate the response rate of *Nab*-sirolimus in the treatment of BCG-refractory NMIBC. The secondary endpoints are to further evaluate the safety of *Nab*-sirolimus per NCI criteria as well as to assess molecular correlates for response to therapy. The results from biomarker analysis, including, but not limited to, p-S6K, p-S6, p-AKT, p-4EBP1, Ki67, mTOR-activating aberrations, and a panel of more than 300 genes and intron regions in the ONCOPNEL^{™} test, could establish the usefulness of these biomarkers in treatment selection for NMIBC patients, as well as surrogate indicators of clinical efficacy for Nab-sirolimus treatment. The approach to phase 3 clinical studies is to conduct controlled, randomized comparative safety and efficacy studies of *Nab*-sirolimus versus the approved standard of care for the target disease (i.e. NMIBC).

Combination regimens of *Nab*-sirolimus with chemotherapeutic agents currently used for intravesical treatment of NMIBC, including mitomycin C, cisplatin, gemcitabine, valrubicin, and docetaxel may be further evaluated in a phase II intravesical *Nab*-sirolimus clinical study as described above, if any of the combination regimens examined in Example 2 is found to be safe and significantly improve antitumor activity over *Nab*-sirolimus as a single agent in a UMUC3 human bladder cancer xenograft mouse model.

### Example 4: Phase II clinical study of Nab-sirolimus in peripheral arterial disease

A prospective, multicenter, 2-stage phase II clinical study is conducted to investigate the safety and effectiveness of adventitial delivery of *Nab*-sirolimus (also referred to as ABI-009) to improve outcomes of femoropopliteal revascularization after balloon angioplasty and provisional stenting of the popliteal and contiguous peripheral arteries.

Male or female patients at least 18 years of age are enrolled in the study, if the patients present with a de novo atherosclerotic lesion > 70% in the popliteal artery, allowing lesion extension into contiguous arteries, that totals up to 15 cm in length, and with a reference vessel diameter of 3 to 8 mm. *Nab*-sirolimus is administered to the adventitia in a dose of 40 to 100 µg/cm of desired vessel treatment length using a BULLFROG^{®} micro-infusion catheter.

The study is conducted in 2 stages: Stage A: open label dose escalation stage with 10 patients, 2 doses 40 µg/cm and 100 µg/cm; and Stage B: 100 patients blinded and randomized 1:1 to receive either the highest safe *Nab*-sirolimus dose established from Stage A or no treatment.

Primary endpoints include acute safety outcomes of Major Adverse Limb Events or Peri-Operative Death (MALE + POD) within 30 days from the procedure and effectiveness outcomes by evaluating duplex ultrasound index lesion binary restenosis (PSVR > 2.4) at 6 and 12 months. Secondary endpoints include long term safety, duplex ultrasound index lesion binary restenosis (PSVR > 4.0) or occlusion at 6 and 12 months, inflammatory biomarkers, target lesion revascularization (TLR) rate, target extremity revascularization (TLR) rate, target extremity revascularization (TER) rate, infusion technical success, procedural success, and healthcare economics.

Biological samples, such as blood sample and tissue biopsy samples may be obtained from patients during the course of the study, which are analyzed to establish biomarkers (such as mTOR-activating aberrations) in treatment selection for PAH patients, as well as surrogate indicators of clinical efficacy for *Nab*-sirolimus treatment.

### Example 5: Phase II clinical study of Nab-sirolimus in PAH

A combined phase 1/2 study is conducted to evaluate the safety and efficacy of *Nab-*sirolimus (also referred to as ABI-009) in patients with severe progressive pulmonary arterial hypertension (PAH) on maximal currently available background therapy.

In the phase 1 portion of the study, 3 dose levels of *Nab*-sirolimus (20 mg/m², 45 mg/m², and 75 mg/m²) are tested in cohorts of 3 patients each to evaluate the safe dose and DLTs of *Nab*-sirolimus in patients with severe PAH. After finding the most optimal dose in the clinical phase I study, the clinical phase II study further explores the safety and efficacy of *Nab-*sirolimus in patients with severe progressive PAH.

Eligible patients have severe PAH (New York Heart Association [NYHA] class III or IV), PVR >5 Woods Units despite best available therapy with at least two drugs including an oral agent, either an endothelin receptor antagonist and/or phosphodiesterase type 5 inhibitor, and/or a prostacyclin analogue (unless unwilling or unable to tolerate).

The exploratory primary efficacy endpoint is the change in PVR after 16 weeks of treatment. This endpoint has been used successfully in all prior open-label proof-of-concept trials in PAH patients as PVR does not improve in the absence of specific therapy. Secondary efficacy endpoints at 16 weeks include additional hemodynamic parameters (cardiac output, pulmonary artery pressures, pulmonary artery occlusion pressure, and central venous pressure), 6-minute walk distance test, change in NYHA functional class, Doppler-echocardiographic imaging to assess right ventricular function, CT-PET to determine the glycolytic activity of the right ventricle and pulmonary vasculature, as well as measurement of brain naturetic peptide and troponin levels, indicative of right ventricular strain. Patients are allowed to remain on treatment up to 24 weeks if there is measured clinical benefit observed after 16 weeks.

Assessments in open label follow-up are determined as clinically indicated. Safety assessments for all patients are conducted. Single point pharmacokinetics are determined weekly prior to the next dose for the first 3 weeks of treatment to determine a basal or trough level. Biological samples, such as blood sample and tissue biopsy samples may be obtained from patients during the course of the study, which are analyzed to establish biomarkers (such as mTOR-activating aberrations) in treatment selection for PAH patients, as well as surrogate indicators of clinical efficacy for *Nab*-sirolimus treatment.

### Example 6: Phase I clinical study of ABI-009 (Nab-sirolimus) in Pediatric Patients with Recurrent or Refractory Solid Tumors, including CNS Tumors as a Single Agent and in Combination with Temozolomide and Irinotecan

A single-arm non- randomized Phase 1 dose escalation study is designed to determine the toxicity profile, maximum tolerated dose, recommended Phase 2 dose, as well as pharmacokinetic and pharmacodynamic parameters of ABI-009 as single agent or in combination with temozolomide and irinotecan for treating recurrent or refractory solid tumors, including central nervous system (CNS) tumors, in pediatric patients. Efficacy of AB-009 in combination with irinotecan and temozolomide in treating solid tumors of the pediatric patients is assessed within the confines of the Phase 1 study. Furthermore, expression of biomarkers, such as S6K1 and 4EBP1 are determined in patients before the treatment. Exemplary solid tumors to be investigated include neuroblastoma (NB), osteosarcoma (OS), Ewing's sarcoma (EWS), rhabdomyosarcoma (RMS), meduloblastoma (MB), gliomas, renal tumors, and hepatic tumors (such as hepatoblastoma and hepatocellular carcinoma).

Primary objectives of the clinical study include: 1) to estimate the maximum tolerated dose (MTD) and/or recommended Phase 2 dose (RP2D) of ABI-009 administered as an intravenous dose over 30 minutes on Days 1 and 8 of a 21-day cycle, in combination with temozolomide and irinotecan (administered on Days 1-5) to pediatric patients with recurrent/ refractory solid tumors, including CNS tumors; 2) to define and describe the toxicities of single agent ABI-009 administered as an intravenous dose over 30 minutes on Days 1 and 8 of a 21-day cycle in pediatric patients with recurrent or refractory cancer; 3) to define and describe the toxicities of ABI-009 administered as an intravenous dose over 30 minutes on Days 1 and 8 of a 21-day cycle in combination with temozolomide and irinotecan (administered on Days 1-5) in pediatric patients with recurrent or refractory cancer; and 4) to characterize the pharmacokinetics of ABI-009 in pediatric patients with recurrent or refractory cancer. Secondary objective of the study is to preliminarily define the antitumor activity of ABI-009 in combination with temozolomide and irinotecan within the confines of a Phase 1 study. Exploratory objective of the study is to examine S6K1 and 4EBP1 expression status in archival tumor tissue from solid tumor pediatric patients using immunohistochemistry.

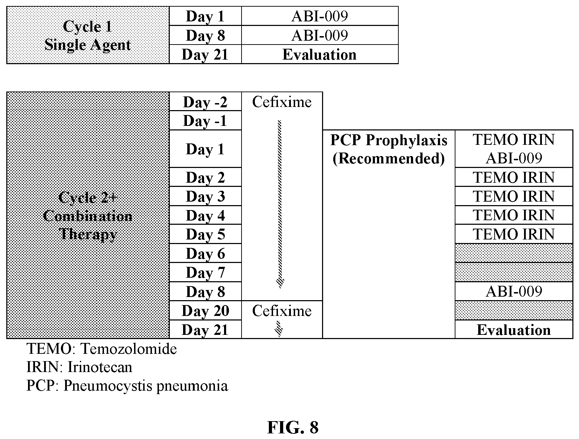

FIG. 8 shows the experimental design schema. ABI-009 is given intravenously over 30 minutes on Days 1 and 8 of each 21-day cycle. During Cycle 2+, ABI-009 is given 1 hour after irinotecan administration during Cycle 2. For subsequent cycles, ABI-009 is given within 8 hours after temozolomide and irinotecan. Temozolomide is administered orally, once daily on Days 1-5 of each 21-day cycle from Cycle 2+. Irinotecan is administered orally, once daily on Days 1-5 one hour after temozolomide of each 21-day cycle from Cycle 2+. Cefixime or an equivalent antibiotic is used as diarrheal prophylaxis and administered 2 days prior to the first dose of irinotecan, during irinotecan administration, and 3 days after the last does of irinotecan of each cycle. A cycle of therapy is considered 21 days. A cycle may be repeated for a total of 35 cycles, up to a total duration of therapy of approximately 24 months.

The dose escalation schema is shown in Table 1 below. Dose level 1 is the starting dose level, which is determined based on the recommended Phase 2 dose of ABI-009, irinotecan and temozolomide in previous clinical studies. If the MTD has been exceeded at Dose Level 1, then the subsequent cohort of patients will be treated at Dose Level -1. If Dose Level -1 is not well tolerated the study will be closed to accrual.

**Table 1. Dosing schema.**

| Dose Level | Cycle 1 | Cycle 2+ | | |
|---|---|---|---|---|
| | ABI-009 (mg/m²) | ABI-009 (mg/m²) | Irinotecan (mg/m²) | Temozolomide (mg/m²) |
| -1 | 20 | 20 | 90 | 125 |
| 1 | 35 | 35 | 90 | 125 |
| 2 | 45 | 45 | 90 | 125 |
| 3 | 55 | 55 | 90 | 125 |

The rolling six design is utilized for dose escalation and patient accrual. *See,* for example, Skolnik JM, Barrett JS, Jayaraman B, et al: "Shortening the timeline of pediatric phase I trials: the rolling six design." J Clin Oncol 26:190-5, 2008. Briefly, two to six patients can be concurrently enrolled onto a dose level, dependent upon (1) the number of patients enrolled at the current dose level, (2) the number of patients who have experienced dose-limiting toxicity (DLT) at the current dose level, and (3) the number of patients entered but with tolerability data pending at the current dose level. For example, when three participants are enrolled onto a dose cohort, if toxicity data is available for all three when the fourth participant entered and there are no DLTs, the dose is escalated and the fourth participant is enrolled to the subsequent dose level. If data is not yet available for one or more of the first three participants and no DLT has been observed, or if one DLT has been observed, the new participant is entered at the same dose level. Lastly, if two or more DLTs have been observed, the dose level is de-escalated. This process is repeated for participants five and six. In place of suspending accrual after every three participants, accrual is only suspended when a cohort of six is filled. When participants are inevaluable for toxicity, they are replaced with the next available participant if escalation or de-escalation rules have not been fulfilled at the time the next available participant is enrolled onto the study.

If two or more of a cohort of up to six patients experience DLT at a given dose level, then the MTD has been exceeded and dose escalation will be stopped. In the unlikely event that two DLTs observed out of 6 evaluable patients are of different classes of Adverse Effects (*e.g.,* hepatotoxicity and myelosuppression), expansion of the cohort to 12 patients will be considered (if one of the DLTs does not appear to be dose-related, the Adverse Effects are readily reversible, AND study chair/DVL leadership/IND sponsor all agree that expansion of the cohort is acceptable). Once the MTD or RP2D has been defined, up to 6 additional patients with relapsed/refractory solid tumors may be enrolled to acquire PK data in a representative number of young patients (i.e., 6 patients < 12 years old and 6 patients ≥ 12 years old).

Patients from Cycle 1 continue onto Cycle 2 if they do not experience a dose-limiting toxicity (DLT) and have again met laboratory parameters as defined in the eligibility section except for the following repeat cycle modified starting criteria: cholesterol ≤ 400 mg/dL OR ≤ 500 mg/dL and on lipid lowering medication, and triglycerides ≤ 300 mg/dL OR ≤ 500 mg/dL and on lipid lowering medication. Patients with progressive disease after Cycle 1 therapy with ABI-009 alone may remain on study provided they do not meet other exclusion criteria.

For Cycles 2+ part of the study, a cycle may be repeated every 21 days if the patient has at least stable disease and has again met laboratory parameters as defined in the eligibility section except for the following repeat cycle modified starting criteria: cholesterol ≤ 400 mg/dL OR ≤ 500 mg/dL and on lipid lowering medication, and triglycerides ≤ 300 mg/dL OR ≤ 500 mg/dL and on lipid lowering medication.

Maximum Tolerated Dose (MTD) for combination therapy is determined as the maximum dose at which ≤ 33% of patients experience DLT during Cycle 2 of therapy. Recommended Phase 2 Dose for combination therapy is determined as the MTD defined in Cycle 2 or in the absence of DLT, or Dose Level 3 (55 mg/m² ABI-009, 90 mg/m² irinotecan, and 125 mg/m2 temozolomide).

The DLT observation period is the first two cycles of therapy. DLTs observed during Cycle 1 are counted towards Cycle 2 combination therapy MTD determination. CTCAE v 4 or current version will be used for grading toxicities. Any patient who receives at least one dose of the study drug(s) is considered evaluable for adverse events. In addition, for the dose-escalation portion, patients must receive at least 100% of the prescribed dose during Cycle 1 and 100% of the prescribed dose during Cycle 2 per protocol guidelines and must have the appropriate toxicity monitoring studies performed during Cycle 1 and Cycle 2 to be considered evaluable for DLT. Patients who are not evaluable for toxicity at a given dose level during either Cycle 1 or Cycle 2 will be replaced.

DLT is defined differently for hematological and non-hematological toxicities. Non-hematological DLT is defined as any Grade 3 or greater non-hematological toxicity attributable to the investigational drug with the specific exclusion of: Grade 3 nausea and vomiting < 3 days duration; Grade 3 liver enzyme elevation, including ALT/AST/GGT, that returns to Grade ≤ 1 or baseline prior to the time for the next treatment cycle. Note: For the purposes of this study the ULN for ALT is defined as 45 U/L; Grade 3 fever; Grade 3 infection; Grade 3 hypophosphatemia, hypokalemia, hypocalcemia or hypomagnesemia responsive to oral supplementation; Grade 3 or 4 hypertriglyceridemia that returns to Grade ≤ 2 prior to the start of the next treatment cycle. The severity (grade) of hypertriglyceridemia is based upon fasting levels. If Grade 3 or 4 triglycerides are detected when routine (non-fasting) laboratory studies are performed, the test should be repeated within 3 days in the fasting state to permit accurate grading; Grade 3 hyperglycemia that returns to ≤ Grade 2 or baseline (with or without the use of insulin or oral diabetic agents) prior to the start of the next treatment cycle. The severity (grade) of hyperglycemia is based upon fasting levels. If Grade 3 hyperglycemia is detected when routine (non-fasting) laboratory studies are performed, the test should be repeated within 3 days in the fasting state to permit accurate grading; Grade 3 or 4 hypercholesterolemia that returns to ≤ Grade 2 after initiation of lipid lowering medication prior to the next treatment cycle. The severity (grade) of hypercholesterolemia is based upon fasting levels. If Grade 3 or 4 hypercholesterolemia is detected when routine (non-fasting) laboratory studies are performed, the test should be repeated within 3 days in the fasting state to permit accurate grading. Non-hematological toxicity also includes a delay of ≥ 14 days between treatment cycles. Allergic reactions that necessitate discontinuation of study drug are not be considered a dose-limiting toxicity.

Hematological DLT is defined as: Grade 4 neutropenia for > 7 days; Platelet count < 20,000/mm³ on 2 separate days, or requiring a platelet transfusion on 2 separate days, within a 7 day period; Myelosuppression that causes a delay of > 14 days between treatment cycles; and Grade 3 or 4 thromboembolic event. Grade 3 or 4 febrile neutropenia is not be considered a dose-limiting toxicity.

Dose modification for elevated fasting triglycerides is as shown in Table 2 below.

**Table 2.**

| Grade | Action |
|---|---|
| Grade 2 | • Continue temsirolimus; if triglycerides are between 301 and 400 mg/dL consider treatment with an HMG-CoA reductase inhibitor depending upon recommendations of institutional hyperlipidemia consultants. HMG-CoA reductase inhibitor is recommended if triglycerides are between 401 and 500 mg/dL |
| Grade 3-4 | • Hold temsirolimus until recovery to ≤ Grade 2 |
| | • An HMG-CoA reductase inhibitor should be started, and dosages should be adjusted based upon recommendations from institutional hyperlipidemia consultants |
| | • Upon retreatment at the same dose level, if Grade 3 or 4 toxicity recurs, lipid lowering medication should be adjusted in consultation with institutional hyperlipidemia consultants. Temsirolimus should be held until recovery to ≤ Grade 2. |
| | • Upon retreatment with temsirolimus concurrent with an HMG-CoA reductase inhibitor, if Grade 3 or 4 elevations recur, temsirolimus should be held until recovery to ≤ Grade 2. Further lipid lowering medication options should be discussed with institutional hyperlipidemia consultants. Upon recovery to ≤ Grade 2, temsirolimus should be restarted at the next lower dose level. If the patient is being treated on the lowest dose level, protocol therapy should be discontinued. |

Disease evaluations are performed at the end of Cycle 1, at the end of Cycle 2, then every other cycle for 2 cycles, then every 3 cycles. Disease response is assessed using the Response Evaluation Criteria in Solid Tumors (RECIST) guideline (version 1.1).

In additional to monitoring toxicity and response, pharmacokinetic and pharmacodynamic studies are performed. ABI-009 pharmacokinetics (PK) are determined using validated LC-MS/MS assays. Irinotecan and temozolomide pharmacokinetics are determined using a validated HPLC assay with fluorescence detection. For ABI-009 PK studies, plasma samples (2 mL per time point) are obtained from patients at the following time points during Cycle 1 (single agent) and Cycle 2 (Combination therapy) of the study: Day 1: pre-dose, end of infusion, and then 1, 2, 4, and 8 hrs after beginning of infusion; Day 2: 24 hours post-D1 ABI-009 dose; Day 4 (± 1 day): 72 hours ( ± 24 hours) post- D1 ABI-009 dose; and Day 8: Pre-ABI-009 dose. Optionally, CSF collection at any time point post-infusion can be obtained and analyzed to determine PK of ABI-009. For irinotecan and temozolomide PK studies, plasma samples (2 mL per time point) are obtained from patients at the following time points during Cycle 2 (Combination Therapy) of the study: Day 1: Pre-dose, and then 10 min, 1 hr, 3 hrs, and 6 hrs post-irinotecan dose; and Day 2: Pre-Day 2 irinotecan dose (24 hours after Day 1 irinotecan dose). Pharmacokinetics parameters (Tₘₐₓ, Cₘₐₓ, t_{1/2}, AUC, Cl/F) are calculated using standard non-compartmental or compartmental methods, as needed.

Additionally, tumor tissue samples are analyzed by immunohistochemistry to evaluate S6K1 and 4EBP1 expression in pediatric solid tumors prior to treatment with ABI-009. Paraffin embedded tissue block or unstained slides are required prior to enrollment. The analysis is performed during Cycle 1 of the study.

### Eligibility

Eligible individuals must meet all of the following inclusion criteria:
(1) Patients must be ≥ 12 months and ≤ 21 years of age;
(2) Patients must be diagnosed with recurrent or refractory solid tumors, including CNS tumors:
(3) Patients must have the following performance status: Karnofsky ≥ 50% for patients >16 year of age and Lansky ≥ 50% for patients ≤ 16 years of age. Neurologic deficits in patients with CNS tumors must have been relatively stable for at least 7 days prior to study enrollment. Patients who are unable to walk because of paralysis, but who are up in a wheelchair, will be considered ambulatory for the purpose of assessing the performance score.
(4) Patients must have fully recovered from the acute toxic effects of all prior anti-cancer chemotherapy and must meet at least the following duration from prior anti-cancer directed therapy prior to enrollment. If after the required timeframe, the numerical eligibility criteria are met, *e.g.* blood count criteria, the patient is considered to have recovered adequately: (i) Cytotoxic chemotherapy or other chemotherapy known to be myelosuppressive: ≥ 21 days after the last dose of cytotoxic or myelosuppressive chemotherapy (42 days if prior nitrosourea); (ii) Anti-cancer agents not known to be myelosuppressive (*e.g*. not associated with reduced platelet or ANC counts): ≥ 7 days after the last dose of agent; (iii) Antibodies: ≥ 3 half-lives for the antibody or ≥ 30 days must have elapsed after the last dose, whichever is shorter, and must have recovered from all acute toxicities; (iv) Hematopoietic growth factors: ≥ 14 days after the last dose of a long-acting growth factor (*e.g*. Neulasta) or 7 days for short-acting growth factor. For agents that have known adverse events occurring beyond 7 days after administration, this period must be extended beyond the time during which adverse events are known to occur; (v) Immunotherapy or Immune Modulatory Drugs: ≥ 42 days after the completion of any type of immunotherapy, immune modulatory drugs (*e.g*. cytokines, adjuvants, etc.) except steroids, or tumor directed vaccines; (vi) Stem cell Infusions (with or without TBI): Allogeneic (non-autologous) bone marrow or stem cell transplant, or any stem cell infusion including DLI or boost infusion: ≥ 84 days after infusion and no evidence of GVHD); autologous stem cell infusion including boost infusion: ≥ 42 days; g) Cellular Therapy: ≥ 42 days after the completion of any type of cellular therapy (e.g. modified T cells, NK cells, dendritic cells, etc.); (vii) XRT/External Beam Irradiation including Protons: ≥ 14 days after local XRT; ≥ 150 days after TBI, craniospinal XRT or if radiation to ≥ 50% of the pelvis; ≥ 42 days if other substantial BM radiation; i) Radiopharmaceutical therapy (*e.g*., radiolabeled antibody, 131I-MIBG): ≥ 42 days after systemically administered radiopharmaceutical therapy; (viii) Irinotecan, temozolomide and mTOR inhibitor exposure: Patients who have received prior single agent therapy with irinotecan, temozolomide, or an mTOR inhibitor, excluding ABI-009, are eligible; Patients who have received prior combination therapy with two of the three agents, excluding ABI-009 are eligible; Patients who have received prior therapy with all three agents in combination (i.e. irinotecan, temozolomide, and a mTOR inhibitor) are not eligible; Patients who have previously received irinotecan and temozolomide and progressed or had significant toxicity with these two drugs are not eligible; and
5) Patients must meet organ function criteria described below:
(i) Adequate Bone Marrow Function Defined as: For patients with solid tumors without known bone marrow involvement: Peripheral absolute neutrophil count (ANC) ≥ 1000/mm³; Platelet count ≥ 100,000/mm³ (transfusion independent, defined as not receiving platelet transfusions for at least 7 days prior to enrollment); Hemoglobin ≥ 8.0 g/dL at baseline (may receive RBC transfusions). For patients with known bone marrow metastatic disease: Peripheral absolute neutrophil count (ANC) ≥ 1000/ mm³; Platelet count ≥ 100,000/ mm³ (transfusion independent, defined as not receiving platelet transfusions for at least 7 days prior to enrollment); May receive transfusions provided they are not known to be refractory to red cell or platelet transfusions. These patients will not be evaluable for hematologic toxicity. At least 5 of every cohort of 6 patients with a solid tumor must be evaluable for hematologic toxicity, for the dose-escalation part of the study. If dose-limiting hematologic toxicity is observed, all subsequent patients enrolled must be evaluable for hematologic toxicity.
(ii) Adequate Renal Function Defined as: Creatinine clearance or radioisotope GFR ≥70ml/min/1.73 m² or a serum creatinine based on age/gender as shown in Table 3 below.

**Table 3.**

| Age | Maximum Serum Creatinine (mg/dL) | |
|---|---|---|
| | Male | Female |
| 1 to < 2 years | 0.6 | 0.6 |
| 2 to < 6 years | 0.8 | 0.8 |
| 6 to < 10 years | 1 | 1 |
| 10 to < 13 years | 1.2 | 1.2 |
| 13 to < 16 years | 1.5 | 1.4 |
| ≥ 16 years | 1.7 | 1.4 |

| | | |
|---|---|---|
| * The threshold creatinine values in this Table were derived from the Schwartz formula for estimating GFR utilizing child length and stature data published by the CDC. | | |

(iii) Adequate Liver Function Defined as: Bilirubin (sum of conjugated + unconjugated) ≤ 1.5 x upper limit of normal (ULN) for age; SGPT (ALT) ≤ 110 U/L. For the purpose of this study, the ULN for SGPT is 45 U/L; Serum albumin ≥ 2 g/dL.
(iv) Adequate Pulmonary Function Defined as: Pulse oximetry > 94% on room air if there is clinical indication for determination (*e.g*. dyspnea at rest).
(v) Adequate Neurologic Function Defined as: Patients with seizure disorder may be enrolled if on non-enzyme inducing anticonvulsants and well controlled; Nervous system disorders (CTCAE v4) resulting from prior therapy must be ≤ Grade 2.
(vi) Adequate Metabolic Function Defined as: Serum triglyceride level ≤ 300 mg/dL; Serum cholesterol level ≤ 300 mg/dL; Random or fasting blood glucose within the upper normal limits for age. If the initial blood glucose is a random sample that is outside of the normal limits, than follow-up fasting blood glucose can be obtained and must be within the upper normal limits for age.
(vii) Adequate Blood Pressure Control Defined as: A blood pressure (BP) ≤ the 95th percentile for age, height, and gender and not receiving medication for treatment of hypertension.
(viii) Adequate Coagulation Defined as: Not actively on any anticoagulants and INR ≤ 1.5.

Patients meeting any one or more of the following exclusion criteria are not eligible for enrolling in the study: (1) Patients with interstitial lung disease and/or pneumonitis are not eligible; (2) Patients must not be receiving any strong CYP3A4 inducers or inhibitors within 7 days prior to enrollment; (3) Patient with a history of allergic reactions attributed to compounds of similar composition temsirolimus/other mTOR inhibitors, temozolomide or irinotecan are not eligible; (4) Patients with hypersensitivity to albumin are not eligible; (5) Patients have a BSA of < 0.2 m² at the time of study enrollment are not eligible; (6) Patients with current or recent deep vein thrombosis are not eligible; and (5) Patients who have had or are planning to have the following invasive procedures are not eligible: Major surgical procedure, laparoscopic procedure, open biopsy or significant traumatic injury within 28 days prior to enrollment; Subcutaneous port placement or central line placement is not considered major surgery. External central lines must be placed at least 3 days prior to enrollment and subcutaneous ports must be placed at least 7 days prior to enrollment; Core biopsy within 7 days prior to enrollment; or Fine needle aspirate within 7 days prior to enrollment. For purposes of this study, bone marrow aspirate and biopsy are not considered surgical procedures and therefore are permitted within 14 days prior to start of protocol therapy.
Also disclosed herein are *inter alia* the following items (said items are not claims):
1. A method of treating a hyperplasia in an individual comprising administering to the individual an effective amount of a composition comprising nanoparticles comprising an mTOR inhibitor and an albumin, wherein the individual is selected for treatment on the basis of having an mTOR-activating aberration.
2. The method of item 1, wherein the method further comprises assessing the mTOR-activating aberration in the individual.
3. A method of selecting an individual having a hyperplasia for treatment with a composition comprising nanoparticles comprising an mTOR inhibitor and an albumin, wherein the method comprises:
   (1) assessing an mTOR-activating aberration in the individual; and
   (2) selecting the individual for treatment based on the individual having the mTOR-activating aberration.
4. The method of item 3, wherein the method further comprises administering the composition comprising nanoparticles comprising an mTOR inhibitor and an albumin to the selected individual.
5. The method of any one of items 1-4, wherein the hyperplasia is selected from the group consisting of cancer, restenosis, and pulmonary hypertension.
6. The method of item 5, wherein the cancer is selected from the group consisting of pancreatic neuroendocrine cancer, endometrial cancer, breast cancer, renal cell carcinoma, lymphangioleiomyomatosis (LAM), prostate cancer, lymphoma, bladder cancer, endometrial cancer, and ovary cancer.
7. The method of any one of items 1-6, wherein the mTOR-activating aberration comprises a mutation in an mTOR-associated gene.
8. The method of item 7, wherein the mTOR-activating aberration comprises a copy number variation of an mTOR-associated gene.
9. The method of item 7 or item 8, wherein the mTOR-activating aberration is assessed by gene sequencing.
10. The method of item 9, wherein the gene sequencing is based on sequencing of DNA in a tumor sample.
11. The method of item 9, wherein the gene sequencing is based on sequencing of circulating DNA or cell-free DNA isolated from a blood sample.
12. The method of any one of items 1-11, wherein the mTOR-activating aberration comprises an aberrant expression level of an mTOR-associated gene.
13. The method of any one of items 1-11, wherein the mTOR-activating aberration comprises an aberrant phosphorylation level of the protein encoded by the mTOR-associated gene.
14. The method of item 13, wherein the mTOR-activating aberration comprises an aberrant phosphorylation level of a protein encoded by an mTOR-associated gene selected from the group consisting of AKT, S6K, S6, 4EBP1, and SPARC.
15. The method of item 13 or item 14, wherein the aberrant phosphorylation level is determined by immunohistochemistry.
16. The method of any one of items 1-15, wherein the mTOR-activating aberration comprises an aberrant activity level of an mTOR-associated gene.
17. The method of any one of items 1-16, wherein the mTOR-activating aberration leads to activation of mTORC1.
18. The method of any one of items 1-17, wherein the mTOR-activating aberration leads to activation of mTORC2.
19. The method of any one of items 1-18, wherein the mTOR-activating aberration is an aberration in at least one mTOR-associated gene selected from the group consisting of AKT1, FLT3, MTOR, PIK3CA, PIK3CG, TSC1, TSC2, RHEB, STK11, NF1, NF2, PTEN, TP53, FGFR4, KRAS, NRAS, and BAP1.
20. The method of item 19, wherein the at leastone mTOR-associated gene comprises MTOR.
21. The method of item 20, wherein the mTOR-activating aberration comprises an activating mutation of MTOR.
22. The method of item 19, wherein the at least one mTOR-associated gene comprises TSC1 or TSC2.
23. The method of item 22, wherein the mTOR-activating aberration comprises a loss of heterozygosity of TSC1 or TSC2.
24. The method of item 22, wherein the mTOR-activating aberration comprises a loss of function mutation in TSC1 or TSC2.
25. The method of item 19, wherein the at least one mTOR-associated gene comprises RHEB.
26. The method of item 25, wherein the mTOR-activating aberration comprises a loss of function mutation in RHEB.
27. The method of item 19, wherein the at least one mTOR-associated gene comprises NF1.
28. The method of item 27, wherein the mTOR-activating aberration comprises a loss of function mutation in NF1,
29. The method of item 19, wherein the at least one mTOR-associated gene comprises NF2.
30. The method of item 29, wherein the mTOR-activating aberration comprises a loss of function mutation of NF2.
31. The method of item 19, wherein the mTOR-associated gene comprises PTEN.
32. The method of item 31, wherein the mTOR-activating aberration comprises a deletion of PTEN.
33. The method of item 19, wherein the mTOR-associated gene comprises PIK3CA.
34. The method of item 33, wherein the mTOR-activating aberration comprises a loss of function mutation in PIK3CA.
35. The method of item 19, wherein the mTOR-associated gene comprises PIK3CG.
36. The method of item 35, wherein the mTOR-activating aberration comprises a loss of function mutation in PTK3CG.
37. The method of item 19, wherein the mTOR-associated gene comprises AKT1.
38. The method of item 37, wherein the mTOR-activating aberration comprises an activating mutation in AKT1.
39. The method of item 19, wherein the mTOR-associated gene comprises TP33.
40. The method of item 39, wherein the mTOR-activating aberration comprises a loss of function mutation in TP53.
41. The method of any one of items 1-40, wherein the mutational status of TFE3 is further used as a basis for selecting the individual.
42. The method of item 41, wherein the mutational status of TFE3 comprises translocation of TFE3.
43. The method of any one of items 1-42, wherein the method further comprises administering to the individual an effective amount of a second therapeutic agent.
44. The method of any one of items 1-43, wherein the individual is human.
45. The method of any one of items 1-44, wherein the composition comprises nanoparticles comprising the mTOR inhibitor and the albumin is administered intravenously.
46. The method of any one of items 1-44, wherein the composition comprises nanoparticles comprising the mTOR inhibitor and the albumin is administered subcutaneously.
47. The method of any one of items 1-46, wherein the nanoparticles in the composition comprise the mTOR inhibitor associated with the albumin.
48. The method of any one of items 1-47, wherein the nanoparticles in the composition have an average diameter of no greater than about 150 nm.
49. The method of any one of items 1-48, wherein the ratio of the mTOR inhibitor to the albumin in the nanoparticles is about 1:1 to about 9:1.
50. The method of any one of items 1-49, wherein the albumin is human serum albumin.
51. The method of any one of items 1-50, wherein the mTOR inhibitor is a limus drug.
52. The method of item 51, wherein the limus drug is sirolimus.
53. The method of any one of items 1-52, wherein the dose of the mTOR inhibitor in the composition is about 10 mg/m² to about 100 mg/m².
54. A kit comprising 1) a composition comprising nanoparticles comprising an mTOR inhibitor and an albumin, and 2) an agent for assessing an mTOR-activating aberration.

## Claims

1. A composition comprising nanoparticles comprising sirolimus and albumin for use in a method of treating a solid tumor in a human individual having a loss-of-function mutation in TSC1 or TSC2, wherein the method comprises administering to the individual an effective amount of the composition.

2. The composition for use of claim 1, wherein the loss-of-function mutation is selected from the group consisting of: a non-sense mutation, a frameshift mutation, a splice site mutation, a splice variant and a deletion.

3. The composition for use of claim 1 or 2, wherein the loss-of-function mutation is a somatic mutation.

4. The composition for use of any one of claims 1 to 3, wherein the loss-of-function mutation is assessed by gene sequencing of nucleic acids from a sample of the individual.

5. The composition for use of claim 4, wherein:
i) the nucleic acids are DNA in a tumor sample; or
ii) the nucleic acids are circulating DNA or cell-free DNA isolated from a blood sample.

6. The composition for use of any one of claims 1 to 5, wherein:
i) the solid tumor is a locally advanced or metastatic solid tumor; and/or
ii) the solid tumor is selected from the group consisting of endometrial cancer, epithelial ovarian cancer, angiosarcoma, and sarcoma.

7. The composition for use of any one of claims 1 to 6, wherein the composition is administered to the individual intravenously or subcutaneously.

8. The composition for use of any one of claims 1 to 7, wherein the dose of sirolimus in the composition is from about 10 mg/m² to about 100 mg/m².

9. The composition for use of any one of claims 1 to 8, wherein the method further comprises the administration of an effective amount of a second therapeutic agent.

10. The composition for use of any one of claims 1 to 9, wherein the individual has progressed on a prior therapy;
preferably wherein the individual is nonresponsive, partially responsive, initially responsive, resistant or refractory to a prior therapy.

11. The composition for use of any one of claims 1 to 10, wherein the individual is no more than about 21 years old;
preferably no more than about 18 years old.

12. The composition for use of any one of claims 1 to 11, wherein the nanoparticles in the composition comprise sirolimus associated with the albumin.

13. The composition for use of any one of claims 1 to 12, wherein the nanoparticles in the composition have an average diameter of no greater than about 150 nm.

14. The composition for use of any one of claims 1 to 13, wherein the ratio of sirolimus to the albumin in the nanoparticles is from about 1:1 to about 9:1.

15. The composition for use of any one of claims 1 to 14, wherein the albumin is human serum albumin.
